# EUROPEAN PATENT SPECIFICATION

(11) **EP 3 914 091 B1**
(45) Date of publication and mention of the grant of the patent: **17.09.2025**
(21) Application number: 20712073.4
(22) Date of filing: 23.01.2020
(51) Int. Cl.: A23K 20/105, A23L 33/12, A23L 33/135, A61K 31/575, A61K 35/742, G01N 33/50, A23K 10/18, A23K 50/40, A23L 33/10, C12Q 1/06, A61P 1/00, A61P 1/12, A61P 37/06

(54) **METHODS AND COMPOSITIONS FOR TREATING INTESTINAL DISORDER**
VERFAHREN UND ZUSAMMENSETZUNGEN ZUR BEHANDLUNG VON INTESTINALEN STÖRUNGEN
PROCÉDÉS ET COMPOSITIONS POUR LE TRAITEMENT D'UN TROUBLE INTESTINAL

(30) Priority: 23.01.2019 US 201962796021 P
(43) Date of publication of application: 01.12.2021
(60) Divisional application: 25189550.4
(73) Proprietor: Mars, Incorporated, McLean, VA 22101 (US); The Trustees of the University of Pennsylvania, Philadelphia, PA 19104 (US)
(72) Inventor: PEREA, Sally Christine, Lewisburg, OH 45338 (US); BEITING, Daniel P., Philadelphia, PA 19104 (US)
(74) Representative: Haseltine Lake Kempner LLP
(86) International application number: PCT/US2020/014823
(87) International publication number: WO 2020/154523

(56) References cited:
- WO-A1-2015/179437
- WO-A1-2018/042304
- WO-A1-2018/042304
- WO-A1-2018/195467
- ALSHAWAQFEH MK ET AL: "A dysbiosis index to assess microbial changes in fecal samples of dogs with chronic inflammatory enteropathy", FEMS MICROBIOLOGY ECOLOGY, vol. 93, no. 11, 1 November 2017 (2017-11-01), pages 136, XP055896962, Retrieved from the Internet <URL:https://academic.oup.com/femsec/article-pdf/93/11/fix136/21574836/fix136.pdf> DOI: 10.1093/femsec/fix136
- ISAIAH ANITHA: "CLOSTRIDIUM HIRANONIS, A BILE ACID 7?-DEHYDROXYLATING BACTERIUM IN DOGS", PHD THESIS, 30 August 2018 (2018-08-30), pages 1 - 133, XP055838670, Retrieved from the Internet <URL:https://oaktrust.library.tamu.edu/bitstream/handle/1969.1/188939/ISAIAH-DISSERTATION-2018.pdf?sequence=1&isAllowed=y> [retrieved on 20210907]
- QINGHONG LI ET AL: "Effects of the Dietary Protein and Carbohydrate Ratio on Gut Microbiomes in Dogs of Different Body Conditions", MBIO, vol. 8, no. 1, 24 January 2017 (2017-01-24), XP055621358, DOI: 10.1128/mBio.01703-16
- CHARLIE G. BUFFIE ET AL: "Precision microbiome reconstitution restores bile acid mediated resistance to Clostridium difficile", NATURE, vol. 517, 22 October 2014 (2014-10-22), London, pages 205 - 208, XP055363406, ISSN: 0028-0836, DOI: 10.1038/nature13828
- FRANCESCA BRESCIANI ET AL: "Effect of an extruded animal protein-free diet on fecal microbiota of dogs with food-responsive enteropathy", JOURNAL OF VETERINARY INTERNAL MEDICINE, vol. 32, no. 6, 23 October 2018 (2018-10-23), US, pages 1903 - 1910, XP055764673, ISSN: 0891-6640, DOI: 10.1111/jvim.15227
- ALSHAWAQFEH MK ET AL: "A dysbiosis index to assess microbial changes in fecal samples of dogs with chronic inflammatory enteropathy", FEMS MICROBIOLOGY ECOLOGY, vol. 93, no. 11, 1 November 2017 (2017-11-01), pages 136, XP055896962, Retrieved from the Internet <URL:https://academic.oup.com/femsec/article-pdf/93/11/fix136/21574836/fix136.pdf> DOI: 10.1093/femsec/fix136
- ISAIAH ANITHA: "CLOSTRIDIUM HIRANONIS, A BILE ACID 7?-DEHYDROXYLATING BACTERIUM IN DOGS", PHD THESIS, 30 August 2018 (2018-08-30), pages 1 - 133, XP055838670, Retrieved from the Internet <URL:https://oaktrust.library.tamu.edu/bitstream/handle/1969.1/188939/ISAIAH-DISSERTATION-2018.pdf?sequence=1&isAllowed=y> [retrieved on 20210907]
- QINGHONG LI ET AL: "Effects of the Dietary Protein and Carbohydrate Ratio on Gut Microbiomes in Dogs of Different Body Conditions", MBIO, vol. 8, no. 1, 24 January 2017 (2017-01-24), XP055621358, DOI: 10.1128/mBio.01703-16
- CHARLIE G. BUFFIE ET AL: "Precision microbiome reconstitution restores bile acid mediated resistance to Clostridium difficile", NATURE, vol. 517, 22 October 2014 (2014-10-22), London, pages 205 - 208, XP055363406, ISSN: 0028-0836, DOI: 10.1038/nature13828
- FRANCESCA BRESCIANI ET AL: "Effect of an extruded animal protein-free diet on fecal microbiota of dogs with food-responsive enteropathy", JOURNAL OF VETERINARY INTERNAL MEDICINE, vol. 32, no. 6, 23 October 2018 (2018-10-23), US, pages 1903 - 1910, XP055764673, ISSN: 0891-6640, DOI: 10.1111/jvim.15227
- DATABASE MEDLINE [online] US NATIONAL LIBRARY OF MEDICINE (NLM), BETHESDA, MD, US; 11 May 2020 (2020-05-11), REED A D ET AL: "Strain-Dependent Inhibition of Clostridioides difficile by Commensal Clostridia Carrying the Bile Acid-Inducible (bai) Operon.", Database accession no. NLM32179626
- REED A D ET AL: "Strain-Dependent Inhibition of Clostridioides difficile by Commensal Clostridia Carrying the Bile Acid-Inducible (bai) Operon.", JOURNAL OF BACTERIOLOGY 11 05 2020, vol. 202, no. 11, 11 May 2020 (2020-05-11), ISSN: 1098-5530
- DATABASE BIOSIS [online] BIOSCIENCES INFORMATION SERVICE, PHILADELPHIA, PA, US; May 2018 (2018-05-01), RIDLON JASON M ET AL: "BILE ACID 7 alpha-DEHYDROXYLATING GUT CLOSTRIDIA: FROM COMPARATIVE GENOMICS TO IN VIVO METATRANSCRIPTOMICS AND METABOLOMICS TO GENE DISCOVERY", Database accession no. PREV201900127784
- GASTROENTEROLOGY, vol. 154, no. 6, Suppl. 1, May 2018 (2018-05-01), ANNUAL MEETING OF THE AMERICAN-SOCIETY-FOR-GASTROINTESTINAL-ENDOSCOPY / DIGESTIVE DISEASE WEEK; WASHINGTON, DC, USA; JUNE 02 -05, 2018, pages S639 - S640, ISSN: 0016-5085(print)
- DATABASE EMBASE [online] ELSEVIER SCIENCE PUBLISHERS, AMSTERDAM, NL; 1 May 2020 (2020-05-01), JOSEPH D ET AL: "DEVELOPMENT OF A TARGETED 7 [alpha]-DEHYDRATASE PCR ASSAY FOR FECAL MICROBIAL TRANSPLANT BILE ACID CHARACTERIZATION", Database accession no. EMB-002005913022
- JOSEPH D ET AL: "DEVELOPMENT OF A TARGETED 7 [alpha]-DEHYDRATASE PCR ASSAY FOR FECAL MICROBIAL TRANSPLANT BILE ACID CHARACTERIZATION", GASTROENTEROLOGY 20200501 W.B. SAUNDERS NLD, vol. 158, no. 6, Supplement 1, 1 May 2020 (2020-05-01), pages S 20200502 to 20200505 Chicago IL - 479 CONF, ISSN: 0016-5085
- DODEN HEIDI ET AL: "Metabolism of Oxo-Bile Acids and Characterization of Recombinant 12[alpha]-Hydroxysteroid Dehydrogenases from Bile Acid 7[alpha]-Dehydroxylating Human Gut Bacteria.", APPLIED AND ENVIRONMENTAL MICROBIOLOGY 15 05 2018, vol. 84, no. 10, 15 May 2018 (2018-05-15), XP002799340, ISSN: 1098-5336
- RIDLON JASON M ET AL: "BILE ACID 7 alpha-DEHYDROXYLATING GUT CLOSTRIDIA: FROM COMPARATIVE GENOMICS TO IN VIVO METATRANSCRIPTOMICS AND METABOLOMICS TO GENE DISCOVERY", GASTROENTEROLOGY, vol. 154, no. 6, Suppl. 1, May 2018 (2018-05-01), & ANNUAL MEETING OF THE AMERICAN-SOCIETY-FOR-GASTROINTESTINAL-ENDOSCOPY / DIGESTIVE DISEASE WEEK; WASHINGTON, DC, USA; JUNE 02 -05, 2018, pages S639 - S640, XP002799341
- RIDLON J M ET AL: "Isolation and characterization of a bile acid inducible 7@?-dehydroxylating operon in Clostridium hylemonae TN271", ANAEROBE, LONDON, GB, vol. 16, no. 2, 1 April 2010 (2010-04-01), pages 137 - 146, XP027006981, ISSN: 1075-9964, [retrieved on 20090521]
- SOLBACH P ET AL: "BaiCD gene abundance is negatively correlated with Clostridium difficile infection", JOURNAL OF CROHN'S AND COLITIS 20180201 OXFORD UNIVERSITY PRESS NLD, vol. 12, no. Supplement 1, 1 February 2018 (2018-02-01), XP002799342, ISSN: 1876-4479
- SHUAI WANG ET AL: "Diet-induced remission in chronic enteropathy is associated with altered microbial community structure and synthesis of secondary bile acids", MICROBIOME, BIOMED CENTRAL LTD, LONDON, UK, vol. 7, no. 1, 31 August 2019 (2019-08-31), pages 1 - 20, XP021272746, DOI: 10.1186/S40168-019-0740-4
- JOSEPH D ET AL: "DEVELOPMENT OF A TARGETED 7 [alpha]-DEHYDRATASE PCR ASSAY FOR FECAL MICROBIAL TRANSPLANT BILE ACID CHARACTERIZATION", GASTROENTEROLOGY 20200501 W.B. SAUNDERS NLD, vol. 158, no. 6, Supplement 1, 1 May 2020 (2020-05-01), pages - 479 CONF, XP002799343, ISSN: 0016-5085
- REED A D ET AL: "Strain-Dependent Inhibition of Clostridioides difficile by Commensal Clostridia Carrying the Bile Acid-Inducible (bai) Operon.", JOURNAL OF BACTERIOLOGY 11 MAY 2020, vol. 202, no. 11, 11 May 2020 (2020-05-11), XP002799344, ISSN: 1098-5530, Retrieved from the Internet <URL:https://jb.asm.org/content/202/11/e00039-20> [retrieved on 20200610]

## Description

### FIELD

The presently disclosed subject matter relates to methods for determining responsiveness of a companion animal having an intestinal disorder to a diet, wherein the companion animal is a dog, wherein the diet is a therapeutic protein hydrolyzed diet and wherein the intestinal disorder is chronic enteropathy.

### BACKGROUND

Inflammatory bowel disease (IBD), including Crohn's disease and ulcerative colitis, is a multi-factorial and debilitating disease characterized by chronic immune-pathology, disruption of intestinal homeostasis and altered composition of the gut microbiome (dysbiosis). Several lines of evidence point to resident gut bacteria as important factors in the etiology of IBD. First, disease is often more severe in areas of the intestine with the highest microbial biomass, and antibiotics are frequently used as an adjunct therapy with immunosuppressants or monoclonal antibodies for managing IBD ^{1,2}. Second, genome-wide associations studies have identified numerous susceptibility loci in genes responsible for recognizing or responding to bacteria ³. Finally, in some mouse models of colitis, disease can be transferred to naive hosts via fecal transplant ⁴⁻⁶, suggesting a causal role for gut microbes in disease. Collectively, these findings have led to a 'two-hit' model for IBD in which both host genetics and microbial factors influence disease presentation, highlighting an opportunity to develop novel treatments for IBD that target the microbiome. Thus, there is a need for novel methods and compositions for treating IBD and other intestinal disorders that target gut microbiome and metabolites thereof.

### SUMMARY OF THE INVENTION

The present invention is as defined by the appended claims.

The present invention provides a method for determining responsiveness of a companion animal having an intestinal disorder to a diet, wherein the intestinal disorder is chronic enteropathy, wherein the diet is a therapeutic hydrolyzed protein diet and wherein the companion animal is a dog. The method comprises:
a) measuring an amount of a intestinal microorganism in the companion animal;
b) comparing the amount of the intestinal microorganism with a reference amount of the intestinal microorganism, wherein the reference amounts of the intestinal microorganisms are determined based on the amounts of the intestinal microorganisms in a plurality of healthy companion animals; and
c) determining that the companion animal is responsive to the diet, when the amount of the intestinal microorganism is lower than the reference amount of the intestinal microorganism, or determining that the companion animal is non-responsive to the diet, when the amount of the intestinal microorganism is higher than the reference amount of the intestinal microorganism, wherein the intestinal microorganism comprises a bacterium comprising a 16S rRNA comprising a nucleotide sequence that is identical to the 16S rRNA nucleotide sequence of FJ957494.1.1454 (SEQ ID NO: 13)

In certain embodiments of the disclosure, the method further comprises administering the diet to the companion animal when companion animal is determined as responsive to the diet. In certain embodiments, the method further comprises administering the diet, a steroid and optionally an antibiotic to the companion animal when companion animal is determined as non-responsive to the diet.

In certain embodiments, the determination in step c) occurs before administering the diet or the diet, the steroid and optionally the antibiotic to the companion animal.

### BRIEF DESCRIPTION OF THE DRAWINGS

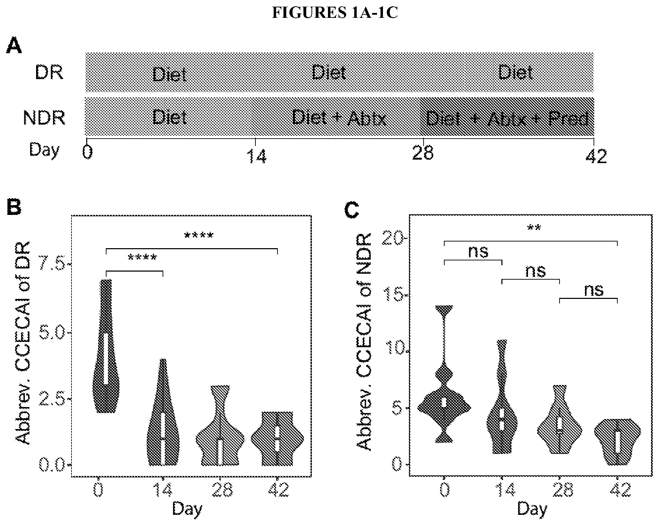

Figures 1A-1C depict that diet therapy induces rapid and durable remission in canine model of chronic enteritis. Figure 1A is a schematic showing clinical study design for identifying diet responsive (DR) and non-diet responsive (NDR) dogs. Antibiotics (Abtx) and Prednisone (Pred) treatments are indicated. Abbreviated Canine Chronic Enteropathy Clinical Activity Index (CCECAI) scores were assessed at four different time points in DR (n=20) (Figure 1B) and NDR (n=9) (Figure 1C) animals. ns = not significant, _{**} p < 0.01, _{****} p < 0.0001 using Wilcoxon rank sum test.

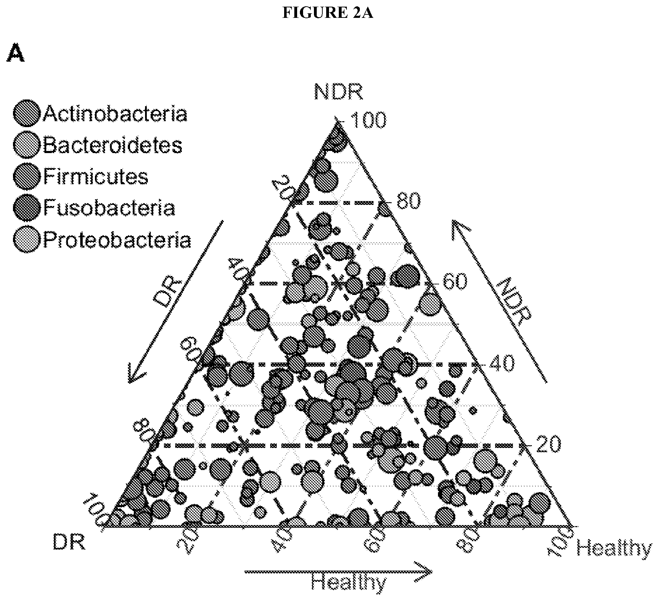

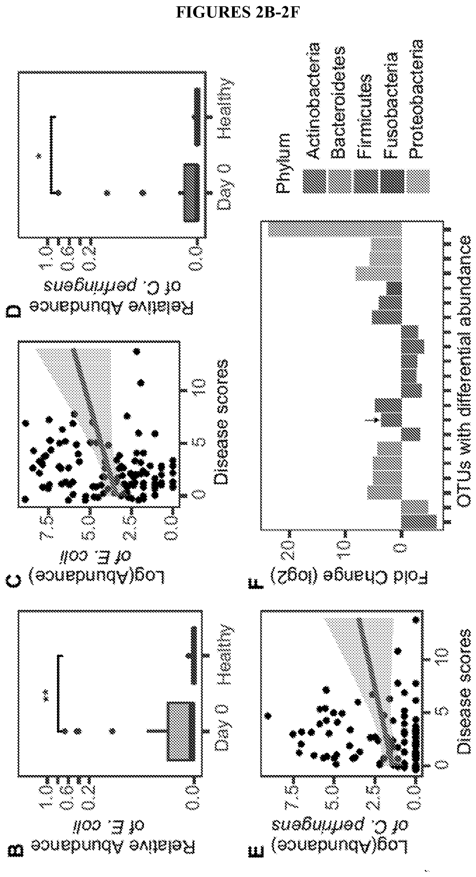

Figures 2A-2F depict identification of microbial community profiles associated with treatment outcome. Figure 2A is a ternary plot of phylum level OTUs from top 5 most abundant phyla among healthy (right), DR (left) and NDR (top) animals. Bubble size represents the log2 OTU abundance. Relative abundance of *E. coli* (Figure 2B) and *C. perfringens* (Figure 2D) in animals with active disease (day 0) and healthy dogs. Spearman correlation between log10 abundance of *E. coli* (Figure 2C) or *Clostridium sp.* (Figure 2E) and CCECAI disease score. Figure 2F depicts differentially abundant OTUs between DR and DNR animals at day 0. Y-axis value represents the log2 fold change for DR versus NDR. Arrow marks the OTU corresponding to *C. perfringens.* _{*} p < 0.05, _{**} p < 0.01 using Wilcoxon rank sum test (or Wilcoxon signed-rank test if available). Spearman correlations in panel C and E are significant (p < 0.05) with correlation coefficients of 0.2109 and 0.2324, respectively.

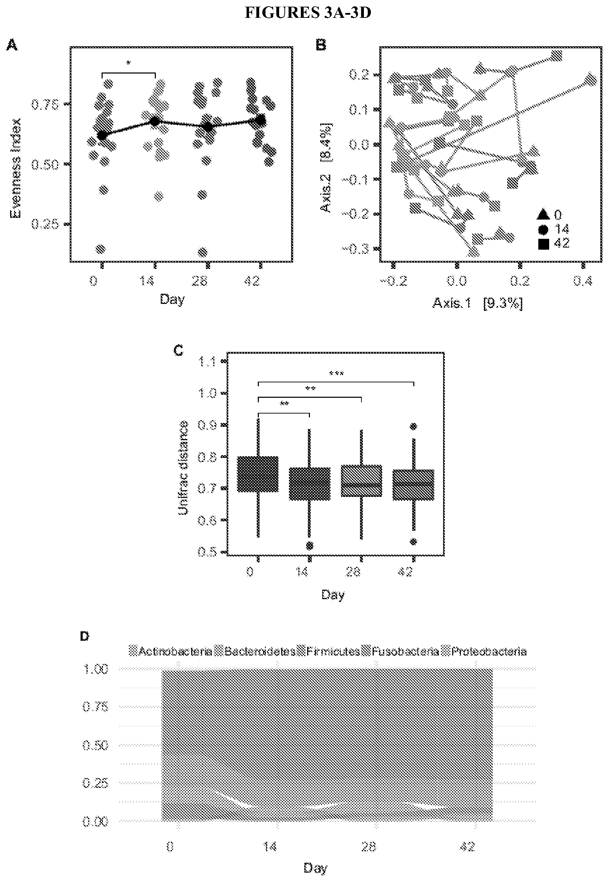

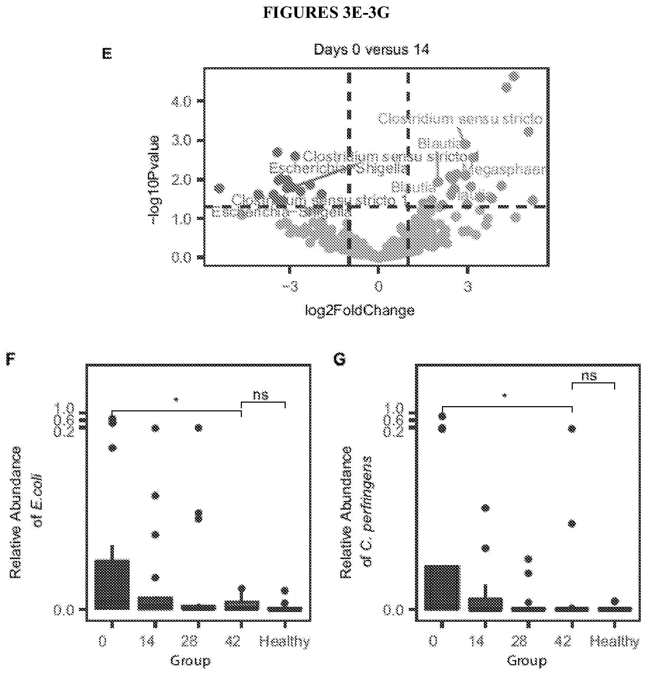

Figures 3A-3F depict that therapeutic diet ameliorates dysbiosis associated with chronic enteritis. Figure 3A depicts Pielou's evenness index for DR animals at different time points in the study. Figure 3B depicts the principal coordinate analysis (PCoA) based on unweighted Unifrac distance for DR. Figure 3C depicts the phylogenetic distance (unweighted Unifrac) to healthy controls for DR animals. Figure 3D depicts the stream plot showing phylum level dynamics of microbiota structure for DR animals throughout the study. Figure 3E depicts the volcano plot showing differentially abundant OTUs enriched in either DR dogs with active disease (day 0, red points) or in remission after diet therapy (day 14, blue points). Selected taxa (e.g., *Escherichia-Shigella spp., Clostridium spp*.) are labeled. The relative abundance of *E. coli* (Figure 3F) and *C. perfringens* (Figure 3G) in DR animals throughout the study and compared to healthy controls. ns = not significant, *p < 0.05, **p < 0.01, ***p < 0.0001 using Wilcoxon rank sum test (or Wilcoxon signed-rank test if paired data was available).

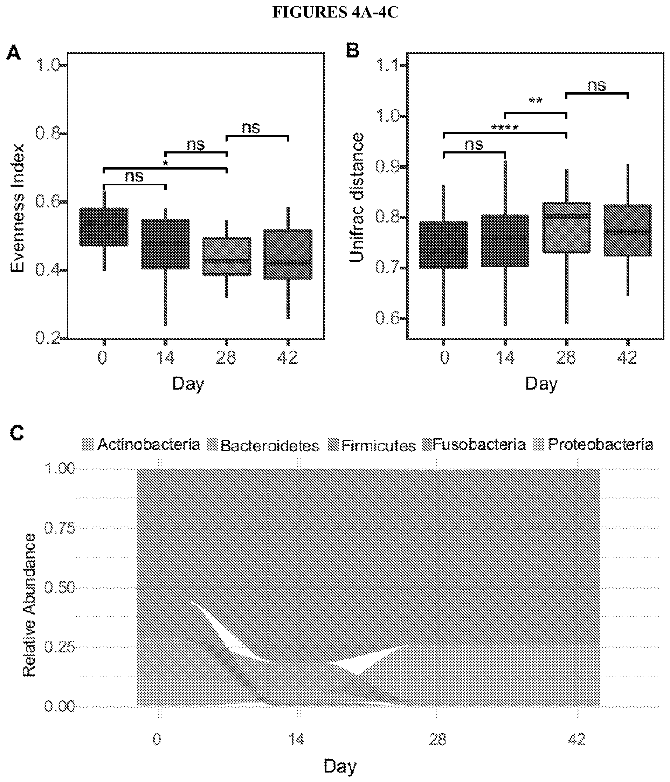

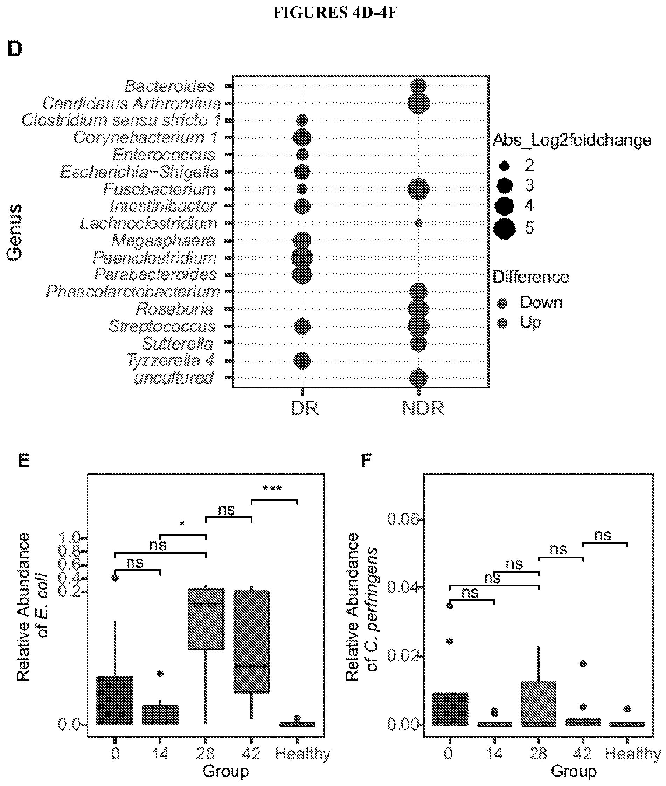

Figures 4A-4F depict diet-induced changes in the microbiome associated with remission. Figure 4A depicts Pielou's evenness index in NDR animals, and their phylogenetic distance (unweighted Unifrac) to healthy dogs is shown in Figure 4B. Figure 4C depicts the stream plot showing phylum level dynamics of microbiota structure for NDR animals throughout the study. Diet therapy began at day 0, metronidazole administration at day 14, and prednisone at day 28 (see methods). Figure 4D depicts the bubble plot showing differentially abundant genera (fold change > 2 and P < 0.05) between day 14 versus day 0 for DR (left) and NDR (right) animals. Bubble size indicates absolute log fold change between day 14 and day 0, and color reflects direction of change. Figure 4E and 4F depict the relative abundance of *E. coli* (Figure 4E) and *C. perfringens* (Figure 4F) in NDR animals throughout the study and compared to healthy controls. ns = not significant, *p < 0.05, **p < 0.01, ***p < 0.0001 using Wilcoxon rank sum test (or Wilcoxon signed-rank test if paired data was available).

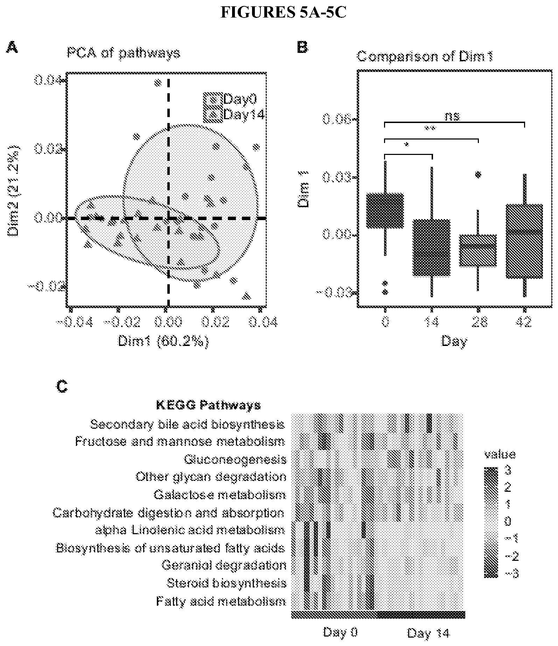

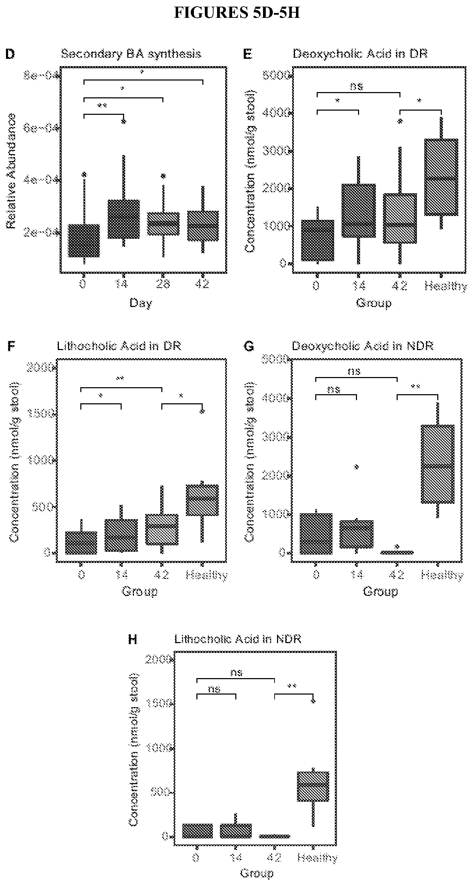

Figures 5A-5H depict that diet-induced remission is associated with metabolic reprogramming and increased levels of secondary bile acids. Figure 5A depicts a PCA analysis of KEGG pathways based on the results of Tax4Fun analysis. Figure 5B depicts the first principal component (Dim 1) from panel A, for all time points. Figure 5C depicts a heatmap showing the shift of metabolic potentials from fat/lipid metabolism to carbohydrate/sugar metabolism as DR animals receive diet therapy. Figure 5D depicts the relative abundance of the KEGG pathway for secondary bile acid biosynthesis, predicted based on 16S sequence data. Figures 5E-5H depict the levels of deoxycholic (Figure 5E) and lithocholic acid (Figure 5F) measured in the stool of DR animals and NDR animals (Figures 5G and 5H). ns = not significant, *p < 0.05, **p < 0.01, ***p < 0.0001 using Wilcoxon rank sum test (or Wilcoxon signed-rank test if paired data was available).

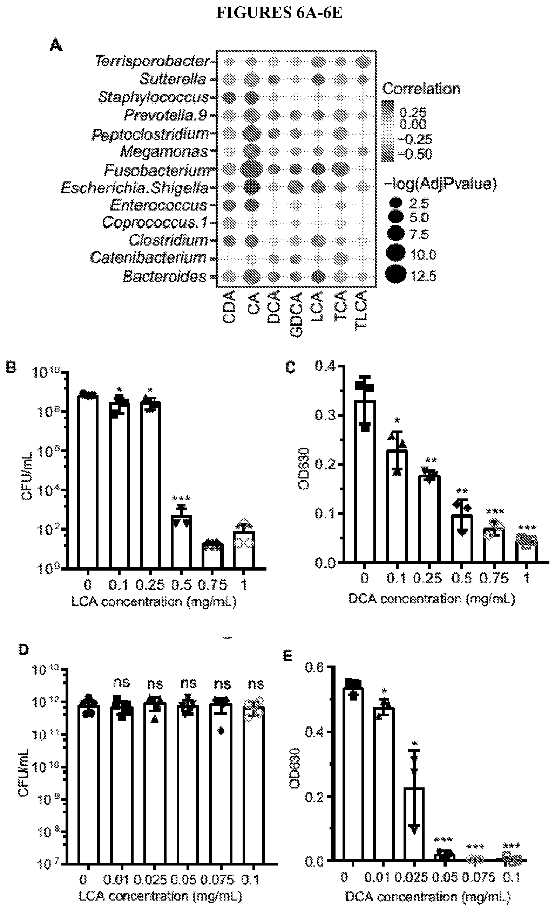

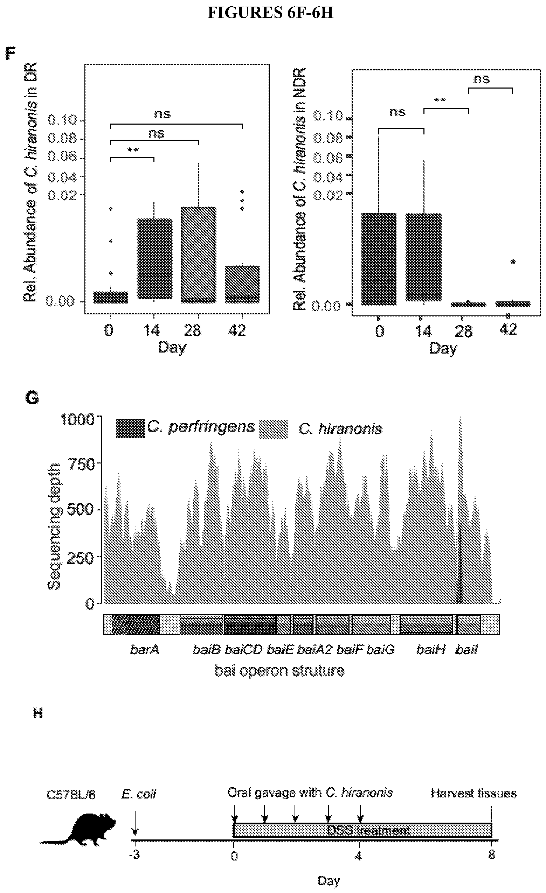

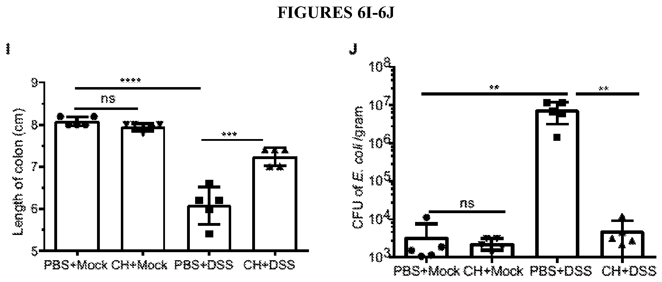

Figures 6A-6J depict that *C. hiranonis* is a diet-responsive species with the ability to produce secondary bile acids that inhibit the expansion of potential pathogens *in vitro* and *in vivo.* Figure 6A depicts the Spearman correlations between the abundance of bacteria genera and the levels of bile acids. Only genera that have significant (P < 0.05) correlations with bile acids are shown. Figures 6B-6E depict the *in vitro* growth of canine clinical isolates of *E. coli* (Figures 6B and 6C) or *C. perfringens* (Figures 6D and 6E) in the presence of varying concentrations of lithocholic acid or deoxycholic acid (mean ± s.d. shown). The *in vitro* inhibition tests were biologically repeated 2 times. Each point in the graphs represent one replicate well in the assay. Figure 6F depicts the relative abundance of the OTU corresponding to *C. hiranonis* (FJ957494.1.1454) in 16S rRNA sequencing data for DR and NDR animals. Figure 6G depicts the coverage of the bile acid operon (*bai*) from the *C. hiranonis* reference (ASM15605v1) with whole genome sequencing reads produced *C. hiranonis* (teal) and *C. perfringens* (red) canine clinical isolates. Figure 6H is a schematic showing experimental design for mouse experiments. Figure 6I depicts the length of colon at day 8. Figure 6J depicts *E. coli* Nissle strain CFUs measured in colon contents at day 8 (mean ± s.d. shown for n=5 mice). Experiments were repeated 3 times with similar results. Data shown are from a representative experiment. ns = not significant, *p < 0.05, **p < 0.01, ***p < 0.0001 using Wilcoxon signed rank sum test for relative abundance comparisons or t test for the *in vitro* culture experiments.

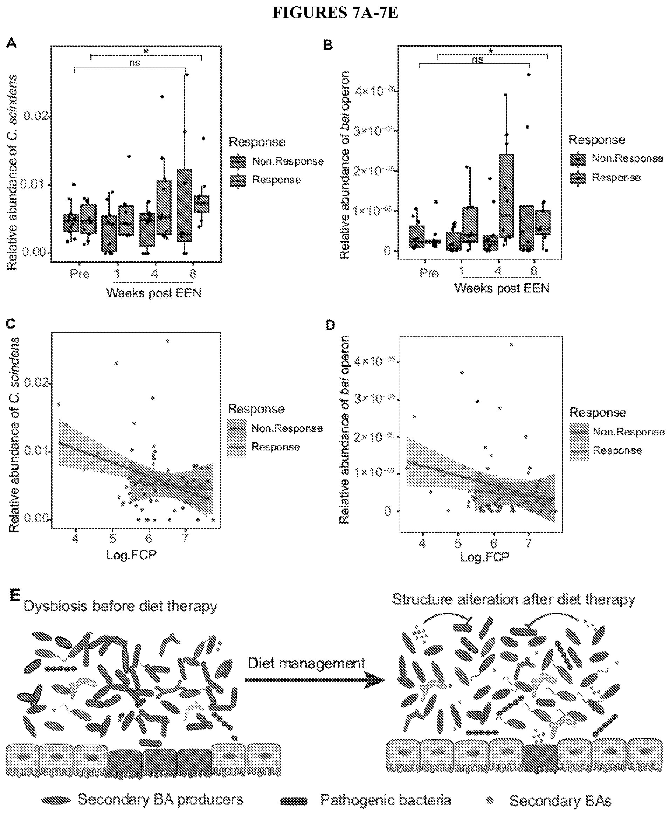

Figures 7A-7E depict that the bile acid producer, *C. scindens,* is associated with diet-induced remission in human pediatric Crohn's disease. Analysis of public data ²³ from human pediatric Crohn's disease patients treated with exclusive enteral nutrition (EEN). Relative abundance of reads (mapping ratio) aligning to *C. scindens* reference (Figure 7A) or *bai* operon (Figure 7B) from 20 patients at pretreatment and 1, 4 and 8 weeks following administration of EEN. Patients that responded to treatment and entered remission (n = 10, red) and those that failed therapy (n = 10, green) are shown. Figures 7C and 7D depict Spearman correlations between log10-transformed fecal calprotectin levels (FCP) and relative abundance of *C. scindens* (Figure 7C) (R= -0.3515 for 'Responsive', P=0.0328; R= -0.0267 for 'Non.Responsive', P=0.8770) or *bai* operon (Figure 7D) (R= -0.3944 for 'Responsive', P=0.0157; R= 0.0490 for 'Non.Responsive', P=0.7766). Figure 7E is a schematic showing proposed model for diet-induced remission. ns = not significant, *p < 0.05, **p < 0.01, ***p < 0.0001 using Wilcoxon rank sum test for relative abundance comparisons.

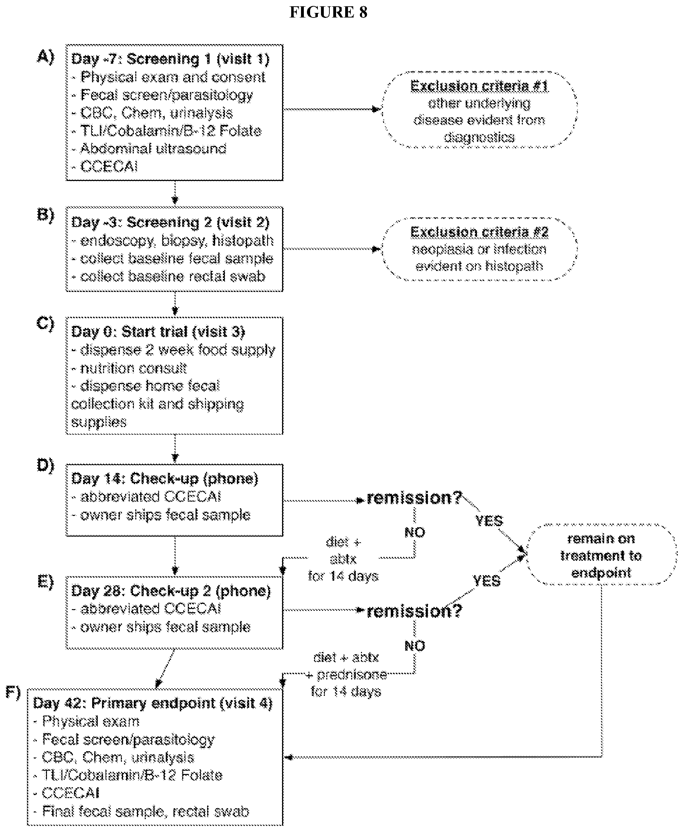

Figure 8 depicts detailed clinical design for canine chronic enteritis study.

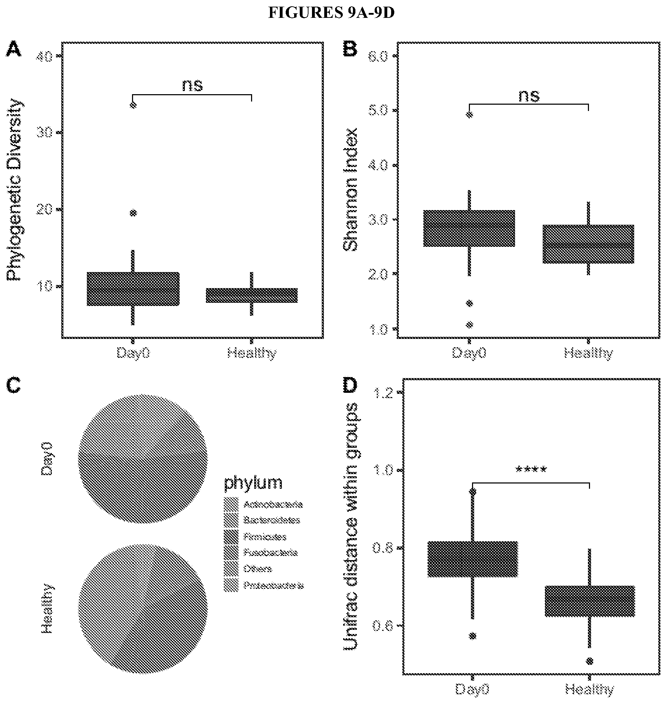

Figures 9A-9D depict community structures of microbiomes in the dogs with CE and in the healthy dogs. Faith's phylogenetic diversity (Figure 9A) and Shannon index (Figure 9B) were compared between the samples from the dogs with CE (day 0) and the samples from healthy dogs. Figure 9C depicts the ratios of microbiota compositions at a phylum level. Figure 9D depicts the Unifrac (unweighted) distances within the microbiomes of the dogs with CE or within those of the healthy dogs. ns = not significant, *p < 0.05, **p < 0.01, ***p < 0.0001 using Wilcoxon rank sum test.

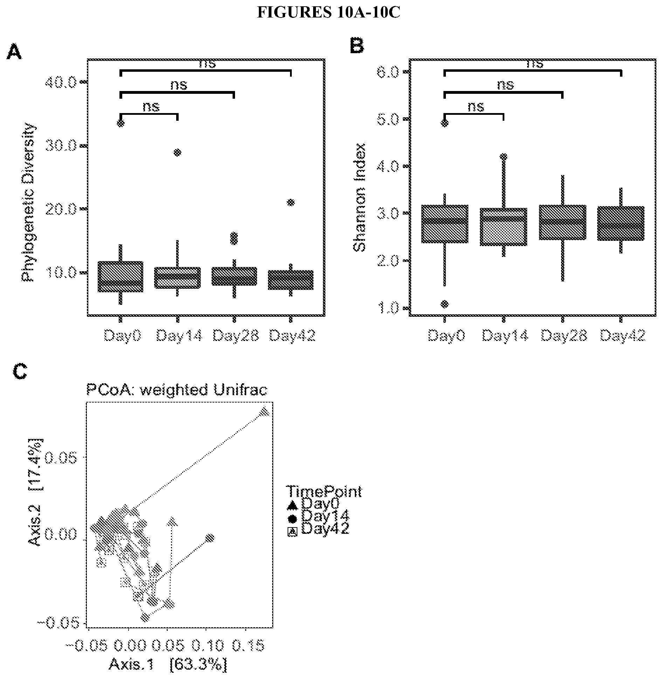

Figures 10A-10C depict that microbiota community structure changes induced by diet therapy in diet responsive dogs. Figure 10A depicts Faith's phylogenetic diversity. Figure 10B depicts Shannon index diversity. Figure 10C depicts principal coordinate Analysis (PCoA) based on Weighted Unifrac distance of the microbiomes. ns = not significant, *p < 0.05, **p < 0.01, ***p < 0.0001 using Wilcoxon rank sum test.

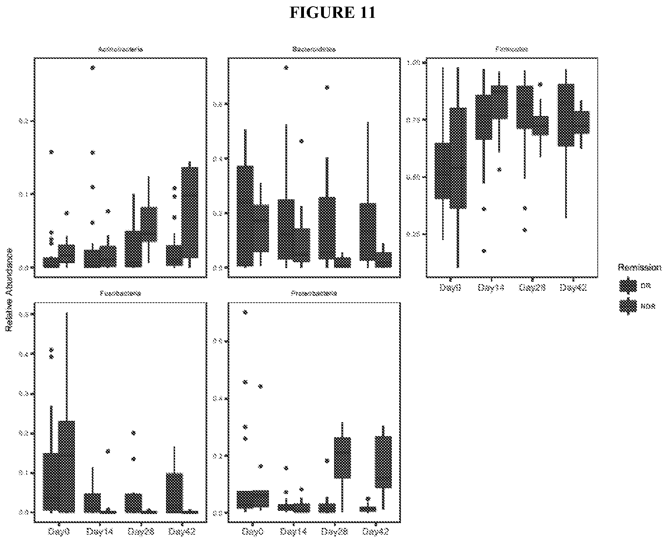

Figure 11 depicts that dynamics of microbiome changes at a phylum level for diet responsive dogs (DRs) and non-diet responsive dogs (NDRs).

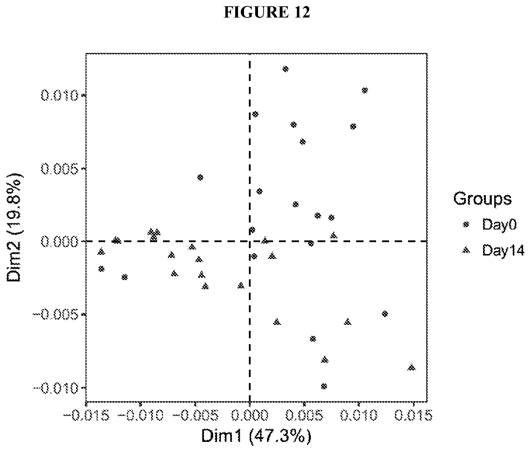

Figure 12 depicts principal component analysis based on the abundances of KO (KEGG Orthology) for the samples of day 0 and day 14.

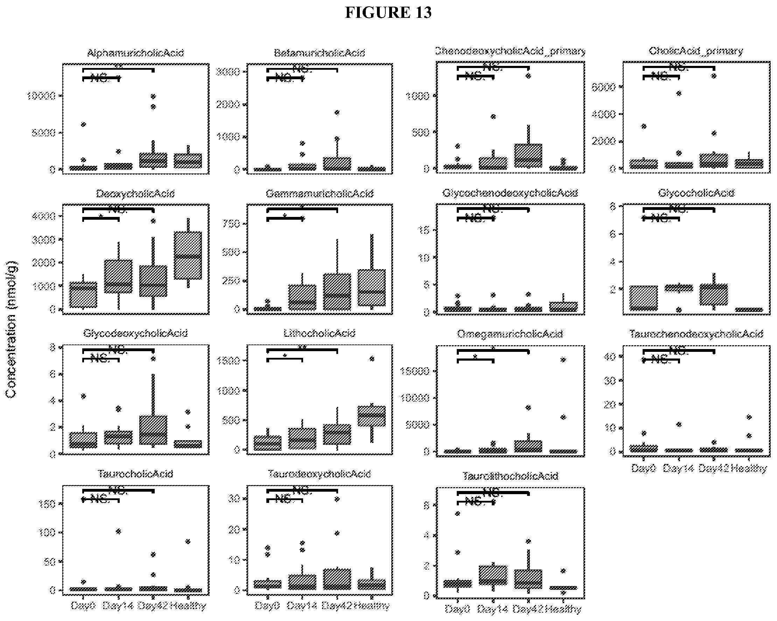

Figure 13 depicts concentrations of bile acids detected in the fecal samples of diet responsive dogs. NS = not significant, *p < 0.05, **p < 0.01, ***p < 0.0001 using Wilcoxon signed-rank test.

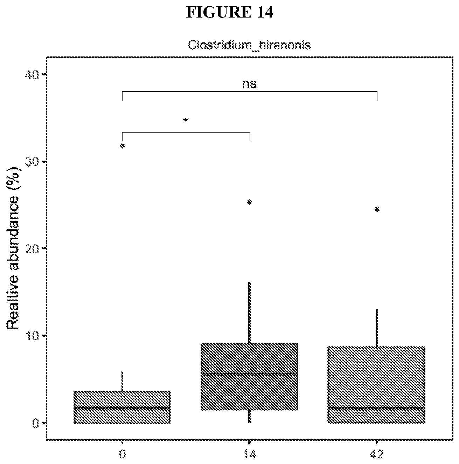

Figure 14 depicts relative abundance of *C. hiranonis* in diet responsive dogs calculated from metagenomic data.

### DETAILED DESCRIPTION OF THE INVENTION

The present invention is defined by the appended claims.

To date, there remains a need for novel methods and compositions for treating IBD and other intestinal disorders that target gut microbiome and metabolites thereof. The present disclosure relates to methods for improving intestinal health, treating intestinal dysbiosis and/or treating an intestinal disorder in a companion animal (i.e., dog), which is based, at least in part, on the discovery that intestinal microorganisms that produce bile acids can promote intestinal health and/or is associated with remission from an intestinal disorder after treatment, and that changes of intestinal microorganism population are associated to intestinal health status.

For clarity and not by way of limitation, the detailed description of the presently disclosed subject matter is divided into the following subsections:
1. Definitions;
2. Intestinal bacteria and health assessment tools relating to the same;
3. Pharmaceutical composition;
4. Food products;
5. Treatment methods; and
6. Kits.

### 1. DEFINITIONS

The terms used in this specification generally have their ordinary meanings in the art, within the context of the present disclosure and in the specific context where each term is used. Certain terms are discussed below, or elsewhere in the specification, to provide additional guidance to the practitioner in describing the methods and compositions of the present disclosure and how to make and use them.

As used herein, the use of the word "a" or "an" when used in conjunction with the term "comprising" in the claims and/or the specification can mean "one," but it is also consistent with the meaning of "one or more," "at least one," and "one or more than one." Still further, the terms "having," "including," "containing" and "comprising" are interchangeable and one of skill in the art is cognizant that these terms are open ended terms.

The term "about" or "approximately" means within an acceptable error range for the particular value as determined by one of ordinary skill in the art, which will depend in part on how the value is measured or determined, i.e., the limitations of the measurement system. For example, "about" can mean within 3 or more than 3 standard deviations, per the practice in the art. Alternatively, "about" can mean a range of up to 20%, or up to 10%, or up to 5%, or up to 1% of a given value. Alternatively, particularly with respect to biological systems or processes, the term can mean within an order of magnitude, e.g., within 5-fold or within 2-fold, of a value.

The term "effective treatment" or "effective amount" of a substance means the treatment or the amount of a substance that is sufficient to effect beneficial or desired results, including clinical results, and, as such, an "effective treatment" or an "effective amount" depends upon the context in which it is being applied. In the context of administering a composition to improving immunity, digestive function and/or decreasing inflammation, an effective amount of a composition described herein is an amount sufficient to improving immunity, digestive function and/or decreasing inflammation, as well as decrease the symptoms and/or reduce the likelihood of a digestive disorder and/or inflammation. An effective treatment described herein is a treatment sufficient to improving immunity, digestive function and/or decreasing inflammation, as well as decrease the symptoms and/or reduce the likelihood of a digestive disorder and/or inflammation. The decrease can be a 1%, 5%, 10%, 20%, 30%, 40%, 50%, 60%, 70%, 80%, 90%, 95%, 98% or 99% decrease in severity of symptoms of a digestive disorder or inflammation, or the likelihood of a digestive disorder or inflammation. An effective amount can be administered in one or more administrations. A likelihood of an effective treatment described herein is a probability of a treatment being effective, *i.e.,* sufficient to treat or ameliorate a digestive disorder and/or inflammation, as well as decrease the symptoms.

As used herein, and as well-understood in the art, "treatment" is an approach for obtaining beneficial or desired results, including clinical results. For purposes of this subject matter, beneficial or desired clinical results include, but are not limited to, alleviation or amelioration of one or more symptoms, diminishment of extent of a disorder, stabilized (*i.e.,* not worsening) state of a disorder, prevention of a disorder, delay or slowing of the progression of a disorder, and/or amelioration or palliation of a state of a disorder. The decrease can be a 1%, 5%, 10%, 20%, 30%, 40%, 50%, 60%, 70%, 80%, 90%, 95%, 98% or 99% decrease in severity of complications or symptoms. "Treatment" can also mean prolonging survival as compared to expected survival if not receiving treatment.

As used herein, and as well-understood in the art, a "probiotic" is a preparation or composition comprising microorganisms that can provide health benefits when consumed. The microorganisms include, but are not limited to bacteria, fungi, yeasts and archaea. In certain embodiments, the probiotic can modify the microbiome in the GI system to enhance the balance of the microbiome in GI system, *e.g.,* by acting as an inoculum for an increased population of beneficial microbes, and/or by antagonizing growth of deleterious microbes. In certain embodiments, the probiotic is an animal probiotic, *e.g.,* a feline probiotic or a canine probiotic.

As used herein, and as well-understood in the art, a "prebiotic" is a substance or a composition that can induce the growth or activity of one or more beneficial microorganism (*e.g.,* one or more probiotics, *e.g.,* bacteria, fungi, yeasts and archaea). In certain embodiments, the prebiotic can modify the microbiome in the GI system to enhance the balance of the microbiome in GI system. In certain embodiments, the prebiotic is indigestible to an animal. In certain embodiments, the prebiotic can induce the growth or activity of one or more animal probiotics, *e.g.,* a canine probiotic.

The term "pet food" or "pet food composition" or "pet food product" or "final pet food product" means a product or composition that is intended for consumption by a companion animal. In the present invention, the companion animal is a "domestic" dog, *e.g., Canis lupus familiaris.* A "pet food" or "pet food composition" or "pet food product" or "final pet food product" includes any food, feed, snack, food supplement, liquid, beverage, treat, toy (chewable and/or consumable toys), meal substitute or meal replacement.

An "individual" or "subject" herein is a vertebrate, such as a human or non-human animal, for example, a mammal. Mammals include, but are not limited to, humans, non-human primates, farm animals, sport animals, rodents and pets. Non-limiting examples of non-human animal subjects include rodents such as mice, rats, hamsters, and guinea pigs; rabbits; dogs; cats; sheep; pigs; goats; cattle; horses; and non-human primates such as apes and monkeys. The present invention relates to methods in dogs.

As used herein, the term *"in vitro"* refers to an artificial environment and to processes or reactions that occur within an artificial environment. In vitro environments exemplified, but are not limited to, test tubes and cell cultures.

As used herein, the term *"in vivo"* refers to the natural environment (e.g., an animal or a cell) and to processes or reactions that occur within a natural environment, such as embryonic development, cell differentiation, neural tube formation, etc.
"Pharmaceutical composition" and "pharmaceutical formulation," as used herein, refer to a composition which is in such form as to permit the biological activity of an active ingredient contained therein to be effective, and which contains no additional components which are unacceptably toxic to a patient to which the formulation would be administered.

"Pharmaceutically acceptable," as used herein, e.g., with respect to a "pharmaceutically acceptable excipient," refers to the property of being nontoxic to a subject. A pharmaceutically acceptable ingredient in a pharmaceutical formulation can be an ingredient other than an active ingredient which is nontoxic. A pharmaceutically acceptable excipient can include a buffer, carrier, stabilizer, and/or preservative.

As used herein, the term "pharmaceutically acceptable salt" refers to any salt of a compound provided herein which retains its biological properties and which is not toxic or otherwise undesirable for pharmaceutical use. Such salts can be derived from a variety of organic and inorganic counter-ions well known in the art. Pharmaceutically acceptable salts further include, by way of example only and without limitation, sodium, potassium, calcium, magnesium, ammonium, tetraalkylammonium and the like, and when the compound contains a basic functionality, salts of non-toxic organic or inorganic acids.

### 2. INTESTINAL MICROORGANISMS AND HEALTH ASSESSMENT TOOLS RELATING TO THE SAME

Disclosed herein, but not claimed as part of the present invention, are intestinal microorganisms and combinations thereof. This disclosure is based, at least in part, on the discovery that intestinal microorganisms that produce bile acids can promote intestinal health and/or is associated with remission from an intestinal disorder after treatment, and that changes of intestinal microorganism population are associated to intestinal health status.

### Intestinal microorganism capable of producing a bile acid

In certain examples, the intestinal microorganism is for use as a medicament. In certain examples, the intestinal microorganism is for the treatment of an intestinal disorder in a subject in need thereof.

In certain examples, the intestinal microorganism is a bacterium capable of producing a bile acid. In certain examples, the bile acid is chenodeoxycholic acid, cholic acid, glycochenodeoxycholic acid, glycocholic acid, taurocholic acid, taurochenodeoxycholic acid, taurodeoxycholic acid, glycodeoxycholic acid, ursodeoxycholic acid, glycoursodeoxycholic acid, tauroursodeoxycholic acid, taurolithocholic acid, alpha-muricholic acid, deoxycholic acid, gamma-muricholic acid, glycolithocholic acid, taurolithocholic acid, lithocholic acid, omega-muricholic acid or any combination thereof.

In certain examples, the bile acid is a primary bile acid. In certain examples, the primary bile acid is chenodeoxycholic acid, cholic acid, glycochenodeoxycholic acid, glycocholic acid, taurocholic acid, taurochenodeoxycholic acid or any combination thereof.

In certain examples, the bile acid is a secondary bile acid. In certain examples, the secondary bile acid is taurodeoxycholic acid, glycodeoxycholic acid, ursodeoxycholic acid, glycoursodeoxycholic acid, tauroursodeoxycholic acid, taurolithocholic acid, alpha-muricholic acid, deoxycholic acid, gamma-muricholic acid, glycolithocholic acid, taurolithocholic acid, lithocholic acid, omega-muricholic acid or any combination thereof. In certain examples, the secondary bile acid is deoxycholic acid and/or lithocholic acid.

In certain examples, the bacterium comprises a bile acid-inducible operon (bai operon). In certain examples, the bacterium comprises an enzyme having 7-dehydroxylation activity. In certain examples, the bai operon comprises a nucleotide sequence that is at least about 80% (e.g., at least about 85%, at least about 90%, or at least about 95%) homologous or identical to SEQ ID NO: 1, or any functional fragment thereof. In certain examples, the bai operon comprises the nucleotide sequence set forth in SEQ ID NO: 1 . SEQ ID NO: 1 represents an exemplary sequence of *C. hiranonis* bile acid-inducible operon. SEQ ID NO: 3 represents an exemplary sequence of *C. scindens* bile acid-inducible operon.

1 gcaaattgat tttgattggt atttctttca ttcaaaatat ctcctttcct ttatttagct
61 gtattaaaat ttataaaaaa ttttcattgt taataaaaaa atattctttg ttagtattat
121 agcataattt ataaaaataa tgataatgtt ttaatattga aataataaat atgtaaaaag
181 gttggaaatt tatttaaaaa tgaccagaga taaaaagctc aggtcatttt ttttattatt
241 acaagtaatt tgaaaaaaat atatgaaatg aatggagaaa atataactga gatacatttg
301 ataatgaaaa aaacatttat cgaaattgta aatagactca ttgttataat taataaatat
361 ttattatggc atagttgtta aaattatacc ctaaagaaac gtttcctcaa aaagtgggtt
421 ataaaataaa tgttttttga cgaaagatgt gattttattt gtaccccttt tgtataaaga
481 ttaaacagta tttttgtata aatatattgt atacagtata gagaatgtcg atgtaaaaaa
541 gtatataaaa gtaaataata atcaaaaaaa ctagttttaa ttattaaaaa tgataaaaaa
601 tattaataaa ataaagagtc aaaaatactt gttagttaaa tcacagattt tgtctaagta
661 tagattaggt tttgtatttg aaaaggtcat ctatagtgtt gtaagaaagc gagttattag

721 cacatattgt atctcaaaaa aatgttaaga taatatcaag atagggcgat aaagaaaaaa
781 gcaaattgaa aaagaaaaaa gtaactataa gtttttacaa taaatcaaaa gagaattgat
841 tttaaaagag ggaggcaaaa taccgatatg aatgatgtga aatgtaaata ttttaataaa
901 tttaatacag gaatgtcaga ttttgttact ccaggaaaac agttagaata tgtagcaaaa
961 tgcaagccag atgaaaaagc tatcatatat atagataaag aagacaatgt gagagatatc
1021 acttggaagg aacttcacat agcttcaaat aaactagctt ggcatttaat gaaaaaggga
1081 tttggaaaag gtcaggtagc aatggtatct ttcccaaatg gtatagaaca tatattagca
1141 acattagctg tttggaaaac aggaggttgc tacatgccag tttcttgtaa gataacagat
1201 acagagcttg gtgatatatg cagaataata aaaccaacag tttcttttac agataaagaa
1261 atgccttgta gaacagaaag tataaaaata ggatcagtat tcgatgtttg taaagacgaa
1321 tcagaagaaa tgccagaaga tatagctgca aatccaaata tgatttctcc atctggagga
1381 acaacaggag agcctaagtt cataaaacag aatgtggcaa gtggcttatc tgatgaaatt
1441 ataaaaagct ggtttgaaat gtcaggtatg gaatttgaac aaagacaatt attagtagga
1501 ccacttttcc atggtgctcc tcatacagca gcatttaatg gattatttgt aggaaataca
1561 ttgataatac ctagaaattt aagacctgaa agtatagtta gatatataaa agaatacaaa
1621 atagaattta tacagatgat cccaacatta atgaatagaa taataaaatt agctgatgtt
1681 gataaagaag attttaaatc aataaaagca ctacaccata ctggtggata ttgttctcca
1741 tatttaaaag aaaagtggat cgatataata ggagctgaaa aagttcacga aatgtactct
1801 atgacagagg caatcggtat cacttgtata agaggagatg aatggcttaa acactatgga
1861 agcgtaggac ttccactagg aggaagcaga atatcaataa gagatgaaga aggaaatgaa
1921 ttaggaccac atgaggttgg agaaattcat atgacttcac caagtgcttg ttgcatgaca
1981 gaatacataa accataaacc acttgaaact aaagatggtg gatttagaag tgttggtgat
2041 ttcggttatg tagatgaaga tggatacctt tacttctcag atagaagaag cgacatgctt
2101 gttataggtg gagaaaacgt atttgcgact gaagttgaac cagtactacc agcttatgaa
2161 aaagtagttg atgctgtggt agttggaata cctgatgaag agtggggaag aagattacac
2221 gcaatagtac agaagaaaga agaagtttca gcagaagaat taatcgagta cttaggaaaa
2281 cacttattac catataaagt tccaaagagc tttacatttg ttccttgcat accaagaggt
2341 gacaatggaa aggtaaacag agataagatg ctaaaaggct taatagaaaa aaatctagtt
2401 aataaagttt gctaggatat aaattcagtt aactatctgc accaagtgca gtggaaaata
2461 aatcaaaatt aataaaataa attaataagg taaatttagg aggtctaaaa tgagttacga
2521 cgcacttttt tcaccattta aaatcagagg attagaactt aaaaacagaa tagttctacc
2581 aggtatgaat acaaaaatgg caaaaaataa acatgattta agcgatgata tgatagctta
2641 ccatgttgca agagcaaaag caggttgtgc attaaatata tttgaatgtg ttgcgctatg
2701 tccagcacct catgcatata tgtacatggg attatacaat gacaatcatg tagctcagtt
2761 aaaaaaatta acagatgctg ttcacgaagt tggcggtaaa atggctgttc agttatggca
2821 tggtggtttc agcccacaga tgttctttga taaaacaaat acattagaaa caccagatac
2881 tataacagtt gaacgtattc atgaaatagt taaagagttt ggagaaggtg caagaagagc
2941 tgttgaagct ggattcgatg cagttgaatt ccatgcagca cacagttact tacctcacga
3001 attcctaagt ccaggaatga acaaaagaac tgacgaatat ggtggaaact tcgaaaatcg
3061 ttgcagattc tgcttcgaag tagttgaagc tatacgtgca aatataccag aagatatgcc
3121 attcttcatg agagttgact gcatagatga gttaatggat gaagtaatga cagaagaaga
3181 aatagtagaa ttcataaata gatgtgctga tctaggagta gacgtagctg acttatcaag
3241 aggtaatgct cagtcattcg caacagttta cgaagttcct cctttcaact tacagcacgg
3301 tttcaatata gaaaacatat acaacatcaa aaaacagata aaaataccag taatgggtgt
3361 tggacgtata aacacaggag aaatggctaa ccaggtaata gcagatggaa aatttgactt
3421 agttggtata ggtcgtgctc agttagcaga tcaggattgg gttgctaaag ttagagaagg
3481 taaagaagat ttaatacgtc attgtatagg atgtgaccag ggatgctacg atgcagttat
3541 aaaccctcag atgactcata taacttgtac aagaaaccct cacttatgct tagaatacaa
3601 aggtatgcca aaaactgatg aacctaaaaa agttatgata atcggtggtg gtatggctgg
3661 tatattagca gctgaagtac ttaaaaaacg tggacatgaa ccagttatat tcgaagcttc
3721 tgatcactta gcaggacagt tcgtattagc aggtaaagct ccaatgaaag aagactgggc
3781 agctgcagct aaatgggaag ctgaagaagt agctcgttta ggaatagaag ttagatacaa
3841 tacaaaagtt actccagaat taatagaaga attcgctcca gaccacgttg ttatagctat
3901 aggatctgat tacgtagctc cagctatacc aggtatagat agtgacaaag tttacactca
3961 gtatcaggta ttaaaaggtg aagtagaacc aaaaggacat gtagcagtag ttggttgtgg
4021 attagttggt acagaagttg ctcagtactt agcagctaga ggagctcagg taacagctat
4081 agaaagaaaa ggtgttggta caggtctaag catgcttaga agaatgttca tgaacccaga
4141 attcaaatac tacaaaataa acaaaatgtc tggaactaac atagttggta tagaaccagg
4201 aaaacttcac tacataatga ctaacaagaa aactcaggaa gttactgaag gtgtgttaga
4261 atgtgatgca gcagtaatct gtacaggtat aactgctaga ccaagtgaag atttacagga
4321 aaaatgtaaa gaattaggtg ttccattcaa cgtaataggt gacgcagctg gtgctagaga
4381 tgctagaata gctactcagg aaggttacga agtaggtatg agtatataat ttaaaaatta
4441 tataattata taaattaaaa gttattaaat tacaagaaag aggcgaataa aatgacttta

4501 gaagcaagaa tagaagcatt agaaaaagaa atacagagat taaacgatat agaagctata
4561 aaacagttaa aagctaaata tttccgttgc ctagatggaa aattatggga tgaattagaa
4621 actactcttt ctcctaacat agaaacttct tactctgatg gaaaattagt attccacagc
4681 ccaaaagaag taactgaata tttagcagca gcaatgccta aagaagaaat aagtatgcac
4741 atgggacata ctccagaaat aactatagac agcgaaaata ctgctacagg aagatggtac
4801 ttagaagata acctaatatt cacagacgga aaatacaaaa acgttggaat aaacggtgga
4861 gcattctaca cagataaata tgaaaaaata gacggacagt ggtacataaa agaaactgga
4921 tatgttcgta tatttgaaga acatttcatg agagatccaa aaatacatat aactagcaac
4981 atgcataaag aaaaataata actgattgct aataaacaag atataaacag ggggctggta
5041 aacagccagc cctctgaaaa ataaactaaa aaactataat cttttaaaat cttaattaaa
5101 gtagaaggag ataagacaat gaacttagta caggacaaaa tagttataat aacaggtgga
5161 acaagtggta taggtctttg cgcagcaaaa atattcatgg ataacggtgc aacagtttct
5221 atattcggaa aaactcagga agaagtagat gctgctaaag cagaattaaa agaaactcac
5281 ccagataaag aagtattagg atttgctcca gatttaacta atagagatga agttatggct
5341 gcagttggtg cagtagctga aaaatacgga agattagacg ttatgataaa caatgctggt
5401 gttactagct caaacgtatt ctcaagagtt agcccagaag aattcacata tttaatggat
5461 ataaacgtta caggtgtatt ccatggtgct tgggctgctt accactgcct gaaaggtgaa
5521 aagaagatta taataaatac tgcttcagta acaggaatac acggatcatt atcaggagtt
5581 ggatacccaa caagtaaatc agctgttgta ggattcactc aggctcttgg tagagaaata
5641 atacgtaaaa acataagagt tgttggtgtt gcaccaggtg ttgttaacac tccaatggtt
5701 ggtaatatac cagatgaaat attagatgga tacctaagct cattcccaat gaagagaatg
5761 ttagaaccag aagaaatagc taacacttac ttattcttag cttctgactt agctagtggt
5821 ataacagcta caactgtaag cgttgacggt gcttatagac catcataaga tttactttaa
5881 tttaaaactg taattagata gataatacga cgattaatat aaaaaatgtt ctttaaaaga
5941 aaaggagaaa taaaatggct ggattaaaag attttcctaa atttggtgca ctttctggat
6001 taaaaatatt agatagtgga tctaacatag ctggacctct aggtggtgga cttttagcag
6061 aatgtggtgc tacagttata cacttcgaag gacctaaaaa acctgacaac cagagaggtt
6121 ggtatggata ccctcagaac cacagaaacc agttatcaat ggttgctgat ataaaatctg
6181 aagaaggtag aaaaatattc ttagacttaa taaaatgggc tgacatatgg gttgaatcat
6241 caaaaggtgg acagtacgac agactaagtc tttctgatga agttatatgg tcagtaaacc
6301 ctaaaatagc tatagttcac gtttctggat acggacaggt tggagatcca tcatacgtaa
6361 caaaagcttc ttatgatgct gttggacagg cattcagtgg atacatgtca ttaaatggtg
6421 ttaatgaagc attaaaaata aatccttacc taagtgactt cgtatgtgtt cttactactt
6481 gctgggcaat gttagcatgc tacgtaagta ctcagttaac tggaaaagga gaatctgtag
6541 acgttgctca gtacgaagca ttagctcgta taatggacgg acgtatgata cagtacgcta
6601 ctgatggtgt aagtgttcca aaaactggta acaaagatgc tcaggcagct ctattcagct
6661 tctatacttg taaagatgga agaactatat tcataggtat gactggtgct gaagtatgta
6721 agagaggatt ccctgtaata gggcttccag ttcctggtac aggtgaccct gacttcccag
6781 aaggattcac aggatggatg ataaatactc cagttggaca gagaatggaa aaagctatgg
6841 aagcattcgt tgctgaaaga actatgccag aagttgaaaa agctatgata gatgctcaga
6901 taccatgcca gagagtttat gatcttgaag actgcttaaa cgaccctcac tggaatgctc
6961 gtggaactat aatggaatgg gatgacccaa tgatgggaca cataaaaggt cttggattaa
7021 taaacaaatt caaaaacaac ccttctgaaa tatggagagg tgctccatta ttcggtatgg
7081 acaacagaga cataattaga gaccttggat attctgagga ggaagttaac gatttatacg
7141 ctaaaggtat tgtaaacgaa ttcgaccttg aaacaactat aaaacgttac aaacttgatc
7201 aggttatacc tcacatggct aaaaaagata aataagaaac gtattaaata ataaaatata
7261 aatgtcgagc ctgccagaat gagaattttg acaggcttga tattataacg aaatgttata
7321 aaaaaaacaa aataaaaatt gcttaaattt tatacaagga gaattgaaat gacagcaaca
7381 aacgcaaact ataaaaaagg ctttatccca tttgctatag cagcgttact agtaggtctt
7441 ataggtggtt tcacagccgt tctagcacct gcattcgtag cagatatggg tcttaacgat
7501 aacaatacta catggatagc actagcgctt gcaatgtcta cagctgcatg tgctccaata
7561 cttggtaaat taggtgacgt acttggacgt cgtaaaactt tattattagg aatcatagta
7621 ttcacaatag gtaacgtatt aacagcaata gcatcttcat taatattcat gctaggtgca
7681 agatttatag ttggggttgg tacagcggct atagctccag ttataatggc ttacatagtt
7741 acagaatatc caccagaaga aactggtaag ggattcgctc tttatatgtt aatatcaagt
7801 gctgcagttg ttgttggtcc aacttgtggt ggattaataa tgcaggcatt tggatggaga
7861 atgatgatgt gggtttgtgt tgccctttgt gtagtaacat tcttcatatg ttcagtaatg
7921 attaagaaaa cagactttga aaagaaaagt cttgataact tcgataaaaa aggtgcagta
7981 tgcgtactaa tattcttcag tttagtatta tgtataccat catttggaca gaatataggt
8041 tggacatcag cgccattcct aggtgttaca gcagtagctt tagtaacatt attcttatta
8101 ataaaagctg aaagcagtgc agaaaaccca atattaagtg gtaaatttat gaaacgtaaa
8161 gaattcatat taccagtatt aatattattc cttactcagg gattaatgca ggctaacatg
8221 actaacgtaa tattattcgt tagagctact cagccagaaa atacaataat atcaagtttc

8281 gcaatatcaa tcctttacat aggtatgtct ttaggttcag tattcatagg acctatggca
8341 gataaaaaag aaccaaaaac tgtacttaca ggatcacttc tattcactgg tataggttgt
8401 gcaatgatgt acttcttcac agaaactgca ccattcgcaa tgttagctgg atctctagga
8461 atgttaggta taggacttgg aggaaatgct acaatactaa tgaaagtttc attatctgga
8521 ttatctcagg cagaagctgg atcaggaaca ggaacatacg gattattcag agatatatca
8581 gctccatttg gtgttgcggt attcgtacca ctatttgcaa acacagttac aacaagaatg
8641 gctggagtaa tggctaacgg aactgcagaa gctgctgcta aatcattagc atctgtttct
8701 tctatacata cattagcatt agttgaagta tgctgtgtaa tattagcaat agttgcagtt
8761 agaatgctac caaaaataca caataaataa tttaaaaata ataacagagt tgaaaaaaca
8821 ctcaattaaa agaggggcct tgagcccctt ttttagtgta aaaatgacaa aatactatca
8881 atttatataa atgataatta aactcgtcaa ccaaagaaat attcacaaag tagataataa
8941 tagatattca aaaagtgata tattattagg caaaaagtgc aagaaattag cgagtattcg
9001 acaacttttt gtccaatggt agaaaagaat atttgttatc ataaatatag acaaagggct
9061 ttgaccaaaa ctaaggaaaa agtttgcata atataaaaaa taaaataaaa taaaaaaata
9121 aaaataaaat aaaagcgaaa ggaaaaaaca acatcatgga tatgaaaaat tctaaactat
9181 tctcaccttt aacaatagga tcattaacat taaacaacag agttggtatg gcaccaatga
9241 gtatggacta cgaagctgct gacggaacag ttccaaaaag attagcagat atatttgttc
9301 gtagagctga aggtggaaca ggatatgtaa caatagacgc ggtaacaata gatagtaaat
9361 ataaatatat gggtaataca actgctttag attctgatga tttagttact cagttcaaag
9421 aatttgcaac aagagttaga gaagcaggaa gcacattaat acctcaggtt atacatccag
9481 gaccagaatc aatatgtgga tacagacaca tagcaccact tggaccatca gttaatacaa
9541 atgctaactg ccacgtgagc cgtgctataa gtgtagatga aatacatgaa ataataaaac
9601 agtttggaca ggctgctaga agagttgaag aagcaggatg cggtggtata ggattacact
9661 gtgcacatgc ttacatgcta ccaggttcat tcttatctcc attaagaaac aaaagaatgg
9721 atgaatacgg cggatgtcta gataacagag caagattcgt aatagaaatg atagaagaag
9781 ttcgtagaaa tgtaagtcct gatttcccaa taatgcttag aatatctggg gatgaaagaa
9841 tgataggagg aaactcttta gaagatatgt tatacttagc tccaaaattt gttgaagctg
9901 gtgtaaatat gtttgaagtt tctggaggta ctcagtacga aggattagaa cacataatac
9961 caagtcagaa caaaagcata ggtgtaaacg tacacgaagc atctgaaatc aaaaaagttg
10021 tagatgttcc agtttacgct gttggtaaaa taaatgacat aagatacgct gctgaaatag
10081 ttgaaagagg actagttgat ggggtatcaa taggtagacc attattagca gatccagact
10141 tatgtaataa agcaaaagaa aacttatttg atgaaataac tccatgtgca agctgtggag
10201 gaagctgtat aagccgtact gcagatagac ctcagtgtcg ttgccatata aacccaagag
10261 ttggattcga atatgattat ccagaagttc cagctgaaaa atctaaaaaa gttctagttg
10321 taggtgctgg acctggtggt atgatggcag cagttacagc agctgaaaga ggacatgatg
10381 taacactttg ggaagctgac actcagatag gtggacagat aaacttagca gtagtagctc
10441 caggtaaaca ggaaatgact aaatggttat ctcacttaaa ctacagagct aaaaaagctg
10501 gagttaaaat ggtattagga aaagaagcta cagtagaaaa cataaaagaa tttgctccag
10561 aagcagttat agttgcaaca ggtgctagac cattagttcc accaataaaa ggaactcagg
10621 actacccagt tcttacagct catgacttct taagaggaaa attcgttata ccaaaaggaa
10681 aagtttgtgt actaggtgga ggagctgttg cttgtgaaac tgcagaaaca gtattagaaa
10741 acgctagacc aaacgcattc actagaggat ttgatgctag tatcggtgat gtagatgtta
10801 cattagtaga aatgttacca cagttattaa caggagtatg tgctccaaat agaactccat
10861 taataagaaa acttaaaaac aaaggtgttc atataaatgt aaatactaaa atattagaag
10921 taactgacca cgacgttaaa gttcagagag ctgacggtgc agaagaatgg ttaaaaggat
10981 tcgactacat actattcgga cttggttcta gaaactacga tccaatatct gaacagataa
11041 aagaattcgt tccagaagta cacgttgttg gggatgctaa gagagctaga caggcaagct
11101 ttgcaatgtg ggaagctttc gaagcagcat acagcttata a [SEQ ID NO: 1]

1 aaaagatatt aagcattaag aaaatgcaca aaaaatcagc gtgtgagagg gagggcaagg
61 agttgaagcg tgactttttt aacaagttta atttggggac atcgaacttt gtcacgccgg
121 gaaaacagtt ggaatacgtt tcggaatgca agccagattc tactgcggtc atttgcttag
181 ataaagaaca gaactgttcc gttattactt ggcatcagct gcacgtctat tccagccagc
241 tggcatggta ccttatagaa aatgagattg gcccggggtc gatcgtactt acaatgtttc
301 cgaacagcat cgagcacatt attgcggtat ttgcaatctg gaaggcgggc gcctgctata
361 tgcccatgtc ctataaggcg gcggaatccg agatcaggga ggcctgcgat accatccacc
421 cgaatgcggc ttttgcggaa tgcaagattc caggattaaa attctgcctt agcgcagacg
481 agatatatga ggcgatggaa ggaagatcca aggagatgcc ttcggaccgt ctggccaatc
541 cgaacatgat atccttatca ggcggaacca gcggaaagat gaagttcatc cgtcagaacc
601 ttccatgcgg gctggacgat gagacgatca gaagctggtc tttgatgtct ggaatgggat
661 ttgagcagcg ccagctgctg gtaggcccgc tgtttcatgg cgcgcctcac tccgcggcgt
721 ttaatggact gttcatgggc aacaccctgg tactgaccag gaacctttgc ccgggaaata

781 tcctgaacat gattaagaaa tataagattg aatttataca gatggtgccg accctgatga
841 accggcttgc caaactggag ggagtcggaa aagaagactt tgcatccctg aaggcgctgt
901 gccatacagg gggcgtctgt tctccctggc ttaagcagat ctggatcgac ctgctggggc
961 ctgaaaagat ctatgagatg tattccatga cggaatgcat cggccttacc tgcatccggg
1021 gagacgagtg ggtgaagcat ccgggaagca tcggacggcc agtgggcgat agcaaggtgt
1081 ctatccggga tgagaatggc aaggaagttg cgccttttga gattggcgag atctatatga
1141 cagcgccggc ctcctatctg gttaccgagt acatcaattg ggaaccgctg gaagtgaaag
1201 agggaggctt ccgaagcgta ggggatatcg gctacgtgga tgagcagggc tatctgtact
1261 tttctgaccg gcgcagcgac atgctggtat caggcggaga aaacgtgttc gccaccgaag
1321 tcgagacggc gcttttgaga tataaggata tcctggacgc tgtagtggta gggataccgg
1381 atgaagatct ggggcgaagg ctccatgcgg tcattgagac agggaaagag ataccggcag
1441 aggaactgaa aacattcctg agaaagtatc tgactccata taagatacca aagacgttcg
1501 agttcgtaag gagcatacga aggggagaca atggaaaggc cgacaggaag cggatcctgg
1561 aagattgtat tgcccgcggg ggatgattct ataaatgcaa agaaaacaaa ttatataaag
1621 gaggagtaac aaaatgagtt acgaagcact tttttcacca ttcaaggtca gaggactgga
1681 acttaaaaac cgtatcgtcc tgcctggaat gaacaccaag atggcaaaga acaagcacga
1741 cataggcgag gatatgatag cctaccatgt tgccagggca aaagcgggat gcgcgttaaa
1801 tatatttgaa tgcgtagcat tatgtccggc gcctcacgct tatatgtata tggggcttta
1861 tacggaccat catgtagaac agcttaagaa attgacggat gcagtccatg aagcaggcgg
1921 caagatgggc atccagctgt ggcatggagg attcagcccg cagatgttct ttgacgagac
1981 caacaccctg gaaactccgg acactcttac ggtagagagg attcatgaga tcgtagaaga
2041 attcggacgc ggcgcaagga tggctgttca ggctggattt gacgcagtag aattccatgc
2101 ggctcacagt tatctgcctc acgagttctt aagccctgga atgaacaaac gtacggatga
2161 gtacggcgga agttttgaga accgctgcag attctgttat gaagtcgttc aggcaatccg
2221 ttccaatatc ccggatgaca tgccattctt tatgcgtgca gactgcatcg acgaattaat
2281 ggaacagacc atgacagagg aagagatcgt tacatttatc aataagtgcg cagaacttgg
2341 cgtggatgtg gcagaccttt cccgtggaaa cgcgacttca ttcgcaaccg tatatgaagt
2401 tccgccattc aacctggctc atggcttcaa catagagaat atttacaaca tcaaaaagca
2461 gatcaatatc ccggttatgg gagttggccg tatcaataca ggagagatgg caaacaaggt
2521 cattgaagaa ggcaagtttg acctggtagg catcggacgc gcccagcttg cagatccaaa
2581 ctggatcacc aaagtaagag aaggcaaaga agacctgatc cgccactgta tcggatgtga
2641 ccagggatgc tatgacgcag tcatcaatcc aaagatgaag catatcacct gcacccacaa
2701 tccaggattg tgcttagagt atcagggaat gccaaagaca gacgctccta agaaagtcat
2761 gatcgtagga ggcggaatgg caggcatgat cgctgcggaa gtattaaaga ccagaggcca
2821 taacccggta atcttcgagg catccgacaa gcttgcagga cagttcaggc tggcaggcgt
2881 agcgccgatg aagcaggatt gggcagatgt tgcagaatgg gaagcaaaag aagtagagcg
2941 ccttggaatc gaagtacgtc tgaataccga agtgactgca gagaccatca aggaattcaa
3001 tccggataat gtcatcatcg cagtaggctc tacctatgcg ctgcctgaga ttccgggaat
3061 cgacagccca agcgtatact cccagtatca ggtactgaaa ggggaagtaa atccgacagg
3121 ccgtgtagcc gttatcggat gcggactggt tggtacggaa gtcgcagaac ttctggcatc
3181 cagaggcgca caggtaatcg cgatcgagag gaagggcgta ggtaccggcc ttagcatgct
3241 tcgcagaatg ttcatgaacc cggaattcaa atattacaag atcgccaaga tgtccggaac
3301 aaatgtcacc gctttagagc agggcaaggt tcactacatc atgacagaca agaagaccaa
3361 agaagtgacg cagggagtcc tggaatgcga cgctaccgtt atctgtacag gaattaccgc
3421 acgtccaagc gatgggctta aggcaagatg cgaagaactt ggaatcccgg ttgaggtgat
3481 cggagacgct gctggcgcaa gagactgcac gatcgcgaca cgcgaaggct atgacgcagg
3541 aatggcaatc tagaaaatca gaacttatca atcttacata tagaaaggat gatacatatg
3601 acattagaag agagagttga agcattagaa aaagaattgc aggagatgaa ggatattgag
3661 gcaatcaagg aactgaaagg aaagtatttc cgctgcctgg acggaaagat gtgggatgag
3721 ctggagacca ccctgtcacc aaatatcgta acctcttatt ccaacgggaa actggtattc
3781 catagcccga aggaagttac cgattactta aagagctcga tgccaaaaga agagatcagc
3841 atgcatatgg gccacacgcc ggagatcacc attgacagcg agactacggc tacgggcaga
3901 tggtatctgg aagatagact gatctttacg gacggtaagt acaaagacgt aggaatcaat
3961 ggcggcgcgt tctatacaga caaatatgag aagatagacg gccagtggta catccttgaa
4021 accggctatg tacgaatcta tgaagaacat ttcatgcgtg atccaaagat ccatatcacg
4081 atgaacatgc acaaataaga atattgtaaa agaaaggcag gagtaagagt atgaatctcg
4141 tacaagacaa agttacgatc atcacaggcg gcacaagagg tattggattc gccgctgcca
4201 aaatatttat cgacaatggc gcaaaagtat ccatcttcgg agagacgcag gaagaagtag
4261 atacagcgct tgcacagtta aaagaacttt atccggaaga agaggttctg ggattcgcgc
4321 cggatcttac atccagagac gcagttatgg cagcggtagg ccaggtagca cagaaatatg
4381 gcagactgga tgtcatgatc aacaatgcag gaattaccag caacaacgta ttctccagag
4441 tgtctgaaga agagttcaag catattatgg acatcaacgt aacaggcgta ttcaacggcg
4501 catggtgcgc ataccagtgc atgaaggatg ccaaaaaggg cgttatcatc aacacggcat

4561 ccgttacagg catcttcgga tcactctcag gcgtaggata tccggccagc aaggcaagcg
4621 tgatcggact cacccatgga cttggaagag agatcatccg caagaatatc cgtgtagtag
4681 gagtggctcc tggagttgtg aacacggata tgaccaatgg caatcctccg gagatcatgg
4741 aaggatatct gaaggcgctt ccgatgaaga gaatgcttga gccggaagag atcgctaatg
4801 tatacctgtt cctggcatct gacttggcaa gcggcattac ggctactacg gtcagcgtag
4861 acggggctta cagaccataa ttttaatttt tactaagtag aatatgtgat atagaaaagg
4921 agatataaaa acatggctgg aataaaagat tttccaaaat tcggagctct tgcagggctt
4981 aagatacttg acagcggatc taacatcgcc ggacctttag gcggaggcct tctggcagaa
5041 tgcggagcaa cggtcatcca ttttgaagga ccaaagaaac ctgataacca gagaggatgg
5101 tacggctatc cacagaatca ccgtaatcag ctgtctatgg tagcagacat caaatctgaa
5161 gaaggaagaa agatcttcct tgatctgatc aaatgggcag atatctgggt agagtcatcc
5221 aaaggcggac agtatgacag gctgggactt tccgatgaag tcatctggga agtaaatcct
5281 aagattgcca tcgtgcacgt atccggatat ggacagacag gagacccgtc ttacgttaca
5341 cgtgcatcct atgacgcagt aggccaggca ttcagcggct atatgtcact gaacggaaca
5401 acggaagcgc tgaagatcaa tccttatctg agcgatttcg tatgcggact taccacatgc
5461 tgggctatgc ttgcctgcta tgtaagcacc attcttaccg gaaaaggcga atctgttgac
5521 gttgcacagt acgaagcgct ggcacgtatc atggacggac gtatgatcca gtacgctaca
5581 gacggcgtga agatgccaag aaccggcaat aaggatgcgc aggctgccct gttcagcttc
5641 tacacctgta aagacggacg tacgatcttt atcggaatga ctggcgcgga agtatgtaag
5701 agaggcttcc cgatcatcgg acttccggta cctggaaccg gagacccgga cttcccggaa
5761 ggcttcacag gctggatgat ctatactcct gtaggacaga gaatggaaaa ggctatggag
5821 aagtatgtat ctgagcatac gatggaagaa gtagaggctg agatgcaggc acaccagatt
5881 ccatgccaga gagtatacga gctggaagac tgcctgaacg atcctcactg gaaagcacgt
5941 ggaactatta cggagtggga tgacccgatg atgggacata tcacaggcct tggactgatc
6001 aacaagttca agagaaatcc ttccgaaatc tggagaggcg ctccgctgtt cggtatggat
6061 aaccgcgata tcctgaaaga cctgggatat gacgatgcaa agatcgatga actctatgag
6121 cagggcatcg tcaatgaatt cgaccttgac actactatca aacgctatag actggatgaa
6181 gtaattccac atatgagaaa gaaagaggag taagagtatg agcaccgtag ccaatccaaa
6241 ttataagaaa ggttttgtcc cctttgcaat tgcagcactc ctggtgagcc tgatcggcgg
6301 ttttaccgcc gttctcggcc cggccttcgt ggcggaccag gggattgact ataataatac
6361 cacatggatt tccctggcgc tggcgatgtc ttccgccgca tgcgctccaa tccttggaaa
6421 actgggagac gtgctaggac gcaggacgac gctgcttctg ggtattgtga tctttgcggc
6481 cggcaatgtg ctgacagccg tagccacgtc cctgatattc atgctggcag cccgttttat
6541 cgtaggtatc ggaacagcag cgatctcacc gatcgttatg gcctatatcg taaccgagta
6601 tccgcaggag gagacaggaa aggcctttgg cctgtatatg ctgatctcca gcggcgccgt
6661 cgtggtagga cctacctgtg gcggcctgat catgaatgcg gctggctgga gagtcatgat
6721 gtgggtatgc gtcgctctgt gcgtcgttgt attcctgatc tgcacattct ccatcaagaa
6781 gactgcattt gagaagaaga gcatggcagg atttgacaag ccgggcgcag ccctggtagt
6841 cgtattcttc agtttgttcc tgtgcatccc atccttcgga cagaatatcg gatggtcttc
6901 cacagcattt atcgcagcag cggcagtagc gctggtagca cttttcatcc tggtaatggt
6961 agaaaagaaa gcgaagagtc cgatcatgaa cggcaagttt atggcacgca aggaattcgt
7021 gcttccagta ttgatcctgt tccttacaca gggacttatg atggcaaata tgaccaatgt
7081 catcgtgttc gtgcgctata cgcagccgga caatgtcatt atatcaagtt ttgcgatctc
7141 catcatgtac ataggaatgt ccttaggctc cgttatcatt ggacctgttg cagataagaa
7201 agagccaaag acggttctga cattctctct ggtactgaca gccatcggct gtgcgctgat
7261 gtatctgttc aaggcagatt cctccgtcgc tatctttgcg gcatccttgg gaatccttgg
7321 atttggcctt ggaggaaatg caaccatctt catgaaggta gcgctttccg gcctgtccag
7381 cgaagtagct ggctctggta ctggaaccta tggcctgttc agagatatct cggcaccatt
7441 cggcgtggca gtgttcgtgc ctatgtttgc caacggcgta acagcgaata ttgcgaaata
7501 cgcgtcaggc ggcatggaag aaggcgccgc tacggtaaaa gcagccatct catccatcca
7561 gacgctgaca ctggttgaac ttggatgtat cgttgtggga atcatccttg tgagaatgct
7621 gccaagaatc tatcagaaga aagaggcata aataagttaa gaaaagaggt aattataaat
7681 ggatatgaaa cattccagat tattttcgcc gcttcagatc ggatccctga cactgtctaa
7741 ccgtgtcggc atggctccca tgagcatgga ctatgaagca gcagacggaa ctgtgcccaa
7801 gaggctggcg gacgtatttg tccgccgcgc cgagggaggc acaggctacg tcatgatcga
7861 cgcggtgacg atagacagca agtatcctta tatgggaaat acaacggccc ttgaccgtga
7921 tgaactggtt ccccagttta aggaatttgc tgacagagta aaagaagcag gcagcacgct
7981 ggtgccgcag atcattcatc cgggtccgga atccgtatgc ggctaccggc atatcgctcc
8041 gcttggacct tctgccaaca ccaatgcaaa ctgccacgtg agcagatcga tcagcataga
8101 tgagatccat gacatcatta agcagttcgg ccaggcggca cgccgcgccg aagaagcagg
8161 atgcggggca atctccctgc actgcgcgca tgcgtatatg ctgccaggat ccttcctgtc
8221 accgcttcgc aacaagcgca tggatgaata tggcggaagc cttgacaacc gtgcccgttt
8281 cgtgatcgag atgattgagg aggcccgcag gaatgtgagt cctgatttcc cgatcttcct

8341 tcgtatctcc ggagacgaga gaatggtagg aggcaacagc cttgaagata tgctctacct
8401 ggcaccgaag ttcgaggctg ccggcgtaag catgctggaa gtatccggcg gaacccagta
8461 tgaaggcctg gaacatatca ttccttgcca gaataagagc aggggcgtca atgtatatga
8521 agcttctgag atcaagaaag tagtgggcat cccggtatac gcagtaggaa agatcaacga
8581 tatacgctat gcggcagaga tcgtagaacg cggcctggta gacggcgtgg ctatgggacg
8641 tccgcttctg gcagatccgg acctttgcaa gaaggcagtg gaaggccagt ttgacgagat
8701 cactccatgc gcaagctgcg gcggaagctg catcagccgt tctgaggcag cgcctgagtg
8761 ccattgccat attaatccaa ggcttggccg ggagtatgaa ttcccggatg tgcctgccga
8821 gaagtccaag aaggtactgg ttatcggcgc aggccctgga ggaatgatgg ctgccgtgac
8881 agctgcggaa cgcggccatg atgttacggt atgggaggct gacgacaaga tcggcggcca
8941 gctgaacctg gcagtagtgg ctcctggcaa gcaggagatg acccagtgga tggtacatct
9001 gaactatcgc gcgaagaaag caggcgtgaa gtttgaattc aataaagaag cgacggcaga
9061 agatgtcaag gcgctggcgc cggaagcagt gatcgttgct acaggcgcga agccgctggt
9121 tcctccgatt aaaggaacac aggattatcc ggtgcttact gcccatgatt tccttcgcgg
9181 caagttcgtg attccgaagg gacgcgtctg cgtgctggga ggaggcgcgg ttgcctgcga
9241 gactgccgag acagccctgg agaatgcacg tccgaattct tataccagag gatacgatgc
9301 aagcatcgga gatatcgatg tcacgcttgt ggagatgctt ccgcagctcc ttaccggcgt
9361 atgcgcgccg aaccgcgagc ctttgatccg caagttaaag agcaagggcg tacacatcaa
9421 cgtcaatacc aagatcatgg aagtaacaga ccatgaagta aaggttcaga gacaggatgg
9481 aacgcaggaa tggctggaag gatttgacta tgtcctcttt ggccttggtt ccagaaatta
9541 cgatccgctt tcagagaccc tcaaggaatt cgttccggaa gtacatgtca tcggcgatgc
9601 cgtaagggcg cgccaggcaa gctacgcaat gtgggaagga tttgagaagg catacagcct
9661 gtaaaagcgg tttgagtaaa aggaggctta agaaatggca gtgaaggcaa tctcaggctg
9721 cgacaaggat caggaactga tca [SEQ ID NO: 3]

In certain examples, the bacterium is transformed with a vector comprising a bile acid-inducible operon (bai operon). In certain examples, the bacterium stably expresses an enzyme having 7-dehydroxylation activity. In certain examples, the enzyme is a bile-acid 7-dehydroxylase. In certain examples, the enzyme is selected from the group consisting of a bile-acid 7-dehydroxylase, bile-acid 7-alpha-dehydroxylase, 7-alpha-dehydratase, bile acid CoA ligase, 3 alpha-HSDH, CoA transferase, 3-dehydro-4-7-alpha-oxidoreductase, 3-dehydro-4-7-beta-oxidoreductase, CA/CDCA transporter, 7-beta-dehydratase and AraC/XyIS. In certain examples, the enzyme comprises an amino acid sequence that is at least about 80% (e.g., at least about 85%, at least about 90%, or at least about 95%) homologous or identical to the amino acid of a bile-acid 7-dehydroxylase, bile-acid 7-alpha-dehydroxylase, 7-alpha-dehydratase, bile acid CoA ligase, 3 alpha-HSDH, CoA transferase, 3-dehydro-4-7-alpha-oxidoreductase, 3-dehydro-4-7-beta-oxidoreductase, CA/CDCA transporter, 7-beta-dehydratase__ or AraC/XyIS.

SEQ ID NO: 2 represents an exemplary sequence of 16S rRNA gene in *C. hiranonis.* SEQ ID NO: 4 represents an exemplary sequence of 16S rRNA gene in *C. scindens.*

1 acatgcaagt cgagcgattc tcttcggaga agagcggcgg acgggtgagt aacgcgtggg
61 taacctgccc tgtacacacg gataacatac cgaaaggtat gctaatacgg gataatatat
121 aagagtcgca tgacttttat atcaaagatt tttcggtaca ggatggaccc gcgtctgatt
181 agcttgttgg cggggtaacg gcccaccaag gcgacgatca gtagccgacc tgagagggtg
241 atcggccaca ttggaactga gacacggtcc aaactcctac gggaggcagc agtggggaat
301 attgcacaat gggcgcaagc ctgatgcagc aacgccgcgt gagcgatgaa ggccttcggg
361 tcgtaaagct ctgtcctcaa ggaagataat gacggtactt gaggaggaag ccccggctaa
421 ctacgtgcca gcagccgcgg taatacgtag ggggctagcg ttatccggat ttactgggcg
481 taaagggtgc gtaggcggtc tttcaagtca ggagttaaag gctacggctc aaccgtagta
541 agctcctgat actgtctgac ttgagtgcag gagaggaaag cggaattccc agtgtagcgg
601 tgaaatgcgt agatattggg aggaacacca gtagcgaagg cggctttctg gactgtaact
661 gacgctgagg cacgaaagcg tggggagcaa acaggattag ataccctggt agtccacgct

 721 gtaaacgatg agtactagtt gtcggaggtt accccttcgg tgccgcagct aacgcattaa
 781 gtactccgcc tggggagtac gcacgcaagt gtgaaactca aaggaattga cggggacccg
 841 cacaagtagc ggagcatgtg gtttaattcg aagcaacgcg aagaacctta cctaggcttg
 901 acatccttct gaccgaggac taatctcctc tttccctccg gggacagaag tgacaggtgg
 961 tgcatggttg tcgtcagctc gtgtcgtgag atgttgggtt aagtcccgca acgagcgcaa
1021 cccttgtctt tagttgccat cattaagttg ggcactctag agagactgcc agggataacc
1081 tggaggaagg tggggatgac gtcaaatcat catgcccctt atgcctaggg ctacacacgt
1141 gctacaatgg gtggtacaga gggcagccaa gccgtgaggt ggagcaaatc ccttaaagcc
1201 attctcagtt cggattgtag gctgaaactc gcctacatga agctggagtt actagtaatc
1261 gcagatcaga atgctgcggt gaatgcgttc ccgggtcttg tacacaccgc ccgtcacacc
1321 atgggagttg gagacacccg aagccgacta tctaaccttt tgggagaagt cgtccccctc
1381 gaatcaatac ccc    [SEQ ID NO: 2]

1 gagagtttga tcctggctca ggatgaacgc tggcggcgtg cctaacacat gcaagtcgaa
61 cgaagcgctt ccgctagatt ttcttcggag atgaaggcgg ctgcgactga gtggcggacg
121 ggtgagtaac gcgtgggcaa cctgccttgc actgggggat aacagccaga aatggctgct
181 aataccgcat aagaccgaag cgccgcatgg cgcagcggcc aaagccccgg cggtgcaaga
241 tgggcccgcg tctgattagg tagttggcgg ggtaacggcc caccaagccg acgatcagta
301 gccgacctga gagggtgacc ggccacattg ggactgagac acggcccaga ctcctacggg
361 aggcagcagt ggggaatatt gcacaatggg ggaaaccctg atgcagcgac gccgcgtgaa
421 ggatgaagta tttcggtatg taaacttcta tcagcaggga agaagatgac ggtacctgac
481 taagaagccc cggctaacta cgtgccagca gccgcggtaa tacgtagggg gcaagcgtta
541 tccggattta ctgggtgtaa agggagcgta gacggcgatg caagccagat gtgaaagccc
601 ggggctcaac cccgggactg catttggaac tgcgtggctg gagtgtcgga gaggcaggcg
661 gaattcctag tgtagcggtg aaatgcgtag atattaggag gaacaccagt ggcgaaggcg
721 gcctgctgga cgatgactga cgttgaggct cgaaagcgtg gggagcaaac aggattagat
781 accctggtag tccacgccgt aaacgatgac tactaggtgt cgggtggcaa ggccattcgg
841 tgccgcagca aacgcaataa gtagtccacc tggggagtac gttcgcaaga atgaaactca
901 aaggaattga cggggacccg cacaagcggt ggagcatgtg gtttaattcg aagcaacgcg
961 aagaacctta cctgatcttg acatcccgat gccaaagcgc gtaacgcgct ctttcttcgg
1021 aacatcggtg acaggtggtg catggttgtc gtcagctcgt gtcgtgaggt gttgggttaa
1081 gtcccgcaac gagcgcaacc cctatcttca gtagccagca tttcggatgg gcactctgga
1141 gagactgcca gggacaacct ggaggaaggt ggggatgacg tcaaatcatc atgcccctta
1201 tgaccagggc tacacacgtg ctacaatggc gtaaacaaag ggaggcgaac ccgcgagggt
1261 gggcaaatcc caaaaataac gtctcagttc ggattgtagt ctgcaactcg actacatgaa
1321 gctggaatcg ctagtaatcg cgaatcagaa tgtcgcggtg aatacgttcc cgggtcttgt
1381 acacaccgcc cgtcacacca tgggagtcag taacgcccga agccggtgac ccaacccgca
1441 agggagggag ccgtcgaagg tgggaccgat aactggggtg aagtcgtaac aaggtagccg
1501 tatcggaagg tgcggctgga tcacctcctt c [SEQ ID NO: 4]

In certain examples, the intestinal microorganism comprises *C. hiranonis.*

By "percentage of identity" between two nucleic acid or amino acid sequences in the sense of the present disclosure, it is intended to indicate a percentage of nucleotides or of identical amino acid residues between the two sequences to be compared, obtained after the best alignment (optimum alignment), this percentage being purely statistical and the differences between the two sequences being distributed randomly and over their entire length. The comparisons of sequences between two nucleic acid or amino acid sequences are traditionally carried out by comparing these sequences after having aligned them in an optimum manner, said comparison being able to be carried out by segment or by "comparison window". The optimum alignment of the sequences for the comparison can be carried out, in addition to manually, by means of the local homology algorithm of Smith and Waterman (1981) [Ad. App. Math. 2:482], by means of the local homology algorithm of Neddleman and Wunsch (1970) [J. Mol. Biol. 48: 443], by means of the similarity search method of Pearson and Lipman (1988) [Proc. Natl. Acad. Sci. USA 85:2444), by means of computer software using these algorithms (GAP, BESTFIT, FASTA and TFASTA in the Wisconsin Genetics Software Package, Genetics Computer Group, 575 Science Dr., Madison, WI, or else by BLAST N or BLAST P comparison software).

The percentage of identity between two nucleic acid or amino acid sequences is determined by comparing these two sequences aligned in an optimum manner and in which the nucleic acid or amino acid sequence to be compared can comprise additions or deletions with respect to the reference sequence for an optimum alignment between these two sequences. The percentage of identity is calculated by determining the number of identical positions for which the nucleotide or the amino acid residue is identical between the two sequences, by dividing this number of identical positions by the total number of positions in the comparison window and by multiplying the result obtained by 100 in order to obtain the percentage of identity between these two sequences.

For example, it is possible to use the BLAST program, "BLAST 2 sequences" (Tatusova et al., "Blast 2 sequences - a new tool for comparing protein and nucleotide sequences", FEMS Microbiol Lett. 174:247-250) available on the site www.ncbi.nlm.nih.gov, the parameters used being those given by default (in particular for the parameters "open gap penalty" : 5, and "extension gap penalty" : 2; the matrix chosen being, for example, the matrix "BLOSUM 62" proposed by the program), the percentage of identity between the two sequences to be compared being calculated directly by the program. It is also possible to use other programs such as "ALIGN" or "Megalign" (DNASTAR) software.

By amino acid sequence having at least about 80%, *e.g.,* at least about 85%, at least about 90%, at least about 95% and at least about 98% identity with a reference amino acid sequence, those having, with respect to the reference sequence, certain modifications, in particular a deletion, addition or substitution of at least one amino acid, a truncation or an elongation are preferred. In the case of a substitution of one or more consecutive or nonconsecutive amino acid(s), the substitutions are preferred in which the substituted amino acids are replaced by "equivalent" amino acids. The expression "equivalent amino acids" is aimed here at indicating any amino acid capable of being substituted with one of the amino acids of the base structure without, however, essentially modifying the biological activities of the corresponding antibodies and such as will be defined later, especially in the examples. These equivalent amino acids can be determined either by relying on their structural homology with the amino acids which they replace, or on results of comparative trials of biological activity between the different antibodies capable of being carried out.

By way of non-limiting example, Table 1 represents the possibilities of substitution capable of being carried out without resulting in a profound modification of the biological activity of the corresponding modified antibody, the reverse substitutions being naturally envisageable under the same conditions.

**Table 1.**

| **Original residue** | **Substitution(s)** |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (G) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

### Intestinal microorganism indicating intestinal health

In certain examples, the intestinal microorganism can be used to indicate intestinal health in a subject. In certain examples, the intestinal microorganism is associated with an intestinal disorder. In certain examples, the intestinal microorganism is associated with a heathy intestinal status. In certain examples, the intestinal microorganism less abundant in a healthy subject compared to a subject having an intestinal disorder.

### Health assessment tools

Also disclosed herein, although not claimed as part of the present invention, is a health assessment tool relating to the microorganisms disclosed herein. In certain examples, the health assessment tool is for monitoring intestinal health status or dysbiosis. In certain examples, the health assessment tool comprises one or more probe for detecting an amount of one or more microorganisms disclosed herein. In certain examples, the health assessment tool comprises a microarray of one or more probe for detecting an amount of one or more microorganism disclosed herein. In certain examples, the probe comprises a nucleic acid probe for detecting a signature gene of a microorganism disclosed herein. In certain examples, the probe detects a 16S rRNA sequence of a microorganism disclosed herein. In certain examples, the probe comprises an antibody. In certain examples, the antibody binds to a surface protein/ antigen of a microorganism disclosed herein.

In certain examples, the amount of the microorganism is measured from a fecal sample of the subject. In certain examples, the health assessment tool monitoring intestinal health status or dysbiosis by comparing the amount of the one or more microorganism with a reference amount of the one or more microorganism.

In certain examples, the health assessment tool comprises probes for detecting at least about 1, at least about 2, at least about 3, at least about 4, at least about 5, at least about 6, at least about 7, at least about 8, at least about 9, at least about 10, at least about 12, at least about 14, at least about 26 or more microorganisms disclosed herein. In certain examples, the health assessment tool comprises probes for detecting about 1, about 2, about 3, about 4, about 5, about 6, about 7, about 8, about 9, about 10, about 12, about 14, or about 26 microorganisms disclosed herein. In certain examples, the health assessment tool comprises probes for detecting between about 1 to about 500, between about 1 to about 100, between about 1 to about 26, between about 5 to about 100, between about 5 to about 26, between about 10 to about 26, between about 15 to about 50, or between about 50 to about 100 microorganisms disclosed herein.

### 3. PHARMACEUTICAL COMPOSITION

Also disclosed herein, but not part of the claimed invention, is a pharmaceutical composition for use as a medicament. In certain examples, the pharmaceutical composition comprises an effective amount of a bacterium capable of producing a first bile acid. In certain examples, the pharmaceutical composition further comprises an effective amount of a second bile acid. In certain embodiments, the bacterium is any bacterium disclosed in the above section. In certain embodiments, the first bile acid and/or the second bile acid is any bile acid disclosed in the above section or a pharmaceutically acceptable salt thereof. In certain examples, the first bile acid and the second bile acid are the same. In certain examples, the first bile acid and the second bile acid are different.

In certain examples, the bacterium comprised in the pharmaceutical composition is between about 1 thousand CFU and about 100 trillion CFU. In certain examples, the bacterium is between about 1 thousand CFU and about 1 trillion CFU, between about 1 million CFU and about 1 trillion CFU, between about 100 million CFU and about 100 billion CFU, between about 1 billion CFU and about 1 trillion CFU, between about 1 billion CFU and about 100 billion CFU, between about 100 million CFU and about 100 billion CFU, between about 1 billion CFU and about 50 billion CFU, between about 100 million CFU and about 50 billion CFU, or between about 1 billion CFU and about 10 billion CFU. In certain examples, the bacterium comprised in the pharmaceutical composition is at least about 1 thousand CFU, at least about 1 million CFU, at least about 10 million CFU, at least about 100 million CFU, at least about 1 billion CFU, at least about 10 billion CFU, at least about 100 billion CFU or more.

In certain examples, the second bile acid comprised in the pharmaceutical composition is between about 1 µg/unit dose and about 1 g/unit dose. In certain examples, the second bile acid comprised in the pharmaceutical composition is between about 10 µg/unit dose and about 1 g/unit dose, between about 10 µg/unit dose and about 500 mg/unit dose, between about 100 µg/unit dose and about 500 mg/unit dose, between about 1 mg/unit dose and about 500 mg/unit dose, between about 10 mg/unit dose and about 500 mg/unit dose, between about 100 mg/unit dose and about 500 mg/unit dose, between about 10 mg/unit dose and about 100 mg/unit dose, between about 50 mg/unit dose and about 300 mg/unit dose. In certain examples, the second bile acid comprised in the pharmaceutical composition is at least about 1 µg/unit dose, at least about 10 µg/unit dose, at least about 100 µg/unit dose, at least about 1 mg/unit dose, at least about 10 mg/unit dose, at least about 100 mg/unit dose, at least about 1 g/unit dose or more

Disclosed herein, but not part of the claimed invention, is provided a bile acid for the treatment of an intestinal disorder in a dog. In certain examples, the bile acid is selected from the group consisting of chenodeoxycholic acid, cholic acid, glycochenodeoxycholic acid, glycocholic acid, taurocholic acid, taurochenodeoxycholic acid, taurodeoxycholic acid, glycodeoxycholic acid, ursodeoxycholic acid, glycoursodeoxycholic acid, tauroursodeoxycholic acid, taurolithocholic acid, alpha-muricholic acid, deoxycholic acid, gamma-muricholic acid, glycolithocholic acid, taurolithocholic acid, lithocholic acid, omega-muricholic acid and any combination thereof. In certain examples, the bile acid is a secondary bile acid. In certain examples, the secondary bile acid is selected from the group consisting of taurodeoxycholic acid, glycodeoxycholic acid, ursodeoxycholic acid, glycoursodeoxycholic acid, tauroursodeoxycholic acid, taurolithocholic acid, alpha-muricholic acid, deoxycholic acid, gamma-muricholic acid, glycolithocholic acid, taurolithocholic acid, lithocholic acid, omega-muricholic acid and any combination thereof. In certain examples, the secondary bile acid is deoxycholic acid and/or lithocholic acid.

In certain examples, the pharmaceutical composition is for the treatment of an intestinal disorder in a subject in need thereof. In certain examples, the subject is a dog. In certain examples, the intestinal disorder is chronic enteropathy. In certain examples, the intestinal disorder is a chronic enteropathy selected from the group consisting of food responsive enteropathy, antibiotic responsive enterophaty, and idiophathic inflammatory bowel disease (IBD).

The exact dose and frequency of administration depends on the particular condition being treated, the age, weight and general physical condition of the particular patient as well as other medication the individual can be taking, as is well known to those skilled in the art. Generally, the daily dose of a pharmaceutical composition disclosed herein can be in the range of between about 0.01 mg to about 1000 mg/day. In certain examples, the pharmaceutical composition can be about 0.05 mg to about 1000 mg/day, about 0.1 mg to about 1000 mg/day, about 1 mg to about 500 mg/day, about 0.01 mg to about 500 mg/day, about 0.05 mg to about 200 mg/day, about 1 mg to about 500 mg/day, about 1 mg to about 200 mg/day, about 5 mg to about 500mg/day, about 50 mg to about 200 mg/day, about 100 mg to about 200 mg/day, about 100 mg to about 1000 mg/day, about 20 mg to about 50 mg/day, or about 20 mg to about 100 mg/day.

In certain examples, the pharmaceutical composition disclosed herein, but which is not part of the claimed invention, can be administered from about 10 times per day to about once per day, from about 5 times per day to about once per day, or from about thrice per day to about once per day. In certain examples, the pharmaceutical composition disclosed herein can be administered once per day. In certain examples, the pharmaceutical composition disclosed herein can be administered once per two days, once per three days, once per four days, once per five days, once per six days, once a week, once per two weeks, once per three weeks, or once per month.

The pharmaceutical composition disclosed herein, but which is not part of the claimed invention, can be administered in a variety of forms. In certain examples, the pharmaceutical composition disclosed herein can be administered orally, parenterally, rectally. In certain examples, orally administered pharmaceutical composition in solid dosage forms can be administered as capsules, dragees, granules, pills, powders, and tablets. In certain examples, the pharmaceutical composition can be administered in liquid form as elixirs, emulsions, microemulsions, solutions, suspensions, and syrups. In certain examples, parenterally administered pharmaceutical composition can be administered as aqueous or oleaginous solutions or aqueous or oleaginous suspensions, which suspensions comprise crystalline, amorphous, or otherwise insoluble forms of the pharmaceutical composition. In certain examples, rectally administered pharmaceutical composition can be administered as creams, gels, lotions, ointments, and pastes.

Depending upon the form of administration, the pharmaceutical composition disclosed herein, but which is not part of the claimed invention, can be formulated or administered with or without a pharmaceutically acceptable excipient. In certain examples, the excipients include encapsulating materials or formulation additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, solution aid, and any combination thereof. In certain examples, the pharmaceutical composition disclosed herein, but which is not part of the claimed invention, is administered without a solubilization aid.

In certain examples, the pharmaceutical composition can separately be provided or packaged as kits.

### 4. FOOD PRODUCTS

Also disclosed herein, but not part of the claimed invention, is a food product for improving intestinal health. In certain examples, the food product comprises an effective amount of a bacterium capable of producing a first bile acid. In certain examples, the food product further comprises an effective amount of a second bile acid. In certain examples, the first bile acid and the second bile acid are the same. In certain examples, the first bile acid and the second bile acid are different.

In certain examples, the food product is a dietary supplement.. In certain examples, the food product is a dog food product. In certain examples, the food product is a pet dietary supplement.

In certain examples, the bacterium comprised in the pharmaceutical composition is between about 10 thousand CFU and about 100 trillion CFU. In certain examples, the bacterium is between about 1 thousand CFU and about 1 trillion CFU, between about 1 million CFU and about 1 trillion CFU, between about 100 million CFU and about 100 billion CFU, between about 1 billion CFU and about 1 trillion CFU, between about 1 billion CFU and about 100 billion CFU, between about 100 million CFU and about 100 billion CFU, between about 1 billion CFU and about 50 billion CFU, between about 100 million CFU and about 50 billion CFU, or between about 1 billion CFU and about 10 billion CFU. In certain examples, the bacterium comprised in the pharmaceutical composition is at least about 1 thousand CFU, at least about 1 million CFU, at least about 10 million CFU, at least about 100 million CFU, at least about 1 billion CFU, at least about 10 billion CFU, at least about 100 billion CFU or more.

In certain examples, the second bile acid comprised in the pharmaceutical composition is between about 1 µg/daily serving dose and about 1 g/ daily serving dose. In certain examples, the second bile acid comprised in the pharmaceutical composition is between about 10 µg/daily serving dose and about 1 g/daily serving dose, between about 10 µg/daily serving dose and about 500 mg/daily serving dose, between about 100 µg/daily serving dose and about 500 mg/daily serving dose, between about 1 mg/daily serving dose and about 500 mg/daily serving dose, between about 10 mg/daily serving dose and about 500 mg/daily serving dose, between about 100 mg/daily serving dose and about 500 mg/daily serving dose, between about 10 mg/daily serving dose and about 100 mg/daily serving dose, between about 50 mg/daily serving dose and about 300 mg/daily serving dose. In certain examples, the second bile acid comprised in the pharmaceutical composition is at least about 1 µg/daily serving dose, at least about 10 µg/daily serving dose, at least about 100 µg/daily serving dose, at least about 1 mg/daily serving dose, at least about 10 mg/daily serving dose, at least about 100 mg/daily serving dose, at least about 1 g/daily serving dose or more.

In certain examples, a formulation of the presently disclosed subject matter, which is not claimed as part of the present invention, can further comprise an additional active agent. Non-limiting examples of additional active agents that can be present within a formulation of the presently disclosed subject matter include a nutritional agent (e.g., amino acids, peptides, proteins, fatty acids, carbohydrates, sugars, nucleic acids, nucleotides, vitamins, minerals, *etc*.), a prebiotic, a probiotic, an antioxidant, and/or an agent that improves animal health.

In certain examples, the food product comprises one or more probiotic. In certain examples, the probiotic is a human probiotic. In certain examples, the probiotic is an animal probiotic. In certain examples, the animal probiotic is a canine probiotic. In certain examples, the probiotic is *bifidobacterium, lactic acid bacterium* and/or *enterococcus.* In certain examples, the probiotic is selected from the group consisting of any organism from lactic acid bacteria and more specifically from the following bacterial genera; *Lactococcus spp., Pediococcus spp., Bifidobacterium spp. (e.g., B. longum B. bifidum, B. pseudolongum, B. animalis), Lactobacillus spp. (e.g. L.bulgaricus, L. acidophilus, L. brevis, L casei, L. rhamnosus, L. plantarum, L. reuteri, L. fermentum, Enterococcus spp. (e.g. E. faecium), Prevotella spp., Fusobacteria spp, Alloprevotella spp,* and any combination thereof. In certain examples, the probiotic is administered to a companion animal in an amount of from about 1 colony forming unit (CFU) to about 100 billion CFUs per day for the maintenance of GI microflora. In certain examples, the probiotic is administered to a companion animal in an amount of from about 1 colony forming unit (CFU) to about 20 billion CFUs per day for the maintenance of GI microflora. In certain examples, the probiotic is administered to a companion animal in an amount of from about 1 billion CFUs to about 20 billion CFUs per day for the maintenance of GI microflora. In certain examples, the probiotic is administered to a companion animal in amounts of from about 0.01 billion to about 100 billion live bacteria per day. In certain examples, the probiotic is administered to a companion animal in amounts of from about 0.1 billion to about 10 billion live bacteria per day.

In certain examples, an additional prebiotic can be included, such as fructooligosaccharides (FOS), xylooligosaccharides (XOS), galactooligosaccharides (GOS), glucans, galactans, arabinogalactan, inulin and/or mannooligosaccharides. In certain examples, the additional prebiotic is administered in amounts sufficient to positively stimulate the GI microflora and/or cause one or more probiotics to proliferate.

In certain examples, the companion animal food product can further contain additives known in the art. In certain examples, such additives are present in amounts that do not impair the purpose and effect provided by the presently disclosed subject matter. Examples of contemplated additives include, but are not limited to, substances that are functionally beneficial to improving health, substances with a stabilizing effect, organoleptic substances, processing aids, substances that enhance palatability, coloring substances, and substances that provide nutritional benefits. In certain examples, the stabilizing substances include, but are not limited to, substances that tend to increase the shelf life of the product. In certain examples, such substances include, but are not limited to, preservatives, synergists and sequestrants, packaging gases, stabilizers, emulsifiers, thickeners, gelling agents, and humectants. In certain examples, the emulsifiers and/or thickening agents include, for example, gelatin, cellulose ethers, starch, starch esters, starch ethers, and modified starches.

In certain examples, the additives for coloring, palatability, and nutritional purposes include, for example, colorants; iron oxide, sodium chloride, potassium citrate, potassium chloride, and other edible salts; vitamins; minerals; and flavoring. The amount of such additives in a product typically is up to about 5% (dry basis of the product).

In certain examples, the companion animal food product is a dietary supplement. In certain examples, the dietary supplements include, for example, a feed used with another feed to improve the nutritive balance or performance of the total. In certain examples, the supplements include compositions that are fed undiluted as a supplement to other feeds, offered free choice with other parts of an animal's ration that are separately available, or diluted and mixed with an animal's regular feed to produce a complete feed. The AAFCO, for example, provides a discussion relating to supplements in the American Feed Control Officials, Incorp. Official Publication, p. 220 (2003). Supplements can be in various forms including, for example, powders, liquids, syrups, pills, tablets, encapsulated compositions, *etc.*

In certain examples, the companion animal food product is a treat. In certain examples, treats include, for example, compositions that are given to an animal to entice the animal to eat during a non-meal time. In certain examples, the companion animal food product is a treat for canines include, for example, dog bones. Treats can be nutritional, wherein the product comprises one or more nutrients, and can, for example, have a composition as described above for food. Non-nutritional treats encompass any other treats that are non-toxic.

In certain examples, a bacterium and/or a bile acid of the presently disclosed subject matter, but which is not part of the claimed invention, can be incorporated into the composition during the processing of the formulation, such as during and/or after mixing of other components of the product. Distribution of these components into the product can be accomplished by conventional means.

In certain examples, companion animal food products of the presently disclosed subject matter, but which are not part of the claimed invention, can be prepared in a canned or wet form using conventional companion animal food processes. In certain examples, ground animal (e.g., mammal, poultry, and/or fish) proteinaceous tissues are mixed with the other ingredients, such as milk fish oils, cereal grains, other nutritionally balancing ingredients, special purpose additives (e.g., vitamin and mineral mixtures, inorganic salts, cellulose and beet pulp, bulking agents, and the like); and water that sufficient for processing is also added. These ingredients are mixed in a vessel suitable for heating while blending the components. Heating of the mixture can be effected using any suitable manner, such as, for example, by direct steam injection or by using a vessel fitted with a heat exchanger. Following the addition of the last ingredient, the mixture is heated to a temperature range of from about 50° F to about 212° F. Temperatures outside this range are acceptable but can be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. This is usually accomplished by heating to temperatures of greater than about 230° F for an appropriate time, which is dependent on, for example, the temperature used and the composition.

In certain examples, companion animal food products of the presently disclosed subject matter, but which are not part of the claimed invention, can be prepared in a dry form using conventional processes. In certain examples, dry ingredients, including, for example, animal protein sources, plant protein sources, grains, *etc.,* are ground and mixed together. In certain examples, moist or liquid ingredients, including fats, oils, animal protein sources, water, *etc.,* are then added to and mixed with the dry mix. In certain examples, the mixture is then processed into kibbles or similar dry pieces. In certain examples, the companion animal food product is kibble. In certain examples, kibble is formed using an extrusion process in which the mixture of dry and wet ingredients is subjected to mechanical work at a high pressure and temperature and forced through small openings and cut off into kibble by a rotating knife. In certain examples, the wet kibble is then dried and optionally coated with one or more topical coatings which can include, for example, flavors, fats, oils, powders, and the like. In certain examples, kibble can also be made from the dough using a baking process, rather than extrusion, wherein the dough is placed into a mold before dry-heat processing.

In certain examples, treats of the presently disclosed subject matter, but which are not part of the claimed invention, can be prepared by, for example, an extrusion or baking process similar to those described above for dry food.

Also disclosed herein, but not part of the claimed invention, is a diet for increase a population of a bacterium capable of producing a bile acid in a companion animal. In certain examples, the diet comprises protein, fat, crude fiber, total dietary fiber, carbohydrate, calcium, phosphorus, sodium, chloride, potassium, magnesium, iron, copper, manganese, zinc, iodine, selenium, vitamin A, vitamin D3, vitamin E, vitamin C, thiamine (vitamin B1), riboflavin (vitamin B2), pantothenic acid, niacin, pyridoxine (vitamin B6), folic acid, biotin, cobalannin (vitamin B12), choline, arginine, lysine, methionine, cystine, taurine, linoleic acid, arachidonic acid, Omega-6 fatty acids, Omega-3 fatty acids, EPA, and/or DHA.

In certain examples, the subject is a dog. In certain examples, the diet is a Royal Canin Veterinary Diet. In certain examples, the diet is selected from the group consisting of Ultamino, Hydrolyzed Protein Adult HP Dry, Hydrolyzed Protein Wet, Hydrolyzed Protein Adult PS Dry, Hydrolyzed Protein Moderate Calorie Dry, Hydrolyzed Protein Small Dog Dry, Hydrolyzed protein Treats, and any combination thereof.

In certain examples, the bacterium comprises a bile acid-inducible operon (bai operon). In certain examples, the bacterium is C. *hiranonis.*

Also disclosed herein, but not part of the claimed invention, is a Royal Canin Veterinary Diet for the treatment of an intestinal disorder in a dog, wherein the dog comprises an intestinal microorganism, and wherein the amount of the intestinal microorganism is lower than a reference amount of the intestinal microorganism, wherein the intestinal microorganism is FJ957494.1.1454.

### 5. TREATMENT METHODS

The present invention is defined by the appended claims.
The presently disclosed subject matter relates to a method for improving intestinal health and/or treating an intestinal disorder of a subject in need thereof.

The present invention provides for a method for determining responsiveness of a companion animal having an intestinal disorder to a diet, wherein the intestinal disorder is chronic enteropathy, wherein the diet is a therapeutic hydrolyzed protein diet and wherein the companion animal is a dog. The method comprises:
a) measuring an amount of a intestinal microorganism in the companion animal;
b) comparing the amount of the intestinal microorganism with a reference amount of the intestinal microorganism, wherein the reference amounts of the intestinal microorganisms are determined based on the amounts of the intestinal microorganisms in a plurality of healthy companion animals; and
c) determining that the companion animal is responsive to the diet, when the amount of the intestinal microorganism is lower than the reference amount of the intestinal microorganism, or determining that the companion animal is non-responsive to the diet, when the amount of the intestinal microorganism is higher than the reference amount of the intestinal microorganism.

In the present invention, the intestinal microorganism comprises a bacterium comprising a 16S rRNA comprising a nucleotide sequence that is identical to the 16S rRNA nucleotide sequence of FJ957494.1.1454 (SEQ ID NO: 13).

In certain embodiments of the disclosure, the method further comprises administering the diet to the companion animal when companion animal is determined as responsive to the diet. In certain embodiments, the method further comprises administering the diet, a steroid and optionally an antibiotic to the companion animal when companion animal is determined as non-responsive to the diet.

In certain embodiments, the determination in step c) occurs before administering the diet or the diet, the steroid and optionally the antibiotic to the companion animal.

In certain embodiments, the reference amount of an intestinal microorganism derived from a mean amount of the intestinal microorganism in a plurality of healthy companion animals. In certain embodiments, the amount of the intestinal bacterium is measured from a fecal sample of the subject.

In certain emobidments of the disclosure, the method comprises administering to the subject an effective amount of a presently disclosed pharmaceutical composition, an effective amount of a presently disclosed food product, or any combination thereof. In certain embodiments, the method further comprises monitoring an intestinal microorganism in the subject. In certain embodiments, the intestinal microorganism is sampled from a fecal sample of the subject.

In certain embodiments, an amount of the intestinal microorganism is increased after administration of the pharmaceutical composition and/or the food product. In certain embodiments, the amount of the intestinal microorganism is increased within about 14 days after administration of the pharmaceutical composition and/or the food product. In certain embodiments, an amount of the intestinal bacterium is increased within about 21 days, within about 14 days, within about 12 days, within about 10 days, within about 7 days, within about 6 days, within about 5 days, within about 4 days, within about 3 days, within about 2 days, or within about 1 day after administration of the pharmaceutical composition. In certain embodiments, an amount of the intestinal bacterium is increased within about 1 day to about 21 days, within about 1 days to about 14 days, within about 3 days to about 14 days, within about 5 days to about 14 days, within about 7 days to about 14 days, within about 10 days to about 14 days, or within about 7 days to about 21 days after administration of the pharmaceutical composition.

In the present invention, the subject is a companion animal, which is a dog (*e.g.,* a domestic dog). In the present invention, the companion animal has an intestinal disorder. In certain non-limiting embodiments, the companion animal is under a treatment for a digestive disorder. In certain non-limiting embodiments, the treatment is a dietary therapy. In the present invention, the companion animal is a dog. In the present invention, the intestinal disorder is chronic enteropathy. In certain embodiments, the intestinal disorder is a chronic enteropathy selected from the group consisting of food responsive enteropathy, antibiotic responsive enterophaty, and idiophathic inflammatory bowel disease (IBD).

In certain embodiments, the pharmaceutical composition and/or the food product can be administered to a subject from 20 times per day to once per day, from 10 times per day to once per day, or from 5 times per day to once per day. In certain embodiments, the pharmaceutical composition and/or the food product can be administered to a subject once per day, twice per day, thrice per day, 4 times per day, 5 times per day, 6 times per day, 7 times per day, 8 times per day, 9 times per day, 10 or more times per day. In certain embodiments, the pharmaceutical composition and/or the food product can be administered to a subject once per two days, once per three days, once per four days, once per five days, once per six days, once a week, once per two weeks, once per three weeks, or once per month. In certain embodiments, the food product can be administered to an animal in a constant manner, e.g., where the animal grazes on a constantly available supply of the subject food product.

In certain embodiments, the dosage of the pharmaceutical composition is between about 1 mg/kg body weight per day and about 5000 mg/kg body weight per day. In certain embodiments, the dosage of the pharmaceutical composition is between about 5 mg/kg body weight per day and about 1000 mg/kg body weight per day, between about 10 mg/kg body weight per day and about 500 mg/kg body weight per day, between about 10 mg/kg body weight per day and about 250 mg/kg body weight per day, between about 10 mg/kg body weight per day and about 200 mg/kg body weight per day, between about 20 mg/kg body weight per day and about 100 mg/kg body weight per day, between about 20 mg/kg body weight per day and about 50 mg/kg body weight per day or any intermediate range thereof. In certain embodiments, the dosage of the pharmaceutical composition is at least about 1 mg/kg body weight per day, at least about 5 mg/kg body weight per day, at least about 10 mg/kg body weight per day, at least about 20 mg/kg body weight per day, at least about 50 mg/kg body weight per day, at least about 100 mg/kg body weight per day, at least about 200 mg/kg body weight per day or more. In certain embodiments, the dosage of the pharmaceutical composition is no more than about 5 mg/kg body weight per day, no more than about 10 mg/kg body weight per day, no more than about 20 mg/kg body weight per day, no more than about 50 mg/kg body weight per day, no more than about 100 mg/kg body weight per day, no more than about 200 mg/kg body weight per day, no more than about 500 mg/kg body weight per day or more.

In certain embodiments, the amount of the pharmaceutical composition and/or the food product decreases over the course of feeding a companion animal. In certain embodiments, the concentration of the pharmaceutical composition and/or the food product increases over the course of feeding a companion animal. In certain embodiments, the concentration of the pharmaceutical composition and/or the food product is modified based on the age of the companion animal.

### 6. KITS

Also disclosed herein but not part of the claimed invention are kits for treating and/or preventing an intestinal disorder in a subject. In certain examples, the kit comprises an effective amount of the presently disclosed pharmaceutical composition, dietary supplement, functional food, food product, diet or any combination thereof. In certain examples, the kit comprises a sterile container; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments.

If desired, the pharmaceutical composition, dietary supplement, functional food, food product, and/or diet are provided together with instructions for administering the same to a subject having or at risk of developing an intestinal disorder. The instructions generally include information about the use of the pharmaceutical composition, dietary supplement, functional food, food product, diet for the treatment and/or prevention of an intestinal disorder. In certain examples, the instructions include at least one of the following: description of the therapeutic agent; dosage schedule and administration for treatment or prevention of an intestinal disorder or symptoms thereof; precautions; warnings; indications; counter-indications; over-dosage information; adverse reactions; animal pharmacology; clinical studies; and/or references. The instructions can be printed directly on the container (when present), or as a label applied to the container, or as a separate sheet, pamphlet, card, or folder supplied in or with the container.

Advantageously, the kit can be packaged in per use groupings such that, for example, a daily prescription of each component can be identified in order to enhance patient compliance. Sets of the pharmaceutical composition can be identified in a variety of ways. For example, in certain examples, a set of the pharmaceutical composition, dietary supplement, functional food, food product, diet can be identified on the package containing the same. In certain examples, external instructions can be provided with a set or sets of the pharmaceutical composition, dietary supplement, functional food, food product, diet that, for example, identify a grouping and instruct a patient/animal owner appropriate times to take the pharmaceutical composition, dietary supplement, functional food, food product, diet of the kit.

### EXAMPLES

The presently disclosed subject matter will be better understood by reference to the following Example, which is provided as exemplary of the present disclosure, and not by way of limitation.

### Example 1

### Introduction

Although a wide range of environmental factors have been shown to influence the microbiome, diet is regarded as one of the most potent modulators of the composition and function of the gut-resident microbial community in healthy humans and other mammals ^{7,8} and can act as both a risk factor and a treatment modality for IBD ^{9,10}. Epidemiologic data and studies in mice have shown that diets high in fat and/or low in fiber, as well as dietary additives such as emulsifiers, are either risk factors for IBD, or in some cases can directly compromise intestinal barrier function leading to disease ¹¹⁻¹³. Diet can be also leveraged to treat IBD, with perhaps the clearest example of this being the use of exclusive enteral nutrition (EEN) as first-line therapy for pediatric Crohn's disease ¹⁴. High remission rates (≥ 60%) are observed following EEN and, compared to corticosteroids, EEN achieves better patient growth along with a reduction in biomarkers of disease, such as fecal calprotectin and C-reactive protein ¹⁵⁻¹⁸. Interestingly, EEN has a marked effect on the microbiome, but the precise nature of this effect has been complicated to discern, with some studies reporting reduced microbiome diversity following EEN therapy ¹⁹⁻²¹, while others point to relatively unchanged ^{22,23} or increased diversity ²⁴.

The mechanisms by which diet impacts the gut microbiome to ameliorate IBD symptoms are unclear and are complicated to dissect from human subject research were diet is challenging to control, necessitating either retrospective studies in conjunction with extensive food intake surveys ²⁵, controlled feeding studies ²⁶, or focusing on populations with different subsistence practices ²⁷⁻²⁹. In contrast, mouse models of colitis have yielded important insights into the pathophysiology of intestinal inflammation, but these often involve chemical or genetic perturbation, rather than spontaneous disease development. Moreover, the ubiquitous use of autoclaved food and acidified water for mouse husbandry, together with the tendency for cage effects to dominate in mouse microbiome studies, raises concerns about clinical relevance of diet-microbiome studies in these models of colitis. As companion animals, dogs share the same environment as humans, and spontaneously develop a chronic enteritis that clinically resembles human IBD, including similar gastrointestinal pathology, responsiveness to similar treatments ^{30,31}, involvement of some of the same susceptibility loci ³⁰⁻³², and shared disease-associated microbial taxa ³³⁻³⁵. Intriguingly, after treatment with dietary therapy, over 50% of dogs with chronic enteritis enter a long-lasting state of remission ³⁶, making the use of prescription diets the first-line treatment for IBD in companion animal medicine. A recent metagenomic study produced a catalog of over one million taxonomically and functionally annotated microbial genes from the canine gut and showed that - compared to other mammals, such as the mouse and pig - the microbial environment in dogs most closely resembles that of humans ³⁷. Furthermore, the canine microbiome was markedly altered by diet change in a manner that resembles what has been reported in humans ³⁷. Together, these data argue that dogs are an ideal animal model in which to study diet-microbiome interactions in the context of intestinal disease.

Despite the fact that the gut microbiome has been implicated in IBD pathogenesis, and that diet profoundly alters the microbiome and can be used to manage symptoms of IBD, there is limited insight into the mechanisms by which this occurs. In this study, treatment-naive dogs were examined with chronic enteritis and changes in their fecal microbial community structure and metabolites in response to treatment were monitored. By comparing changes over time in diet-responsive dogs, versus animals that failed diet therapy and required subsequent combination therapy, it was shown that diet induces rapid remission by shaping the community structure and re-programming the metabolic function of the microbiome. Notably, it was demonstrated that secondary bile acids, likely produced by clostridia, are involved in the diet induced alterations of microbiota community by inhibiting the growth of potential pathogens. These findings provide a general mechanism by which diet can modulate microbial communities to reduce GI disease.

### Methods

### Diagnosis and treatment of Canine Chronic Enteritis (CCE)

Client-owned animals presenting with clinical signs of CCE were screened at the Ryan Veterinary Hospital of the University of Pennsylvania. All animal work was carried out in accordance within the guidelines of the University of Pennsylvania IACUC (Protocol 805283), and signed owner consent was obtained before enrollment. Dogs were screened if they had any one of the following clinical signs for ≥ 3 weeks' duration: vomiting, diarrhea or weight loss despite adequate caloric intake. Dogs were excluded from screening if they had been treated with a hydrolyzed protein diet, antibiotics, corticosteroids or probiotics within the previous two weeks. At the time of screening, the following were performed on each animal: complete physical examination, routine fecal screening (including zinc sulfate flotation for parasite identification, gram stain and culture for *Salmonella* spp. and *Campylobacter* spp.), complete blood count, serum biochemical profile, serum measurement of canine trypsin-like immunoreactivity, cobalamin and folate, urinalysis, abdominal ultrasound examination, and disease severity scoring using the Canine Chronic Enteropathy Clinical Activity Index (CCECAI) ³⁶. If these initial screening tests failed to identify a cause for the clinical signs, upper and/or lower gastrointestinal endoscopy with mucosal biopsies was performed. Biopsies were fixed in formalin, embedded in paraffin, and sections were stained with hematoxylin and eosin, and slides were examined by a board-certified veterinary pathologist. Dogs were enrolled if histopathology revealed intestinal inflammation with no identifiable underlying cause (such as infectious agents). Dogs were excluded if another histopathologic diagnosis was identified.

Three 14-day treatment tiers were included in the trial **(****Fig 1A** **and Fig S1),** and dogs were evaluated for a therapeutic response at the conclusion of each tier using CCECAI. Remission was determined using an abbreviated CCECAI that included scores to the first five indices (attitude/activity, appetite, vomiting, stool consistency, and stool frequency), and was defined as an abbreviated CCECAI score ≤ 2, with no score > 1 for any of the five indices. Animals were first administered a therapeutic hydrolyzed protein diet (Royal Canin HP). Dogs that entered remission following this treatment were designated as diet-response (DR) and were maintained on therapeutic diet for the reminder of the trial. Animals that did not respond to therapeutic diet (NDR) subsequently began a two-week course of metronidazole (10 mg/kg PO q 12 hours) while being maintained on the therapeutic diet. Dogs that entered remission following antibiotic treatment were maintained on the combination of antibiotics and therapeutic diet for the reminder of the trial. Animals that still failed to show a favorable response remained on diet and metronidazole but received prednisone (1 mg/kg PO q 12 hours) (Tier 3) for the final 14 days of the trial. Dogs that presented with hypoalbuminemia (protein-losing enteropathy) at the initial screening were presumed to have more severe disease and poorer prognoses and thus were immediately administered all three interventions and were not included in the analyses. All dogs in which serum cobalamin was low at screening were supplemented with cyanocobalamin (50 mcg/kg SQ q 7 days) for the duration of the study. At the conclusion of the study, all animals returned to the clinic for the primary endpoint, which included a full re-evaluation of dogs including complete physical examination, complete blood count, serum chemistry, serum measurement of cobalamin and folate (if low at screening visit), urinalysis, CCECAI scoring and final fecal collections.

### 16S rRNA gene sequencing and data analysis

Genomic DNA was extracted from stool using the PowerSoil DNA Isolation Kit (MO BIO Laboratories, Carlsbad, CA) following the manufacturer's recommendations. A mock community pool containing purified genomic DNA from 12 known bacterial isolates was amplified and sequenced as a quality control. Additional controls included extraction of blank-processed samples (in which the DNA extraction process was followed without addition of input material), and water only, to determine background microbial signal. A dual-index amplicon sequencing method was employed for PCR amplification of the V4 region of the 16S *rRNA* gene ⁶¹. Pico-green based Amplicons were sequenced on a MiSeq platform (Illumina, San Diego, CA) using 250 base pair paired-end chemistry. Reads were filtered to remove sequences with average Phred quality score ≤ 20 using Quantitative Insights into Microbial Ecology (QIIME) ⁶² with filtering options (-q 20 - p 0.75 -r 3). Homopolymers > 10 bp in length and sequences < 248 bp and > 255 bp were removed using Mothur ⁶³. Chimeric sequences were identified and removed by usearch61 ⁶⁴ against the representative 16S sequences of SILVA128 (97_otus_16S.fasta) ^{65,66}. Quality-controlled sequences were then clustered against the SILVA128 database (SILVA_128_QIIME_release) using the open-reference OTU picking as implemented in QIIME with default parameters. The OTU table was rarefied to 10600 sequences per sample. In order to get a taxonomic assignment at species level for the OTUs from *Clostridium sensu stricto* 1, the corresponding representative sequences in SILVA database were used to search against NCBI 'nr' database. Species were temporarily assigned by the best hits (P< 1e-5) and further confirmation were done by comparing the relative abundances of these species determined by metagenomic shotgun sequencing method and by 16S sequencing method. The OTU 'New.ReferenceOTU52' represents *C. perfringens,* which is the most dominant OTU in some dogs, and the OTU 'FJ957494.1.1454' is corresponding to *C. hiranonis.*

Analysis of OTU tables was carried out using the R statistical environment ⁶⁷, the bioconductor suite of software ⁶⁸, and the Phyloseq2 package ⁶⁹. Singletons and OTUs with ambiguous annotations were removed from the OTU table. Alpha diversity (Shannon diversity index and Faith's Phylogenetic Diversity) and Beta diversity (weighted and unweighted UniFrac) were calculated using Phyloseq2. Pielou 's evenness index was calculated according to the literature ⁷⁰. Functional potential of microbial communities (KEGG pathways and KEGG Orthologs) was predicted by Tax4Fun ⁷¹ with default parameters against SILVA123 database. Wilcoxon sum rank test was used for comparisons of KEGG pathways at different timepoints (FDR < 0.05). Principal component analysis for KEGG pathways and orthologs was performed by the R package factoextra. For differential abundance analysis and association analysis, filtering was carried out to remove taxa with a max abundance < 0.1% across all samples and present in < 10% of all samples. The resulting 381 species accounted for an average 96.23% of the total microbial composition. DESeq2 ⁷² implemented in Phyloseq2 (test="Wald", fitType="parametric") was used for differential abundance analysis on different taxonomy levels (Fold change >2 and P value <0.05) using un-rarefied reads. The Spearman correlation was computed between the abundance of each microbial composition (Log-transformed) and the values of different factors (i.e., CCECAI for each dog, time points, concentration of each metabolite). To avoid taking log of the zero value, 1 read was added to the abundance for each composition before calculating the Spearman correlation. All p values in the above analysis were adjusted by the FDR (Benjamini-Hochberg) method for multiple comparisons except where noted.

### Metagenomic sequencing and data analysis

Sequencing libraries were prepared using Illumina Nextera XT with 1ng of canine stool collected at days 0, 14 and 42 from 19 out of the 20 diet-responsive dogs in the study. Sizing and quantification of libraries was carried out using a Tapestation 4200 (Agilent) and Qubit 3 (Thermo Fisher), respectively. Equimolar amounts of each library were pooled and sequenced on an Illumina NextSeq 500 instrument to produce 150 bp, paired-end sequences. Sequencing adapters and low quality reads were trimmed and filtered by Trimmomatic (v0.36) (leading:3 trailing:3 slidingwindow:4:15 minlen:36). High quality reads were mapped to the canine reference genome (CanFam3.1), using Bowtie2 v2.3.4.1 (--very-sensitive), and aligned reads were removed using SamTools ⁷³. After host filtering, each sample was sequenced to a depth of > 10 million paired-end reads (median depth = 35.8 million). Taxonomic annotation for each sample was generated using Metaphlan2 ⁴⁶. The identified *Clostridium* spp. and *Eubacterium* spp. were further searched for the existence of genes involved in secondary bile acid production (*bai* operon) using tBlastn against the reference genomes of these species in GeneBank with the protein sequence of genes in 7α-dehydroxylation pathway (*baiG, baiB, baiA, baiF, baiCD and baiE*) (p-value ≤ 1e-5).

Metagenomic data from pediatric Crohn's disease patients before and after exclusive enteral nutrition (EEN) have been described previously ²³ and were downloaded from European Nucleotide Archive (ENA) (SRP057027). The same filtering steps and settings for the metagenomic data analysis above in this study were used for these datasets. After filtering out human reads, taxonomic annotation for each sample using Metaphlan2 showed the presence of *Clostridium.* Among them, *Clostridium scindens* has been well known for the secondary BA producing ability. Paired reads with PCR duplicates removed by samtools ⁷³ were aligned to the*C*. *scindens* reference genome (ASM15450v1, strain ATCC 35704) as well as strain VE202-05 (ASM47184v1) using bwa-mem (v0.7.17-r1188) ⁷⁴ with default settings to estimate the abundances of bacteria among different samples (proportion of mapped reads in total reads). Wilcoxon sum rank test was used to test for significant differences in read mapping, and Spearman correlation was used to compare number of reads mapped with log-transformed fecal calprotectin (FCP) levels ²³.

### Anaerobic culture and identification of bacterial isolates by whole genome sequencing

Rectal swabs freshly collected from dogs with active disease (day 0) and/or in remission at the end of the study (day 42) were transferred to an anaerobic chamber (97.5% nitrogen, 2.5% hydrogen; Coy Labs, Grass Lake, MI) within one hour of collection. The tip of the swabs was homogenized in 1 mL of pre-reduced PBS with 1% cysteine (PBSc). Serial dilutions made in PBSc (down to 10 ⁻⁵) were plated on brain-heart infusion (BHI), yeast casitone fatty acid with carbohydrate (YCFAC) ⁷⁵, gut microbiota medium (GMM) ⁷⁶, and De Man, Rogosa and Sharpe (MRS) ⁷⁷ agars (Anaerobe Systems, Morgan Hill, CA). After incubation at 37 ° C for 1-3 days, single colonies were picked from plates and grown overnight in BHI, YCFAC, GMM, or MRS broth (Anaerobe Systems, Morgan Hill, CA). Overnight cultures were saved as glycerol stocks (25% glycerol) and frozen neat for DNA extraction. DNA was purified from bacterial isolates using the High Pure PCR template kit (Roche) and used for PCR with primers specific for the bacterial 16S rRNA gene, including 27F (5'-AGAGTTTGATCMTGGCTCAG-3'), 515F (5'-GTGCCAGCMGCCGCGGTAA-3'), and 1492R (5'-CGGTTACCTTGTTACGACTT-3'). PCR products were purified using QiaQuick PCR Purification kit (Qiagen), Sanger sequenced, and sequences were assembled using Geneious software v11.1.5 (Biomatters Inc.). The longest high quality stretch of assembled sequence (at least 800 bp) was used for BLAST to find closest the match in Genbank. In addition, for selected *C*. *hiranonis, C. perfringens* and *E. coli* isolates, 1 ng of DNA was used to construct sequencing libraries using Illumina Nextera XT. Libraries were sized and quantified as described above for metagenomic sequencing. For each sample, at least ~ 10 million, 150bp single-end reads were generated using an Illumina NextSeq 500 instrument. Quality control steps were the same as the metagenomic analysis above. High quality reads were mapped to the genome of *C. hiranonis* (ASM15605v1) using Stampy ⁷⁸ (--substitutionrate = 0.1), which allows mapping of reads that are highly divergent from the reference genome. PCR duplicates were removed by Samtools. Coverage of genomic regions representing the *bai* operon were calculated for each isolate to show the existence of genes in 7α-dehydroxylation pathway.

### Metabolomics and In vitro bacterial growth inhibition assays

Bile acids were quantified in stool using a Waters Acquity uPLC System with a QDa single quadrupole mass detector and an autosampler (192 sample capacity) as described previously ⁷⁹. Briefly, fecal samples were suspended in methanol (5 µL/mg stool), vortexed for 1 minute, and centrifuged at 13,000g for 5 minutes. The supernatant was transferred to a new vial and analyzed on an Acquity uPLC with a Cortecs UPLC C-18+ 1.6 mm 2.1 x 50 mm column. All chemicals and reagents were mass spectrometry grade. Canine isolates of *C. perfringens* (n=3) and *E. coli* were revived from glycerol stocks in Modified Reinforced Clostridial Broth (MRCB, Fisher Scientific) or Luria broth (LB, Fisher Scientific), respectively and grown overnight in the anaerobic chamber at 37 ° C. Lithocholic and deoxycholic acids (Sigma) were dissolved in 100% ethanol (30 mg/mL). Growth inhibition by deoxycholic acid was determined by microbroth dilution and assessed by OD ₆₃₀ after overnight growth. Due to low solubility (<1 mg/L), inhibition by lithocholic acid was assessed by counting colonies on agar plates with LCA (0, 0.1, 0.25, 0.5, 0.75, or 1 mg/mL and LB plates for *E. coli,* and 0, 0.01, 0.025, 0.05, 0.075, or 0.01 mg/mL and Columbia blood agar supplemented with 5% defibrinated sheep's blood for *C. perfringens* that were incubated anaerobically, at 37 ° C for 24 (*E. coli*) or 48 (*C. perfringens*) hours.

### Mouse experiments

Female C57BL/6 (7 weeks old) (Jackson Laboratory) were orally pre-colonized with a kanamycin-resistant *E. coli* strain (Nissle 1917) (1×10 ⁹ CFU/mouse) 4 days prior to the to the start of dextran sulfate sodium (DSS) treatment. Animals were randomly assigned to groups (cages) at baseline and drinking water was replaced with either filter-sterilized water (mock-treatment), or a filter-sterilized solution of 2.5% (w/v) DSS (relative molecular mass ~40,000; Sigma-Aldrich) in water. The mice treated with mock or DSS were orally gavaged *C. hiranonis* (1×10 ⁸ CFU/mouse, in anaerobic PBS) or PBS (control) from days 0 to 4. *C. hiranonis* was grown overnight in MRCB, anaerobically, at 37 ° C. Culture density was assessed via optical density (630 nm) and the required volume of culture was spun at 10,000g for 15 min. Bacterial pellets were resuspended in PBS to obtain a dose of 1x10 ⁸ CFU/mouse. All procedures were performed in accordance with the guidelines of the University of Pennsylvania Institutional Animal Care and Use Committee. The mice were then euthanized at day eight and colon contents and tissues were collected. Colon contents were weighted and cultured on LB agar plates with kanamycin (100 µ g /mL) for 16 hours. Stool samples from baseline and colon contents from day eight were collected and stored at -80°C for the detection of bile acid levels. Colons were fixed in formalin and sections stained with haematoxylin and eosin (H&E). Pathology was blindly evaluated by an board-certified veterinary pathologist (C.B.) according to standard criteria for DSS colitis.

### Data availability

Raw 16S rRNA gene sequences for canine stool samples have been deposited in the Sequence Read Archive (SRA ₈₀ ; accession number pending). Processed OTU tables and metadata can be accessed through MicrobiomeDB ⁵⁶. Metagenomic and whole genome sequence data are also available on SRA (accession numbers pending).

### Results

### Dietary therapy induces rapid and durable remission

To investigate the impact of a therapeutic diet on disease and the microbiome, treatment-naive dogs (n=29) with chronic enteritis (CE) were enrolled in a study to evaluate the impact of diet on disease and the microbiome. Dogs with active disease were switched from their current diet to a commercially-available therapeutic hydrolyzed protein diet **(****Fig. 1A****).** Impact of treatment on disease was monitored using the Canine Chronic Enteropathy Clinical Activity Index (CCECAI; hereafter referred to as 'disease score'), which is positively correlated with poor clinical outcome ³⁶. After two weeks on therapeutic diet, 69% (20/29) of animals entered remission, marked by a reduction in the mean disease score from 4.1 (95% CI = 4.8 - 3.3) to 1.3 (95% CI = 1.8 - 0.7). These diet-responsive (DR) animals were maintained on diet for the remainder of the study with no additional interventions **(****Fig. 1B****).** At the conclusion of the study (day 42), DR animals had an mean disease score of 0.9 (95% CI = 1.3 - 0.6), constituting an > 4-fold reduction in disease severity compared to day 0 **(****Fig. 1B****). In** contrast, 31% (9/29) of animals failed to show a significant reduction in disease score after two weeks on therapeutic diet **(****Fig. 1C****).** These non-diet-responsive (NDR) animals presented with more severe disease scores (mean score = 6.1; 95% CI = 7.4 - 4.7) than DR animals (P < 0.05 at day 0) and did not show a significant reduction after 2 week diet therapy **(****Fig. 1C****).** NDR animals were maintained on diet therapy for the reminder of the study, but also received combination therapy that included antibiotics (at day 14) and prednisone (at day 28) **(****Fig. 1A** **and** **Fig. 8****, see methods),** but showed only incremental improvement in disease scores **(****Fig. 1C****).** These data highlight a rapid clinical response to hydrolyzed diet in the majority of dogs with chronic enteritis.

### Identification of microbial community profiles associated with treatment outcome

To determine whether treatment with hydrolyzed diet alone is sufficient to alter the microbial community in the gut, 16S rRNA gene profiling was carried out on fecal samples collected from DR, NDR and healthy control animals (n=11). Consistent with previous reports ³⁸, it was found that the diversity of the canine fecal microbiome was not significantly altered in dogs with CE, compared to healthy controls **(****Fig. 9A-B**and that the communities in both groups were predominantly comprised of Firmicutes, Bacteroidetes, Proteobacteria, Actinobacteria, and Fusobacteria **(****Fig. 9C****).** However, compared to healthy dogs, animals with CE showed greater between-individual distance in microbial community structure by unweighted Unifrac **(****Fig. 9D****).** Using a ternary plot visualization, an enrichment of Operational Taxonomic Units (OTUs) was observed from Firmicutes and Proteobacteria in animals with active disease, while Bacteroidetes were enriched in healthy animals **(****Fig. 2A****).** Interestingly, a subset of proteobacterial OTUs was highly enriched in DR animals compared to both NDR and healthy controls **(****Fig. 2A****),** tan points in lower left corner).

These differences prompted us to carry out a formal differential abundance analysis, identifying 55 OTUs that distinguish healthy animals from those with disease **(Table 2).** For example, *Escherichia coli,* which is commonly associated with intestinal diseases, was over-represented in animals with CE **(****Fig. 2B****),** showing a significant, albeit weak, positive correlation with disease score (R=0.2109, P=0.02626) **(****Fig. 2C****).** OTUs from *Clostridium sensu stricto* 1 were also enriched in CE, including *Clostridium perfringens* **(****Fig. 2D****),** which was also positively correlated with disease scores **(****Fig. 2E****)** (R=0.2324, P=0.01412). These bacteria have been implicated in large bowel diarrhea/colitis in dogs ³⁹. Taken together with previously published work ⁴⁰, these data demonstrate that dysbiosis during CE is marked by the presence of pathobionts. Next, whether the microbiome in DR and NDR animals differed prior to the start of treatment (day 0) was investigated. Although no differences were observed between the two groups in community diversity, evenness or distance from healthy controls (unweighted or weighted Unifrac), 21 OTUs were identified that were differentially abundant between DR and NDR animals, 13 of which were enriched in animals that ended up responding to diet treatment **(****Fig. 2F** **and Table 3).** Interestingly, Proteobacteria and *C. perfringens* were found to be more abundant in DR animals **(****Fig. 2F****).** Collectively, these results highlight distinct microbial signatures during disease that are associated with different clinical outcomes following diet therapy.

### Therapeutic diet ameliorates dysbiosis associated with chronic enteritis

To assess whether diet-induced remission is accompanied by alterations in dysbiosis, the microbial community structures were compared before and after administration of therapeutic diet in DR animals. No significant change was observed in phylogenetic distance or shannon diversity **(****Fig. 10A-B****)** but did see a marked increase in community evenness following diet administration **(****Fig. 3A****)** when focusing on the top 40 most abundant OTUs among the samples, which account for 83% of the total reads. Principal coordinate analysis based on unweighted **(****Fig. 3B****)** or weighted **(****Fig. 10C****)** UniFrac showed a clear separation between dogs, even at day 0, before diet therapy was administered, highlighting heterogeneity in dysbiosis associated with clinical disease. Despite this baseline difference between animals, community structure underwent a marked shift away from disease-state by 14 and 42 days after diet therapy **(****Fig. 3B****).** Comparing unweighted Unifrac distances between DR and healthy animals at each time point, it was observed that diet-induced remission was marked by decreased phylogenetic distance relative to healthy controls, a trend that continued through day 42, when the phylogenetic similarity to day 0 was lowest and similarity to healthy dogs was highest **(****Fig. 3C****).**

Given that therapeutic diet shifted the community structure of the microbiome in DR animals, it was reasoned that composition of the fecal microbiome would be rapidly altered by dietary intervention. Administration of therapeutic diet was broadly characterized by an increase of Firmicutes and a decrease of Proteobacteria **(****Fig. 3D****).** Fourteen days after beginning diet therapy, ten genera were differentially abundant compared to pre-treatment (day 0) in DR animals **(Table 4).** Potential pathogenic genera associated with IBD were found under-represented after diet treatment. For example, *Escherichia-Shigella, Clostridium sensu stricto* 1, *Enterococcus* and *Fusobacterium* had a higher relative abundance at Day 0 and were significantly reduced after 14 days on therapeutic diet. When evaluated at species level, 36 OTUs were significantly differential abundant between samples collected at day 0 compared to day 14 **(****Fig. 3E****) (Table 5).** *E. coli* was typically enriched in the animals at day 0 in this study **(****Fig. 3E****),** and its relative abundance declined dramatically after two-weeks on therapeutic diet, eventually reaching levels nearly undetectable by day 42 that were also indistinguishable from levels observed in healthy dogs **(****Fig. 3F****).** *C. perfringens* also showed significant lower prevalence in the samples at day 14 and in healthy dogs, compared to day 0 samples **(****Fig. 3G****).** In turn, several increased OTUs were from the genera that have been suggested as beneficial commensals in human studies, such as *Blautia* ⁴¹ **(Table 5).** Taken together, these results point to ameliorated dysbiosis with a reduction of pathobionts and increase of beneficial commensal taxa as a hallmark of diet therapy.

### Remission-specific changes in the microbiome following diet therapy

It was hypothesized that the changes observed following diet therapy in DR animals are associated with remission, rather than merely a response to diet that is independent of clinical outcome. To test this hypothesis, the impact of therapeutic diet on dogs that entered remission following diet therapy alone (DR), was compared with changes observed in non-diet-responsive (NDR) animals that failed to enter remission after diet therapy, and which require additional therapies after day 14. Whereas diet therapy in DR animals was associated with increased community evenness **(****Fig. 3A****)** and a decreased phylogenetic distance from health dogs **(****Fig. 3C****),** the same treatment in NDR dogs did not significantly affect the microbial community evenness or Unifrac distance to healthy dogs **(****Fig. 4A and 4B****).** Just as it was observed in DR animals **(****Fig. 3D****),** diet also altered the gut microbiota compositions in NDR animals **(****Fig. 4C****).** Differential abundance analysis comparing NDR animals at day 0 versus day 14, when they received only therapeutic diet, identified 24 OTUs **(Table 6).** However, this shift was distinct from that observed in DR animals **(****Fig. 4D** **and** **Fig. 11****).** For example, diet therapy was associated with a decrease in the relative abundance of *Fusobacterium* and *Phascolarctobacterium* in NDR animals at day 14, compared to day 0, while these taxa were either unchanged or more modestly altered by diet therapy in DR animals. Conversely, *Escherichia-Shigella, Enterococcus* and some of *Clostridium sensu stricto* 1 are only reduced in animals that enter remission after diet treatment **(****Fig. 4D****).** The disease associated bacteria *E. coli* and *C. perfringens* were not significantly changed in NDR animals after diet therapy **(****Fig. 4E and 4F****).** After 14 days on therapeutic diet, NDR dogs were maintained on diet, but were also administered metronidazole, an antibiotic that largely targets anaerobes. Interestingly, antibiotic treatment exacerbated dysbiosis, resulting in a precipitous decline in community evenness **(****Fig. 4A****),** increased distance from healthy controls **(****Fig. 4B****),** and increased relative abundance of potential pathogens **(****Fig. 4E and 4F****).**

### Diet-induced remission is associated with metabolic reprogramming and increased levels of secondary bile acids.

To determine if diet induced changes in microbial community structure would translate to altered microbial metabolism the 16S rRNA gene sequencing data were used to assess the relative abundance of predicted KEGG pathways. Principal component analysis of metabolic pathway abundance data showed a separation between samples from animals with active disease (day 0) and those in remission (day 14) **(****Fig. 12****,** **Fig. 5A and 5B****).** Differential abundance analysis identified 36 pathways were increased in relative abundance as a result of diet treatment in DR animals **(Table** 7), including several involved in carbohydrate metabolism and secondary bile acid synthesis **(****Fig. 5C** **and** **5D****).** In contrast, 50 pathways were reduced, including fatty acid and steroid biosynthesis **(****Fig. 5C****).** This shift in metabolic potential away from lipid biosynthesis toward carbohydrate and bile acid synthesis as animals entered remission prompted us to quantify bile acids in stool. Using targeted metabolomics, levels of 15 bile acids in stool of healthy dogs were measured, compared with stool collected at days 0, 14 and 42 in the study **(****Fig. 13****, Table 10).** Consistent with the 16S data, the secondary bile acids deoxycholic acid **(****Fig. 5E**) and lithocholic acid **(****Fig. 5F**) were high in healthy controls but low in animals with active disease (day 0) and were significantly increased after the diet treatment in DR animals at day 14 and 42 **(****Fig. 5E and 5F****, and Table 8).** Notably, levels of these secondary bile acids were not elevated by diet treatment in NDR animals **(****Fig. 5G and 5H****),** suggesting that this metabolic shift is linked to diet-induced remission.

### Lithocholic and deoxycholic acid inhibit the growth of E. coli and C. perfringens, in vitro.

Diet-induced remodeling of the microbiome could be due, at least in part, to the inhibitory role of secondary bile acids on harmful bacteria. Correlation analysis of the metabolomics and microbiome data identified thirteen genera significantly associated with at least one bile acid (Spearman, R > 0.04 or < -0.04) **(****Fig. 6A****).** The primary bile acid, cholic acid, was negatively correlated with 11 OTUs, consistent with the reported ability of this bile acid to negatively regulate bacterial growth ⁴². It was also observed that the increase in secondary bile acids following diet treatment correlated with a reduction in relative abundance of certain bacteria (*e.g*., OTUs from *Escherichia-Shigella, Clostridium* and *Fusobacterium)* **(Table 9).** To directly test this hypothesis, lithocholic or deoxycholic acid were assessed for their ability to inhibit the *in vitro* growth of *E*. *coli* (*n =* 1) or *C. perfringens* (n = 3) isolates derived from the dogs with active disease, since these species or their genera were associated with disease in the animal model. Deoxycholic acid blocked the growth of both species at a concentration comparable to what was detected in the fecal samples **(****Fig. 6C and 6E****),** while lithocholic acid blocked the growth of *E. coli* but not *C. perfringens* **(****Fig. 6B and 6D****, respectively).** Collectively, these results show that the inhibitory activity of these bile acids varies for different bacteria and suggest that elevated secondary bile acids observed following diet therapy can contribute to the decrease of potentially harmful bacteria.

### C. hiranonis is a diet-responsive species with the ability to produce secondary bile acids.

Next, the source of lithocholic and deoxycholic acids after diet treatment was identified. Production of these from primary bile acids requires the 7-dehydroxylation activity conferred by the bile acid-inducible (*bai*) operon - an activity unique to a limited number of anaerobes representing a small fraction of the microbiome, including some Clostridial and Eubacterial species ⁴³. Given the finding that certain clostridial OTUs, as well as levels of lithocholic and deoxycholic acids, increase after diet-induced remission **(****Fig. 3G** **and** **Fig. 5E-F****,** respectively), potential bile acid producers in DR animals were identified. Stool samples collected day 0, 14 and 42 after starting diet therapy were subjected to metagenomic sequencing. Taxonomic assignment of reads identified six *Clostridium* species (*C. perfringens, C. hiranonis, C. nexile, C. colicanis, C. glycolicum* and *C*. *ramosum*) and 2 *Eubacterium* species (*Eubacterium biforme* and *E*. *dolichum)* present in these samples at a relative abundance ≥ 0.01% in at least 10% samples. Of these species only *C*. *hiranonis* has been reported to have the *bai* operon ⁴⁴, and this was confirmed by BLAST against the reference genomes of these species in GenBank. Moreover, the metagenomic data **(****Fig. 14****)** and 16S sequencing data showed that the relative abundance of C. *hiranonis* was significantly increased after diet treatment in DR animals **(****Fig. 6F****, left)** but not in NDR animals that failed diet therapy **(****Fig. 6F****, right).** Since the *Clostridium* genus exhibits a high level of genetic divergence, even at the species level, that canine *C*. *hiranonis* possesses the *bai* operon was confirmed. Anaerobic culture of rectal swabs collected during the study, followed by isolate picking and Sanger sequencing of full-length 16S rRNA gene, was used to assemble a canine culture collection from 7 dogs with chronic enteritis before and/or after treatment. In total, *49 Clostridium* isolates belonging to 5 species were identified (*C*. *baratii, C. perfringens, C. sartagoforme, C. hiranonis,* and *C*. *lactatifermentans*). 82% (31/39) of the clostridial isolates from animals with active disease were *C. perfringens,* consistent with the reported involvement of this organism in canine ³⁹ and human ⁴⁵ gastrointestinal disease. Two *C*. *hiranonis* isolates were obtained from independent diet-responsive animals in remission at day 42. These *C*. *hiranonis* isolates and three *C. perfringens* isolates were selected for whole genome sequencing. Reads were aligned to the reference *C*. *hiranoni,* revealing an intact *bai* operon in canine *C*. *hiranonis,* but not *C. perfringens* **(****Fig. 6G****).** Taken together, these data point to *C*. *hiranonis,* a species originally isolated from human stool ⁴⁴, as a likely bile acid producer associated with diet-induced remission in dogs.

### C. hiranonis inhibits inflammation-induced expansion of E. coli in a mouse model of DSS colitis

The ability of *C*. *hiranonis* to produce secondary bile acids, combined with the observation that lithocholic and deoxycholic acids were potent inhibitors of *E. coli* and *C. perfringens* growth, *in vitro,* prompted us to test whether *C*. *hiranonis* could restrict expansion of potential pathogens *in vivo* during inflammation. Mice were first colonized with drug-selectable *E. coli* (Nissle 1917 strain), inflammation was triggered by administration of dextran sodium sulfate (DSS) in the drinking water, and animals were either orally administered PBS daily (mock) or *C*. *hiranonis* **(****Fig. 6H****).** DSS treatment resulted in reduced colon length **(****Fig. 6I**), and a dramatic bloom of the *E. coli* Nissle strain **(****Fig. 6J****).** In contrast, DSS-treated mice that received *C*. *hiranonis* daily showed a marked reduction of colonic shortening, and a near complete abrogation of *E. coli* expansion. Taken together with the finding that lithocholic and deoxycholic acids can inhibit growth of pathobionts, these data suggest that *C*. *hiranonis* or secondary bile acids produced by this species, mediate colonization resistance during enteritis.

### C. scindens is associated with diet-induced remission in pediatric Crohn's disease

Given that high remission rates are observed in both dogs and humans following dietary therapy, it was hypothesized that a similar induction of *bai* operon-containing clostridia can occur in pediatric Crohn's disease patients being treated with exclusive enteral nutrition (EEN). To test this, publicly available data from a recent study that examined approximately 20 patients before and after treatment with EEN were analyzed ²³, in which half responded to treatment while other half failed EEN therapy. Classifications of bacterial taxa present in each sample using standard metagenomic analysis methods ⁴⁶ revealed the presence of *C. scindens,* which is recognized for having high 7-dehydroxylation activity ^{44,47}. The relative abundance was further estimated using the proportion of total reads that map the reference genome of *C. scindens.* As shown in **Fig. 7A****,** this bacterium increased significantly from pretreatment to 8 weeks post-EEN, as did the number of reads mapping to the *bai* operon **(****Fig. 7B****).** Remarkably, this increase was only observed in patients that entered remission following EEN (Responsive, n =10) but not those that failed therapy (Non.Responsive, n = 10) **(****Fig.7A and 7B****).** In addition, the correlation analysis between the relative abundance of *C. scindens* and fecal calprotectin (FCP), a biomarker of disease activity for IBD ²³, indicated a significantly negative correlation **(****Fig. 7C****)** in EEN 'Responsive' patients (R= -0.3515, P = 0.03287), but not in EEN 'Non.Responsive' patients (R=-0.0267, P=0.877). Similarly, a significant negative correlation between *bai* operon and FCP was observed in diet-responsive (R= -0.3944, P= 0.0157), but not non-responsive patients (R= 0.0490, P= 0.7766) **(****Fig. 7D****).** These results collectively point to bile acid producing clostridia as key features of diet-induced remission and potent inhibitors of pathobiont colonization in both animals and humans **(****Fig 7E****).**

### Discussion

Using a diet-responsive animal model to study the role of the microbiome in chronic enteritis, remission-specific changes in microbiome composition and function were identified. All animals enrolled in the study had active disease, yet their baseline microbiome composition differed greatly **(****Fig. 3****),** perhaps reflecting variation in their environment, genetic background (breed), age and weight. This variation in composition supports the idea that enteric disease is driven not by a single dysbiotic state, but rather dysbiosis reflects a loss of community stability ⁴⁸. Rather than dramatic changes in microbial community structure following diet therapy, a shift from lipid metabolism to carbohydrate and bile acid synthesis was observed. Although the metabolomics analysis was focused on bile acids, a broader picture of the metabolites produced before and after diet therapy, as well as the macro- and micro-nutrients present in the diets themselves, can improve the understanding of the mechanisms by which diet achieves remission.

One important open question is precisely how therapeutic diets such as EEN or prescription pet foods alter the microbiome and whether there are general principles that could be used to guide the development of better dietary therapies. Studies in pediatric Crohn's disease have reported higher remission rates with EEN compared to partial enteral nutrition (PEN), which includes some table food. These observations have led some to postulate that a highly monotonous diet that is reduced in complexity can constitute an essential part of nutritional therapies for IBD. Consistent with this notion, mice fed a monotonous diet exhibited lower microbial diversity and were more susceptible to DSS colitis than mice fed an alternating diet ⁴⁹. However, the prevalence and treatment of chronic enteritis in veterinary medicine highlights that disease routinely develops even when diets are monotonous and that rapid and robust remission can be achieved with solid food. Hydrolyzed protein diets, such as the one used in the study, have been shown to be effective in the management of canine chronic enteropathies ^{50,51}, and have previously been shown to be more effective for long term management when compared to a highly digestible diet formulated with non-hydrolyzed protein sources ^{50,51}. While it is uncertain what characteristic of these hydrolyzed formulas are driving the response, the low molecular weight of hydrolyzed proteins can reduce their ability to be recognized by the immune system while providing improved digestibility. In summary, the results suggest that the dog would be a useful model to dissect the beneficial and harmful roles of different diets, particularly since formulated diets have long been a standard of care for treating numerous diseases in companion animals.

Secondary bile acids and bile acid-producing clostridial species were identified as key features of diet-induced remission in humans and dogs. These findings complement recent studies examining the mechanisms by which fecal microbiota transplant (FMT) cure *Clostridium difficile* infection ⁵². Buffy *et al.* identified *Clostridium scindens* as associated with resistance to *C. difficile* infection in both humans and mice, and they showed that transfer of *C. scindens,* or a consortium containing this organism, protected mice from *C. difficile* challenge. Moreover, inhibition of *C. difficile* growth *in vitro* by *C. scindens* was associated with secondary bile acid production. These data are consistent with microbiological studies showing that primary bile acids induce germination of *C. difficile,* while certain secondary bile acids can block vegetative growth ⁵³. Although *C. difficile* was not observed in the animals, *C. perfringens* and *E. coli* were identified as major disease-associated taxa, and it was shown that physiologic levels of secondary bile acids potently block *in vitro* growth of these organisms. It is not known whether bile acids can restrict these organisms *in vivo* in the canine model but elucidating these mechanisms could have important health implications beyond veterinary medicine. Although *C*. *difficile* is the leading cause of nosocomial diarrhea in humans, *C. perfringens* and *E. coli* are both common human commensals and have been implicated in both diarrheal disease and colitis in humans and dogs. Moreover, the ability of *C. perfringens* to produce numerous toxins make it a leading cause of foodborne illness and soft tissue infections. Interestingly, when data from a cohort of pediatric Crohn's disease patients before and after diet therapy were examined, it was found that *C*. *scindens* was associated with diet-induced remission **(****Fig. 7****),** and a related study showed that sustained remission following EEN was characterized by low levels of proteobacteria, while patients that relapsed showed a marked increase in proteobacteria ⁵⁴. The data, together with previous studies in dogs ^{33,35}, highlight the importance of leveraging animal models and advocate for the use of newly developed analytical methods ⁵⁵ and database approaches ^{56,57} for comparing across multiple microbiome studies to take a 'One Health' approach that could identify conserved themes in host-microbiome interactions.

*C. difficile* infections frequently arise after antibiotic treatment, a phenomenon attributed to the effect of antibiotics on secondary bile acid levels ⁵⁸. Interestingly, it was also observed that antibiotics antagonized the diet-induced shifts in microbiome composition and function, promoting a more dysbiotic state coincident with dramatically reduced levels of lithocholic and deoxycholic acid **(****Fig. 4** **and** **Fig. 5****).** Taken together, these data support a more general model for microbe-microbe interactions in the gut in which bile acid producing clostridia restrict the growth of a range of bile acid-sensitive pathobionts to limit disease and highlight that these processes are exquisitely sensitive to antimicrobials. The parallels between the findings and those reported for FMT and *C. scindens* would suggest that FMT might also be beneficial for treating enteritis. Clinical trials testing this hypothesis in IBD patients have shown moderate success, in marked contrast to *C. difficile* infections where FMT is curative for the vast majority of patients ⁵⁹. This discrepancy can be related to different pathobionts contributing to IBD pathogenesis. Interestingly, colitis is a common side-effect observed in cancer patients undergoing immune checkpoint blockade, and a recent study demonstrated complete resolution of this colitis following FMT ⁶⁰, raising the possibility that bile acid producers can be important in treating certain types of colitis.

**Table 2. OTUs with differential abundances between samples of healthy dogs and dogs with CCE at day 0 (Fold change >2 and P-value <0.05).**

| **OTU** | **Bas e Me an** | **log 2 Fol d Ch ang e** | **P valu e** | **Kin gdo m** | **Phylu m** | **Class** | **Order** | **Family** | **Genus** | **Species** |
|---|---|---|---|---|---|---|---|---|---|---|
| HQ80298 3.1.1440 | 134. 78 | - 9.1 4 | 3.28 E-10 | Bact eria | Firmic utes | Clostrid ia | Clostridi ales | Lachnospir aceae | Tyzzerella 4 | NA |
| FJ950694. 1.1472 | 205 2.29 | - 5.5 6 | 6.45 E-09 | Bact eria | Proteo bacteri a | Gamma proteo bacteria | Enteroba cteriales | Enterobacte riaceae | Escherichi a-Shigella | Escheric hia coli |
| HG79845 1.1.1400 | 30.7 4 | - 6.7 0 | 1.14 E-07 | Bact eria | Firmic utes | Bacilli | Lactobac illales | Enterococc aceae | Enterococ cus | Enteroco ccus durans |
| New Refer ence OTU52 | 676. 55 | - 6.5 1 | 8.89 E-07 | Bact eria | Firmic utes | Clostrid ia | Clostridi ales | Clostridiace ae 1 | Clostridiu m sensu stricto 1 | perfring ens |
| New Refer ence OTU82 | 35.4 9 | - 4.8 7 | 6.27 E-05 | Bact eria | Firmic utes | Clostrid ia | Clostridi ales | Clostridiace ae 1 | Clostridiu m sensu stricto 1 | NA |
| GQ44909 2.1.1375 | 69.3 0 | - 7.0 4 | 7.97 E-05 | Bact eria | Firmic utes | Clostrid ia | Clostridi ales | Lachnospir aceae | Tyzzerella | NA |
| FJ506371. 1.1371 | 34.8 8 | - 5.4 0 | 0.00 012 | Bact eria | Firmic utes | Erysipel otrichia | Erysipe lotrichal es | Erysipelotri chaceae | Erysipelat o clostridiu m | NA |
| GQ44874 41.1393 | 81.0 6 | - 6.8 6 | 0.00 011 | Bact eria | Bacter oi detes | Bacteroi dia | Bacteroi dales | Bacteroidac eae | Bacteroide s | Ambigu ous_taxa |
| FJ957494. 1.1454 | 19.1 8 | - 6.3 3 | 0.00 037 | Bact eria | Firmic utes | Clostrid ia | Clostridi ales | Clostridiace ae 1 | Clostridiu m sensu stricto 1 | Ambigu ous_taxa |
| HQ76091 1.1.1437 | 11.6 6 | - 5.9 1 | 0.00 045 | Bact eria | Firmic utes | Clostrid ia | Clostridi ales | Lachnospir aceae | Anaerosti pes | NA |
| GQ00632 4.1.1342 | 10.7 3 | - 5.7 7 | 0.00 084 | Bact eria | Actino bacteri a | Actinob acteria | Coryne bacterial es | Corynebact eriaceae | Corynebac terium 1 | uncultur ed bacteriu m |
| GQ44824 6.1.1389 | 313. 30 | - 3.8 7 | 0.00 079 | Bact eria | Bacter oidetes | Bacteroi dia | Bacteroi dales | Bacteroidac eae | Bacteroide s | Ambigu ous_taxa |
| KC245406 .1.1465 | 3.79 | - 3.7 0 | 0.00 07 | Bact eria | Firmic utes | Clostrid ia | Clostridi ales | Lachnospir aceae | Lachno clostridiu m | uncultur ed bacteriu m |
| New Refer ence OTU54 | 39.9 5 | - 5.9 1 | 0.00 09 | Bact eria | Firmic utes | Clostrid ia | Clostridi ales | Lachnospir aceae | Blautia | uncultur ed bacteriu m |
| HQ75154 9.1.1448 | 10.4 1 | - 4.7 0 | 0.00 12 | Bact eria | Firmic utes | Clostrid ia | Clostridi ales | Lachnospir aceae | uncultured | NA |
| JF712675. 1.1540 | 6.38 | - 5.0 3 | 0.00 12 | Bact eria | Proteo bacteri a | Gamma proteo bacteria | Entero bacterial es | Enterobacte riaceae | Escherichi a-Shigella | uncultur ed bacteriu m |
| JQ208181. 1.1352 | 148. 49 | - 2.9 9 | 0.00 12 | Bact eria | Firmic utes | Clostrid ia | Clostridi ales | Lachnospir aceae | [Ruminoc occus] gauvreauii group | Ambigu ous_taxa |
| GX18240 4.8.1529 | 3.29 | - 4.0 8 | 0.00 20 | Bact eria | Proteo bacteri a | Gamma proteo bacteria | Enteroba cteriales | Enterobacte riaceae | Escherichi a-Shigella | NA |
| FP929060. 38375503 | 375. 58 | - 1.8 2 | 0.00 20 | Bact eria | Firmic utes | Clostrid ia | Clostridi ales | Lachnospir aceae | NA | NA |
| FN667392 1.1495 | 12.1 9 | - 5.9 7 | 0.00 25 | Bact eria | Firmic utes | Bacilli | Lactobac illales | Lactobacill aceae | Lactobacil lus | uncultur ed bacteriu m |
| FN667422 1.1495 | 5.84 | - 4.3 3 | 0.00 34 | Bact eria | Firmic utes | Bacilli | Lactobac illales | Lactobacill aceae | Lactobacil lus | Ambigu ous_taxa |
| HK55708 9.3.1395 | 134 0.69 | - 3.0 2 | 0.00 46 | Bact eria | Firmic utes | Bacilli | Lactobac illales | Streptococe aceae | Streptococ cus | uncultur ed bacteriu m |
| HQ80396 4.1.1435 | 335. 70 | - 2.9 0 | 0.00 46 | Bact eria | Firmic utes | Clostrid ia | Clostridi ales | Lachnospir aceae | Lachno clostridiu m | uncultur ed bacteriu m |
| AM27675 91.1484 | 6.84 | - 2.8 7 | 0.00 45 | Bact eria | Firmic utes | Clostrid ia | Clostridi ales | Lachnospir aceae | Blautia | uncultur ed bacteriu m |
| HK55593 8.1.1357 | 21.7 2 | - 6.4 0 | 0.00 54 | Bact eria | Actino bacteri a | Corioba cteriia | Coriobac teriales | Coriobacter iaceae | Collinsella | uncultur ed bacteriu m |
| KF842598 1.1394 | 22.6 0 | - 6.8 0 | 0.00 54 | Bact eria | Bacter oidetes | Bacteroi dia | Bacteroi dales | Porphyrom onadaceae | Parabacter oides | NA |
| HQ79277 81.1436 | 5.38 | 3.6 2 | 0.00 58 | Bact eria | Bacter oidetes | Bacteroi dia | Bacteroi dales | Bacteroidac eae | Bacteroide s | uncultur ed bacteriu m |
| FM86590 5.1.1392 | 8.52 | - 5.4 5 | 0.00 66 | Bact eria | Firmic utes | Clostrid ia | Clostridi ales | Clostridiace ae 1 | Clostridiu m sensu stricto 1 | NA |
| FN563300 .1.1447 | 114 7.14 | - 1.9 2 | 0.00 64 | Bact eria | Firmic utes | Clostrid ia | Clostridi ales | Lachnospir aceae | Blautia | uncultur ed bacteriu m |
| HQ75468 0.1.1441 | 10.1 5 | - 2.2 0 | 0.00 65 | Bact eria | Firmic utes | Clostrid ia | Clostridi ales | Lachnospir aceae | NA | NA |
| GQ86742 61.1494 | 3.36 | - 4.0 8 | 0.00 72 | Bact eria | Proteo bacteri a | Gamma proteo bacteria | Entero bacterial es | Enterobacte riaceae | Escherichi a-Shigella | uncultur ed bacteriu m |
| EU470512 .1.1400 | 2.07 | - 3.4 0 | 0.00 79 | Bact eria | Proteo bacteri a | Gamma proteo bacteria | Entero bacterial es | Enterobacte riaceae | Escherichi a-Shigella | uncultur ed bacteriu m |
| AY23946 2.1.1500 | 2.71 | - 3.2 0 | 0.00 80 | Bact eria | Firmic utes | Clostrid ia | Clostridi ales | Lachnospir aceae | [Ruminoc occus] gauvreauii group | Ambigu ous_taxa |
| New.Refer ence OTU114 | 8.57 | - 3.1 0 | 0.00 91 | Bact eria | Firmic utes | Bacilli | Lactobac illales | Streptococe aceae | Streptococ cus | uncultur ed bacteriu m |
| FN668375 .4306350. 4307737 | 9.14 | - 4.0 9 | 0.00 93 | Bact eria | Firmic utes | Clostrid ia | Clostridi ales | Peptostrept ococcaceae | NA | NA |
| AB009242 .1.1451 | 8.33 | - 4.8 1 | 0.00 97 | Bact eria | Spiroc haetae | Spiroch aetes | Spirocha etales | Spirochaeta ceae | Treponem a 2 | NA |
| HQ79278 7.1.1438 | 1.61 | 3.4 5 | 0.01 28 | Bact eria | Bacter oidetes | Bacteroi dia | Bacteroi dales | Bacteroidac eae | Bacteroide s | uncultur ed bacteriu m |
| AB506370 .1.1516 | 5.92 | - 4.6 3 | 0.01 94 | Bact eria | Bacter oidetes | Bacteroi dia | Bacteroi dales | Prevotellac eae | Prevotella ceae UCG-001 | Ambigu ous_taxa |
| DQ05736 5.1.1393 | 5.11 | - 4.4 2 | 0.02 02 | Bact eria | Firmic utes | Clostrid ia | Clostridi ales | Lachnospir aceae | Lachno clostridiu m | Ambigu ous_taxa |
| FN667084 .1.1493 | 8.26 | - 3.5 3 | 0.02 16 | Bact eria | Firmic utes | Bacilli | Lactobac illales | Lactobacill aceae | Lactobacil lus | uncultur ed bacteriu m |
| DQ11376 5.1.1450 | 150 0.29 | 3.8 2 | 0.02 30 | Bact eria | Bacter oidetes | Bacteroi dia | Bacteroi dales | Bacteroidac eae | Bacteroide s | NA |
| HK69402 9.9.1487 | 6.57 | - 2.6 6 | 0.02 44 | Bact eria | Firmic utes | Erysipe lotrichia | Erysipel o trichales | Erysipelotri chaceae | Faecalitale a | NA |
| AJ270486. 1.1241 | 10.9 2 | - 4.2 4 | 0.02 90 | Bact eria | Firmic utes | Clostrid ia | Clostridi ales | Lachnospir aceae | Coprococc us 1 | Ambigu ous_taxa |
| EU768569 .1.1352 | 5.69 | - 3.3 7 | 0.03 14 | Bact eria | Firmic utes | Clostrid ia | Clostridi ales | Ruminococ caceae | Ruminiclo stridium 9 | uncultur ed bacteriu m |
| FM17975 2.1.1686 | 1.66 | - 3.1 1 | 0.03 24 | Bact eria | Proteo bacteri a | Gamma proteo bacteria | Entero bacterial es | Enterobacte riaceae | Pseudocitr obacter | NA |
| JF807116. 1.1260 | 2.54 | - 3.7 0 | 0.03 51 | Arch aea | Euryar chaeot a | Methan o bacteria | Methano bacterial es | Methanoba cteriaceae | Methanob revibacter | uncultur ed archaeon |
| FJ957528. 1.1445 | 14.7 5 | - 5.0 9 | 0.03 56 | Bact eria | Firmic utes | Clostrid ia | Clostridi ales | Clostridiace ae 1 | Sarcina | uncultur ed bacteriu m |
| KC504009 .1.1465 | 3.67 | - 3.1 5 | 0.03 50 | Bact eria | Proteo bacteri a | Gamma proteo bacteria | Entero bacterial es | Enterobacte riaceae | Escherichi a-Shigella | NA |
| GQ44850 6.1.1374 | 304. 58 | - 1.5 8 | 0.03 35 | Bact eria | Firmic utes | Clostrid ia | Clostridi ales | Lachnospir aceae | Blautia | uncultur ed bacteriu m |
| JF224013. 1.1362 | 2.89 | - 3.8 9 | 0.03 90 | Bact eria | Bacter oidetes | Bacteroi dia | Bacteroi dales | Porphyrom onadaceae | Porphyro monas | uncultur ed bacteriu m |
| EU774020 1.1361 | 12.8 5 | 2.5 1 | 0.03 91 | Bact eria | Fusoba cteria | Fusobac teriia | Fusobact eriales | Fusobacteri aceae | Fusobacte rium | uncultur ed bacteriu m |
| GQ44848 6.1.1387 | 48.9 5 | - 2.7 2 | 0.03 84 | Bact eria | Firmic utes | Clostrid ia | Clostridi ales | Lachnospir aceae | Blautia | uncultur ed bacteriu m |
| HQ79376 3.1.1451 | 13.4 1 | 3.3 2 | 0.04 34 | Bact eria | Bacter oidetes | Bacteroi dia | Bacteroi dales | Bacteroidac eae | Bacteroide s | NA |
| JN387556. 1.1324 | 164. 12 | - 2.7 8 | 0.04 59 | Bact eria | Firmic utes | Clostrid ia | Clostridi ales | Peptostrept ococcaceae | Intestiniba cter | NA |
| New.Refer ence OTU109 | 34.4 7 | 3.5 4 | 0.04 88 | Bact eria | Bacter oidetes | Bacteroi dia | Bacteroi dales | Bacteroidac eae | Bacteroide s | NA |

**Table 3. OTUs with differential abundances between day 0-samples of diet responsive dogs and diet non-responsive dogs.**

| **OTU ID** | **Ba se Me an** | **log2Fo ldCha nge** | **P value** | **Kin gdo m** | **Phylu m** | **Class** | **Order** | **Family** | **Genus** | **Specie s** |
|---|---|---|---|---|---|---|---|---|---|---|
| JRPJ0100000 2.1034290.10 35971 | 11 1.7 2 | 8.05 | 0.000 3301 85 | Bac teri a | Prote obact eria | Epsilonpr oteobacte ria | Campyl obacter ales | Helicoba eteraceae | Helicob acter | Ambig uous_t axa |
| JF920309.1.1 340 | 22. 79 | 5.56 | 0.004 09 | Bac teri a | Prote obact eria | Epsilonpr oteobacte ria | Campyl obacter ales | Campylo bacterace ae | Campyl obacter | NA |
| FJ978526.1.1 378 | 8.9 3 | 5.36 | 0.040 34 | Bac teri a | Prote obact eria | Gammap roteobact eria | Aeromo nadales | Succinivi brionacea e | Succini vibrio | uncultu red bacteri um |
| New.Referenc eOTU45 | 47. 39 | 23.74 | 3.66 E-14 | Bac teri a | Prote obact eria | Gammap roteobact eria | Aeromo nadales | Succinivi brionacea e | Anaerob iospirill um | Ambig uous_t axa |
| HK555938.1.1 357 | 19. 48 | -6.16 | 0.044 81 | Bac teri a | Actin obact eria | Coriobac teriia | Corioba cteriales | Coriobact eriaceae | Collinse lla | uncultu red bacteri um |
| FJ957494.1.1 454 | 20. 74 | -3.37 | 0.014 29 | Bac teri a | Firmi cutes | Clostridi a | Clostrid iales | Clostridia ceae 1 | Clostrid ium sensu stricto 1 | Ambig uous_t axa |
| New.Referenc eOTU52 | 11 15. 31 | 3.54 | 0.012 42 | Bac teri a | Firmi cutes | Clostridi a | Clostrid iales | Clostridia ceae 1 | Clostrid ium sensu stricto 1 | NA |
| DQ797046.1.1 403 | 14. 74 | 4.64 | 0.023 61 | Bac teri a | Firmi cutes | Negativic utes | Seleno monada les | Veillonel laceae | Allisone lla | uncultu red bacteri um |
| GQ449092.1.1 375 | 15. 00 | -3.58 | 0.045 61 | Bac teri a | Firmi cutes | Clostridi a | Clostrid iales | Lachnosp iraceae | Tyzzere lla | NA |
| AMCI010016 31.341456 | 77. 88 | -4.66 | 0.003 49 | Bac teri a | Bacte roidet es | Bacteroid ia | Bactero idales | Bacteroid aceae | Bacteroi des | uncultu red bacteri um |
| KF842598.1.1 394 | 6.5 2 | 5.98 | 0.029 31 | Bac teri a | Bacte roidet es | Bacteroid ia | Bactero idales | Porphyro monadac eae | Parabact eroides | NA |
| HQ793763.1.1 451 | 5.5 7 | 5.09 | 0.013 96 | Bac teri a | Bacte roidet es | Bacteroid ia | Bactero idales | Bacteroid aceae | Bacteroi des | NA |
| DQ113765.1.1 450 | 20 1.8 7 | 5.02 | 0.004 53 | Bac teri a | Bacte roidet es | Bacteroid ia | Bactero idales | Bacteroid aceae | Bacteroi des | NA |
| ACBW01000 012.3536.505 4 | 4.3 7 | 4.18 | 0.029 09 | Bac teri a | Bacte roidet es | Bacteroid ia | Bactero idales | Bacteroid aceae | Bacteroi des | uncultu red bacteri um |
| HK693629.1.1 491 | 23. 00 | -2.59 | 0.010 37 | Bac teri a | Firmi cutes | Clostridi a | Clostrid iales | Lachnosp iraceae | Blautia | NA |
| JQ208053. 1.1 336 | 14. 50 | 2.58 | 0.044 54 | Bac teri a | Fusob acteri a | Fusobact eriia | Fusobac teriales | Fusobact eriaceae | Fusobac terium | NA |
| GQ493166.1.1 359 | 23 1.2 8 | -2.75 | 0.003 91 | Bac teri a | Firmi cutes | Clostridi a | Clostrid iales | Lachnosp iraceae | NA | NA |
| GQ448486.1.1 387 | 39. 63 | -4.02 | 0.000 23 | Bac teri a | Firmi cutes | Clostridi a | Clostrid iales | Lachnosp iraceae | Blautia | uncultu red bacteri um |
| GQ491426.1.1 332 | 46 1.0 1 | -2.89 | 0.038 94 | Bac teri a | Firmi cutes | Clostridi a | Clostrid iales | Lachnosp iraceae | Blautia | uncultu red bacteri um |
| New.Referenc eOTU54 | 18. 10 | 5.21 | 0.001 52 | Bac teri a | Firmi cutes | Clostridi a | Clostrid iales | Lachnosp iraceae | Blautia | uncultu red bacteri um |
| JN387556.1.1 324 | 16 7.4 7 | 3.99 | 0.007 91 | Bac teri a | Firmi cutes | Clostridi a | Clostrid iales | Peptostre ptococca ceae | Intestini bacter | NA |

**Table 4. Genera with differential abundances between samples of dogs at day 14 and day 0 (day 14 versus day 0) for diet responsive dogs.**

| **OUT IDs** | **Base Mean** | **log2Fol dChan ge** | **P value** | **Kingdo m** | **Phylu m** | **Class** | **Order** | **Family** | **Genus** |
|---|---|---|---|---|---|---|---|---|---|
| GQ0063 24.1.134 2 | 15.64 | -3.74 | 0.0013 7 | Bacteria | Actino bacteria | Actinoba cteria | Corynebac teriales | Coryneb acteriace ae | Corynebac terium 1 |
| New.Re ference OTU52 | 1991.15 | -2.20 | 0.0122 2 | Bacteria | Firmic utes | Clostridi a | Clostridial es | Clostridi aceae 1 | Clostridiu m sensu stricto 1 |
| HG7984 51.1.140 0 | 25.03 | -2.31 | 0.0091 1 | Bacteria | Firmic utes | Bacilli | Lactobacil lales | Enteroco ccaceae | Enterococ cus |
| HK5570 89.3.139 5 | 6043.88 | 3.13 | 0.0012 4 | Bacteria | Firmic utes | Bacilli | Lactobacil lales | Streptoc occaceae | Streptococ cus |
| GQ4483 36.1.141 8 | 34.22 | 3.62 | 0.0208 0 | Bacteria | Firmic utes | Negativi cutes | Selenomo nadales | Veillonel laceae | Megaspha era |
| KF8425 98.1.139 4 | 4.25 | -4.15 | 0.0234 9 | Bacteria | Bactero idetes | Bacteroi dia | Bacteroida les | Porphyro monadac eae | Parabacter oides |
| FJ95069 41.1472 | 1259.08 | -3.07 | 0.0010 9 | Bacteria | Proteob acteria | Gammap roteobac teria | Enterobact eriales | Enteroba cteriacea e | Escherichi a-Shigella |
| HQ8029 83.1.144 0 | 40.41 | -3.23 | 0.0028 03 | Bacteria | Firmic utes | Clostridi a | Clostridial es | Lachnos piraceae | Tyzzerella 4 |
| GQ4484 68.1.136 6 | 2058.39 | -2.10 | 0.0103 6 | Bacteria | Fusoba cteria | Fusobact eriia | Fusobacter iales | Fusobact eriaceae | Fusobacter ium |
| JN3875 56.1.132 4 | 161.95 | -3.09 | 0.0117 2 | Bacteria | Firmic utes | Clostridi a | Clostridial es | Peptostre ptococca ceae | Intestiniba eter |

**Table 5. OTUs with differential abundances between samples day 14 and day 0 in diet responsive dogs (day 14 versus day 0).**

| **OTU IDs+A2:K3 9** | **Bas e Me an** | **log2F oldCh ange** | **P val ue** | **Ki ng do m** | **Phyl um** | **Class** | **Order** | **Family** | **Genus** | **Species** |
|---|---|---|---|---|---|---|---|---|---|---|
| JRPJ010000 02.1034290. 1035971 | 38.0 4 | -3.39 | 0.0 34 | Ba cter ia | Prote obact eria | Epsilon proteob acteria | Campyl obacter ales | Helicob acterace ae | Helicobacter | Ambiguo us_taxa |
| New.Referen ceOTU45 | 32.8 6 | -5.37 | 0.0 17 | Ba cter ia | Prote obact eria | Gamma proteob acteria | Aerom onadale s | Succini vibriona ceae | Anaerobiospi rillum | Ambiguo us_taxa |
| GQ006324.1 .1342 | 10.9 9 | -3.41 | 0.0 02 | Ba cter ia | Actin obact eria | Actinob acteria | Coryne bacteria les | Coryneb acteriac eae | Corynebacter ium 1 | unculture d bacteriu m |
| HK555938.1 .1357 | 13.4 9 | 5.19 | 0.0 34 | Ba cter ia | Actin obact eria | Corioba cteriia | Corioba cteriale s | Corioba cteriace ae | Collinsella | unculture d bacteriu m |
| FJ957551.1. 1489 | 5.64 | 2.44 | 0.0 23 | Ba cter ia | Firmi cutes | Clostrid ia | Clostrid iales | Clostrid iaceae 1 | Sarcina | unculture d bacteriu m |
| FJ957494.1. 1454 | 22.5 2 | 2.91 | 0.0 01 | Ba cter ia | Firmi cutes | Clostrid ia | Clostrid iales | Clostrid iaceae 1 | Clostridium sensu stricto 1 | Ambiguo us_taxa |
| New.Referen ceOTU52 | 899. 64 | -2.89 | 0.0 15 | Ba cter ia | Firmi cutes | Clostrid ia | Clostrid iales | Clostrid iaceae 1 | Clostridium sensu stricto 1 | NA |
| FM865905.1 .1392 | 21.5 1 | -3.54 | 0.0 24 | Ba cter ia | Firmi cutes | Clostrid ia | Clostrid iales | Clostrid iaceae 1 | Clostridium sensu stricto 1 | NA |
| GQ016239.1 .1362 | 12.5 5 | 3.10 | 0.0 15 | Ba cter ia | Firmi cutes | Erysipel otrichia | Erysipe lotricha les | Erysipel otrichac eae | Faecalitalea | Ambiguo us_taxa |
| HG798451.1 .1400 | 21.6 3 | -1.92 | 0.0 23 | Ba cter ia | Firmi cutes | Bacilli | Lactoba cillales | Enteroc occacea e | Enterococcus | Enteroco ccus durans |
| EU461791.1. 1414 | 5.48 | 2.98 | 0.0 43 | Ba cter ia | Firmi cutes | Bacilli | Lactoba cillales | Streptoc occacea e | Streptococcu s | unculture d bacteriu m |
| GU303759.1 .1517 | 22.2 6 | 2.50 | 0.0 10 | Ba cter ia | Firmi cutes | Bacilli | Lactoba cillales | Streptoc occacea e | Streptococcu s | unculture d bacteriu m |
| New.Referen ceOTU114 | 33.2 6 | 3.17 | 0.0 02 | Ba cter ia | Firmi cutes | Bacilli | Lactoba cillales | Streptoc occacea e | Streptococcu s | unculture d bacteriu m |
| AB506154.1 .1541 | 7.45 | 2.91 | 0.0 08 | Ba cter ia | Firmi cutes | Bacilli | Lactoba cillales | Streptoc occacea e | Streptococcu s | unculture d bacteriu m |
| EU774370. 1. 1398 | 2.18 | 3.43 | 0.0 29 | Ba cter ia | Firmi cutes | Bacilli | Lactoba cillales | Streptoc occacea e | Streptococcu s | unculture d bacteriu m |
| HK557089.3 .1395 | 412 3.56 | 2.62 | 0.0 07 | Ba cter ia | Firmi cutes | Bacilli | Lactoba cillales | Streptoc occacea e | Streptococcu s | unculture d bacteriu m |
| HQ807346.1 .1456 | 11.8 8 | 4.56 | 2.3 5E-05 | Ba cter ia | Firmi cutes | Bacilli | Lactoba cillales | Streptoc occacea e | Streptococcu s | unculture d bacteriu m |
| HQ748204.1 .1442 | 15.1 3 | 4.30 | 4.5 8E-05 | Ba cter ia | Firmi cutes | Bacilli | Lactoba cillales | Streptoc occacea e | Streptococcu s | unculture d bacteriu m |
| GU179917.1 .1382 | 29.7 2 | 2.15 | 0.0 44 | Ba cter ia | Firmi cutes | Erysipel otrichia | Erysipe lotricha les | Erysipel otrichac eae | Erysipelatocl ostridium | Ambiguo us_taxa |
| GQ448336.1 .1418 | 49.6 8 | 4.13 | 0.0 14 | Ba cter ia | Firmi cutes | Negativ icutes | Seleno monada les | Veillone llaceae | Megasphaera | unculture d bacteriu m |
| DQ804865.1 .1390 | 32.0 2 | 3.80 | 0.0 30 | Ba cter ia | Firmi cutes | Clostrid ia | Clostrid iales | Lachnos piraceae | NA | NA |
| GQ491757.1 .1361 | 6.02 | 1.79 | 0.0 34 | Ba cter ia | Firmi cutes | Clostrid ia | Clostrid iales | Lachnos piraceae | Blautia | unculture d bacteriu m |
| New.Referen ceOTU56 | 33.7 1 | 5.05 | 0.0 006 04 | Ba cter ia | Bact eroid etes | Bacteroi dia | Bactero idales | Prevotel laceae | Alloprevotell a | Ambiguo us_taxa |
| KF842598.1. 1394 | 4.29 | -4.06 | 0.0 24 | Ba cter ia | Bact eroid etes | Bacteroi dia | Bactero idales | Porphyr omonad aceae | Parabacteroi des | NA |
| HQ802052.1 .1445 | 3.40 | -3.37 | 0.0 10 | Ba cter ia | Bact eroid etes | Bacteroi dia | Bactero idales | Bacteroi daceae | Bacteroides | Ambiguo us_taxa |
| GX182404.8 .1529 | 2.29 | -3.22 | 0.0 35 | Ba cter ia | Prote obact eria | Gamma proteob acteria | Enterob acterial es | Enterob acteriac eae | Escherichia-Shigella | NA |
| FJ950694.1. 1472 | 116 5.27 | -2.81 | 0.0 02 | Ba cter ia | Prote obact eria | Gamma proteob acteria | Enterob acterial es | Enterob acteriac eae | Escherichia-Shigella | Escheric hia coli |
| GQ448506.1 .1374 | 489. 25 | 2.00 | 0.0 11 | Ba cter ia | Firmi cutes | Clostrid ia | Clostrid iales | Lachnos piraceae | Blautia | unculture d bacteriu m |
| HQ802983.1 .1440 | 25.9 5 | -2.62 | 0.0 20 | Ba cter ia | Firmi cutes | Clostrid ia | Clostrid iales | Lachnos piraceae | Tyzzerella 4 | NA |
| DQ793824.1 .1370 | 11.8 4 | -3.29 | 0.0 09 | Ba cter ia | Firmi cutes | Clostrid ia | Clostrid iales | Lachnos piraceae | [Ruminococc us] gauvreauii group | unculture d bacteriu m |
| GQ448468.1 .1366 | 275 6.51 | -2.30 | 0.0 13 | Ba cter ia | Fuso bacte ria | Fusobac teriia | Fusoba cteriale s | Fusobac teriacea e | Fusobacteriu m | unculture d bacteriu m |
| EU774020. 1. 1361 | 2.57 | -3.07 | 0.0 11 | Ba cter ia | Fuso bacte ria | Fusobac teriia | Fusoba cteriale s | Fusobac teriacea e | Fusobacteriu m | unculture d bacteriu m |
| GQ491183.1 .1360 | 618. 96 | 1.51 | 0.0 40 | Ba cter ia | Firmi cutes | Clostrid ia | Clostrid iales | Lachnos piraceae | NA | NA |
| GQ491426.1 .1332 | 506. 28 | 2.55 | 0.0 17 | Ba cter ia | Firmi cutes | Clostrid ia | Clostrid iales | Lachnos piraceae | Blautia | unculture d bacteriu m |
| GQ493039.1 .1311 | 82.9 3 | -3.08 | 0.0 16 | Ba cter ia | Firmi cutes | Clostrid ia | Clostrid iales | Peptostr eptococ caceae | NA | NA |
| JN387556.1. 1324 | 135. 02 | -3.10 | 0.0 10 | Ba cter ia | Firmi cutes | Clostrid ia | Clostrid iales | Peptostr eptococ caceae | Intestinibacte r | NA |
| EU775983.1. 1288 | 2.84 | 2.40 | 0.0 08 | Ba cter ia | Firmi cutes | Clostrid ia | Clostrid iales | Peptostr eptococ caceae | Peptoclostrid ium | unculture d bacteriu m |

**Table 6. OTUs with differential abundances between samples of day 0 and day 14 (day 0 versus day 14) for die non-responsive dogs (Fold change >2 and P-value <0.05).**

| **OTU ID** | **Base Mean** | **log2F oldC hang e** | **P valu e** | **King dom** | **Phyl um** | **Class** | **Order** | **Family** | **Genus** | **Species** |
|---|---|---|---|---|---|---|---|---|---|---|
| GQ44913 7.1.1391 | 461.1 5 | -3.51 | 0.01 87 | Bacte ria | Prote obact eria | Betapr oteoba cteria | Burkh olderia les | Alcalige naceae | Sutterella | NA |
| HK55593 8.1.1357 | 30.11 | -6.18 | 0.01 21 | Bacte ria | Actin obact eria | Coriob acteriia | Coriob acterial es | Corioba cteriace ae | Collinsel la | uncultured bacterium |
| GQ35824 6.1.1466 | 302.4 1 | -3.70 | 0.01 82 | Bacte ria | Firmi cutes | Negati vicutes | Seleno monad ales | Acidam inococc aceae | Phascola rctobacte rium | uncultured Veillonellace ae bacterium |
| New.Refe renceOTU 82 | 61.40 | -3.34 | 0.02 62 | Bacte ria | Firmi cutes | Clostri dia | Clostri diales | Clostrid iaceae 1 | Clostridi um sensu stricto 1 | NA |
| New.Refe renceOTU 52 | 222.7 1 | -4.55 | 0.00 22 | Bacte ria | Firmi cutes | Clostri dia | Clostri diales | Clostrid iaceae 1 | Clostridi um sensu stricto 1 | NA |
| GQ13861 5.1.1402 | 321.5 4 | -3.56 | 0.00 59 | Bacte ria | Firmi cutes | Erysip elotric hia | Erysip elotric hales | Erysipel otrichac eae | Turicibac ter | uncultured bacterium |
| JN681884 .1.1409 | 384.6 8 | 3.09 | 0.00 84 | Bacte ria | Firmi cutes | Bacilli | Lactob acillale s | Streptoc occacea e | Streptoco ccus | NA |
| GU30375 9.1.1517 | 48.18 | 2.96 | 0.01 80 | Bacte ria | Firmi cutes | Bacilli | Lactob acillale s | Streptoc occacea e | Streptoco ccus | uncultured bacterium |
| New.Refe renceOTU 114 | 53.48 | 4.21 | 8.04 E-05 | Bacte ria | Firmi cutes | Bacilli | Lactob acillale s | Streptoc occacea e | Streptoco ccus | uncultured bacterium |
| EU77488 1.1.1422 | 3.84 | 3.39 | 0.02 24 | Bacte ria | Firmi cutes | Bacilli | Lactob acillale s | Streptoc occacea e | Streptoco ccus | uncultured bacterium |
| AB46955 9.1.1551 | 13.56 | 5.00 | 0.00 16 | Bacte ria | Firmi cutes | Bacilli | Lactob acillale s | Streptoc occacea e | Streptoco ccus | uncultured bacterium |
| HK55708 9.3.1395 | 9232. 07 | 4.47 | 2.88 E-05 | Bacte ria | Firmi cutes | Bacilli | Lactob acillale s | Streptoc occacea e | Streptoco ccus | uncultured bacterium |
| EU35871 9.1.1513 | 12.02 | 2.71 | 0.01 80 | Bacte ria | Firmi cutes | Bacilli | Lactob acillale s | Streptoc occacea e | Streptoco ccus | uncultured bacterium |
| HQ74820 4.1.1442 | 17.52 | 2.85 | 0.00 45 | Bacte ria | Firmi cutes | Bacilli | Lactob acillale s | Streptoc occacea e | Streptoco ccus | uncultured bacterium |
| GQ33872 7.1.1397 | 9.95 | 6.38 | 0.03 13 | Bacte ria | Firmi cutes | Clostri dia | Clostri diales | Lachnos piraceae | Anaerost ipes | uncultured bacterium |
| HQ80396 4.1.1435 | 247.4 1 | -2.80 | 0.02 94 | Bacte ria | Firmi cutes | Clostri dia | Clostri diales | Lachnos piraceae | Lachnocl ostridium | uncultured bacterium |
| FJ951866. 1.1493 | 7.83 | -5.45 | 0.01 77 | Bacte ria | Firmi cutes | Clostri dia | Clostri diales | Lachnos piraceae | Roseburi a | NA |
| EU77287 0.1.1289 | 34.63 | -4.27 | 0.00 79 | Bacte ria | Fusob acteri a | Fusoba cteriia | Fusoba cteriale s | Fusobac teriacea e | Fusobact erium | uncultured bacterium |
| GQ44846 8.1.1366 | 4335. 90 | -4.03 | 0.01 25 | Bacte ria | Fusob acteri a | Fusoba cteriia | Fusoba cteriale s | Fusobac teriacea e | Fusobact erium | uncultured bacterium |
| EU77402 0.1.1361 | 7.55 | -4.76 | 0.01 12 | Bacte ria | Fusob acteri a | Fusoba cteriia | Fusoba cteriale s | Fusobac teriacea e | Fusobact erium | uncultured bacterium |
| HQ78265 8.1.1415 | 506.9 2 | -6.12 | 0.00 01 | Bacte ria | Fusob acteri a | Fusoba cteriia | Fusoba cteriale s | Fusobac teriacea e | Fusobact erium | Ambiguous_t axa |
| DQ79463 3.1.1395 | 23.54 | -3.68 | 0.02 09 | Bacte ria | Firmi cutes | Clostri dia | Clostri diales | Lachnos piraceae | NA | NA |
| FN66837 5.4306350 .4307737 | 12.13 | -5.24 | 0.00 16 | Bacte ria | Firmi cutes | Clostri dia | Clostri diales | Peptostr eptococ caceae | NA | NA |
| GQ86744 5.1.1457 | 24.68 | -2.23 | 0.01 50 | Bacte ria | Firmi cutes | Clostri dia | Clostri diales | Lachnos piraceae | NA | NA |

**Table 7. Comparisons of KEGG pathways between different timepoints in the trial for diet responsive dogs.**

| **KEGG pathways** | **Days0vs14_ lfc** | **Days0vs14_pva lue** | **Days0vs14_fdr** |
|---|---|---|---|
| ko00100; Steroid biosynthesis | -2.04 | 0.000209808 | 0.005895615 |
| ko00312; beta-Lactam resistance | 0.44 | 7.25E-05 | 0.005895615 |
| ko00524; Butirosin and neomycin biosynthesis | 0.31 | 0.000164032 | 0.005895615 |
| ko00630; Glyoxylate and dicarboxylate metabolism | -0.39 | 0.000125885 | 0.005895615 |
| ko00910; Nitrogen metabolism | -0.39 | 0.000209808 | 0.005895615 |
| ko03070; Bacterial secretion system | -0.46 | 0.000125885 | 0.005895615 |
| ko04144; Endocytosis | -1.76 | 0.000209808 | 0.005895615 |
| ko04912; GnRH signaling pathway | -1.76 | 0.000209808 | 0.005895615 |
| ko05210; Colorectal cancer | -1.63 | 0.000209808 | 0.005895615 |
| ko05416; Viral myocarditis | -1.63 | 0.000209808 | 0.005895615 |
| ko00640; Propanoate metabolism | -0.25 | 0.000267029 | 0.006821372 |
| ko00010; Glycolysis / Gluconeogenesis | 0.19 | 0.000419617 | 0.006936017 |
| ko00311; Penicillin and cephalosporin biosynthesis | 0.32 | 0.000419617 | 0.006936017 |
| ko00920; Sulfur metabolism | -0.38 | 0.000419617 | 0.006936017 |
| ko04721; Synaptic vesicle cycle | -1.15 | 0.000419617 | 0.006936017 |
| ko04962; Vasopressin-regulated water reabsorption | -1.15 | 0.000419617 | 0.006936017 |
| ko05150; Staphylococcus aureus infection | 0.86 | 0.000335693 | 0.006936017 |
| ko00020; Citrate cycle (TCA cycle) | -0.31 | 0.000644684 | 0.006967545 |
| ko00052; Galactose metabolism | 0.40 | 0.000644684 | 0.006967545 |
| ko00240; Pyrimidine metabolism | 0.12 | 0.000522614 | 0.006967545 |
| ko00410; beta-Alanine metabolism | -0.41 | 0.000644684 | 0.006967545 |
| ko00473; D-Alanine metabolism | 0.29 | 0.000644684 | 0.006967545 |
| ko00592; alpha-Linolenic acid metabolism | -1.38 | 0.000644684 | 0.006967545 |
| ko00633; Nitrotoluene degradation | -0.67 | 0.000644684 | 0.006967545 |
| ko03410; Base excision repair | 0.10 | 0.000522614 | 0.006967545 |
| ko04115; p53 signaling pathway | -1.57 | 0.000522614 | 0.006967545 |
| ko00550; Peptidoglycan biosynthesis | 0.18 | 0.000965118 | 0.008748331 |
| ko00909; Sesquiterpenoid and triterpenoid biosynthesis | -1.52 | 0.000965118 | 0.008748331 |
| ko04621; NOD-like receptor signaling pathway | 0.43 | 0.000965118 | 0.008748331 |
| ko04930; Type II diabetes mellitus | 0.17 | 0.000965118 | 0.008748331 |
| ko05168; Herpes simplex infection | -1.28 | 0.000965118 | 0.008748331 |
| ko00281; Geraniol degradation | -0.58 | 0.001411438 | 0.01166512 |
| ko00540; Lipopolysaccharide biosynthesis | -0.53 | 0.001411438 | 0.01166512 |
| ko04622; RIG-I-like receptor signaling pathway | 0.57 | 0.001411438 | 0.01166512 |
| ko00071; Fatty acid metabolism | -0.55 | 0.001693726 | 0.012863159 |
| ko00120; Primary bile acid biosynthesis | 0.51 | 0.001693726 | 0.012863159 |
| ko00121; Secondary bile acid biosynthesis | 0.51 | 0.001693726 | 0.012863159 |
| ko00430; Taurine and hypotaurine metabolism | -0.32 | 0.00202179 | 0.013856655 |
| ko00590; Arachidonic acid metabolism | -0.39 | 0.00202179 | 0.013856655 |
| ko05012; Parkinsons disease | -0.60 | 0.00202179 | 0.013856655 |
| ko05111; Vibrio cholerae pathogenic cycle | -0.58 | 0.00202179 | 0.013856655 |
| ko00051; Fructose and mannose metabolism | 0.18 | 0.002399445 | 0.014046748 |
| ko00310; Lysine degradation | -0.27 | 0.002399445 | 0.014046748 |
| ko00351; DDT degradation | -0.89 | 0.002399445 | 0.014046748 |
| ko00520; Amino sugar and nucleotide sugar metabolism | 0.14 | 0.002399445 | 0.014046748 |
| ko00561; Glycerolipid metabolism | 0.27 | 0.002399445 | 0.014046748 |
| ko01040; Biosynthesis of unsaturated fatty acids | -0.32 | 0.002399445 | 0.014046748 |
| ko04011; MAPK signaling pathway - yeast | 0.27 | 0.002399445 | 0.014046748 |
| ko00130; Ubiquinone and other terpenoid-quinone biosynthesis | -0.38 | 0.002838135 | 0.014241355 |
| ko00380; Tryptophan metabolism | -0.58 | 0.002838135 | 0.014241355 |
| ko00680; Methane metabolism | -0.15 | 0.002838135 | 0.014241355 |
| ko02060; Phosphotransferase system (PTS) | 0.52 | 0.002838135 | 0.014241355 |
| ko04626; Plant-pathogen interaction | -0.09 | 0.002838135 | 0.014241355 |
| ko04940; Type I diabetes mellitus | 0.09 | 0.002838135 | 0.014241355 |
| ko05110; Vibrio cholerae infection | -1.3 | 0.002838135 | 0.014241355 |
| ko05145; Toxoplasmosis | -1.25 | 0.002838135 | 0.014241355 |
| ko00300; Lysine biosynthesis | 0.07 | 0.003341675 | 0.015915434 |
| ko02040; Flagellar assembly | -0.63 | 0.003341675 | 0.015915434 |
| ko04973; Carbohydrate digestion and absorption | 0.46 | 0.003341675 | 0.015915434 |
| ko05340; Primary immunodeficiency | 0.29 | 0.003917694 | 0.018347867 |
| ko00511; Other glycan degradation | 0.45 | 0.004577637 | 0.020098686 |
| ko00791; Atrazine degradation | -0.24 | 0.004577637 | 0.020098686 |
| ko00983; Drug metabolism - other enzymes | 0.14 | 0.004577637 | 0.020098686 |
| ko03430; Mismatch repair | 0.13 | 0.004577637 | 0.020098686 |
| ko00230; Purine metabolism | 0.08 | 0.005329132 | 0.022689184 |
| ko04260; Cardiac muscle contraction | -0.76 | 0.005329132 | 0.022689184 |
| ko00620; Pyruvate metabolism | -0.06 | 0.00617981 | 0.025918306 |
| ko00190; Oxidative phosphorylation | -0.11 | 0.007144928 | 0.028277814 |
| ko00330; Arginine and proline metabolism | -0.13 | 0.007144928 | 0.028277814 |
| ko00943; Isoflavonoid biosynthesis | 0.55 | 0.007144928 | 0.028277814 |
| ko04614; Renin-angiotensin system | 0.52 | 0.007144928 | 0.028277814 |
| ko00062; Fatty acid elongation | 0.43 | 0.008232117 | 0.030437168 |
| ko02020; Two-component system | -0.19 | 0.008232117 | 0.030437168 |
| ko03008; Ribosome biogenesis in eukaryotes | -0.22 | 0.008232117 | 0.030437168 |
| ko04122; Sulfur relay system | -0.23 | 0.008232117 | 0.030437168 |
| ko04910; Insulin signaling pathway | 0.22 | 0.008232117 | 0.030437168 |
| ko00471; D-Glutamine and D-glutamate metabolism | 0.15 | 0.00945282 | 0.033623321 |
| ko00500; Starch and sucrose metabolism | 0.20 | 0.00945282 | 0.033623321 |
| ko00860; Porphyrin and chlorophyll metabolism | -0.24 | 0.00945282 | 0.033623321 |
| ko05132; Salmonella infection | -0.33 | 0.010826111 | 0.038026714 |
| ko00603; Glycosphingolipid biosynthesis - globo series | 0.37 | 0.012359619 | 0.041844012 |
| ko03030; DNA replication | 0.08 | 0.012359619 | 0.041844012 |
| ko05142; Chagas disease (American trypanosomiasis) | -0.63 | 0.012359619 | 0.041844012 |
| ko00720; Carbon fixation pathways in prokaryotes | -0.12 | 0.014068604 | 0.045968344 |
| ko01057; Biosynthesis of type II polyketide products | -0.50 | 0.014068604 | 0.045968344 |
| ko04146; Peroxisome | -0.21 | 0.014068604 | 0.045968344 |

**Table 8. Comparisons of bile acids between samples at different timepoints for diet responsive dogs.**

| Bile acid | Days0vs14_fc | Days0vs14_pval ue | Days0vs42_fc | Days0vs42_pvalue |
|---|---|---|---|---|
| AlphamuricholicAcid | 1.74 | 0.192517572 | 2.91 | 0.006835938 |
| DeoxycholicAcid | 1.74 | 0.019058892 | 1.70 | 0.1015625 |
| GammamuricholicAcid | 16.18 | 0.024390241 | 18.33 | 0.014266187 |
| LithocholicAcid | 1.50 | 0.053710938 | 2.02 | 0.010826921 |
| OmegamuricholicAcid | 8.22 | 0.022494271 | 33.55 | 0.042315275 |

**Table 9. Spearman correlations between abundance of OTUs and concentration of Bile acids in diet responsive dogs.**

| **Taxa** | **Bile acid** | **Spearman correlatio n** | **Pvalue** | **Adjusted Pvalue** |
|---|---|---|---|---|
| Fusobacterium_uncultured.bacterium | Chenodeoxycholic. Acid _primary | - 0.4680450 36 | 0.00053 3 | 0.02204240 5 |
| Bacteroides_NA | Chenodeoxycholic. Acid _primary | - 0.4744310 34 | 0.00043 6 | 0.02204240 5 |
| Fusobacterium_uncultured.bacterium | Cholic.Acid_primary | - 0.6468425 27 | 3.11E-08 | 3.86E-06 |
| Fusobacterium_Ambiguous_taxa | Cholic.Acid_primary | - 0.6074896 4 | 3.36E-07 | 2.09E-05 |
| Bacteroides_NA | Cholic.Acid_primary | - 0.5925650 32 | 7.64E-07 | 2.37E-05 |
| Peptoclostridium_uncultured.bacterium | Cholic.Acid_primary | - 0.5617381 93 | 3.67E-06 | 9.11E-05 |
| Megamonas_uncultured.bacterium | Cholic Acid_primary | - 0.5186559 46 | 2.57E-05 | 0.000532 |
| Bacteroides_uncultured.bacterium | Cholic.Acid_primary | - 0.4992762 41 | 5.69E-05 | 0.00100767 4 |
| Prevotella.9_uncultured.bacterium | Cholic.Acid_primary | -0.4938089 | 7.05E-05 | 0.00109338 1 |
| Escherichia.Shigella Escherichia.coli | Cholic.Acid_primary | 0.4877971 89 | 8.90E-05 | 0.00122617 7 |
| Sutterella_NA | Cholic.Acid_primary | - 0.4565076 09 | 0.00027 9 | 0.00345892 5 |
| Staphylococcus _Ambiguous_taxa | Cholic.Acid_primary | 0.4181062 28 | 0.00098 4 | 0.01108803 |
| Escherichia.Shigella_uncultured.bacterium | Cholic.Acid_primary | 0.4073204 92 | 0.00136 6 | 0.01411137 3 |
| Enterococcus_Enterococcus.durans | Cholic.Acid_primary | 0.3944912 05 | 0.00199 | 0.01897972 7 |
| Fusobacterium_uncultured.organism | Cholic.Acid_primary | - 0.3916293 35 | 0.00216 | 0.01912951 5 |
| Faecalibacterium_uncultured.bacterium | Cholic.Acid_primary | - 0.3829826 02 | 0.00275 5 | 0.02277260 9 |
| Clostridium.sensu.stricto.1_NA | Cholic.Acid_primary | 0.3651581 78 | 0.00445 9 | 0.03071782 7 |
| Phascolarctobacterium_uncultured.Veillonell aceae.bacterium | Cholic.Acid_primary | - 0.3651561 22 | 0.00445 9 | 0.03071782 7 |
| Erysipelatoclostridium_NA | Cholic.Acid_primary | 0.3693630 09 | 0.00398 9 | 0.03071782 7 |
| Lactobacillus_uncultured.bacterium | Cholic.Acid_primary | 0.3626514 94 | 0.00476 1 | 0.03074124 8 |
| Enterobacter_NA | Cholic.Acid_primary | 0.3610920 59 | 0.00495 8 | 0.03074124 8 |
| Fusobacterium _Ambiguous_taxa | Deoxycholic. Acid | 0.5442708 84 | 5.29E-05 | 0.00439602 |
| uncultured.bacterium_uncultured.bacterium | Deoxycholic. Acid | 0.5364548 36 | 7.09E-05 | 0.00439602 |
| Enterococcus_NA | Glycochenodeoxycholic Acid | - 0.5513703 53 | 0.00027 5 | 0.03407158 1 |
| Fusobacterium_uncultured.bacterium | Glycocholic. Acid | -0.4919575 | 0.00038 3 | 0.04752498 9 |
| Escherichia.Shigella Escherichia.coli | Glycodeoxycholic.Acid | - 0.5000108 73 | 0.00016 | 0.00663291 4 |
| Escherichia.Shigella_uncultured.bacterium | Glycodeoxycholic.Acid | - 0.5036782 78 | 0.00014 1 | 0.00663291 4 |
| Escherichia.Shigella_NA | Glycodeoxycholic.Acid | - 0.5136984 49 | 9.83E-05 | 0.00663291 4 |
| Bacteroides_NA | Lithocholic. Acid | 0.6022167 45 | 1.21E-05 | 0.00149501 1 |
| Escherichia.Shigella Escherichia.coli | Lithocholic. Acid | - 0.5051369 52 | 0.00040 2 | 0.01214716 |
| Clostridium.sensu.stricto.1_uncultured.bacter ium | Lithocholic. Acid | - 0.4986443 83 | 0.00049 | 0.01214716 |
| Alloprevotella_NA | Lithocholic. Acid | 0.5256513 52 | 0.00020 9 | 0.01214716 |
| Fusobacterium_uncultured.bacterium | Lithocholic. Acid | 0.4655440 21 | 0.00127 | 0.02250386 6 |
| Terrisporobacter_uncultured.bacterium | Lithocholic. Acid | - 0.4689023 52 | 0.00115 8 | 0.02250386 6 |
| Fusobacterium _Ambiguous_taxa | Taurocholic. Acid | - 0.5970782 23 | 9.46E-07 | 0.00011732 2 |
| Bacteroides_uncultured.bacterium | Taurocholic. Acid | - 0.4784149 77 | 0.00016 7 | 0.00690818 1 |
| Fusobacterium_uncultured.bacterium | Taurocholic. Acid | - 0.4674250 93 | 0.00024 7 | 0.00764160 2 |
| Peptoclostridium_uncultured.bacterium | Taurocholic. Acid | - 0.4473670 36 | 0.00048 5 | 0.01202289 2 |
| Prevotella.9_uncultured.bacterium | Taurocholic. Acid | - 0.4321733 93 | 0.00078 8 | 0.01501759 2 |
| Catenibacterium_uncultured.bacterium | Taurocholic. Acid | - 0.4298120 58 | 0.00084 8 | 0.01501759 2 |
| Bacteroides_NA | Taurocholic. Acid | - 0.4193080 54 | 0.00116 8 | 0.01810278 9 |
| Megamonas_uncultured.bacterium | Taurocholic. Acid | - 0.3953182 61 | 0.00233 9 | 0.03221999 7 |
| Sutterella_NA | Taurocholic. Acid | - 0.3793226 34 | 0.00361 4 | 0.04481940 3 |
| Parasutterella_uncultured.organism | Taurocholic. Acid | 0.3722514 42 | 0.00435 2 | 0.04906268 9 |
| Terrisporobacter_uncultured.bacterium | Taurolithocholic. Acid | - 0.4728739 3 | 0.00010 4 | 0.01289612 6 |
| Escherichia. Shigella NA | Taurolithocholic. Acid | - 0.4080859 79 | 0.00099 3 | 0.04607011 7 |
| Prevotellaceae.UCG.003_uncultured.bacteriu m | Taurolithocholic. Acid | - 0.4043972 84 | 0.00111 5 | 0.04607011 7 |

**Table 10. Comparison of the amount of bile acids in the fecal samples of healthy dogs, diet responsive dogs with CCE and diet non-responsive dogs with CCE.**

| **Group** | **Timepoi nt** | **Bile Acid** | **Mean (nmol/g)** | **CI lower** | **CI upper** | **Std. Error** | **(mg/g)** | **#%** |
|---|---|---|---|---|---|---|---|---|
| DR | 0 | Deoxycholi c | 753.4492 | 385.5109 | 1121.3876 | 167.16 98 | 0.29578305 9 | 0.0295 78 |
| DR | 14 | Deoxycholi c | 1232.6501 | 787.5515 | 1677.7488 | 209.96 16 | 0.48390391 5 | 0.0483 9 |
| DR | 42 | Deoxycholi c | 1229.1665 | 663.6408 | 1794.6923 | 266.76 93 | 0.48253635 1 | 0.0482 54 |
| NDR | 0 | Deoxycholi c | 487.2588 | - 186.5451 | 1161.0627 | 242.68 58 | 0.19128416 2 | 0.0191 28 |
| NDR | 14 | Deoxycholi c | 736.7662 | - 124.4111 | 1597.9436 | 335.01 26 | 0.28923378 1 | 0.0289 23 |
| NDR | 42 | Deoxycholi c | 33.16975 | - 57.53601 | 123.87551 | 32.669 75 | 0.01302151 5 | 0.0013 02 |
| Health y | | Deoxycholi c | 2328.5853 | 1519.088 3 | 3138.0823 | 357.84 29 | 0.91413738 8 | 0.0914 14 |
| DR | 0 | Lithocholic | 132.0341 | 46.31136 | 217.75684 | 38.947 44 | 0.04972042 4 | 0.0049 72 |
| DR | 14 | Lithocholic | 209.8332 | 120.1777 8 | 299.48862 | 42.292 18 | 0.07901743 4 | 0.0079 02 |
| DR | 42 | Lithocholic | 266.74216 | 153.5088 6 | 379.97547 | 53.414 32 | 0.10044778 9 | 0.0100 45 |
| NDR | 0 | Lithocholic | 56.86471 | - 39.08019 | 152.80961 | 34.556 74 | 0.02141369 2 | 0.0021 41 |
| NDR | 14 | Lithocholic | 74.4107 | - 46.58682 | 195.40822 | 47.070 09 | 0.02802103 1 | 0.0028 02 |
| NDR | 42 | Lithocholic | 1.718967 | - 1.665428 | 5.103362 | 1.2189 67 | 0.00064731 6 | 6.47E-05 |
| Health y | | Lithocholic | 601.7535 | 314.6058 | 888.9013 | 126.93 54 | 0.22660388 | 0.0226 6 |

| | | | | | | | | |
|---|---|---|---|---|---|---|---|---|
| Note: The values <1nmol/g were under the limit of detection; so an appropriate value 0.5 was used for the calculation. | | | | | | | | |

**Table 11. 16S rRNA Sequences of OTUs in Tables 2-5**

| **OTUs in Table 2** |
|---|
| JRPJ01000002.1034290.1035971 |
| AGAGTTTGATCCTGGCTCAGAGTGAACGCTGGCGGCGTGCCTAATACATGCAAGTCGAACGATGAAACTTCTAGCT TGCTAGAAGTGGATTAGTGGCGCACGGGTGAGTAATGCATAGGTAACATGCCCTTTAGTCTGGGATAGCCACTGGA AACGGTGATTAATACTGGATACTCCCTACGGGGGAAAGGGGCTTTCAATAAAGAATTTCTCTTTTTAGTGTTTTGT GTTGTTGGCACAAAATTCTAGTATTTGGAATGAGAAATTGGTGTTGTGAAGCAATTTGTGCGGAGATTAGACTTAG TGTCTGTCGTGTCAGCAAATTGCGAACTCATCGATTTATCATCCAAAGACGAATTTTTTATTGAAAGCCTTCGCTA AAGGATTGGCCTATGTCCTATCAGCTTGTTGGTGAGGTAATGGCTCACCAAGGCTATGACGGGTATCCGGCCTGAG AGGGTGATCGGACACACTGGAACTGAGACACGGTCCAGACTCCTACGGGAGGCAGCAGTAGGGAATATTGCTCAAT GGGGGAAACCCTGAAGCAGCAACGCCGCGTGGAGGATGAAGGTTTTAGGATTGTAAACTCCTTTTGTAAGAGAAGA TTATGACGGTATCTTACGAATAAGCACCGGCTAACTCCGTGCCAGCAGCCGCGGTAATACGGAGGGTGCAAGCGTT ACTCGGAATCACTGGGCGTAAAGAGCGCGTAGGCGGGTGGTCAAGTCAGATGTGAAATCCTGTAGCTTAACTACAG AACTGCATTTGAAACTGACCATCTAGAGTATGGGAGAGGTAGGTGGAATTCTTGGTGTAGGGGTAAAATCCGTAGA GATCAAGAGGAATACTCATTGCGAAGGCGACCTGCTGGAACATTACTGACGCTGATGCGCGAAAGCGTGGGGAGCA AACAGGATTAGATACCCTGGTAGTCCACGCCCTAAACGATGAATGCTAGTTGTTGTGAGGCTTGTCCTTGCAGTAA TGCAGCTAACGCATTAAGCATTCCGCCTGGGGAGTACGGTCGCAAGATTAAAACTCAAAGGAATAGACGGGGACCC GCACAAGCGGTGGAGCATGTGGTTTAATTCGATGATACGCGAAGAACCTTACCTAGGCTTGACATTGATAGAATCT ACTAGAGATAGTGGAGTGCCCTTTTAGGGAGCTTGAAAACAGGTGCTGCACGGCTGTCGTCAGCTCGTGTCGTGAG ATGTTGGGTTAAGTCCCGCAACGAGCGCAACCCTCGTCCTTAGTTGCTAGCAGTTTGGCTGAGCACTCTAAGGAGA CTGCCTTCGTAAGGAGGAGGAAGGTGAGGACGACGTCAAGTCATCATGGCCCTTACGCCTAGGGCTACACACGTGC TACAATGGGGTGCACAAAGAGATGCAATAGTGTGAGCTGGAGCCAATCTCTAAAACATCTCTCAGTTCGGATTGTA GTCTGCAACTCGACTACATGAAGCTGGAATCGCTAGTAATCGCAAATCAGCAATGTTGCGGTGAATACGTTCCCGG GTCTTGTACTCACCGCCCGTCACACCATGGGAGTTGTATTTGCCTTAAGTCGGAATGCTAAATTGGCTACCGCCCA CGGCAGATGCAGCGACTGGGGTGAAGTCGTAACAAGGTAACCGTAGGTGAACCTGCGGTTG |
| JF920309.1.1340 |
| AGTGAACGCTGGCGGCGTGCCTAATACATGCAAGTCGAACGATGAAGCTTCTAGCTTGCTAGAAGTGGATTAGTGG CGCACGGGTGAGTAAGGTATAGTTAATCTGCCCTACACAAGAGGACAACACCTAGAAATGGGTGCTAATACTCTAT ACTCCTGCTTAACACAAGTTGAGTAGGGAAAGTTTTTCGGTGTAGGATGAGACTATATAGTATCAGCTAGTTGGTA AGGTAAAGGCTTACCAAGGCTATGACGCTTAAGAGGTCTGAGAGGATGATCTCTCACACTGGAACTGAGACACGGT CCAGACTCCTACGGGAGGCAGCAGTAGGGAATATTGCGCAATGGGCGAAAGCCTGACGCAGCAACGCCGCGTGGAG GATGACACTTTTAGGAGCGTAAACTCCTTTTCTTAGGGAAGAATTCTGACGGTACCTAAGGAATAAGCACCGGCTA ACTCCGTGCCAGCAGCCGCGGTAATACGGAGGGTGCAAGCGTTACTCGGAATCACTGGGCGTAAAGGGCGCGTAGG CGGATTATCAAGTCTCTTGTGAAATCTAATGGCTTAACCATTAAACTGCTTGGGAAACTGATAGTCTAGAGTGAGG GAGAGGCAGATGGAATTGGTGGTGTAGGGGTAAAATCCGTAGATATCACCAAGAATACCCATTGCGAAGGCGATCT GCTGGAACTCAACTGACGCTAAGGCGCGAAAGCGTGGGGAGCAAACAGGATTAGATACCCTGGTAGTCCACGCCCT AAACGATGTATGCTAGTTGTTGGGCTGCTAGTCAGCTCAGTAATGCAGCTAACGCATTAAGCATACCGCCTGGGGA GTACGGTCGCAAGATTAAAACTCAAAGGAATAGACGGGGACCCGCACAAGCGGTGGAGCATGTGGTTTAATTCGAA GATACGCGAAGAACCTTACCTAGGCTTGATATCCAACAAAGCTTCTAGAGATAGAAGTGTGCTAGCTTGCTAGAAT GTTGAGACAGGTGCTGCACGGCTGTCGTCAGCTCGTGTCGTGAGATGTTGGGTTAAGTCCCGCAACGAGCGCAACC CACGTATTTAGTTGCTAACACTTCGGGTGAGCACTCTAAATAGACTGCCTTCGTAAGGAGGAGGAAGGTGTGGACG ACGTCAAGTCATCATGGCCCTTATGCCTAGGGCGACACACGTGCTACAATGGCATATACAATGAGACGCAATACCG CGAGGTGGAGCAAATCTATAAAATATGTCCCAGTTCGGATTGTTCTCTGCAACTCGAGAGCATGAAGCCGGAATCG CTAGTAATCGCAAATCAGCCATGTTGCGGTGAATACGTTCCCGGGTCT |
| FJ978526.1.1378 |
| CATGCAAGTCGAACGGTAACATAGAGGAAGCTTGCTTTCTCTGATGACGAGTGGCGGACGGGTGAGTAAGGTCTGG GAAACTGCCTGACAGAGGGGGACAACAACTGGAAACGGTTGCTAATACCGCATACACCCTGAGGGGGAAAGTCGAA AGACGCTGTCAGATGTGCCCAGATGGGATTAGCTAGTAGGTGAGGTAAAGGCTCACCTAGGCGACGATCTCTAGCT GGTCTGAGAGGATGATCAGCCACATTGGGACTGAGACACGGCCCAGACTCCTACGGGAGGCAGCAGTAGGGAATAT TGCACAATGGGGGGAACCCTGATGCAGCCATGCCGCGTGTGTGAAGAAGGCCTTCGGGTTGTAAAGCACTTTCAGA GGGGAGGAAAATGACGTTACCCTCAGAAGAAGCACCGGCTAACTCCGTGCCAGCAGCCGCGGTAATACGGAGGGTG CAAGCGTTAATCGGAATAACTGGGCGTAAAGGGCATGCAGGCGGTTCTGCAAGTAGGGTGTGAAAGCCCGGGCTC AACCTCGGAATTGCACTCTAAACTGTGGGACTAGAGTATTGCAGGGGGAGACGGAATTCCAGGTGTAGCGGTGGAA TGCGTAGAGATCTGGAAGAACACCAAAGGCGAAGGCAGTCTCCTGGGCAAATACTGACGCTCATATGCGAAAGCGT GGGTAGCAAACAGGATTAGATACCCTGGTAGTCCACGCCGTAAACGATGTTGATTAGAAGCTTGCTTGTAAGAGTG GGTTTCGCAGCTAACGCGATAAATCAACCGCCTGGGGAGTACGGCCGCAAGGTTAAAACTCAAATGAATTGACGGG GGCCCGCACAAGCGGTGGAGCATGTGGTTTAATTCGACGCAACGCGATGAACCTTACCTGATCTTGACATCGCGAG AATTACTTGTAATGAGTAAGTGCCTTCGGGAACTCGCAGACAGGTGCTGCATGGCTGTCGTCAGCTCGTGTCGTGA GATGTTGGGTTAAGTCCCGCAACGAGCGCAACCCTTGTCCTTTGTTGCCAGCGGGTAGAGCCGGGAACTCAAAGGA GACTGCCAGTGATAAACTGGAGGAAGGTAGGGATGACGTCAAGTCATCATGGCCCTTACGGTCAGGGCTACACACG TGCTACAATGGGGCGTACAGAGGGAAACGAAACTGCGAGGTGGAGTGGAACCCAGAAAGCGTCCCTAAGTTCGGAT |
| TGGAGTCTGCAACTCGACTCCATGAAGTCGGAATCGCTAGTAATCGCAAATCAGAATGTTGCGGTGAATACGTTCC CGGGCCTTGTACACACCGCCCGTCACACCATGGGAGTGGATTGCACCAGAAGTGGCCAGCCTAACTGCAAAGAGGG CGGTACCACG |
| New.ReferenceOTU45 |
| TACGGAGGGTGCAAGCGTTAATCGGAATAACTGGGCGTAAAGGGCATGTAGGCGGAAAGGCAAGCAAGATGTGAAA GACCTGGGCTCAACCTGGGTTGGTCATTTTGAACTACCTTTCTAGAGTATTGCAGAGGGAGATGGAATTTCAGGTG TAGCGGTGGAATGCGTAGATATCTGAAAGAACACCAGAGGCGAAGGCGGTCTCCTGGGCAAATACTGACGCTGAGG TGCGAAAGCGTGGGGAGCAAACAGG |
| HK555938.1.1357 |
| ACGGCACCCCTCTCCGGAGGGAAGCGAGTGGCGAACGGCTGAGTAACACGTGGAGAACCTGCCCCCTCCCCCGGGA TAGCCGCCCGAAAGGACGGGTAATACCGGATACCCCCGGGCGCCGCATGGCGCCCGGGCTAAAGCCCCGACGGGAG GGGATGGCTCCGCGGCCCATCAGGTAGACGGCGGGGTGACGGCCCACCGTGCCGACAACGGGTAGCCGGGTTGAGA GACCGACCGGCCAGATTGGGACTGAGACACGGCCCAGACTCCTACGGGAGGCAGCAGTGGGGAATCTTGCGCAATG GGGGGAACCCTGACGCAGCGACGCCGCGTGCGGGACGGAGGCCTTCGGGTCGTAAACCGCTTTCAGCAGGGAAGAG TCAAGACTGTACCTGCAGAAGAAGCCCCGGCTAACTACGTGCCAGCAGCCGCGGTAATACGTAGGGGGCGAGCGTT ATCCGGATTCATTGGGCGTAAAGCGCGCGTAGGCGGCCCGGCAGGCCGGGGGTCGAAGCGGGGGGCTCAACCCCCC GAAGCCCCCGGAACCTCCGCGGCTTGGGTCCGGTAGGGGAGGGTGGAACACCCGGTGTAGCGGTGGAATGCGCAGA TATCGGGTGGAACACCGGTGGCGAAGGCGGCCCTCTGGGCCGAGACCGACGCTGAGGCGCGAAAGCTGGGGGAGCG AACAGGATTAGATACCCTGGTAGTCCCAGCCGTAAACGATGGACGCTGGGTGTGGGGGGACGATCCCCCCGTGCCG CAGCCNACGCATTAAGCGTCCCGCCTGGGGAGTACGGCCGCAAGGCTAAAACTCAAAGGAATTGACGGGGGCCCGC ACAAGCAGCGGAGCATGTGGCTTAATTCGAAGCAACGCGAAGAACCTTACGGCGCATCCCCCCGAGGCCCACGGGG GGTCCGCCGCGTGGGTCAGAGGAGCGCATACGGGAGGTGCATGGTTGTCGTCAGCTCGTGTCGTGAGATGTTGGGT TAAGTCCCGCAACGAGCGCAACCCCCGCCGCGTGTTGCCATCGGGTGATGCCGGAACCCACGCGGACCGCCGCC GTCAAGGCGGAGGAGGGCGGGGACGACGTCAAGTCATCATGCCCCTTATGCCCTGGGCTGCACACGTGCTACAATG GCCGGTACAGAGGGATGCCACCCCGCGAGGGGGAGCGGATCCCGGAAAGCCGGCCCCAGTTCGGATTGGGGGCTGC AACCCGCCCCCATGAAGTCGGAGTTGCTAGTAATCGCGGATCAGCATGCCGCGGTGAATGCGTTCCCGGGCCTTGT ACACACCGCCCGTCACACCACCCGAGTCGTCTGCACCCGAAGTCGCCGGCCCAACCGCAAGGGGG |
| FJ957494.1.1454 |
| TGAGTTTGATCATGGCTCAGGACGAACGCTGGCGGCGTGCCTAACACATGCAAGTCGAGCGATGAAATTTTCTTCG GAAAATGGATTAGCGGCGGACGGGTGAGTAACACGTGGGTAACCTGCCCTATAGAGAGGGATAGCCTTCCGAAAGG GAGATTAATACCTCATAATATCCTAGTATCGCATGATACATGGATTAAAGGAGCAATCCGCTATAGGATGGACCCG CGGCGCATTAGCTAGTTGGTGAGGTAACGGCTCACCAAGGCGACGATGCGTAGCCGACCTGAGAGGGTGATCGGCC ACATTGGGACTGAGACACGGCCCAGACTCCTACGGGAGGCAGCAGTGGGGAATATTGCACAATGGGGGAAACCCTG ATGCAGCAACGCCGCGTGAGTGATGACGGTCTTCGGATTGTAAAGCTCTGTCTTTAGGGACGATAATGACGGTACC TAAGGAGGAAGCCACGGCTAACTACGTGCCAGCAGCCGCGGTAATACGTAGGTGGCAAGCGTTGTCCGGATTTACT GGGCGTAAAGGGAGCGTAGGCGGATCTTTAAGTGGGATGTGAAATACTCGGGCTCAACCTGGGGGCTGCATTCCAA ACTGGGGATCTAGAGTACAGGAGGGGNGAGTGGAATTCCTAGTGTAGCGGTGAAATGCGTAGAGATTAGGAAGAAC ACCAGTGGCGAAGGCGACTNTCTGGACTGTAACTGACGCTGAGGCTCGAAAGCGTGGGGAGCAAACAGGATTAGAT ACCCTGGTAGTCCACGCCGTAAACGATGAATACTAGGTGTAGGGGGTGTCAACTCCCCCTGTGCCGCCGCTAACGC ATTAAGTATTCCGCCTGGGGAGTACGGTCGCAAGATTAAAACTCAAAGGAATTGACGGGGGCCCGCACAAGTAGCG GAGCATGTGGTTTAATTCGACGCAACGCGAAGAACCTTACCTAGACTTGACATCTTCTGCATTACCCTTAATCGGG GAAGTTCCTTCGGGGACAGAATGACAGGTGGTGCATGGTTGTCGTCAGCTCGTGTCGTGAGATGTTGGGTTAAGTC CCGCAACGAGCGCAACCCTTAAGCTTAGTTGCCATCATTAAGTTGGGCACTCTAAGTTGACTGCCGGTGACAAACC GGAGGAAGGTGGGGATGACGTCAAATCATCATGCCCCTTATGTCTAGGGCTACACACGTGCTACAATGGCAAGTAC AAAGAGAAGCAATACTGTGAAGTGGAGCAAAACTCAAAAACTTGTCTCAGTTCGGATTGTAGGCTGAAACTCGCCT ACATGAAGCTGGAGTTGCTAGTAATCGCGAATCAGAATGTCGCGGTGAATACGTTCCCGGGTCTTGTACACACCGC CCGTCACACCATGAGAGTTGGCAATACCCGAAGTCCGTAAGCTAACCGTAAGGAGGCAGCGGCCGAAGGTAGGGTC AGCGATGGGG |
| New.ReferenceOTU52 |
| TACGTAGGTGGCGAGCGTTATCCGGATTTACTGGGCGTAAAGGGAGCGTAGGCGGATGATTAAGTGGGATGTGAAA TACCCGGGCTCAACTTGGGTGCTGCATTCCAAACTGGTTATCTAGAGTGCAGGAGAGGAGAGTGGAATTCCTAGTG TAGCGGTGAAATGCGTAGAGATTAGGAAGAACACCAGTGGCGAAGGCGACTCTCTGGACTGTAACTGACGCTGAGG CTCGAAAGCGTGGGGAGCAAACAGG |
| DQ797046.1.1403 |
| AGAGTTTGATCCTGGCTCAGGACGAACGCTGGCGGCATGCTTAACACATGCAAGTCGAACGGACTGATTCCTTCGG GATGAAAGTTAGTGGCGAACGGGTGAGTAATGTATGAGCAACCTGCCTCTGTCAACGGGATAACAGTTGGAAACGA CTGCTAATACGGTATATGACCACGGCACCGCATGGTGCAGCGGTAAAAGATTTATCGGACAGAGATGGGCTCATA TCCCATTAGGTAGTTGGTGAGATAACAGCCCACCAAGCCGACGATCAGTAGCCGGTCTGAGAGGATGAACGGCCAC ACTGGAACTGAGACACGGTCCAGACTCCTACGGGAGGCAGCAGTGGGGAATCTTCCGCAATGGACGAAAGTCTGAC GGAGCAACGCCGCGTGAACGATGAAGGTCTTCGGATTGTAAAGTTCTGTGATCCGGGACGAAGGCATTGATTGAGA ACATTGATTGATGTTGACGGTACCGGAAAAGCAAGCCACGGCTAACTACGTGCCAGCAGCCGCGGTAATACGTAGG |
| TGGCAAGCGTTGTCCGGAATTATTGGGCGTAAAGCGCGCGCAGGCGGCCGTGCAAGTCCATCTTAAAAGCGTGGGG CTTAACCCCATGAGGGGATGGAAACTGCAGGGCTGGAGTGTCGGAGGGGAAAGTGGAATTCCTAGTGTAGCGGTGA AATGCGTAGAGATTAGGAAGAACACCGGTGGCGAAGGCGACTTTCTAGACGACAACTGACGCTGAGGCGCGAAAGC GTGGGGAGCAAACAGGATTAGATACCCTGGTAGTCCACGCCGTAAACGATGGATACTAGGTGTAGGAGGTATCGAC CCCTTCTGTGCCGGAGTTAACGCAATAAGTATCCCGCCTGGGAAGTACGATCGCAAGATTAAAACTCAAAGGAATT GACGGGGGCCCGCACAAGCGGTGGAGTATGTGGTTTAATTCGACGCAACGCGAAGAACCTTACCAAGCCTTGACAT TGATCGCAATCCGCAGAAATGCGGAGTTCCTCTTCGGAGGACGAGAAAACAGGTGGTGCACGGCTGTCGTCAGCTC GTGTCGTGAGATGTTGGGTTAAGTCCCGCAACGAGCGCAACCCCTATCTTCTGTTGCCAGCACGTAAAGGTGGGAA CTCAGGAGAGACCGCCGCGGACAACGCGGAGGAAGGCGGGGATGACGTCAAGTCATCATGCCCCTTATGGCTTGGG CTACACACGTACTACAATGGGTGCAAACAAAGAGAAGCGAAGTCGCGAGATGGAGCGGACCTCATAAACGCACTCC CAGTTCAGATTGCAGGCTGCAACCCGCCTGCATGAAGTAGGAATCGCTAGTAATCGCGGGTCAGCATACCGCGGTG AATACGTTCCCGGGCCTTGTACACACCGCCCGTCA |
| GQ449092.1.1375 |
| CTGGCTCAGGATGAACGCTGGCGGCGTGCTTAACACATGCAAGTCGAACGAAGAGGTTAGAATGAGAGCTTCGGC AGGATTTCTTTCCATCTTAGTGGCGGACGGGTGAGTAACGTGTGGGCAACCTGCCCTGTACTGGGGGATAATCATT GGAAACGATGACTAATACCGCATGTGGTTCTCGGAAGGCATCTTCTGAGGAAGAAAGGATTTATTCGGTACAGGAT GGGCCCGCATCTGATTAGCTAGTTGGTGAGATAACAGCCCACCAAGGCGACGATCAGTAGCCGACCTGAGAGGGTG ATCGGCCACATTGGGACTGAGACACGGCCCAAACTCCTACGGGAGGCAGCAGTGGGGAATATTGCACAATGGGCGA AAGCCTGATGCAGCAACGCCGCGTGAAGGATGAAGGGTTTCGGCTCGTAAACTTCTATCAATAGGGAAGAAACAAA TGACGGTACCTAAATAAGAAGCCCCGGCTAACTACGTGCCAGCAGCCGCGGTAATACGTAGGGGGCAAGCGTTATC CGGAATTACTGGGTGTAAAGGGAGCGTAGGCGGCATGGTAAGCCAGATGTGAAAGCCTTGGGCTTAACCCGAGGAT TGCATTTGGAACTATCAAGCTAGAGTACAGGAGAGGAAAGCGGAATTCCTAGTGTAGCGGTGAAATGCGTAGATAT TAGGAAGAACACCAGTGGCGAAGGCGGCTTTCTGGACTGAAACTGACGCTGAGGCTCGAAAGCGTGGGGAGCAAAC AGGATTAGATACCCTGGTAGTCCACGCCGTAAACGATGAGTGCTAGGTGTCGGGGAGGAATCCTCGGTGCCGTAGC TAACGCAATAAGCACTCCACCTGGGGAGTACGACCGCAAGGTTGAAACTCAAAGGAATTGACGGGGGCCCGCACAA GCGGTGGAGCATGTGGTTTAATTCGAAGCAACGCGAAGAACCTTACCAAGGCTTGACATCCCGATGACCGTCCTAG AGATAGGACTTCTCTTCGGAGCATCGGTGACAGGTGGTGCATGGTTGTCGTCAGCTCGTGTCGTGAGATGTTGGGT TAAGTCCCGCAACGAGCGCAACCCTTGTCACTAGTTGCTACGAAAGGGCACTCTAGTGAGACTGCCGGTGACAAAC CGGAGGAAGGTGGGGATGACGTCAAGTCCTCATGGCCCTTATGGGTAGGGCTTCACACGTCATACAATGGTCGGAA CAGAGGGCAGCGAAGCCGTGAGGCGGAGCCAATCCCAGAAAACCGATCGTAGTCCGGATTGCAGTCTGCAACTCGA CTGCATGAAGTCGGAATCGCTAGTAATCGCGGATCAGCATGCCGCGGTGAATACGTTCCCGGGTCTTGTACACACC GCCCGTA |
| AMCI01001631.34.1456 |
| GGCGCACGGGTGAGTAACACGTATCCAACCTGCCGATAACTCGGGGATAGCCTTTCGAAAGAAAGATTAATACCCG ATGGCATGTAAAGACCTCCTGGTCTTTACATTAAAGAATTTCGGTTATCGATGGGGATGCGTTCCATTAGATAGTA GGCGGGGTAACGGCCCACCTAGTCCACGATGGATAGGGGTTCTGAGAGGAAGGTCCCCCACATTGGAACTGAGACA CGGTCCAAACTCCTACGGGAGGCAGCAGTGAGGAATATTGGTCAATGGACGCGAGTCTGAACCAGCCAAGTAGCGT GAAGGAAGACTGCCCTATGGGTTGTAAACTTCTTTTATACGGGAATAAAGTATTCCACGTGTGGGATTTTGTATGT ACCGTATGAATAAGGATCGGCTAACTCCGTGCCAGCAGCCGCGGTAATACGGAGGATCCGAGCGTTATCCGGATTT ATTGGGTTTAAAGGGAGCGTAGGTGGAAGATTAAGTCAGCCTGTGAAAGTTTGCGGCTTAACCGTAAAATTGCAGT TGATACTGGTTTTCTTGAGTGCAGTAGAGGTGGGCGGAATTCGTGGTGTAGCGGTGAAATGCTTAGATATCACGAA GAACTCCGATTGCGAAGGCAGCTCACTGGACTGTAACTGACACTGATGCTCGAAAGTGTGGGTATCAAACAGGATT AGATACCCTGGTAGTCCACACAGTAAACGATGAATACTCGCTGTTTGCGATATACAGTAAGCGGCCAAGCGAAAGC GTTAAGTATTCCACCTGGGGAGTACGCCGGCAACGGTGAAACTCAAAGGAATTGACGGGGGCCCGCACAAGCGGAG GAACATGTGGTTTAATTCGATGATACGCGAGGAACCTTACCCGGGCTTAAATTACACCTGAATAGATTGGAAACAT TTTAGCCGCAAGGCAGGTGTGAAGGTGCTGCATGGTTGTCGTCAGCTCGTGCCGTGAGGTGTCGGCTTAAGTGCCA TAACGAGCGCAACCCTTATCTTCAGTTACTAACAGTTATAGCTGAGGACTCTGAAGAGACTGCCGTCGTAAGATGT GAGGAAGGTGGGGATGACGTCAAATCAGCACGGCCCTTACGTCCGGGGCTACACACGTGTTACAATGGGGGGTACA GAAGGCTGCTACCTGGCGACAGGATGCCAATCCTTAAATCCTCTCTCAGTTCGGACTGGAGTCTGCAACCCGACTC CACGAAGCTGGATTCGCTAGTAATCGCGCATCAGCCATGGCGCGGTGAATACGTTCCCGGGCCTTGTACACACCGC CCGTCAAGCCATGAAAGCCGGGGGTACCTGAAGTGCGTAACCGCAAGGAGCGTCCTAGGGTAAAACTGGTAATTGG GGCTAAGTCGTAACAAGGTAGCCGTACCGGAAGGTGCGGCTG |
| KF842598.1.1394 |
| AGAGTTTGATCCTGGCTCAGGATGAACGCTAGCGACAGGCTTAACACATGCAAGTCGAGGGGCAGCATGATTTGTA GCAATACAGATTGATGGCGACCGGCGCACGGGTGAGTAACGCGTATGCAACTTACCTATCAGAGGGGGATAGCCCG GCGAAAGTCGGATTAATACCCCATAAAACAGGGGTCCCGCATGGGAATATTTGTTAAAGATTCATCGCTGATAGAT AGGCATGCGTTCCATTAGGCAGTTGGCGGGGTAACGGCCCACCAAACCGACGATGGATAGGGGTTCTGAGAGGAAG GTCCCCCACATTGGTACTGAGACACGGACCAAACTCCTACGGGAGGCAGCAGTGAGGAATATTGGTCAATGGCCGA GAGGCTGAACCAGCCAAGTCGCGTGAAGGAAGAAGGATCTATGGTCTGTAAACTTCTTTTATAGGGGAATAAAGTG GAGGACGTGTCCTTTTTTGTATGTACCCTATGAATAAGCATCGGCTAACTCCGTGCCAGCAGCCGCGGTAATACGG AGGATGCGAGCGTTATCCGGATTTATTGGGTTTAAAGGGTGCGTAGGTGGTGATTTAAGTCAGCGGTGAAAGTTTG TGGCTCAACCATAAAATTGCCGTTGAAACTGGGTTACTTGAGTGTGTTTGAGGTAGGCGGAATGCGTGGTGTAGCG |
| GTGAAATGCATAGATATCACGCAGAACTCCGATTGCGAAGGCAGCTTACTAAACCATAACTGACACTGAAGCACGA AAGCGTGGGGATCAAACAGGATTAGATACCCTGGTAGTCCACGCAGTAAACGATGATTACTAGGAGTTTGCGATAC AATGTAAGCTCTACAGCGAAAGCGTTAAGTAATCCACCTGGGGAGTACGCCGGCAACGGTGAAACTCAAAGGAATT GACGGGGGCCCGCACAAGCGGAGGAACATGTGGTTTAATTCGATGATACGCGAGGAACCTTACCCGGGTTTGAACG TAGTCTGACCGGAATGGAAACACTCCTTCTAGCAATAGCAGATTACAAGGTGCTGCATGGTTGCCTCAACTCCGGC CCGGAAGGTCCGGCTTAATTGCCATAACAAGCGCACCCTTTTACCAAGGTTCAAACAGGTGAAGCTTGAAGACTCT GTGGAACCTCCCCCCTAACCTGTGAGAAGAAGTGGGGATACACTCAATAAACCACGGCCCTTAATCCCGGGGGGAA CACTGGTTACAATGGGTTGGGAAAGGGGGCTTCCTGGCGACAGGATGCTAATCTCCAAACCATGTCTCAGTTCGGA TCGGAGTCTGCAACTCGACTCCGTGAAGCTGGATTCGCTAGTAATCGCGCATCAGCCATGGCGCGGTGAATACGTT CCCGGGCCTTGTACACACCGCCCGTC |
| HQ793763.1.1451 |
| GATGAACGCTAGCTACAGGCTTAACACATGCAAGTCGAGGGGCAGCATGGTCTTAGCTTGCTAAGGCTGATGGCGA CCGGCGCACGGGTGAGTAACACGTATCCAACCTGCCGTCTACTCTTGGCCAGCCTTCTGAAAGGAAGATTAATCCA GGATGGGATCATGAGTTCACATGTCCGCATGATTAAAGGTATTTTCCGGTAGACGATGGGGATGCGTTCCATTAGA TAGTAGGCGGGGTAACGGCCCACCTAGTCAACGATGGATAGGGGTTCTGAGAGGAAGGTCCCCCACATTGGAACTG AGACACGGTCCAAACTCCTACGGGAGGCAGCAGTGAGGAATATTGGTCAATGGGCGCGAGCCTGAACCAGCCAAGT AGCGTGAAGGATGACTGCCCTATGGGTTGTAAACTTCTTTTGTCCGGGAATAAAACCGCCTACGTGTAGGCGCTTG TATGTACCGGTACGAATAAGCATCGGCTAACTCCGTGCCAGCAGCCGCGGTAATACGGAGGATGCGAGCGTTATCC GGATTTATTGGGTTTAAAGGGAGCGCAGACGGGTTTTTAAGTCAGCTGTGAAAGTTTGGGGCTCAACCTTAAAATT GCAGTTGATACTGGAGACCTTGAGTGCAGTTGAGGCAGGCGGAATTCGTGGTGTAGCGGTGAAATGCTTAGATATC ACGAAGAACTCCGATTGCGAAGGTAGCTTGCTAAAGTGTAACTGACGTTCATGCTCGAAAGTGTGGGTATCAAACA GGATTAGATACCCTGGTAGTCCACACGGTAAACGATGGATACTCGCTGTTGGCGATATACGGTCAGCGGCTTAGCG AAAGCGTTAAGTATCCCACCTGGGGAGTACGCCGGCAACGGTGAAACTCAAAGGAATTGACGGGGGCCCGCACAAG CGGAGGAACATGTGGTTTAATTCGATGATACGCGAGGAACCTTACCCGGGCTTAAATTGCACTGGACTATTCTGGA AACAGGATATTCTTCGGACCAGTGTGAAGGTGCTGCATGGTTGTCGTCAGCTCGTGCCGTGAGGTGTCGGCTTAAG TGCCATAACGAGCGCAACCCTTGCTGCCAGTTACTAACAGGTAATGCTGAGGACTCTGGCGGGACTGCCATCGTAA GATGCGAGGAAGGTGGGGATGACGTCAAATCAGCACGGCCCTTACGTCCGGAGCTACACACGTGTTACAATGGTAG GTACAGAGGGTAGCTACCCAGCGATGGGATGCGAATCTCGAAAGCCTATCTCAGTTCGGATTGGAGGCTGAAACCC GCCTCCATGAAGTTGGATTCGCTAGTAATCGCGCATCAGCCATGGCGCGGTGAATACGTTCCCGGGCCTTGTACAC ACCGCCCGTCAAGCCATGGGAGCCGGGGGTACCTGAAGTACGTAACCGCAAGGATCGTCCTAGGGTAAAACTGGTG ACTGGGG |
| DQ113765.1.1450 |
| GATGAACGCTAGCTACAGGCTTAACACATGCAAGTCGAGGGGCAGCATGAAGTTTGCTTGCAAACTTTGATGGCGA CCGGCGCACGGGTGAGTAACGCGTATCCAACCTCCCGCATACTCGGGGATAGCCTTCTGAAAGGAAGATTAATACC CGATGGTATCTTAAGCGCACATGCAATTAAGATTAAAGAATTTCGGTATGCGATGGGGATGCGTTCCATTAGGTAG TAGGCGGGGTAACGGCCCACCTAGCCATCGATGGATAGGGGTTCTGAGAGGAAGGTCCCCCACATTGGAACTGAGA CACGGTCCAAACTCCTACGGGAGGCAGCAGTGAGGAATATTGGTCAATGGGCGCGAGCCTGAACCAGCCAAGTAGC GTGAAGGATGACTGCCCTATGGGTTGTAAACTTCTTTTGTCCGGGAATAAAACCGCCTACGTGTAGGCGCTTGTAT GTACCGGTACGAATAAGCATCGGCTAACTCCGTGCCAGCAGCCGCGGTAATACGGAGGATGCGAGCGTTATCCGGA TTTATTGGGTTTAAAGGGAGCGCAGACGGGTTTTTAAGTCAGCTGTGAAAGTTTGGGGCTCAACCTTAAAATTGCA GTTGATACTGGAGACCTTGAGTGCAGTTGAGGCAGGCGGAATTCGTGGTGTAGCGGTGAAATGCTTAGATATCACG AAGAACTCCGATTGCGAAGGCAGCTTGCTAAAGTGTAACTGACGTTCATGCTCGAAAGTGTGGGTATCAAACAGGA TTAGATACCCTGGTAGTCCACACGGTAAACGATGGATACTCGCTGTTGGCGATATACGGTCAGCGGCTTAGCGAAA GCGTTAAGTATCCCACCTGGGGAGTACGCCGGCAACGGTGAAACTCAAAGGAATTGACGGGGGCCCGCACAAGCGG AGGAACATGTGGTTTAATTCGATGATACGCGAGGAACCTTACCCGGGCTTAAATTGCACTGGACTTTCCCGGAAAC GGGATTTTCTTCGGACCAGTGTGAAGGTGCTGCATGGTTGTCGTCAGCTCGTGCCGTGAGGTGTCGGCTTAAGTGC CATAACGAGCGCAACCCTTGCTGCCAGTTACTAACAGGTAATGCTGAGGACTCTGGCGGGACTGCCATCGTAAGAT GCGAGGAAGGTGGGGATGACGTCAAATCAGCACGGCCCTTACGTCCGGGGCTACACACGTGTTACAATGGGGGGTA CAGAAGGCCGCTACCCGGCAACGGGATGCCAATCTCCAAAACCCCTCTCAGTTCGGACTGGAGTCTGCAACCCGAC TCCACGAAGCTGGATTCGCTAGTAATCGCGCATCAGCCACGGCGCGGTGAATACGTTCCCGGGCCTTGTACACACC GCCCGTCAAGCCATGAAAGCCGGGGGTACCTGAAGTGCGTAACCGCAAGGAGCGCCCTAGGGTAAAACTGGTAATT GGGGCT |
| ACBW01000012.3536.5054 |
| AGAGTTTGATCCTGGCTCAGGATGAACGCTAGCTACAGGCTTAACACATGCAAGTCGAGGGGCAGCGGGATTGAAG CTTGCTTCAATTGCCGGCGACCGGCGCACGGGTGAGTAACGCGTATCCAACCTTCCGCTTACTCGGGGATAGCCTT TCGAAAGAAAGATTAATACCCGATGGTATCTTAAGCACGCATGAGATTAAGATTAAAGATTTATCGGTAAGCGATG GGGATGCGTTCCATTAGGCAGTTGGCGGGGTAACGGCCCACCAAACCTACGATGGATAGGGGTTCTGAGAGGAAGG TCCCCCACATTGGAACTGAGACACGGTCCAAACTCCTACGGGAGGCAGCAGTGAGGAATATTGGTCAATGGGCGAG AGCCTGAACCAGCCAAGTAGCGTGAAGGATGACGGCCCTACGGGTTGTAAACTTCTTTTGTGCGGGAATAAAGGAA CCTACGTGTAGGTTTTTGCATGTACCGTAACGAATAAGCATCGGCTAACTCCGTGCCAGCAGCCGCGGTAATACGG AGGATGCGAGCGTTATCCGGATTTATTGGGTTTAAAGGGAGCGTAGACGGGTTTTTAAGTCAGCTGTGAAAGTTTG GGGCTCAACCTTAAAATTGCAGTTGAAACTGGAGACCTTGAGTACGGTTGAGGCAGGCGGAATTCGTGGTGTAGCG |
| GTGAAATGCTTAGATATCACGAAGAACCCCGATTGCGAAGGCAGCCTGCTAAGCCGCCACTGACGTTGAGGCTCGA AAGTGCGGGTATCAAACAGGATTAGATACCCTGGTAGTCCGCACGGTAAACGATGGATACTCGCTGTTGGCGATAG ACAGTCAGCGGCCAAGCGAAAGCGTTAAGTATCCCACCTGGGGAGTACGCCGGCAACGGTGAAACTCAAAGGAATT GACGGGGGCCCGCACAAGCGGAGGAACATGTGGTTTAATTCGATGATACGCGAGGAACCTTACCCGGGCTTGAACT GCAGTGGAATTATCCGGAAACGGATAAGCGAGCAATCGCCGCTGTGGAGGTGCTGCATGGTTGTCGTCAGCTCGTG CCGTGAGGTGTCGGCTTAAGTGCCATAACGAGCGCAACCCTTGCTGCCAGTTACTAACAGGTCATGCTGAGGACTC TGGCAGGACTGCCATCGTAAGATGCGAGGAAGGTGGGGATGACGTCAAATCAGCACGGCCCTTACGTCCGGGGCTA CACACGTGTTACAATGGGGAGTACAGAGGGCAGCTACCGGGCGACCGGATGCGAATCCCGAAAGCTCCTCTCAGTT CGGACTGGAGTCTGCAACCCGACTCCACGAAGCTGGATTCGCTAGTAATCGCGCATCAGCCACGGCGCGGTGAATA CGTTCCCGGGCCTTGTACACACCGCCCGTCAAGCCATGAAAGCCGGGGGTACCTGAAGTACGTAACCGCGAGGATC GTCCTAGGGTAAAACCGGTAATTGGGGCTAAGTCGTAACAAGGTAGCCGTACCGGAAGGTGCGGCTG |
| HK693629.1.1491 |
| AGAGTTTGATCCTGGCTCAGGATGAACGCTGGCGGCGTGCTTAACACATGCAAGTCGAACGAGAAACATTTTAATG AAGCTTCGGCAGATTTAGTTTGTTTCTAGTGGCGGACGGGTGAGTAACGCGTGGGTAACCTGCCTCACACTGGGGG ATAACAGTCAGAAATGACTGCTAATACCGCATAAGCGCACGGAACCGCATGGTTTTGTGTGAAAAACTCCGGTGGT GTGAGATGGACCCGCGTTGGATTAGCCAGTTGGCAGGGTAACGGCCTACCAAAGCGACGATCCATAGCCGGCCTGA GAGGGTGAACGGCCACATTGGGACTGAGACACGGCCCAGACTCCTACGGGAGGCAGCAGTGGGGAATATTGCACAA TGGGGGAAACCCTGATGCAGCGACGCCGCGTGAAGGAAGAAGTATCTCGGTATGTAAACTTCTATCAGCAGGGAAG ATAATGACGGTACCTGACTAAGAAGCCCCGGCTAACTACGTGCCAGCAGCCGCGGTAATACGTAGGGGGCAAGCGT TATCCGGATTTACTGGGTGTAAAGGGAGCGTAGACGGAGCAGCAAGTCTGATGTGAAAGGCAGGGGCTCAACCCCT GGACTGCATTGGAAACTGTTGATCTTGAGTACCGGAGGGGTAAGCGGAATTCCTAGTGTAGCGGTGAAATGCGTAG ATATTAGGAGGAACACCAGTGGCGAAGGCGGCTTACTGGACGGTAACTGACGTTGAGGCTCGAAAGCGTGGGGAGC AAACAGGATTAGATACCCTGGTAGTCCACGCCGTAAACGATGAATACTAGGTGTCGGGTGGCAGAGCCATTCGGTG CCGCAGCAAACGCAGTAAGTATTCCACCTGGGGAGTACGTTCGCAAGAATGAAACTCAAAGGAATTGACGGGGACC CGCACAAGCGGTGGAGCATGTGGTTTAATTCGAAGCAACGCGAAGAACCTTACCAAGTCTTGACATCCCTCTGACC GGTCCTTAACCGGACCTTTCCTTCGGGACAGAGGAGACAGGTGGTGCATGGTTGTCGTCAGCTCGTGTCGTGAGAT GTTGGGTTAAGTCCCGCAACGAGCGCAACCCCTATCCCCAGTAGCCAGCATTTAAGGTGGGCACTCTGAGGAGACT GCCAGGGATAACCTGGAGGAAGGCGGGGATGACGTCAAATCATCATGCCCCTTATGATTTGGGCTACACACGTGCT ACAATGGCGTAAACAAAGGGAAGCAGAGCGGTGACGCCGAGCAAATCCCAAAAATAACGTCCCAGTTCGGACTGCA GTCTGCAACTCGACTGCACGAAGCTGGAATCGCTAGTAATCGCGGATCAGAATGCCGCGGTGAATACGTTCCCGGG TCTTGTACACACCGCCCGTCACACCATGGGAGTCAGTAACGCCCGAAGTCAGTGACCTAACCGAAAGGAGGAGCT GCCGAAGGCGGGACGGATGACTGGGGTGAAGTCGTAACAAGGTAACC |
| JQ208053.1.1336 |
| GATGAACGCTGACAGAATGCTTAACACATGCAAGTCTACTTGAATTCACTTCGGTGATAGTAAGGTGGCGGACGGG TGAGTAACACGTAAAGAACTTGCCTTACAGTCTGGGACAACTATTGGAAACGATAGCTAATACCGGATATTATGCG AGAGTCGCATGACTCTTGTATGAAAGCTATATGCGCTGTAAGAGAGCTTTGCGTCCCATTAGCTAGTTGGTGAGGT AACGGCTCACCAAGGCCACGATGGGTAGCCGGCCTGAGAGGGTGAACGGCCACAAGGGGACTGAGACACGGCCCTT ACTCCTACGGGAGGCAGCAGTGGGGAATATTGGACAATGGACCAAAAGTCTGATCCAGCAATTCTGTGTGCACGAT GACGGTCTTAGGATTGTAAAGTGCTTTCAATTGGGAAGAAAAAAATGACGGTACCAATAGAAGAAGCGACGGCTAA ATACGTGCCAGCAGCCGCGGTAATACGTATGTCGCAAGCGTTATCCGGATTTATTGGGCGTAAAGCGCGTCTAGGT GGTTTGGTAAGTCTGATGTGAAAATGCGGGGCTCAACTCCGTATTGCGTTGGAAACTGCCTAACTAGAGTATCGGA GAGGTGGGCGGAACTACAAGTGTAGAGGTGAAATTCGTAGATATTTGTAGGAATGCCGATAGAGAAGTCAGCTCAC TGGACGAATACTGACACTGAAGCGCGAAAGCATGGGGAGCAAACAGGATTAGATACCCTGGTAGTCCATGCTGTAA ACGATGATTACTAAGCGTCGGGGGTCGAACCTCGGCACTCAAGCTAACGCGATAAGTAATCCGCCTGGGGAGTACG TACGCAAGTATGAAACTCAAAGGAATTGACGGGGACCCGCACAAGTGGTGGAGCATGTGGTTTAATTCGACGCAAC GCGAGGAACCTTACCAGCGTTTGACATCCTAGGAATGAGAAAGAGATTTCTTAGTGCTCCTTCGGGAGAACCTAGA GACAGGTGGTGCATGGCTGTCGTCAGCTCGTGTCGTGAGATGTTGGGTTAAGTCCCGCAACGAGCGCAACCCCTAT TGTATGTTGCCATCATTAAGTTGGGCACTCATGCGATACTGCCTGCGATGAGCAGGAGGAAGGTGGGGATGACGTC AAGTCATCATGCCCCTTATACGCTGGGCTACACACGTGCTACAATGGGCAGTACAGAGAGAAGCAAATCTGCGAGG AGGAGCAAATCTCACAAAGCTGTTCGTAGTTCGGATTGTACTCTGCAACTCGAGTACATGAAGTTGGAATCACTAG TAATCGCAAATCAGCTATGTTGCGGTGAATACGTTCTCGGGTCT |
| GQ493166.1.1359 |
| GATGAACGCTGGCGGCGTGCTTAACACATGCAAGTCGAACGGAAACATTTAATGAAGCTTCGGCAGATTTAGCT TGTTTCTAGTGGCGGACGGGTGAGTAACGCGTGGGTAACCTGCCTCACACTGGGGGATAACAGTCAGAAATGACTG CTAATACCGCATAAGCGCACGGAACCGCATGGTTTTGTGTGAAAAACTCCGGTGGTGTGAGATGGACCCGCGTTGG ATTAGCCAGTTGGCAGGGTAACGGCCTACCAAAGCGACGATCCATAGCCGGCCTGAGAGGGTGAACGGCCACATTG GGACTGAGACACGGCCCAGACTCCTACGGGAGGCAGCAGTGGGGAATATTGCACAATGGGGGAAACCCTGATGCAG CGACGCCGCGTGAAGGAAGAAGTATCTCGGTATGTAAACTTCTATCAGCAGGGAAGAAAATGACGGTACCTGACTA AGAAGCCCCGGCTAACTACGTGCCAGCAGCCGCGGTAATACGTAGGGGGCAAGCGTTATCCGGATTTACTGGGTGT AAAGGGAGCGTAGACGGAATGGCAAGTCTGATGTGAAAGGCAGGGGCTCAACCCCTGGACTGCATTGGAAACTGTC AGTCTTGAGTACCGGAGGGGTAAGCGGAATTCCTAGTGTAGCGGTGAAATGCGTAGATATTAGGAGGAACACCAGT GGCGAAGGCGGCTTACTGGACGGTAACTGACGTTGAGGCTCGAAAGCGTGGGGAGCAAACAGGATTAGATACCCTG |
| GTAGTCCACGCCGTAAACGATGAATACGAGGTGTCGGGTGGCAAAGCCATTCGGTGCCGCAGCAAACGCAAAAAG TAATCCACCTGGGGAGTACGTTCCCAAGAATGAAACTCAAAGGAAATAGCGGGGACCCGCACAAGCGGTGGAGC ATGTGGTGTATTTGAAGCAACGCGAAGAACCTTACCAAGTCTTGACATCCCTCTGACCGGTCCTTAACCGGACCTC TCCTTCGGGACAGGGGAGACAGGTGGTGCATGGTTGTCGTCAGCTCGTGTCGTGAGATGTTGGGTTAAGTCCCGCA ACGAGCGCAACCCCTATCCTTAGTAGCCAGCATCTGAGGTGGGCACTCTGAGGAGACTGCCAGGGATAACCTGGAG GAAGGCGGGGAGGACGTCAAATCATCATGCCCCCTATGATTTGGGCTACACACGTGCTACAATGGCGTAAACAAAG GGAAGCAGAGCGGTGACGCCGAGCAAATCCCAAAAATAACGTCCCAGTTCGGACTGCAGTCTGCAACTCGACTGCA CGAAGCTGGAATCGCTAGTAATCGCGGATCAGAATGCCGCGGTGAATAAAGCCCGGGTCTTGCACT |
| GQ448486.1.1387 |
| AGAGTTTGATCATGGCTCAGGATGAACGCTGGCGGCGTGCTTAACACATGCAAGTCGAACGGAATTACTTTATTG AAGCTTTGGTCGATTTAATTTAATTATAGTGGCGGACGGGTGAGTAACGCGTGGGTAACCTGCCTTATACAGGGGG ATAACAGTCAGAAATGGCTGCTAATACCGCATAAGCGCACAGAGCTGCATGGCTCAGTGTGAAAAACTCCGGTGGT ATAAGATGGACCCGCGTTGGATTAGTTGGTTGGTGGGGTAACGGCCCACCAAGGCGACGATCCATAGCCGGCCTGA GAGGGTGAACGGCCACATTGGGACTGAGACACGGCCCAGACTCATACGGGAGGCAGCAGTGGGGAATATTGCACAA TGGGGGAAACCCTGATGCAGCGACGCCGCGTGAAGGAAGAAGTATCTCGGTATGTAAACTTCTATCAGCAGGGAAG ATAGTGACGGTACCTGACTAAGAAGCCCCGGCTAACTACGTGCCAGCAGCCGCGGTAATACGTAGGGGGCAAGCGT TATCCGGATTTACTGGGTGTAAAGGGAGCGTAGACGGTGTGGCAAGTCTGATGTGAAAGGCATGGGCTCAACCTGT GGACTGCATTGGAAACTGTCATACTTGAGTGCCGGAGGGGTAAGCGGAATTCCTAGTGTAGCGGTGAAATGCGTAG ATATTAGGAGGAACACCAGTGGCGAAGGCGGCTTACTGGACGGTAACTGACGTTGAGGCTCGAAAGCGTGGGGAGC AAACAGGATTAGATACCCTGGTAGTCCACGCCGTAAACGATGAATACTAGGTGTCGGGTGGCAAAGCCATTCGGTG CCGTCGCAAACGCAGTAAGTATTCCACCTGGGGAGTACGTTCGCAAGAATGAAACTCAAAGGAATTGACGGGGACC CGCACAAGCGGTGGAGCATGTGGTTTAATTCGAAGCAACGCGAAGAACCTTACCAAGTCTTGACATCCGCCTGACC GATCCTTAACCGGATCTTTCCTTCGGGACAGGCGAGACAGGTGGTGCATGGTTGTCGTCAGCTCGTGTCGTGAGAT GTTGGGTTAAGTCCCGCAACGAGCGCAACCCCTATCCTCAGTAGCCAGCATTAAGTTGGGCACTCATGCGATACTG CCTGCGATGAGCAGGAGGAAGGTGGGGATGACGTCAAGTCATCATGCCCCTTATACGCTGGGCTACACACGTGCTA CAATGGGTAGTACAGAGAGTCGCAAACCTGCGAGGGGGAGCTAATCTCAGAAAACTATTCTCAGTTCGGATTGTAC TCTGCAACTCGAGTACATGAAGTTGGAATCGCTAGTAATCGCAAATCAGCTATGTTGCGGTGAATACGTTCTCGGG TCTTGCACTCACCGCCCGT |
| GQ491426.1.1332 |
| GCTCAGGATGAACGCTGGCGGCGTGCTTAACACATGCAAGTCGAGCGAAGCACTTGCCATTGACTCTTCGGAAGAT TTGGCATTTGACTGAGCGGCGGACGGGTGAGTAACGCGTGGGTAACCTGCCTCATACGGGGGAATAACAGTTAGAA ATGGCTGCTAATGCCGCATAACCGCACAGGACCGCATGGACTGGTGTGAAAAACTGAGGTGGTATGAGATGGGCCC GCGTCTGATTAGGTTAGTTGGCGGGGTAACGGCCCACCAAGCCGACGATCAGTAGCCGACCTGAGAGGGACCGGCC ACATTGGGACTGAGACATGGCCCAGACTCCTACGGGAGGCAGCAGTGGGGAATATTGCACAATGGAGGAAACTCTG ATGCAGCGACGCCGCATGAAGGAAGAAGTATCTCGGTATGTAAACTTCTATCAGCAGGGAAGAAAATGACGGTACC TGACTAAGAAGCCCCGGCTAACTACGTGCCAGCAGCCGCGGTAATACGTAGGGGGCAAGCGTTATCCGGATTTACT GGGTGTAAAGGGAGCGTAGACGGACGGGCAAGTCTGATGTGAAAGCCCGGGGCTTAACCCCGGGACTGCATTGGAA ACTGTCCATCTTGAGTGCCGGAGAGGTAAGCGGAATTCCTAGTGTAGCGGTGAAATGCGTAGATATTAGGAGGAAC ACCAGTGGCGAAGGCGGCTTACTGGACGGTAACTGACGTTGAGGCTCGAAAGCGTGGGGAGCAAACAGGATTAGAT ACCCTGGTAGTCCACGCCGTAAACGATCAATAATGGGTGTCGGGTTGCAAAGCAATCCGGTGCCGCAGCAAACGCA GTAAGTATTCCCCCTCGGGAGTACGTTCGCAAGAATGAAACTCAAAGGAAGGGACGGGGATCCGCACAAGCGGCGG AGCATGTGGTTTAATTAGAAGCAACGCGAAGAACCTTACCAAGTCTTGACATCTGCCTGACCGTTCCTTAACCGGA ACTATCTTTCGGGACAGGCAAGACAGGTGGTGCATGGTTGTCGTCAGCTCGTGTCGTGAGATGTTGGGTTAAGTCC CGCAACGAGCGCAACCCCTGTCCTTAGTAGCCAGCAGTCCGGCTGGGCACTCTAGGGAGACTGCCGGGGGTAACCC GGAGGAAGGCGGGGAGGAGGTCAAATCATCATGCCCCCCCTGATTTGGGCTACACACGTGGTACAATGGCGTAAAC AAAGGGAAGCGGAGTGGTGACGCTGAGCAAATCTCAAAAATAACGTCCCACTTCGGACTGCAGTCTGCAACTCGAC TGCACGAAGCTGGAATCGCTAGTAATCGCGAATCAGAATG |
| New.ReferenceOTU54 |
| TACGTAGGGGGCAAGCGTTATCCGGATTTACTGGGTGTAAAGGGAGCGTAGACGGCATGGCAAGTCTGATGTGAAA GGCAGGGGCTCAACTCCTGGACTGCATTGGAAACTGCCAGGCTTGAGTGCCGGAGGGGTAAGCGGAATTCCTAGTG TAGCGGTGAAATGCGTAGATATTAGGAGGAACACCAGTGGCGAAGGCGGCTTACTGGACGGTAACTGACGTTGAGG CTCGAAAGCGTGGGGAGCAAACAGG |
| JN387556.1.1324 |
| CGTAAGTAACCTGCCCTGTACACACGGATAACATACCGAAAGGTATGCTAATACGGATAATATATTTTGATCGCA TGGTCGAGATATCAAAGCTCCGGCGGTACACCAGGGACCCCCGACAGAGGAGCTAGTTGGTAGTAATGTCACCAAG GCGACGATCAGAAGCCGAACTGAGAGGGGGATCCGCACATGACTGAGACACGGTCAAACTCCTACGGGAGGCAGCA GTGGGGAATATGCCAATGGGCGAAAGCTGATGCAGCACGCGCGTGAGCGATGAGGCTCGGGTCGTAAAGCTCGTCT CAAGGAAGATAATGACGGTACTTGAGGAGGAAGCCCCGGCTAACTACGTGCCAGCAGCCGCGGTAATACGTAGGGG GCTAGCGTTATCCGGAATTACTGGGCGTAAAGGGTGCGTAGGCGGTCTTTCAAGTCAGGAGTGAAAGGCTACGGCT CAACCGTAGTAAGCTCTTGAAACTGTAAGACTTGAGTGCAGGAGAGGAGAGTGGAATTCCTAGTGTAGCGGTGAAA TGCGTAGATATTAGGAGGAACACCAGTTGCGAAGGCGGCTCTCTGGACTGTAACTGACGCTGAGGCACGAAAGCGT |
| GGGGAGCAAACAGGATTAGATACCCTGGTAGTCCACGCCGTAAACGATGAGTACTAGCTGTCGGAGGTTACCCCCT TCGGTGGCGCAGCTAACGCATTAAGTACTCCGCCTGGGAAGTACGCTCGCAAGAGTGAAACTCAAAGGAATTGACG GGGACCCGCACAAGTAGCGGAGCATGTGGTTTAATTCGAAGCAACGCGAAGAACCTTACCTAAGCTTGACATCCCA CTGACCCTTCCCTAATCGGAAGCTTCCCTTCGGGACAGTGGTGACAGGTGGTGCATGGTTGTCGTCAGCTCGTGTC GTGAGATGTTGGGTTAAGTCCCGCAACGAGCGCAACCCTTGCCTTTAGTTGCCAGCATTAAGTTGGGCACTCTAGA GGGACTGCCAGGGATAACCCGGAGGAGTGGGGATGACGTCAAATCATCATGCCCTTATGCTAGGCTACACACGTGC TACAATGGGTGGTCAGAGGCCAGCCAGTCGTGAGGCCGAGCTATCCCATAAGCCATTCTCGTCCGGATTGTAGGCT GAACTCGCCTACATGAGCTGGAATTACAAGTATGCGATCGATGCTGCGTGATGCGTCCGGGTCTTGTACACACCGC CCGTCACACCATGGGAGTTGGGGGCGCCCGAAGCCGGATTGCTAACCTTTTGGAAGCGTCCGTCGAAGGTGAAACC AATAACTGGGGTGAAGTCGTAACAAGGTAACC |
| |

| **OTUs in Table 3** |
|---|
| GQ006324.1.1342 |
| GACGAACGCTGGCGGCGTGCTTAACACATGCAAGTCGAACGGAAAGGCCCAGCTCGCTGGGGTACTCGAGTGGCG AACGGGTGAGTAACACGTGGGTGATCTGCCTTGCACTCTGGGATAAGCTTGGGAAACTGGGTCTAATACCGGATAT GAACTGCCTTTAGTGTGGTGGTTGGAAAGTTTTTCGGTGCAAGATGAGCTCGCGGCCTATCAGCTTGTTGGTGGG GTAATGGCCTACCAAGGCGTCGACGGGTAGCCGGCCTGAGAGGGTGTACGGCCACATTGGGACTGAGATACGGCCC AGACTCCTACGGGAGGCAGCAGTGGGGAATATTGCACAATGGGCGGAAGCCTGATGCAGCGACGCCGCGTGGGGGA TGACGGCCTTCGGGTTGTAAACTCCTTTCGACAGGGACGAAGCTTTTTGTGACGGTACCTGTATAAGAAGCACCGG CTAACTACGTGCCAGCAGCCGCGGTAATACGTAGGGTGCGAGCGTTGTCCGGAATTACTGGGCGTAAAGAGCTCGT AGGTGGTTTGTCGCGTCGTCTGTGAAATTCCGGGGCTTAACTCCGGGCGTGCAGGCGATACGGGCATAACTTGAGT ACTGTAGGGGAGACTGGAATTCCTGGTGTAGCGGTGAAATGCGCAGATATCAGGAGGAACACCGGTGGCGAAGGCG GGTCTCTGGGCAGTAACTGACGCTGAGGAGCGAAAGCATGGGGAGCGAACAGGATTAGATACCCTGGTAGTCCATG CCGTAAACGGTGGGCGCTAGGTGTGGGTTTCCTTCCACGGGATCCGTGCCGTAGCTAACGCATTAAGCGCCCCGCC TGGGGAGTACGGCCGCAAGGCTAAAACTCAAAGGAATTGACGGGGGCCCGCACAAGCGGCGGAGCATGTGGATTAA TTCGATGCAACGCGAAGAACCTTACCTGGGCTTGACATACACTGGATCGGGCTAGAGATAGTCTTTCCCTTTGTGG CTGGTGTACAGGTGGTGCATGGTTGTCGTCAGCTCGTGTCGTGAGATGTTGGGTTAAGTCCCGCAACGAGCGCAAC CCTTGTCTTATGTTGCCAGCATTTGGTTGGGGACTCATGAGAGACTGCCGGGGTCAACTCGGAGGAAGGTGGGGAT GACGTCAAATCATCATGCCCCTTATGTCCAGGGCTTCACACATGCTACAATGGTCGGTACAACGCGCAGCGACACT GTGAGGTGGAGCGAATCGCTGAAAGCCGGCCTTAGTTCGGATTGGGGTCTGCAACTCGACCCCATGAAGTCGGAGT CGCTAGTAATCGCAGATCAGCAATGCTGCGGTGAATACGTTCCCGGGCCT |
| New.ReferenceOTU52 |
| TACGTAGGTGGCGAGCGTTATCCGGATTTACTGGGCGTAAAGGGAGCGTAGGCGGATGATTAAGTGGGATGTGAAA TACCCGGGCTCAACTTGGGTGCTGCATTCCAAACTGGTTATCTAGAGTGCAGGAGAGGAGAGTGGAATTCCTAGTG TAGCGGTGAAATGCGTAGAGATTAGGAAGAACACCAGTGGCGAAGGCGACTCTCTGGACTGTAACTGACGCTGAGG CTCGAAAGCGTGGGGAGCAAACAGG |
| HG798451.1.1400 |
| CTTGTGTCACCAACCATAGGGAGGGGAAAACATGGAAACGGGGTTCATACCGCATAACTTTTTAGCCCAATGCA TAAGAAGAAAGGCCTTTCGGGTTTCGGTAAAGGAGGCCCCCGCGGCTCTTATAGTGTGTGGAAGTAACCGCTTC CACAAGGCCCAGGTTTCATACCCGACTGGAGAGTGTGTTCGCCACACTGGGGAAAGGACCCCCGGCCCAGTCTCTC TAGGGGAGGCAGCAGTAGGAATTTTCGGCAAAGGAAAAAATTTCTGACCGAACAACGCCGGTTGAATGAAGAAGTT TTTCGGATCGAAAAACTCTGTTGTTAGAGAAGAACAAGGACGTTAGTAACTGAACGTCCCCTGACGGTATCTAACC AGAAAGCCACGGCTAATTACGTGCCAGCAGCCGCGGTAATACGTAGGTGGCAAGCGTTGTCCGGATTTATTGGGCG TAAAGCGAGCGCAGGCGGTTTCTTAAGTCTGATGTGAAAGCCCCCGGCTCAACCGGGGAGGGTCATTGGAAACTGG GAGACTTGAGTGCAGAAGAGGAGAGTGGAATTCCATGTGTAGCGGTGAAATGCGTAGATATATGGAGGAACACCAG TGGCGAAGGCGGCTCTCTGGTCTGTAACTGACGCTGAGGCTCGAAAGCGTGGGGAGCAAACAGGATTAGATACCCT GGTAGTCCACGCCGTAAACGATGAGTGCTAAGTGTTGGAGGGTTTCCGCCCTTCAGTGCTGCAGCAAACGCATTAA GCACTCCGCCTGGGGAGTACGACCGCAAGGTTGAAACTCAAAGGAATTGACGGGGGCCCGCACAAGCGGTGGAGCA TGTGGTTTAATTCGAAGCAACGCGAAGAACCTTACCAGGTCTTGACATCCTTTGACCACTCTAGAGATAGAGCTTT CCCTTCGGGGACAAAGTGACAGGTGGTGCATGGTTGTCGTCAGCTCGTGTCGTGAGATGTTGGGTTAAGTCCCGCA ACGAGCGCAACCCTTATTGTTAGTTGCCATCATTTAGTTGGGCACTCTAGCGAGACTGCCGGTGACAAACCGGAGG AAGGTGGGGATGACGTCAAATCATCATGCCCCTTATGACCTGGGCTACACACGTGCTACAATGGAAGTACAACGA GTCGCTAGACCGCGAGGTCATGCAAATCTCTTAAAGCTTCTCTCAGTTCGGATTGCAGGCTGCAACTCGCCTGCAT GAAGCCGGAATCGCTAGTAATCGCGGATCAGCACGCCGCGGTGAATACGTTCCCGGGCCTTGTACACACCGCCCGT CACACCACGAGAGTTTGTAACACCCGAAGTCGGTGAGGTAACCTTTTTGGAGCCAGCCGCCTAAGGTGGGATAGAT GATTGGGGTGAAGTCGTAACCAACGTATGCC |
| HK557089.3.1395 |
| AGACTTTAGCTTGCTAAAGTTGGAAGAGTTGCGAACGGGTGAGTAACGCGTAGGTAACCTGCCTACTAGCGGGGGA TAACTATTGGAAACGATAGCTAATACCGCATAACAGCATTTAACCCATGTTAGATGCTTGAAAGGAGCAATTGCTT CACTAGTAGATGGACCTGCGTTGTATTAGCTAGTTGGTGAGGTAACGGCTCACCAAGGCGACGATACATAGCCGAC CTGAGAGGGTGATCGGCCACACTGGGACTGAGACACGGCCCAGACTCCTACGGGAGGCAGCAGTAGGGAATCTTCG |
| GCAATGGGGGCAACCCTGACCGAGCAACGCCGCGTGAGTGAAGAAGGTTTTCGGATCGTAAAGCTCTGTTGTAAGA GAAGAACGTGTGTGAGAGTGGAAAGTTCACACAGTGACGGTAACTTACCAGAAAGGGACGGCTAACTACGTGCCAG CAGCCGCGGTAATACGTAGGTCCCGAGCGTTGTCCGGATTTATTGGGCGTAAAGCGAGCGCAGGCGGTTTAATAAG TCTGAAGTTAAAGGCAGTGGCTTAACCATTGTTCGCTTTGGAAACTGTTAGACTTGAGTGCAGAAGGGGAGAGTGG AATTCCATGTGTAGCGGTGAAATGCGTAGATATATGGAGGAACACCGGTGGCGAAAGCGGCTCTCTGGTCTGTAAC TGACGCTGAGGCTCGAAAGCGTGGGGAGCAAACAGGATTAGATACCCTGGTAGTCCACGCCGTAAACGATGAGTGC TAGGTGTTAGGCCCTTTCCGGGGCTTAGTGCCGCAGCTAACGCATTAAGCACTCCGCCTGGGGAGTACGACCGCAA GGTTGAAACTCAAAGGAATTGACGGGGGCCCGCACAAGCGGTGGAGCATGTGGTTTAATTCGAAGCAACGCGAAGA ACCTTACCAGGTCTTGACATCCCGATGCTATTCCTAGAGATAGGAAGTTTCTTCGGAACTGTGAGACTTGAGGGCA GAAGGTAGAGTGCACTTGTATGGGGAGCTGTGGAATGCGTTCCCGCAACGAGCGCAACCCCTATTGTTAGTTGCC ATCATTAAGTTGGGCACTCTAGCGAGACTGCCGGTAATAAACCGGAGGAAGGTGGGGATGACGTCAAATCATCATG CCCCTTATGACCTGGGCTACACACGTGCTACAATGGTTGGTACAACGAGTCGCGAGTCGGTGACGGCAAGCAAATC TCTTAAAGCCAATCTCAGTTCGGATTGTAGGCTGCAACTCGCCTACATGAAGTCGGAATCGCTAGTAATCGCGGAT CAGCACGCCGCGGTGAATACGTTCCCGGGCCTTGTACACACCGCCCGTCACACCACGAGAGTTTGTAACACCCGAA GTCGGTGAGGTANCCTTTTAGGAGC |
| GQ448336.1.1418 |
| AGAGTTTGATCATGGCTCAGGACGAACGCTGGCGGCGTGCTTAACACATGCAAGTCGAACGAAGAGATGAGAAG CTTGCTTCTTATCTCTTCGAGTGGCAAACGGGTGAGTAACGCGTAAGCAACCTGCCCTTCAGATGGGGACAACAGC TGGAAACGGCTGCTAATACCGAATACGTTCTTTTTGTCGCATGGCAGAGGGAAGAAAGGGAGGCTCTTCGGAGCTT TCGCTGAAGGAGGGGCTTGCGTCTGATTAGCTAGTTGGAGGGGTAACGGCCCACCAAGGCGACGATCAGTAGCCGG TCTGAGAGGATGAACGGCCACATTGGGACTGAGACACGGCCCAGACTCCTACGGGAGGCAGCAGTGGGGAATCTTC CGCAATGGACGAAAGTCTGACGGAGCAACGCCGCGTGAACGATGACGGCCTTCGGGTTGTAAAGTTCTGTTATACG GGACGAATGGCGTAGCGGTCAATACCCGTTACGAGTGACGGTACCGTAAGAGAAAGCCACGGCTAACTACGTGCCA GCAGCCGCGGTAATACGTAGGTGGCAAGCGTTGTCCGGAATTATTGGGCGTAAAGGGCGCGCAGGCGGCGTCGTAA GTCGGTCTTAAAAGTGCGGGGCTTAACCCCGTGAGGGGACCGAAACTGCGATGCTAGAGTATCGGAGAGGAAAGCG GAATTCCTAGTGTAGCGGTGAAATGCGTAGATATTAGGAGGAACACCAGTGGCGAAAGCGGCTTTCTGGACGACAA CTGACGCTGAGGCGCGAAAGCCAGGGGAGCAAACGGGATTAGATACCCCGGTAGTCCTGGCCGTAAACGATGGATA CTAGGTGTAGGAGGTATCGACCCCTTCTGTGCCGGAGTTAACGCAATAAGTATCCCGCCTGGGGAGTACGGCCGCA AGGCTGAAACTCAAAGGAATTGACGGGGGCCCGCACAAGCGGTGGAACATGTGGTTTAATTCGATGATACGCGAGG AACCTTACCCGGGCTTAAATTGCAGTGGAATGATGTGGAAACATGTCAGTGAGCAATCACCGCTGTGAAGGTGCTG CATGGTTGTCGTCAGCTCGTGCCGTGAGGTGTCGGCTTAAGTGCCATAACGAGCGCAACCCTTATCTTCAGTTACT AACAGGTCATGCTGAGGACTCTGGAGAGACTGCCGTCGTAAGATGTGAGGAAGGTGGGGATGACGTCAAATCAGCA CGGCCCTTACGTCCGGGGCTACACACGTGTTACAATGGGGGGTACAGAGGGCCGCTACCACGCGAGTGGATGCCAA TCCCAAAAACCTCTCTCAGTTCGGACTGGAGTCTGCAACCCGACTCCACGAAGCTGGATTCGCTAGTAATCGCGCA TCAGCCACGGCGCGGTGAATACGTTCCCGGGCCTTGCACTCACCGCCCGT |
| KF842598.1.1394 |
| AGAGTTTGATCCTGGCTCAGGATGAACGCTAGCGACAGGCTTAACACATGCAAGTCGAGGGGCAGCATGATTTGTA GCAATACAGATTGATGGCGACCGGCGCACGGGTGAGTAACGCGTATGCAACTTACCTATCAGAGGGGGATAGCCCG GCGAAAGTCGGATTAATACCCCATAAAACAGGGGTCCCGCATGGGAATATTTGTTAAAGATTCATCGCTGATAGAT AGGCATGCGTTCCATTAGGCAGTTGGCGGGGTAACGGCCCACCAAACCGACGATGGATAGGGGTTCTGAGAGGAAG GTCCCCCACATTGGTACTGAGACACGGACCAAACTCCTACGGGAGGCAGCAGTGAGGAATATTGGTCAATGGCCGA GAGGCTGAACCAGCCAAGTCGCGTGAAGGAAGAAGGATCTATGGTCTGTAAACTTCTTTTATAGGGGAATAAAGTG GAGGACGTGTCCTTTTTTGTATGTACCCTATGAATAAGCATCGGCTAACTCCGTGCCAGCAGCCGCGGTAATACGG AGGATGCGAGCGTTATCCGGATTTATTGGGTTTAAAGGGTGCGTAGGTGGTGATTTAAGTCAGCGGTGAAAGTTTG TGGCTCAACCATAAAATTGCCGTTGAAACTGGGTTACTTGAGTGTGTTTGAGGTAGGCGGAATGCGTGGTGTAGCG GTGAAATGCATAGATATCACGCAGAACTCCGATTGCGAAGGCAGCTTACTAAACCATAACTGACACTGAAGCACGA AAGCGTGGGGATCAAACAGGATTAGATACCCTGGTAGTCCACGCAGTAAACGATGATTACTAGGAGTTTGCGATAC AATGTAAGCTCTACAGCGAAAGCGTTAAGTAATCCACCTGGGGAGTACGCCGGCAACGGTGAAACTCAAAGGAATT GACGGGGGCCCGCACAAGCGGAGGAACATGTGGTTTAATTCGATGATACGCGAGGAACCTTACCCGGGTTTGAACG TAGTCTGACCGGAATGGAAACACTCCTTCTAGCAATAGCAGATTACAAGGTGCTGCATGGTTGCCTCAACTCCGGC CCGGAAGGTCCGGCTTAATTGCCATAACAAGCGCACCCTTTACCAAGGTTCAAACAGGTGAAGCTTGAAGACTCT GTGGAACCTCCCCCCTAACCTGTGAGAAGAAGTGGGGATACACTCAATAAACCACGGCCCTTAATCCCGGGGGGAA CACTGGTTACAATGGGTTGGGAAAGGGGGCTTCCTGGCGACAGGATGCTAATCTCCAAACCATGTCTCAGTTCGGA TCGGAGTCTGCAACTCGACTCCGTGAAGCTGGATTCGCTAGTAATCGCGCATCAGCCATGGCGCGGTGAATACGTT CCCGGGCCTTGTACACACCGCCCGTC |
| FJ950694.1.1472 |
| CGCCCTGATTGACGGCTATACACATGCAAGTCGAACGGTAACAGGAAACAGCTTGCTTCTTTGCTGACGAGTGGCG GACGGGTGAGTAATGTCTGGGAAACTGCCTGATGGAGGGGGATAACTACTGGAAACGGTAGCTAATACCGCATAAC GTCGCAAGACCAAAGAGGGGGACCTTCGGGCCTCTTGCCATCGGATGTGCCCAGATGGGATTAGCTAGTAGGTGGG GTAACGGCTCCATCCCTAGGCGAGCCGAATCCTTAGCCTGGTCTGAGAGGAATGACCAGCCACACTGGGACTGAGA ACACGGTCCAGACTCCTACGGGAGGCAGCAGTGGGGAATATTGCACAATGGGCGCAAGCCTGATGCAGCCATGCCG CGTGTATGAAGAAGGCCTTCGGGTTGTAAAGTACTTTCAGCGGGGAGGAAGGGAGTAAAGTTAATACCCTTTGCTC |
| ATTGACGTTACCCGCAGAAGAAGCACCGGCTAACTCCGTGCCAGCAGCCGCGGTAATACGGAGGGTGCAAGCGTTA ATCGGAATTACTGGGCGTAAAGCGCACGCAGGCGGTTTGTTAAGTCAGATGTGAAATCCCCGGGCTCAACCTGGGA ACTGCATCTGATACTGGCAAGCTTGAGTCTCGTAGAGGGGGGTAGAATTCCAGGTGTAGCGGTGAAATGCGTAGAG ATCTGGAGGAATACCGGTGGCGAAGGCGGCCCCCTGGACGAAGACTGACGCTCAGGTGCGAAAGCGTGGGGAGCAA ACAGGATTAGATACCCTGGTAGTCCACGCCGTAAACGATGTCGACTTGGAGGTTGTGCCCTTGAGGCGTGGCTTCC GGAGCTAACGCGTTAAGTCGACCGCCTGGGGAGTACGGCCGCAAGGTTAAAACTCAAATGAATTGACGGGGGCCCG CACAAGCGGTGGAGCATGTGGTTTAATTCGATGCAACGCGAAGAACCTTACCTGGTCTTGACATCCACGGAAGTT TTCAGAGATGAGAATGTGCCTTCGGGAACCGTGAGACAGGTGCTGCATGGCTGTCGTCAGCTCGTGTTGTGAAATG TTGGGTTAAGTCCCGCAACGAGCGCAACCCTTATCCTTTGTTGCCAGCGGTCCGGCCGGGAACTCAAAGGAGACTG CCAGTGATAAACTGGAGGAAGGTGGGGATGACGTCCAGGTCATCATGGCCCTTACGAACCAGGGCTACACACGTGC CTACAATGGACGCATCCAAAGAGAGAGCGAACCCTGCCCGCGAGAGCAAGCGGACCTCATAAAGTGCGTCGTAGTC CGGATTGGAGTCTGCAACTCGACTCCATGAAGTCGGAATCGCTAGTAATCGTGGATCAGAATGCCACGGTGAATAC GTTCCCGGGCCTTGTACACACCGCCCGTCACACCATGGGAGTGGGTTGCAAAAGAAGTAGGTAGCTTAACCTTCGG GAGGGCGCTTACCACTTTGGATGCGAGG |
| HQ802983.1.1440 |
| TAAGATGAACGCTGGCGGCGTGCTTAACACATGCAAGTCCTATGAAGCGCTTAAACGGATTTCTTCGGATTGAAGT TTTTGTGACTGAGTGGCGGACGGGTGAGTAACGCGTGGGTAACTTGCCTCATACAGGGGGATAACAGTTAGAAATG ACTGCTAATACCGCATAAGCGCACAGTGCTGCATGGCACAGTGTGAAAAACTCCGGTGGTATGAGATGGACCCGCG TCTGATTAGCTAGTTGGTGGGGTAACGGCCTACCAAGGCGACGATCAGTAGCCGGCCTGAGAGGGTGAACGGCCAC ATTGGGACTGAGACACGGCCCAAACTCCTACGGGAGGCAGCAGTGGGGAATATTGCACAATGGGGGAAACCCTGAT GCAGCGACGCCGCGTGAGCGAAGAAGTATTTCGGTATGTAAAGCTCTATCAGCAGGGAAGAAAATGACGGTACCTG ACTAAGAAGCACCGGCTAAATACGTGCCAGCAGCCGCGGTAATACGTATGGTGCAAGCGTTATCCGGATTTACTGG GTGTAAAGGGAGCGTAGACGGTTGTGTAAGTCTGATGTGAAAGCCCGGGGCTCAACCCCGGGACTGCATTGGAAAC TATGTAACTAGAGTGTCGGAGAGGTAAGCGGAATTCCTAGTGTAGCGGTGAAATGCGTAGATATTAGGAGGAACAC CAGTGGCGAAGGCGGCTTACTGGACGATCACTGACGTTGAGGCTCGAAAGCGTGGGGAGCAAACAGGATTAGATAC CCTGGTAGTCCACGCCGTAAACGATGACTACTAGGTGTCGGGGCCCATAAGGGCTTCGGTGCCGCAGCAAACGCAA TAAGTATTCCACCTGGGGAGTACGTTCGCAAGAATGAAACTCAAAGGAATTGACGGGGACCCGCACAAGCGGTGGA GCATGTGGTTTAATTCGAAGCAACGCGAAGAACCTTACCTGGTCTTGACATCCCACTGACCGGACAGTAATGTGTC CTTTCCTCCGGGACAGTGGAGACAGGTGGTGCATGGTTGTCGTCAGCTCGTGTCGTGAGATGTTGGGTTAAGTCCC GCAACGAGCGCAACCCCTATCCTTAGTAGCCAGCAGTAAGATGGGCACTCTAGGGAGACTGCCAGGGATAACCTGG AGGAAGGTGGGGATGACGTCAAATCATCATGCCCCTTATGACTTGGGCTACACACGTGCTACAATGGCGTAAACAA AGTGAAGCGAAGTCGTGAGGCCAAGCAAATCACAAAAATAACGTCTCAGTTCGGATTGTAGTCTGCAACTCGACTA CAAGAAGCTGGAATCGCTAGTAATCGCAGATCAGAATGCTGCGGTGAATACGTTCCCGGGTCTTGTACACACCGCC CGTCACACCATGGGAGTCGAAAATGCCCGAAGTCGGTGACCTAACGAAAGAAGGAGCCGCCGAAGGCAGGTT |
| GQ448468.1.1366 |
| AGAGTTTGATCCTGGCTCAGGATGAACGCTGACAGAATGCTTAACACATGCAAGTATACTTGATCCTTCGGGTGAT GGTGGCGGACGGGTGAGTAACGCGTAAAGAACTTGCCCTGCAGTCTGGGACAACATTTGGAAACGAATGCTAATCC CGCATAAGCCCACAGCTCGGCATCGAGCAGAGGGAAAAGGAGTGATCTGCTTTGAGATGGCCTCGCGTCCGATTAG CTGGTTGGTGAGGTGACGGCCCATCAAGGCAACGATCGGTAGCCGGACTGAGAGGTTGAACGGCCACATTGGGATT GAGACACGGCCCTTACTCCTACGGGAGGCAGCAGTGGGGAATATTGGACAATGGACCAAAAGTCTGATCCAGCAAT TCTGTGTGCACGATGAAGTTTTTCGGAATGTAAAGTGCTTTCAGTTGGGACGAAGTAAGTGACGGTACCAACAGAA GAAGCGACGGCTAAATACGTGCCAGCAGCCGCGGTAATACGTATGTCGCAAGCGTTATCCGGATTTATTGGGCGTA AAGCGCGTCTAGGCGGTTTGGTAAGTCTGATGTGAAAATGCGGGGCTCAACTCCGTATTGCGTTGGAAACTGCCAA ACTAGAGTACTGGAGAGGTGGGCGGAACTACAAGTGTAGAGGTGAAATTCGTAGATATTTGTAGGAATGCCGATGG GGAAGCCAGCCCACTGGACAGATACTGACGCTAAAGCGCGAAAGCGTGGGTAGCAAACAGGATTAGATACCCTGGT AGTCCACGCCGTAAACGATGATTACTAGGTGTTGGGGGTCGAACCTCAGCGCCCAAGCTAACGCGATAAGTAATCC GCCTGGGGAGTACGTACGCAAGTATGAAACTCAAAGGAATTGACGGGGACCCGCACAAGCGGTGGAGCATGTGGTT TAATTCGACGCAACGCGAGGAACCTTACCAGCGTTTGACATCCTAAGAAATTAGCAGAGATGCTTTTGTGCCCCTT CGGGGGAACTTAGTGACAGGTGGTGCATGGCTGTCGTCAGCTCGTGTCGTGAGATGTTGGGTTAAGTCCCGCAACG AGCGCAACCCCTTTCGTATGTTGCCATCATTAAGTTGGGCACTCATGCGATACTGCCTGCGATGAGCAGGAGGAAG GTGGGGATGACGTCAAGTCATCATGCCCCTTATACGCTGGGCTACACACGTGCTACAATGGGTAGTACAGAGAGTC GCAAACCTGCGAGGGGGAGCTAATCTCAGAAAACTATTCTCAGTTCGGATTGTACTCTGCAACTCGAGTACATGAA GTTGGAATCGCTAGTAATCGCAAATCAGCTATGTTGCGGTGAATACGTTCTCGGGTCTTGTACACACCGCCCGT |
| JN387556.1.1324 |
| CGTAAGTAACCTGCCCTGTACACACGGATAACATACCGAAAGGTATGCTAATACGGATAATATATTTTGATCGCA TGGTCGAGATATCAAAGCTCCGGCGGTACACCAGGGACCCCCGACAGAGGAGCTAGTTGGTAGTAATGTCACCAAG GCGACGATCAGAAGCCGAACTGAGAGGGGGATCCGCACATGACTGAGACACGGTCAAACTCCTACGGGAGGCAGCA GTGGGGAATATGCCAATGGGCGAAAGCTGATGCAGCACGCGCGTGAGCGATGAGGCTCGGGTCGTAAAGCTCGTCT CAAGGAAGATAATGACGGTACTTGAGGAGGAAGCCCCGGCTAACTACGTGCCAGCAGCCGCGGTAATACGTAGGGG GCTAGCGTTATCCGGAATTACTGGGCGTAAAGGGTGCGTAGGCGGTCTTTCAAGTCAGGAGTGAAAGGCTACGGCT CAACCGTAGTAAGCTCTTGAAACTGTAAGACTTGAGTGCAGGAGAGGAGAGTGGAATTCCTAGTGTAGCGGTGAAA TGCGTAGATATTAGGAGGAACACCAGTTGCGAAGGCGGCTCTCTGGACTGTAACTGACGCTGAGGCACGAAAGCGT |
| GGGGAGCAAACAGGATTAGATACCCTGGTAGTCCACGCCGTAAACGATGAGTACTAGCTGTCGGAGGTTACCCCCT TCGGTGGCGCAGCTAACGCATTAAGTACTCCGCCTGGGAAGTACGCTCGCAAGAGTGAAACTCAAAGGAATTGACG GGGACCCGCACAAGTAGCGGAGCATGTGGTTTAATTCGAAGCAACGCGAAGAACCTTACCTAAGCTTGACATCCCA CTGACCCTTCCCTAATCGGAAGCTTCCCTTCGGGACAGTGGTGACAGGTGGTGCATGGTTGTCGTCAGCTCGTGTC GTGAGATGTTGGGTTAAGTCCCGCAACGAGCGCAACCCTTGCCTTTAGTTGCCAGCATTAAGTTGGGCACTCTAGA GGGACTGCCAGGGATAACCCGGAGGAGTGGGGATGACGTCAAATCATCATGCCCTTATGCTAGGCTACACACGTGC TACAATGGGTGGTCAGAGGCCAGCCAGTCGTGAGGCCGAGCTATCCCATAAGCCATTCTCGTCCGGATTGTAGGCT GAACTCGCCTACATGAGCTGGAATTACAAGTATGCGATCGATGCTGCGTGATGCGTCCGGGTCTTGTACACACCGC CCGTCACACCATGGGAGTTGGGGGCGCCCGAAGCCGGATTGCTAACCTTTTGGAAGCGTCCGTCGAAGGTGAAACC AATAACTGGGGTGAAGTCGTAACAAGGTAACC |
| |

| **OTUs in Table 4** |
|---|
| JRPJ01000002.1034290.1035971 |
| AGAGTTTGATCCTGGCTCAGAGTGAACGCTGGCGGCGTGCCTAATACATGCAAGTCGAACGATGAAACTTCTAGCT TGCTAGAAGTGGATTAGTGGCGCACGGGTGAGTAATGCATAGGTAACATGCCCTTTAGTCTGGGATAGCCACTGGA AACGGTGATTAATACTGGATACTCCCTACGGGGGAAAGGGGCTTTCAATAAAGAATTTCTCTTTTTAGTGTTTTGT GTTGTTGGCACAAAATTCTAGTATTTGGAATGAGAAATTGGTGTTGTGAAGCAATTTGTGCGGAGATTAGACTTAG TGTCTGTCGTGTCAGCAAATTGCGAACTCATCGATTTATCATCCAAAGACGAATTTTTTATTGAAAGCCTTCGCTA AAGGATTGGCCTATGTCCTATCAGCTTGTTGGTGAGGTAATGGCTCACCAAGGCTATGACGGGTATCCGGCCTGAG AGGGTGATCGGACACACTGGAACTGAGACACGGTCCAGACTCCTACGGGAGGCAGCAGTAGGGAATATTGCTCAAT GGGGGAAACCCTGAAGCAGCAACGCCGCGTGGAGGATGAAGGTTTTAGGATTGTAAACTCCTTTTGTAAGAGAAGA TTATGACGGTATCTTACGAATAAGCACCGGCTAACTCCGTGCCAGCAGCCGCGGTAATACGGAGGGTGCAAGCGTT ACTCGGAATCACTGGGCGTAAAGAGCGCGTAGGCGGGTGGTCAAGTCAGATGTGAAATCCTGTAGCTTAACTACAG AACTGCATTTGAAACTGACCATCTAGAGTATGGGAGAGGTAGGTGGAATTCTTGGTGTAGGGGTAAAATCCGTAGA GATCAAGAGGAATACTCATTGCGAAGGCGACCTGCTGGAACATTACTGACGCTGATGCGCGAAAGCGTGGGGAGCA AACAGGATTAGATACCCTGGTAGTCCACGCCCTAAACGATGAATGCTAGTTGTTGTGAGGCTTGTCCTTGCAGTAA TGCAGCTAACGCATTAAGCATTCCGCCTGGGGAGTACGGTCGCAAGATTAAAACTCAAAGGAATAGACGGGGACCC GCACAAGCGGTGGAGCATGTGGTTTAATTCGATGATACGCGAAGAACCTTACCTAGGCTTGACATTGATAGAATCT ACTAGAGATAGTGGAGTGCCCTTTTAGGGAGCTTGAAAACAGGTGCTGCACGGCTGTCGTCAGCTCGTGTCGTGAG ATGTTGGGTTAAGTCCCGCAACGAGCGCAACCCTCGTCCTTAGTTGCTAGCAGTTTGGCTGAGCACTCTAAGGAGA CTGCCTTCGTAAGGAGGAGGAAGGTGAGGACGACGTCAAGTCATCATGGCCCTTACGCCTAGGGCTACACACGTGC TACAATGGGGTGCACAAAGAGATGCAATAGTGTGAGCTGGAGCCAATCTCTAAAACATCTCTCAGTTCGGATTGTA GTCTGCAACTCGACTACATGAAGCTGGAATCGCTAGTAATCGCAAATCAGCAATGTTGCGGTGAATACGTTCCCGG GTCTTGTACTCACCGCCCGTCACACCATGGGAGTTGTATTTGCCTTAAGTCGGAATGCTAAATTGGCTACCGCCCA CGGCAGATGCAGCGACTGGGGTGAAGTCGTAACAAGGTAACCGTAGGTGAACCTGCGGTTG |
| New.ReferenceOTU45 |
| TACGGAGGGTGCAAGCGTTAATCGGAATAACTGGGCGTAAAGGGCATGTAGGCGGAAAGGCAAGCAAGATGTGAAA GACCTGGGCTCAACCTGGGTTGGTCATTTTGAACTACCTTTCTAGAGTATTGCAGAGGGAGATGGAATTTCAGGTG TAGCGGTGGAATGCGTAGATATCTGAAAGAACACCAGAGGCGAAGGCGGTCTCCTGGGCAAATACTGACGCTGAGG TGCGAAAGCGTGGGGAGCAAACAGG |
| GQ006324.1.1342 |
| GACGAACGCTGGCGGCGTGCTTAACACATGCAAGTCGAACGGAAAGGCCCAGCTCGCTGGGTACTCGAGTGGCG AACGGGTGAGTAACACGTGGGTGATCTGCCTTGCACTCTGGGATAAGCTTGGGAAACTGGGTCTAATACCGGATAT GAACTGCCTTTAGTGTGGTGGTTGGAAAGTTTTTCGGTGCAAGATGAGCTCGCGGCCTATCAGCTTGTTGGTGGG GTAATGGCCTACCAAGGCGTCGACGGGTAGCCGGCCTGAGAGGGTGTACGGCCACATTGGGACTGAGATACGGCCC AGACTCCTACGGGAGGCAGCAGTGGGGAATATTGCACAATGGGCGGAAGCCTGATGCAGCGACGCCGCGTGGGGGA TGACGGCCTTCGGGTTGTAAACTCCTTTCGACAGGGACGAAGCTTTTTGTGACGGTACCTGTATAAGAAGCACCGG CTAACTACGTGCCAGCAGCCGCGGTAATACGTAGGGTGCGAGCGTTGTCCGGAATTACTGGGCGTAAAGAGCTCGT AGGTGGTTTGTCGCGTCGTCTGTGAAATTCCGGGGCTTAACTCCGGGCGTGCAGGCGATACGGGCATAACTTGAGT ACTGTAGGGGAGACTGGAATTCCTGGTGTAGCGGTGAAATGCGCAGATATCAGGAGGAACACCGGTGGCGAAGGCG GGTCTCTGGGCAGTAACTGACGCTGAGGAGCGAAAGCATGGGGAGCGAACAGGATTAGATACCCTGGTAGTCCATG CCGTAAACGGTGGGCGCTAGGTGTGGGTTTCCTTCCACGGGATCCGTGCCGTAGCTAACGCATTAAGCGCCCCGCC TGGGGAGTACGGCCGCAAGGCTAAAACTCAAAGGAATTGACGGGGGCCCGCACAAGCGGCGGAGCATGTGGATTAA TTCGATGCAACGCGAAGAACCTTACCTGGGCTTGACATACACTGGATCGGGCTAGAGATAGTCTTTCCCTTTGTGG CTGGTGTACAGGTGGTGCATGGTTGTCGTCAGCTCGTGTCGTGAGATGTTGGGTTAAGTCCCGCAACGAGCGCAAC CCTTGTCTTATGTTGCCAGCATTTGGTTGGGGACTCATGAGAGACTGCCGGGGTCAACTCGGAGGAAGGTGGGGAT GACGTCAAATCATCATGCCCCTTATGTCCAGGGCTTCACACATGCTACAATGGTCGGTACAACGCGCAGCGACACT GTGAGGTGGAGCGAATCGCTGAAAGCCGGCCTTAGTTCGGATTGGGGTCTGCAACTCGACCCCATGAAGTCGGAGT CGCTAGTAATCGCAGATCAGCAATGCTGCGGTGAATACGTTCCCGGGCCT |
| HK555938.1.1357 |
| ACGGCACCCCTCTCCGGAGGGAAGCGAGTGGCGAACGGCTGAGTAACACGTGGAGAACCTGCCCCCTCCCCCGGGA TAGCCGCCCGAAAGGACGGGTAATACCGGATACCCCCGGGCGCCGCATGGCGCCCGGGCTAAAGCCCCGACGGGAG GGGATGGCTCCGCGGCCCATCAGGTAGACGGCGGGGTGACGGCCCACCGTGCCGACAACGGGTAGCCGGGTTGAGA GACCGACCGGCCAGATTGGGACTGAGACACGGCCCAGACTCCTACGGGAGGCAGCAGTGGGGAATCTTGCGCAATG GGGGGAACCCTGACGCAGCGACGCCGCGTGCGGGACGGAGGCCTTCGGGTCGTAAACCGCTTTCAGCAGGGAAGAG TCAAGACTGTACCTGCAGAAGAAGCCCCGGCTAACTACGTGCCAGCAGCCGCGGTAATACGTAGGGGGCGAGCGTT ATCCGGATTCATTGGGCGTAAAGCGCGCGTAGGCGGCCCGGCAGGCCGGGGGTCGAAGCGGGGGGCTCAACCCCCC GAAGCCCCCGGAACCTCCGCGGCTTGGGTCCGGTAGGGGAGGGTGGAACACCCGGTGTAGCGGTGGAATGCGCAGA TATCGGGTGGAACACCGGTGGCGAAGGCGGCCCTCTGGGCCGAGACCGACGCTGAGGCGCGAAAGCTGGGGGAGCG AACAGGATTAGATACCCTGGTAGTCCCAGCCGTAAACGATGGACGCTGGGTGTGGGGGGACGATCCCCCCGTGCCG CAGCCNACGCATTAAGCGTCCCGCCTGGGGAGTACGGCCGCAAGGCTAAAACTCAAAGGAATTGACGGGGGCCCGC ACAAGCAGCGGAGCATGTGGCTTAATTCGAAGCAACGCGAAGAACCTTACGGCGCATCCCCCCGAGGCCCACGGGG GGTCCGCCGCGTGGGTCAGAGGAGCGCATACGGAGGTGCATGGTTGTCGTCAGCTCGTGTCGTGAGATGTTGGGT TAAGTCCCGCAACGAGCGCAACCCCCGCCGCGTGTTGCCATCGGGTGATGCCGGGAACCCACGCGGGACCGCCGCC GTCAAGGCGGAGGAGGGCGGGGACGACGTCAAGTCATCATGCCCCTTATGCCCTGGGCTGCACACGTGCTACAATG GCCGGTACAGAGGGATGCCACCCCGCGAGGGGGAGCGGATCCCGGAAAGCCGGCCCCAGTTCGGATTGGGGGCTGC AACCCGCCCCCATGAAGTCGGAGTTGCTAGTAATCGCGGATCAGCATGCCGCGGTGAATGCGTTCCCGGGCCTTGT ACACACCGCCCGTCACACCACCCGAGTCGTCTGCACCCGAAGTCGCCGGCCCAACCGCAAGGGGG |
| FJ957551.1.1489 |
| AGAGTTTGATCCTGGCTCAGGACGAACGCTGGCGGCGTGCCTAATACATGCAAGTCGAGCGGAGTTACTTTGAGAG CTTGCTTTCAAAGTAACTTAGCGGCGGACGGGTGAGTAACACGTAGGCAACCTGCCCCTTAGACTGGGATAACTAC CGGAAACGGTAGCTAATACCGGATAATTTCTTTTTTCTCCTGAAGGAAGAATGAAAGACGGAGCAATCTGTCACTG AGGGATGGGCCTGCGGCGCATTAGCTAGTTGGTGGGGTAACGGCCCACCAAGGCGACGATGCGTAGCCGACCTGAG AGGGTGATCGGCCACATTGGAACTGAGATACGGTCCAGACTCCTACGGGAGGCAGCAGTGGGGAATATTGCACAAT GGGGGAAACCCTGATGCAGCAACGCCGCGTGAGTGATGAAGGTCTTCGGATTGTAAAGCTCTGTCTTTAGGGACGA TAATGACGGTACCTAAGGAGGAAGCCACGGCTAACTACGTGCCAGCAGCCGCGGTAATACGTAGGTGGCAAGCGTT ATCCGGATTTACTGGGCGTAAAGGGAGCGTAGGCGGATATTTAAGTGGGATGTGAAATACCCGAGCTTAACTTGGG AGCTGCATTCCAAACTGGATATCTAGAGTGCAGGAGAGGAGAATGGAATTCCTAGTGTAGCGGTGAAATGCGTAGA GATTAGGAAGAACACCAGTGGCGAAGGCGATTCTCTGGACTGTAACTGACGCTGAGGCTCGAAAGCGTGGGGAGCA AACAGGATTAGATACCCTGGTAGTCCACGCCGTAAACGATGAATACCAGGTGTAGGGGCCCCAAGCCTCTGTGCCG CCGCTAACGCATTAAGTATTCCGCCTGGGGAGTACGGTCGCAAGATTAAAACTCAAAGGAATTGACGGGGACCCGC ACAAGCAGCGGAGCATGTGGTTTAATTCGAAGCAACGCGAAGAACCTTACCTAGACTTGACATGTCCTGAATTACC AGTAATGTGGGAAGTTCCTTCGGGAACAGGAACACAGGTGGTGCATGGTTGTCGTCAGCTCGTGTCGTGAGATGTT GGGTTAAGTCCCGCAACGAGCGCAACCCTTATTGTTAGTTGGTACCATTAAGTTGACCACTCTAGCGAGACTGCCC GGGTTAACCGGGAGGAAGGTGGGGATGACGTCAAATCATCATGCCCCTTATGTCTAGGGCTACACACGTGCTACAA TGGCAAGTACAAAGAGAAGCAATACTGTGAAGTGGAGCAAAACTCAAAAACTTGTCTCAGTTCGGATTGTAGGCTG AAACTCGCCTACATGAAGCTGGAGTTGCTAGTAATCGCGAATCAGAATGTCGCGGTGAATACGTTCCCGGGTCTTG TACACACCGCCCGTCACACCATGAGAGTTGGCAATACCCGAAGTCCGTAAGCTAACCGTAAGGAGGCAGCGGCCGA AGGTAGGGTCAGCGATTGGGGTGAAGTCGTAACAAGGTAACCAA |
| FJ957494.1.1454 |
| TGAGTTTGATCATGGCTCAGGACGAACGCTGGCGGCGTGCCTAACACATGCAAGTCGAGCGATGAAATTTTCTTCG GAAAATGGATTAGCGGCGGACGGGTGAGTAACACGTGGGTAACCTGCCCTATAGAGAGGGATAGCCTTCCGAAAGG GAGATTAATACCTCATAATATCCTAGTATCGCATGATACATGGATTAAAGGAGCAATCCGCTATAGGATGGACCCG CGGCGCATTAGCTAGTTGGTGAGGTAACGGCTCACCAAGGCGACGATGCGTAGCCGACCTGAGAGGGTGATCGGCC ACATTGGGACTGAGACACGGCCCAGACTCCTACGGGAGGCAGCAGTGGGGAATATTGCACAATGGGGGAAACCCTG ATGCAGCAACGCCGCGTGAGTGATGACGGTCTTCGGATTGTAAAGCTCTGTCTTTAGGGACGATAATGACGGTACC TAAGGAGGAAGCCACGGCTAACTACGTGCCAGCAGCCGCGGTAATACGTAGGTGGCAAGCGTTGTCCGGATTTACT GGGCGTAAAGGGAGCGTAGGCGGATCTTTAAGTGGGATGTGAAATACTCGGGCTCAACCTGGGGGCTGCATTCCAA ACTGGGGATCTAGAGTACAGGAGGGGNGAGTGGAATTCCTAGTGTAGCGGTGAAATGCGTAGAGATTAGGAAGAAC ACCAGTGGCGAAGGCGACTNTCTGGACTGTAACTGACGCTGAGGCTCGAAAGCGTGGGGAGCAAACAGGATTAGAT ACCCTGGTAGTCCACGCCGTAAACGATGAATACTAGGTGTAGGGGGTGTCAACTCCCCCTGTGCCGCCGCTAACGC ATTAAGTATTCCGCCTGGGGAGTACGGTCGCAAGATTAAAACTCAAAGGAATTGACGGGGGCCCGCACAAGTAGCG GAGCATGTGGTTTAATTCGACGCAACGCGAAGAACCTTACCTAGACTTGACATCTTCTGCATTACCCTTAATCGGG GAAGTTCCTTCGGGGACAGAATGACAGGTGGTGCATGGTTGTCGTCAGCTCGTGTCGTGAGATGTTGGGTTAAGTC CCGCAACGAGCGCAACCCTTAAGCTTAGTTGCCATCATTAAGTTGGGCACTCTAAGTTGACTGCCGGTGACAAACC GGAGGAAGGTGGGGATGACGTCAAATCATCATGCCCCTTATGTCTAGGGCTACACACGTGCTACAATGGCAAGTAC AAAGAGAAGCAATACTGTGAAGTGGAGCAAAACTCAAAAACTTGTCTCAGTTCGGATTGTAGGCTGAAACTCGCCT ACATGAAGCTGGAGTTGCTAGTAATCGCGAATCAGAATGTCGCGGTGAATACGTTCCCGGGTCTTGTACACACCGC CCGTCACACCATGAGAGTTGGCAATACCCGAAGTCCGTAAGCTAACCGTAAGGAGGCAGCGGCCGAAGGTAGGGTC AGCGATGGGG |
| New.ReferenceOTU52 |
| TACGTAGGTGGCGAGCGTTATCCGGATTTACTGGGCGTAAAGGGAGCGTAGGCGGATGATTAAGTGGGATGTGAAA TACCCGGGCTCAACTTGGGTGCTGCATTCCAAACTGGTTATCTAGAGTGCAGGAGAGGAGAGTGGAATTCCTAGTG TAGCGGTGAAATGCGTAGAGATTAGGAAGAACACCAGTGGCGAAGGCGACTCTCTGGACTGTAACTGACGCTGAGG CTCGAAAGCGTGGGGAGCAAACAGG |
| FM865905.1.1392 |
| GGGAATCTCCAGGATCTGATTAGCGGCGGACGGGTGAGTACACGTGGGTAACCTGCCTCATAGAGTGGAATAGCCT TCCGAAAGGAAGATTAATACCGCATAACGTTGAAAGATGGCATCATCATTCAACCAAAGGAGCAATCCGCTATGAG ATGGACCCGCGGCGCATTAGCTAGTTGGTGGGGTAACGGCCTACCAAGGCGACGATGCGTAGCCGACCTGAGAGGG TGATCGGCCACATTGGGACTGAGACACGGCCCAGACTCCTACGGGAGGCAGCAGTGGGGAATATTGCACAATGGGG GAAACCCTGATGCAGCAACGCCGCGTGAGTGATGAAGGTTTTCGGATCGTAAAGCTCTGTCTTTGGGGAAGATAAT GACGGTACCCAAGGAGGAAGCCACGGCTAACTACGTGCCAGCAGCCGCGGTAATACGTAGGTGGCGAGCGTTATCC GGATTTACTGGGCGTAAAGGGAGCGTAGGCGGATGATTAAGTGGGATGTGAAATACCCGGGCTCAACTTGGGTGCT GCATTCCAAACTGGTTATCTAGAGTGCAGGAGAGGAGAGTGGAATTCCTAGTGTAGCGGTGAAATGCGTAGAGATT AGGAAGAACACCAGTGGCGAAGGCGACTCTCTGGACTGTAACTGACGCTGAGGCTCGAAAGCGTGGGGAGCAAACA GGATTAGATACCCTGGTAGTCCACGCCGTAAACGATGAATACTAGGTGTGGGGGTTTCAACACCTCCGTGCCGCCG CTAACGCATTAAGTATTCCGCCTGGGGAGTACGGTCGCAAGATTAAAACTNAAAGGAATTGACGGGGATCNNCACN AGTAGCGGAGCATGTGGTTTAATTCGAAGCAACGCGAAGAACCTTACCTACACTTGACATCCCTTGCATTACTCTT AATCGAGGAAATCTCTTCGGGGACAAGGTGACAGGTGGTGCATGGTTGTCGTCAGCTCGTGTCGTGAGATGTTGGG TTAAGTCCCGNAACGAGGGGAACCNTTGTCGTTAGTTACTACCATTAAGTTGAGGACTNTAGNGAGACNGCTGGGT TAACNAGGAGGAAGGTGGGGATGACTCAATCTCTGGNCNTTATGTGTAGGGNTACACACGTGCTACAATGGCTGGT ACAGAGAGATGCATACCGGGAGGTGGANTCAATTTAAAAACAGTNTCNTTCGGATTGTAGGNTGAANTNNCCTACT GAAGNTGGAGTTANTAGTAATCGCGAATCAGAATGTCGCGGTGAATACGTTCCCGGGTCTTGTACACNCCNCCCGT CACNCCATGAGAGTTGGCAATACCCGAAGTCCGTGAGCTAACCGCAAGGAGGCAGCGGCCGAAGGTAGGGTCAGCG ATTGGGGTGAAGTCGTAACAGGNA |
| GQ016239.1.1362 |
| GATGAACGCTGGCGGCATGCCTAATACATGCAAGTCGAACGGAGCGAATATGGAAGCTTGCTTCCGTAAGAGCTCA GTGGCGAACGGGTGAGTAACACGTAGGTAACCTGCCCATGTGCCCGGGATAACTGCTGGAAACGGTAGCTAAAACC GGATAGGTGAATAGGAGGCATCTCTTATTCATTAAAGGACCTGTAAGGGTGCGAACATGGATGGACCTGCGGCGCA TTAGCTGGTTGGAGTGGTAACGGCACACCAAGGCGACGATGCGTAGCCGACCTGAGAGGGCGAACGGCCACATTGG GACTGAGACACGGCCCAAACTCCTACGGGAGGCAGCAGTAGGGAATTTTCGTCAATGGGGGGAACCCTGAACGAGC AATGCCGCGTGAGTGAAGAAGGTCTTCGGATCGTAAAGCTCTGTTGTAAGTGAAGAACGGTCAGTAGAGGAAATGA TACTGAAGTGACGGTAGCTTACCAGAAAGCCACGGCTAACTACGTGCCAGCAGCCGCGGTAATACGTAGGTGGCGA GCGTTATCCGGAATCATTGGGCGTAAAGGGTGCGCAGGTGGTACATTAAGTCCGAAGTAAAAGGCAGCAGCTCAAC TGCTGTTGGCTTTGGAAACTGGTGAACTGGAGTGCAGGAGAGGGCGATGGAATTCCATGTGTAGCGGTAAAATGCG TAGATATATGGAGGAACACCAGTGGCGAAGGCGGTCGCCTGGCCTGCAACTGACACTGAGGCACGAAAGCGTGGGG AGCAAATAGGATTAGATACCCTAGTAGTCCACGCCGTAAACGATGAGAACTAAGTGTTGGGGGAGACTCAGTGCTGC AGTTAACGCAATAAGTTCTCCGCCTGGGGAGTATGCACGCAAGTGTGAAACTCAAAGGAATTGACGGGGGCCCGCA CAAGCGGTGGAGTATGTGGTTTAATTCGAAGCAACGCGAAGAACCTTACCAGGCCTTGACATGGATGTAAATGTTC TAGAGATAGAAAGATAGCTATACATCACACAGGTGGTGCATGGTTGTCGTCAGCTCGTGTCGTGAGATGTTGGGTT AAGTCCCGCAACGAGCGCAACCCTTATCGCATGTTACCAGTATTGAGTTAGGGACTCATGCGAGACTGCCGGTGAC AAACCGGAGGAAGGTGGGGATGACGTCAAATCATCATGCCCCTTATGGCCTGGGCTACACACGTACTACAATGGCG GCTACAAAGAGAAGCGAACCTGCGAGGGGGAGCGGAACTCATAAAGGCCGTCTCAGTTCGGATTGGAGTCTGCAAC TCGACTCCATGAAGTCGGAATCGCTAGTAATCGCAGATCAGCATGCTGCGGTGAATACGTTCTCGGGCCT |
| HG798451.1.1400 |
| CTTGTGTCACCAACCATAGGGAGGGGAAAACATGGAAACGGGGTTCATACCGCATAACTTTTTAGCCCAATGCA TAAGAAGAAAGGCCTTTCGGGTTTCGGTAAAGGAGGCCCCCGCGGCTCTTATAGTGTGTGTGGAAGTAACCGCTTC CACAAGGCCCAGGTTTCATACCCGACTGGAGAGTGTGTTCGCCACACTGGGGAAAGGACCCCCGGCCCAGTCTCTC TAGGGGAGGCAGCAGTAGGAATTTTCGGCAAAGGAAAAAATTTCTGACCGAACAACGCCGGTTGAATGAAGAAGTT TTTCGGATCGAAAAACTCTGTTGTTAGAGAAGAACAAGGACGTTAGTAACTGAACGTCCCCTGACGGTATCTAACC AGAAAGCCACGGCTAATTACGTGCCAGCAGCCGCGGTAATACGTAGGTGGCAAGCGTTGTCCGGATTTATTGGGCG TAAAGCGAGCGCAGGCGGTTTCTTAAGTCTGATGTGAAAGCCCCCGGCTCAACCGGGGAGGGTCATTGGAAACTGG GAGACTTGAGTGCAGAAGAGGAGAGTGGAATTCCATGTGTAGCGGTGAAATGCGTAGATATATGGAGGAACACCAG TGGCGAAGGCGGCTCTCTGGTCTGTAACTGACGCTGAGGCTCGAAAGCGTGGGGAGCAAACAGGATTAGATACCCT GGTAGTCCACGCCGTAAACGATGAGTGCTAAGTGTTGGAGGGTTTCCGCCCTTCAGTGCTGCAGCAAACGCATTAA GCACTCCGCCTGGGGAGTACGACCGCAAGGTTGAAACTCAAAGGAATTGACGGGGGCCCGCACAAGCGGTGGAGCA TGTGGTTTAATTCGAAGCAACGCGAAGAACCTTACCAGGTCTTGACATCCTTTGACCACTCTAGAGATAGAGCTTT CCCTTCGGGGACAAAGTGACAGGTGGTGCATGGTTGTCGTCAGCTCGTGTCGTGAGATGTTGGGTTAAGTCCCGCA ACGAGCGCAACCCTTATTGTTAGTTGCCATCATTTAGTTGGGCACTCTAGCGAGACTGCCGGTGACAAACCGGAGG AAGGTGGGGATGACGTCAAATCATCATGCCCCTTATGACCTGGGCTACACACGTGCTACAATGGAAGTACAACGA GTCGCTAGACCGCGAGGTCATGCAAATCTCTTAAAGCTTCTCTCAGTTCGGATTGCAGGCTGCAACTCGCCTGCAT GAAGCCGGAATCGCTAGTAATCGCGGATCAGCACGCCGCGGTGAATACGTTCCCGGGCCTTGTACACACCGCCCGT |
| CACACCACGAGAGTTTGTAACACCCGAAGTCGGTGAGGTAACCTTTTTGGAGCCAGCCGCCTAAGGTGGGATAGAT GATTGGGGTGAAGTCGTAACCAACGTATGCC |
| EU461791.1.1414 |
| AGAGTTTGATCCTGGCTCAGGACTAACGCTGGCGGCGTGCCTAATACATGCAAGTAGAACGCTGAAGACTTTAGCT TGCTAAAGTTGGAAGAGTTGCGAACGGGTGAGTAACGCGTAGGTAACCTGCCTACTAGCGGGGGATAACTATTGGA AACGATAGCTAATACCGCATAACAGCATTTAACCCATGTTAGATGCTTGAAAGGAGCAATTGCTTCACTAGTAGAT GGACCTGCGTTGTATTAGCTAGTTGGTGAGGTAACGGCTCACCAAGGCGACGATACATAGCCGACCTGAGAGGGTG ATCGGCCACACTGGGACTGAGACACGGCCCAGACTCCTACGGGAGGCAGCAGTAGGGAATCTTCGGCAATGGGGGC AACCCTGACCGAGCAACGCCGCGTGAGTGAAGAAGGTTTTCGGATCGTAAAGCTCTGTTGTAAGAGAAGAACGTGT GTGAGAGTGGAAAGTTCACACAGTGACGGTAACTTACCAGAAAGGGACGGCTAACTACGTGCCAGCAGCCGCGGTA ATACGTAGGTCCCGAGCGTTGTCCGGATTTATTGGGCGTAAAGCGAGCGCAGGCGGTTTAATAAGTCTGAAGTTAA AGGCAGTGGCTTAACCATTGTTCGCTTTGGAAACTGTTAGACTTGAGTGCAGAAGGGGAGAGTGGAATTCCATGTG TAGCGGTGAAATGCGTAGATATATGGAGGAACACCGGTGGCGAAAGCGGCTCTCTGGTCTGTAACTGACGCTGAGG CTCGAAAGCGTGGGGAGCAAACAGGATTAGATACCCTGGTAGTCCACGCCGTAAACGATGAGTGAAAGGTGTTAGG CCCTTTCCGGGGCTTAGTTGCTGCACGCTAACTGCATTATGACACTCCGCCAGGGGAGTACGACCGCTAGGTTGAA ACTCAAAGGAGTTGACGGGGGCCAGCACAACCGGTGGAGCATGTGGTTGAATTGGAAGCAACGCGAAGAGCCTTAC CAGGTCTTGACATCCCGACGCTATTCCTAGAGATAGGAAGTTTCTTCGGGACATTCGGTGGCAGGTGGTGCATGGT AGTCGTCAGCTCGTGTCGTGAGATGTTGGGTTAAGTCCCGCAACGAGCGCAACCCCTATTGTTAGTTGCCATACAT TAAGTTGGGCACTCTAGCGAGACTGCCGGTAATAAACCGGAGGAAGGTGGGGATGACGTCAAATCATCATGCCCCT TATGACCTGGGCTACACACGACGCTACAATGGTTGGTACAACGAGTCGCGAGTCGGTGACGGCAAGCAAATCTCTT AAAGCCAATCTCAGTTCGGATTGTAGGCTGCAACTCGCCTACATGAAGTCGGAATCGCTAGTAATCGCGGATCAGC ACGCCGCGGTGAATACGTTCCCGGGCCTTGCACTCACCGCCCGTCA |
| GU303759.1.1517 |
| AGAGTTTGATCATGGCTCAGGACGAACGCCGGCGGCGTGCCTAATACATGCAAGTAGAACGCTGAAGACTTTAGCT TGCTAAAGTTGGAAGAGTTGCGAACGGGTGAGTAACGCGTAGGTAACCTGCCTACTAGCGGGGGATAACTATTGGA AACGATAGCTAATACCGTATAACAGCATTTAACACATGTTAGATGCTTGAAAGGAGCAATTGCTTCACTAGTAGAT GGACCTGCGTTGTATTAGCTAGTTGGTGAGGTAACGGCTCACCAAGGCGACGATACATAGCCGACCTGAGAGGGTG ATCGGCCACACTGGGACTGAGACACGGCCCAGACTCCTACGGGAGGCAGCAGTAGGGAATCTTCGGCAATGGGGGC AACCCTGACCGAGCAACGCCGCGTGAGTGAAGAAGGTTTTCGGATCGTAAAGCTCTGTTGTAAGAGAAGAACGTGT GTGAGAGTGGAAAGTTCACACAGTGACGGTAACTTACCAGAAAGGGACGGCTAACTACGTGCCAGCAGCCGCGGTA ATACGTAGGTCCCGAGCGTTGTCCGGATTTATTGGGCGTAAAGCGAGCGCAGGCGGTTTAATAAGTCTGAAGTTAA AGGCAGTGGCTTAACCATTGTTCGCTTTGGAAACTGTTAGACTTGAGTGCAGAAGGGGAGAGTGGAATTCCATGTG TAGCGGTGAAATGCGTAGATATATGGARGGAAACACCGGTGGCGAAAGCGGCTCTCTGGTCTGTAACTGACGCTGA GGCTCGAGAAGCGTGGGGAGCAAACAGGATTAGATACCCTGGTAGTCCACGCCGTAAGCGATGAGTGCTAGGTGTT AGGCCCTTTCCGGGGCTTAGTGCCGCAGCTAACGCATTAAGCACTCCGCCTGGGGAGTACGACCGCAAGGTTGAAA CTCAAAGGAATTGACGGGGGCCCGCACAAGCGGTGGAGCATGTGGTTTAATTCGAAGCAACGCGAAGAACCTTACC AGGTCTTGACATCCCGATGCTATTCCTAGAGATAGGAAGTTTCTTCGGAACATCGGTGACAGGTGGTGCATGGTTG TCGTCAGCTCGTGTCGTGAGATGTTGGGTTAAGTCCCGCAACGAGCGCAACCCCTATTGTTAGTTGCCATCATTAA GTTGGGCACTCTAGCGAGACTGCCGGTAATAAACCGGAGGAAGGTGGGGATGACGTCAAATCATCATGCCCCTTAT GACCTGGGCTACACACGTGCTACAATGGCGGTCAACAGAGGGAAGCAATACTGTGAAGTGGAGCAAACCCCTAAAA GCCGTCCCAGTTCGGATTGCAGGCTGCAACCCGCCTGTATGAAGTTGGAATCGCTAGTAATCGCGGATCAGCATGC CGCGGTGAATACGTTCCCGGGCCTTGTACACACCGCCCGTCACACCATGAGAGTCGGGAACACCCGAAGTCCGTAG CCTAACTTTCACGAGGGGGCGCGGCCGAAGGTGGGTTCGATAATTGGGGTGAAGTCGTAACAAGGTAACCGTA |
| New.ReferenceOTU114 |
| TACGTAGGTCCCGAGCGTTGTCCGGATTTATTGGGCGTAAAGCGAGCGCAGGCGGTTTAATAAGTCTGAAGTTAAA GGCAGTGGCTTAACCATTTTTCGCTTTGGAAACTGTTAGACTTGAGTGCAGAAGGGGAGAGTGGAATTCCATGTGT AGCGGTGAAATGCGTAGATATATGGAGGAACACCGGTGGCGAAAGCGGCTCTCTGGTCTGTAACTGACGCTGAGGC TCGAAAGCGTGGGGAGCAAACAGG |
| AB506154.1.1541 |
| AGAGTTTGATCCTGGCTCAGGACGAACGCTGGCGGCGTGCCTAATACATGCAAGTAGAACGCTGAAGAAAGGAGCT TGCTTCTTTTGGATGAGTTGCGAACGGGTGAGTAACGCGTAGGTAACCTGCCTTGTAGCGGGGGATAACTATTGGA AACGATAGCTAATACCGCATAACAGCTTTTGACACATGTTAGAAGCTTGAAAGATGCAATTGCATCACTACGAGAT GGACCTGCGTTGTATTAGCTAGTAGGTAGGGTAACGGCCTACCTAGGCGACGATACATAGCCGACCTGAGAGGGTG ATCGGCCACACTGGGACTGAGACACGGCCCAGACTCCTACGGGAGGCAGCAGTAGGGAATCTTCGGCAATGGGGGC AACCCTGACCGAGCAACGCCGCGTGAGTGAAGAAGGTTTTCGGATCGTAAAGCTCTGTTGTAAGAGAAGAACGTGT GTGAGAGTGGAAAGTTCACACAGTGACGGTAACTTACCAGAAAGGGACGGCTAACTACGTGCCAGCAGCCGCGGTG ATACGTAGGTCCCGAGCGTTGTCCGGATTTATTGGGCGTAAAGCGAGCGCAGGCGGTTTAATAAGTCTGAAGTTAA AGGCAGTGGCTTAACCATTGTTCGCTTTGGAAACTGTTAAACTTGAGTGCAGAAGGGGAGAGTGGAATTCCATGTG TAGCGGTGAAATGCGTAGATATATGGAGGAACACCGGTGGCGAAAGCGGCTCTCTGGTCTGTAACTGACGCTGAGG CTCGAAAGCGTGGGGAGCAAACAGGATTAGATACCCTGGTAGTCCACGCCGTAAACGATGAGTGCTAGGTGTTAGG CCCTTTCCGGGGCTTAGTGCCGCAGCTAACGCATTAAGTATTCCGCCTGGGGAGTACGGTCGCAAGATTAAAACTC |
| AAAGGAATTGACGGGGGCCCGCACAAGCAGCGGAGCATGTGGTTTAATTCGAAGCAACGCGAAGAACCTTACCTAG ACTTGACATCTCCTGCATTACTCTTAATCGAGGAAGTCCCTTCGGGGACAGGATGACAGGTGGTGCATGGTTGTCG TCAGCTCGTGTCGTGAGATGTTGGGTTAAGTCCCGCAACGAGCGCAACCCTTATTGTTAGTTGCCATCATTAAGTT GGGCACTCTAGCGAGACTGCCCGGGTTAACCGGGAGGAAGGTGGGGATGACGTCAAATCATCATGCCCCTTATGTC TAGGGCTACACACGTGCTACAATGGTCGGTACAATAAGACGCAAGCCCGCGAGGGGGAGCAAAACTGGAAAACCGA TCTCAGTTCGGATTGTAGGCTGAAACTCGCCTACATGAAGCTGGAGTTGCTAGTAATCGCGAATCAGCATGTCGCG GTGAATACGTTCCCGGGCCTTGTACACACCGCCCGTCACACCATGAGAGTTGGCAATACCCAAAGTACGTGATCTA ACCCGCAAGGGAGGAAGCGTCCTAAGGTAGGGTCAGCGATTGGGGTGAAGTCGTAACAAGGTAGCCGTAGGAGAAC CTGCGGCTG |
| EU774370.1.1398 |
| AGAGTTTGCTCTTGGGTCAGGATGAACGCTGGCGGCGTGCTTAACACATGCAAGTCGAGCGAAAAGTTCTTCGG AGCTTTTCTAGCGGCGGACGGGTGAGTAACACGTGGGCAACCTGCCTCATAGAGGGGAATAGCCTTCCGAAAGGAA GATTAATACCGCATAACATTGTTGAAAGGCATCTTTTAACAATCAAAGGAGCAATCCGCTATGAGATGGGCCCGCG GCGCATTAGCTAGTTGGTGAGGTAACGGCTCACCAAGGCGACGATGCGTAGCCGACCTGAGAGGGTGATCGGCCAC ATTGGAACTGAGACACGGTCCAGACTCCTACGGGAGGCAGCAGTGGGGAATATTGCACAATGGGGGAAACCCTGAT GCAGCGACGCCGCGTGAGTGAAGAAGTATTTCGGTATGTAAAGCTCTGTTGTAAGAGAAGAACGTGTGTGAGAGTG GAAAGTTCACACAGTGACGGTAACTTACCAGAGAGGGACGGCTAACTACGTGCCAGCAGCCGCGGTAATACGTAGG TCCCGAGCGTTGTCCGGATTTATTGGGCGTAAAGCGAGCGCAGGCGGTTTAATAAGTCTGAAGTTAAAGGCAGTGG CTTAACCATTGTTCGCTTTGGAAACTGTTAGACTTGAGTGCAGAAGGGGAGAGTGGAATTCCATGTGTAGCGGTGA AATGCGTAGATATATGGAGGAACACCGGTGGCGAAAGCGGCTCTCTGGTCTGTAACTGACGCTGAGGCTCGAAAGC GTGGGGAGCAAACAGGATTAGATACCCTGGTAGTCCACGCCGTAAACGATGAGTGCTAGGTGTTAGGCCCTTTCCG GGGCTTAGTGCCGCAGCTAACGCATTAAGCACTCCGCCTGGGGAGTACGACCGCAAGGTTGAAACTCAAAGGAATT GACGGGGGCCCGCACAAGCGGTGGAGCATGTGGTTTAATTCGAAGCAACGCGAAGAACCTTACCAGGTCTTGACAT CCCGATGCTATTCCTAGAGATAGGAAGTTTCTTCGGAACATCGGTGACAGGTGGTGCATGGTTGTCGTCAGCTCGT GTCGTGAGATGTTGGGTTAAGTCCCGCAACGAGCGCAACCCCTATTGTTAGTTGCCATCATTAAGTTGGGCACTCT AGCGAGACTGCCGGTAATAAACCGGAGGAAGGTGGGGATGACGTCAAATCATCATGCCCCTTATGACCTGGGCTAC ACACGTGCTACAATGGTTGGTACAACGAGTCGCGAGTCGGTGACGGCAAGCAAATCTCTTAAAGCCAATCTCAGTT CGGATTGTAGGCTGCAACTCGCCTACATGAAGTCGGAATCGCTAGTAATCGCGGATCAGCACGCCGCGGTGAATAC GTTCCCGGGCCTTGCACTCACCGCCCGTCA |
| HK557089.3.1395 |
| AGACTTTAGCTTGCTAAAGTTGGAAGAGTTGCGAACGGGTGAGTAACGCGTAGGTAACCTGCCTACTAGCGGGGGA TAACTATTGGAAACGATAGCTAATACCGCATAACAGCATTTAACCCATGTTAGATGCTTGAAAGGAGCAATTGCTT CACTAGTAGATGGACCTGCGTTGTATTAGCTAGTTGGTGAGGTAACGGCTCACCAAGGCGACGATACATAGCCGAC CTGAGAGGGTGATCGGCCACACTGGGACTGAGACACGGCCCAGACTCCTACGGGAGGCAGCAGTAGGGAATCTTCG GCAATGGGGGCAACCCTGACCGAGCAACGCCGCGTGAGTGAAGAAGGTTTTCGGATCGTAAAGCTCTGTTGTAAGA GAAGAACGTGTGTGAGAGTGGAAAGTTCACACAGTGACGGTAACTTACCAGAAAGGGACGGCTAACTACGTGCCAG CAGCCGCGGTAATACGTAGGTCCCGAGCGTTGTCCGGATTTATTGGGCGTAAAGCGAGCGCAGGCGGTTTAATAAG TCTGAAGTTAAAGGCAGTGGCTTAACCATTGTTCGCTTTGGAAACTGTTAGACTTGAGTGCAGAAGGGGAGAGTGG AATTCCATGTGTAGCGGTGAAATGCGTAGATATATGGAGGAACACCGGTGGCGAAAGCGGCTCTCTGGTCTGTAAC TGACGCTGAGGCTCGAAAGCGTGGGGAGCAAACAGGATTAGATACCCTGGTAGTCCACGCCGTAAACGATGAGTGC TAGGTGTTAGGCCCTTTCCGGGGCTTAGTGCCGCAGCTAACGCATTAAGCACTCCGCCTGGGGAGTACGACCGCAA GGTTGAAACTCAAAGGAATTGACGGGGGCCCGCACAAGCGGTGGAGCATGTGGTTTAATTCGAAGCAACGCGAAGA ACCTTACCAGGTCTTGACATCCCGATGCTATTCCTAGAGATAGGAAGTTTCTTCGGAACTGTGAGACTTGAGGGCA GAAGGGTAGAGTGCACTTGTATGGGGAGCTGTGGAATGCGTTCCCGCAACGAGCGCAACCCCTATTGTTAGTTGCC ATCATTAAGTTGGGCACTCTAGCGAGACTGCCGGTAATAAACCGGAGGAAGGTGGGGATGACGTCAAATCATCATG CCCCTTATGACCTGGGCTACACACGTGCTACAATGGTTGGTACAACGAGTCGCGAGTCGGTGACGGCAAGCAAATC TCTTAAAGCCAATCTCAGTTCGGATTGTAGGCTGCAACTCGCCTACATGAAGTCGGAATCGCTAGTAATCGCGGAT CAGCACGCCGCGGTGAATACGTTCCCGGGCCTTGTACACACCGCCCGTCACACCACGAGAGTTTGTAACACCCGAA GTCGGTGAGGTANCCTTTTAGGAGC |
| HQ807346.1.1456 |
| TTCAGGACGAACGCTGGCGGCGTGCCTAATACATGCAAGTAGAACGCTGAAGACTTTAGCTTGCTAAAGTTGGAAG AGTTGCGAACGGGTGAGTAACGCGTAGGTAACCTGCCTACTAGCGGGGGATAACTATTGGAAACGATAGCTAATAC CGCATAACAGCATTTAACCCATGTTAGATGCTTGAAAGGAGCAATTGCTTCACTAGTAGATGGACCTGCGTTGTAT TAGCTAGTTGGTGAGGTAACGGCTCACCAAGGCGACGATACATAGCCGACCTGAGAGGGTGATCGGCCACACTGGG ACTGAGACACGGCCCAGACTCCTACGGGAGGCAGCAGTAGGGAATCTTCGGCAATGGGGGCAACCCTGACCGAGCA ACGCCGCGTGAGTGAAGAAGGTTTTCGGATCGTAAAGCTCTGTTGTAAGAGAAGAACGTGTGTGAGAGTGGAAAGT TCACACAGTGACGGTAACTTACCAGAAAGGGACGGCTAACTACGTGCCAGCAGCCGCGGTAATACGTAGGTCCCGA GCGTTGTCCGGATTTATTGGGCGTAAAGCGAGCGCAGGCGGTTTAATAAGTCTGAAGTTAAAGGCAGTGGCTTAAC CATTGTTCGCTTTGGAAACTGTTAGACTTGAGTGCAGAAGGGGAGAGTGGAATTCCATGTGTAGCGGTGAAATGCG TAGATATATGGAGGAACACCGGTGGCGAAAGCGGCTCTCTGGTCTGTAACTGACGCTGAGGCTCGAAAGCGTGGGG AGCAAACAGGATTAGATACCCTGGTAGTCCACGCCGTAAACGATGAGTGCTAGGTGTTAGGCCCTTTCCGGGGCTT AGTGCCGCAGCTAACGCATTAAGCACTCCGCCTGGGGAGTACGACCGCAAGGTTGAAACTCAAAGGAATTGACGGG |
| GGCCCGCACAAGCGGTGGAGCATGTGGTTTAATTCGAAGCAACGCGAAGAACCTTACCAGGTCTTGACATCCTTTG ACCACTCTAGAGATAGAGCTTCCCCTTCGGGGGCAAAGTGACAGGTGGTGCATGGTTGTCGTCAGCTCGTGTCGTG AGATGTTGGGTTAAGTCCCGCAACGAGCGCAACCCTTATTGTTAGTTGCCATCATTTAGTTGGGCACTCTAGCGAG ACTGCCGGTGACAAACCGGAGGAAGGTGGGGATGACGTCAAATCATCATGCCCCTTATGACCTGGGCTACACACGT GCTACAATGGGAAGTACAACGAGTTGCGAAGTCGCGAGGCTAAGCTAATCTCTTAAAGCTTCTCTCAGTTCGGATT GTAGGCTGCAACTCGCCTACATGAAGCCGGAATCGCTAGTAATCGCGGATCAGCACGCCGCGGTGAATACGTTCCC GGGCCTTGTACACACCGCCCGTCACACCACGAGAGTTTGTAACACCCGAAGTCGGTGAGGTAACCTTTTAGGAGCC AGCCGCCTAAGG |
| HQ748204.1.1442 |
| CTAATACATGCGAGGAGAACGCTGAAGACTTTCTTTTGCTATAGTTGGAGAGTTGCTAACGGGTGAGTAACGCGT AGGTGACCTGCCTACTAGCGGGGGATAACTATTGCAAACGATAGCTAATACCGCATAACAGCCTTTAACCCATGTT AGATGCTTGAAAGGAGCAATTGCTTCACTAGTAGATGGACCTGCGTTGTATTAGCTAGTTGGTGAGGTAACGGCTC ACCAAGGCGACGATACATAGCCGACCTGAGAGGGTGATCGGCCACACTGGGACTGAGACACGGCCCATACTCCTAC GGGAGGCACCAGTAGGGAATCTTCGGGAATGGGGGCAACCCTGACCGAGCAACGCCGCGTGAGTGAAGAAGGTTTT CGGATCGTAAAGCTCTGTTGTAAGAGAAGAACGTGTGTGAGAGTGGAAAGTTCACACTGTGACGGTAACTTACCAG AAAGGGACGGCTAACTACGTGCCAGCAGCCGCGGTAATACGTAGGTCCCGAGCGTTGTCCGGATTTATTGGGCGTA AAGCGAGCGCAGGCGGTTTAATAAGTCTGAAGTTAAAGGCAGTGGCTTAACCATTGTTCGCTTTGGAAACTGTTAG ACTTGAGTGCATAAGGGGAGAGTGGAATTCCATGTGTAGCGGTGAAATGCGTAGATATATGGAGGAACACCGGTGG CGAAAGCGGCTCTCTGGTCTGTAACTGACGTTGAGGCTCGAAAGCGTGGGGAGCAAACAGGATTAGATACCCTGGT AGTCCACGCTGTAAACGATGAGTGGTAGGTGTTAGGCCCTTTCTGGGGTTTAGTGCCGCAGATTACGCATTAAGCC ATTCGCCTGGGGAGTACGACCGCAAGGTTGAAACTTAAAGGAATTGACGGGGGCCCGCACAAGCGGTGGAGCATGT GGTTTAATTAGAAGCAACGCGAAGAACCTTACCAGGTCTTGACATCCCGATGCTATTCTTAGAGATAGGAAGTTTC TTCGGAACATCGGTGACAGGTGGTGCATGGTTGTCGTCAGCTCGTGTCGAGAGATGTTGGGTTAAGTCCCTCAACG AGCGCAACCCCTATTTTTATTTGCCATCATTAAGTTGGGCAATCTAGCGAGACTGCCGGTAATAAACCGGAGGAAG GTGGGGATGACGTCAAATCATCATGCTCCTTATGTCATGGGTACACACGTGGTACAATGGTTGGTACAACGAGTC GCGAGTTGGTGAAGGCAAGCAAATCTCTTAAAGCCAATATCAGTTCGGATTGTAGGCTGCAAATAGCCTACATGTA GTCGGAATTGTTAGTAATCGGGGATCAGCACTCCGCGGTGAATACGTTTCCGGGCCTTGTACACCCCGCCCGTCTA CACCACGAGAGTTTGTAACACCCGAAGTCGGTGAGGTAACTCTTTAGGAGCCAGCCGCCTAAGGTGGGATAGA |
| GU179917.1.1382 |
| AGAGTTTGATTATGGCTCAGGATGAACGCTGGCGGCGTGCCTAATACATGCAAGTCGAACGCGAGCAGCAATGCTC GAGTGGCGAACGGGTGAGTAATACATAAGTAACCTGCCCTAGACAGGGGGATAACTGCTGGAAACGGCAGCTAAGA CCGCATAGGTATGGACACTGCATGGTGACCATATTAAAAGTGCCAAGGCACTGGTAGAGGATGGACTTATGGCGCA TTAGCTGGTTGGTGAGGTAACGGCTCACCAAGGCGACGATGCGTAGCCGACCTGAGAGGGTGACCGGCCACACTGG GACTGAGACACGGCCCAGACTCCTACGGGAGGCAGCAGTAGGGAATTTTCGGCAATGGGGGGAACCCTGACCGAGC AACGCCGCGTGAAGGAAGAAGGAATTCGTTCTGTAAACTTCTGTTATAAAGGAAGAACGGCGGATATAGGGAATGA TATCCGAGTGACGGTACTTTATGAGAAAGCCACGGCTAACTACGTGCCAGCAGCCGCGGTAATACGTAGGTGGCGA GCGTTATCCGGAATTATTGGGCGTAAAGAGGGAGCAGGCGGCGGCAGAGGTCTGTGGTGAAAGACTGAAGCTTAAC TTCAGTAAGCCATAGAAACCGGGCTGCTAGAGTGCAGGAGAGGATCGTGGAATTCCATGTGTAGCGGTGAAATGCG TAGATATATGGAGGAACACCAGTGGCGAAGGCGACGGTCTGGCCTGTAACTGACGCTCATTCCCGAAAGCGTGGGG AGCAAATAGGATTAGATACCCTAGTAGTCCACGCCGTAAACGATGAGTACTAAGTGTTGGGAGTCAAATTTCAGTG CTGCAGTTAACGCAATAAGTACTCCGCCTGAGTAGTACGTTCGCAAGAATGAAACTCAAAGGAATTGACGGGGGCC CGCACAAGCGGTGGAGCATGTGGTTTAATTCGATGATACGCGAGGAACCTTACCAGGGCTTAAATGTGACTGACAG GTCCGGAAACGGACTTTTCTTCGGACAGTTACAGGTGCTGCATGGTTGTCGTCAGCTCGTGCCGTGAGGTGTCAGG TTAAGTCCTATAACGAGCGCAACCCCTGTCGCTAGTTGCCAGCGAGTAATGTCGGGAACTCTAGCGAGACTGCCAG TGCAAACTGCGAGGAAGGTGGGGATGACGTCAAATCATCACGGCCCTTACGCCCTGGGCTACACACGTGCTACAAT GGCCGGTACAGAGAGCAGCCACCCCGCGAGGGGGAGCGAATCTACAAAACCGGTCACAGTTCGGATCGGAGTCTGC AACTCGACTCCGTGAAGCTGGAATCGCTAGTAATCGGATATCAGCCATGATCCGGTGAATACGTTCCCGGGCCTTG TACACACCCCCGTC |
| GQ448336.1.1418 |
| AGAGTTTGATCATGGCTCAGGACGAACGCTGGCGGCGTGCTTAACACATGCAAGTCGAACGAAGAGATGAGAAG CTTGCTTCTTATCTCTTCGAGTGGCAAACGGGTGAGTAACGCGTAAGCAACCTGCCCTTCAGATGGGGACAACAGC TGGAAACGGCTGCTAATACCGAATACGTTCTTTTTGTCGCATGGCAGAGGGAAGAAAGGGAGGCTCTTCGGAGCTT TCGCTGAAGGAGGGGCTTGCGTCTGATTAGCTAGTTGGAGGGGTAACGGCCCACCAAGGCGACGATCAGTAGCCGG TCTGAGAGGATGAACGGCCACATTGGGACTGAGACACGGCCCAGACTCCTACGGGAGGCAGCAGTGGGGAATCTTC CGCAATGGACGAAAGTCTGACGGAGCAACGCCGCGTGAACGATGACGGCCTTCGGGTTGTAAAGTTCTGTTATACG GGACGAATGGCGTAGCGGTCAATACCCGTTACGAGTGACGGTACCGTAAGAGAAAGCCACGGCTAACTACGTGCCA GCAGCCGCGGTAATACGTAGGTGGCAAGCGTTGTCCGGAATTATTGGGCGTAAAGGGCGCGCAGGCGGCGTCGTAA GTCGGTCTTAAAAGTGCGGGGCTTAACCCCGTGAGGGGACCGAAACTGCGATGCTAGAGTATCGGAGAGGAAAGCG GAATTCCTAGTGTAGCGGTGAAATGCGTAGATATTAGGAGGAACACCAGTGGCGAAAGCGGCTTTCTGGACGACAA CTGACGCTGAGGCGCGAAAGCCAGGGGAGCAAACGGGATTAGATACCCCGGTAGTCCTGGCCGTAAACGATGGATA CTAGGTGTAGGAGGTATCGACCCCTTCTGTGCCGGAGTTAACGCAATAAGTATCCCGCCTGGGGAGTACGGCCGCA AGGCTGAAACTCAAAGGAATTGACGGGGGCCCGCACAAGCGGTGGAACATGTGGTTTAATTCGATGATACGCGAGG |
| AACCTTACCCGGCTTAAATTGCAGTGGAATGATGTGGAAACATGTCAGTGAGCAATCACCGCTGTGAAGGTGCTG CATGGTTGTCGTCAGCTCGTGCCGTGAGGTGTCGGCTTAAGTGCCATAACGAGCGCAACCCTTATCTTCAGTTACT AACAGGTCATGCTGAGGACTCTGGAGAGACTGCCGTCGTAAGATGTGAGGAAGGTGGGGATGACGTCAAATCAGCA CGGCCCTTACGTCCGGGGCTACACACGTGTTACAATGGGGGGTACAGAGGGCCGCTACCACGCGAGTGGATGCCAA TCCCAAAAACCTCTCTCAGTTCGGACTGGAGTCTGCAACCCGACTCCACGAAGCTGGATTCGCTAGTAATCGCGCA TCAGCCACGGCGCGGTGAATACGTTCCCGGGCCTTGCACTCACCGCCCGT |
| DQ804865.1.1390 |
| AGAGTTTGATCCTGGCTCAGGATGAACGCTGGCGGCGTGCTTAACACATGCAAGTCGAACGGAAACTTTTCATTG AAGCTTCGGCAGATTTGGTCTGTTTCTAGTGGCGGACGGGTGAGTAACGCGTGGGTAACCTGCCTTATACAGGGGG ATAACAACCAGAAATGGTTGCTAATACCGCATAAGCGCACAGGACCGCATGGTCCGGTGTGAAAAACTCCGGTGGT ATAAGATGGACCCGCGTTGGATTAGCTAGTTGGCAGGGTAACGGCCTACCAAGGCGACGATCCATAGCCGGCCTGA GAGGGTGAACGGCCACATTGGGACTGAGACACGGCCCAGACTCCTACGGGAGGCAGCAGTGGGGAATATTGCACAA TGGGGGAAACCCTGATGCAGCGACGCCGCGTGAAGGAAGAAGTATCTCGGTATGTAAACTTCTATCAGCAGGGAAG ATAGTGACGGTACCTGACTAAGAAGCCCCGGCTAAATACGTGCCAGCAGCCGCGGTAATACGTATGGTTCAAGCGT TATCCGGATTTACTGGGTGTAAAGGGTGAGTAGGCGGTTATGCAAGTCATATGTGAAATGTCGGGGCTCAACTCCG GCCTGCATAAGAAACTGTATAACTAGAGTGCAGGAGAGGCAAGCGGAATTCCTAGTGTAGCGGTGAAATGCGTAGA TATTAGGAAGAACACCGGTGGCGAAGGCGGCTTGCTGGACTGTTACTGACGCTGAGTCACGAAAGCGTGGGGAGCA AACAGGATTAGATACCCTGGTAGTCCACGCCGTAAACGATGAATACTAGGTGTCGGGTGGCAAAGCCATTCGGTGC CGCAGCAAACGCAATAAGTATTCCACCTGGGGAGTACGTTCGCAAGAATGAAACTCAAAGGAATTGACGGGGACCC GCACAAGCGGTGGAGTATGTGGTTTAATTCGAAGCAACGCGAAGAACCTTACCAGGCCTTGACATGGATATAAATG TTCTAGAGATAGAAAGATAGCTATATATCACACAGGTGGTGCATGGTTGTCGTCAGCTCGTGTCGTGAGATGTTGG GTTAAGTCCCGCAACGAGCGCAACCCTTGTCTTCTGTTACCAGCATTGAGTTGGGGACTCAGGAGAGACTGCCGGT GACAAACCGGAGGAAGGTGGGGATGACGTCAAATCATCATGCCCCTTATGGCCTGGGCTACACACGTACTACAATG GCGCCTACAAAGAGCAGCGACACCGCGAGGTGAAGCGAATCTCATAAAGGGCGTCTCAGTTCGGATTGAAGTCTGC AACTCGACTTCATGAAGTCGGAATCGCTAGTAATCGCAGATCAGCATGCTGCGGTGAATACGTTCCCGGGTCTTGT ACTCACCGCCCGTCA |
| GQ491757.1.1361 |
| GATGAACGCTGGCGGCGTGCTTAACACATGCAAGTCGAGCGAAGCACTTAAGTGGATCTCTTCGGATTGAAACTTA TTTGACTGAGCGGCGGACGGGTGAGTAACGCGTGGGTAACCTGCCTCATACAGGGGGATAACAGTTAGAAATGGCT GCTAATACCGCATAAGCGCACAGGACCGCATGGTCTGGTGTGAAAAACTCCGGTGGTATGAGATGGACCCGCGTCT GATTAGCTAGTTGGAGGGGTAACGGCCCACCAAGGCGACGATCAGTAGCCGGCCTGAGAGGGTGAACGGCCACATT GGGACTGAGACACGGCCCAGACTCCTACGGGAGGCAGCAGTGGGGAATATTGCACAATGGGGGAAACCCTGATGCA GCGACGCCGCGTGAAGGAAGAAGTATCTCGGTATGTAAACTTCTATCAGCAGGGAAGAAAATGACGGTACCTGACT AAGAAGCCCCGGCTAACTACGTGCCAGCAGCCGCGGTAATACGTAGGGGGCAAGCGTTATCCGGATTTACTGGGTG TAAAGGGAGCGTAGACGGAAGAGCAAGTCTGATGTGAAAGGCTGGGGCTTAACCCCAGGACTGCATTGGAAACTGT TTTTCTAGAGTGCCGGAGAGGTAAGCGGAATTCCTAGTGTAGCGGTGAAATGCGTAGATATTAGGAGGAACACCGG TGGCGAAGGCGGCTTACTGGACGACCACTGACGCTGAGGCTCGAAAGCGTGGGGAGCAAACAGGATTAGATACCCT GGTAGTCCACGCCGTAAACCGATGAATAATAGGTGTCGGGGAACAATAGTTCTTTGGTGCCGCAGCAAAACGCATT AAGTATTCCACCTGGGGAGTACGTTCGCAAGAATGAAACTCAAAGGAATTGACGGGGACCCGCACAAGCGGTGGAG CATGTGGTTTAATTCGATGCAACGCGAAGAACCTTACCTGCTCTTGACATCCCACTGACCGGACAGTAATGTGTCC TTTTCTTCTGAACAGTGGAGACAGGTGGTGCATGGTTGTCGTCAGCTCGTGTCGTGAGATGTTGGGTTAAGTCCCG CAACGAGCGCAACCCTCGTCTTTAGTAGCCAGCAGTCCGGCTGGGCACTCTAGAGAGACTGCCAGGGATAACCTGG AGGAAGGCGGGGAGGACGTCAAATCATCATGCCCCTTACGAGCAGGGCTACACACGTGCTACAATGGCGTAAACAA AGGGAAGCGACCCCGTGAAGGTGAGCAAATCTCAAAAATAACGTCTCAGTTCGGATTGTAGTCTGCAACTCGACTA CATGAAGCTGGAATCGCTAGTAATCGCGAATCAGAATGTCGCGGTGAATAAAGGCCGGGTCTTGCACA |
| New.ReferenceOTU56 |
| TACGGAAGGTCCAGGCGTTATCCGGATTTATTGGGTTTAAAGGGAGCGCAGGCGGACTCTTAAGTCAGTTGTGAAA TACGGCGGCTCAACCGTCGGACTGCAGTTGATACTGGGAGTCTTGAGTACACGCAGAGATACTGGAATTCATGGTG TAGCGGTGAAATGCTCAGATATCATGAGGAACTCCGATCGCGAAGGCAGGTATCTGGAGTGTAACTGACGCTGAGG CTCGAAAGTGCGGGTATCAAACAGG |
| KF842598.1.1394 |
| AGAGTTTGATCCTGGCTCAGGATGAACGCTAGCGACAGGCTTAACACATGCAAGTCGAGGGGCAGCATGATTTGTA GCAATACAGATTGATGGCGACCGGCGCACGGGTGAGTAACGCGTATGCAACTTACCTATCAGAGGGGGATAGCCCG GCGAAAGTCGGATTAATACCCCATAAAACAGGGGTCCCGCATGGGAATATTTGTTAAAGATTCATCGCTGATAGAT AGGCATGCGTTCCATTAGGCAGTTGGCGGGGTAACGGCCCACCAAACCGACGATGGATAGGGGTTCTGAGAGGAAG GTCCCCCACATTGGTACTGAGACACGGACCAAACTCCTACGGGAGGCAGCAGTGAGGAATATTGGTCAATGGCCGA GAGGCTGAACCAGCCAAGTCGCGTGAAGGAAGAAGGATCTATGGTCTGTAAACTTCTTTTATAGGGGAATAAAGTG GAGGACGTGTCCTTTTTTGTATGTACCCTATGAATAAGCATCGGCTAACTCCGTGCCAGCAGCCGCGGTAATACGG AGGATGCGAGCGTTATCCGGATTTATTGGGTTTAAAGGGTGCGTAGGTGGTGATTTAAGTCAGCGGTGAAAGTTTG TGGCTCAACCATAAAATTGCCGTTGAAACTGGGTTACTTGAGTGTGTTTGAGGTAGGCGGAATGCGTGGTGTAGCG GTGAAATGCATAGATATCACGCAGAACTCCGATTGCGAAGGCAGCTTACTAAACCATAACTGACACTGAAGCACGA |
| AAGCGTGGGGATCAAACAGGATTAGATACCCTGGTAGTCCACGCAGTAAACGATGATTACTAGGAGTTTGCGATAC AATGTAAGCTCTACAGCGAAAGCGTTAAGTAATCCACCTGGGGAGTACGCCGGCAACGGTGAAACTCAAAGGAATT GACGGGGGCCCGCACAAGCGGAGGAACATGTGGTTTAATTCGATGATACGCGAGGAACCTTACCCGGGTTTGAACG TAGTCTGACCGGAATGGAAACACTCCTTCTAGCAATAGCAGATTACAAGGTGCTGCATGGTTGCCTCAACTCCGGC CCGGAAGGTCCGGCTTAATTGCCATAACAAGCGCACCCTTTTACCAAGGTTCAAACAGGTGAAGCTTGAAGACTCT GTGGAACCTCCCCCCTAACCTGTGAGAAGAAGTGGGGATACACTCAATAAACCACGGCCCTTAATCCCGGGGGGAA CACTGGTTACAATGGGTTGGGAAAGGGGGCTTCCTGGCGACAGGATGCTAATCTCCAAACCATGTCTCAGTTCGGA TCGGAGTCTGCAACTCGACTCCGTGAAGCTGGATTCGCTAGTAATCGCGCATCAGCCATGGCGCGGTGAATACGTT CCCGGGCCTTGTACACACCGCCCGTC |
| HQ802052.1.1445 |
| TACAGGCTTAACACATGCAAGTCGAGGGGCAGCATGATTGAAGCTTGCTTCAATTGATGGCGACCGGCGCACGGGT GAGTAACACGTATCCAACCTTCCGTACACTCAGGGATAGCCTTTCGAAAGAAAGATTAATACCTGATGGTATCTTA AGCACACATGTAATTAAGATTAAAGATTTATCGGTGTACGATGGGGATGCGTTCCATTAGGTAGTAGGCGGGGTAA CGGCCCACCTAGCCTACGATGGATGGGGGTTCTGAGAGGAAGGTCCCCCACATTGGAACTGAGACACGGTCCAAAC TCCTACGGGAGGCAGCAGTGAGGAATATTGGTCAATGGACGAGAGTCTGAACCAGCCAAGTAGCGTGAAGGATGAA GGTCCTACGGATTGTAAACTTCTTTTATAAGGGAATAAAACCTCCCACGTGTGGGAGCTTGTATGTACCTTATGAA TAAGCATCGGCTAACTCCGTGCCAGCAGCCGCGGTAATACGGAGGATGCGAGCGTTATCCGGATTTATTGGGTTTA AAGGGAGCGCAGACGGGTCGTTAAGTCAGCTGTGAAAGTTTGGGGCTCAACCTTAAAATTGCAGTTGATACTGGCG TCCTTGAGTGCGGTTGAGGTGTGCGGAATTCGTGGTGTAGCGGTGAAATGCTTAGATATCACGAAGAACTCCGATT GCGAAGGCAGCACACTAAGCCGTAACTGACGTTCATGCTCGAAAGTGTGGGTATCAAACAGGATTAGATACCCTGG TAGTCCACACAGTAAACGATGAATACTCGCTGTTTGCGATATACAGTAAGCGGCCAAGCGAAAGCATTAAGTATTC CACCTGGGGAGTACGCCGGCAACGGTGAAACTCAAAGGAATTGACGGGGGCCCGCACAAGCGGAGGAACATGTGGT TTAATTCGATGATACGCGAGGAACCTTACCCGGGCTTAAATTGCATTTGAATATATTGGAAACAGTATAGCCGTAA GGCAAATGTGAAGGTGCTGCATGGTTGTCGTCAGCTCGTGCCGTGAGGTGTCGGCTTAAGTGCCATAACGAGCGCA ACCCTTATCTTCAGTTACTAACAGGTCATGCTGAGGACTCTGGAGAGACTGCCGTCGTAAGATGTGAGGAAGGTGG GGATGACGTCAAATCAGCACGGCCCTTACGTCCGGGGCTACACACGTGTTACAATGGGGGGTACAGAAGGCCGCTA CCTGGTGACAGGATGCTAATCCCAAAAGCCTCTCTCAGTTCGGATCGAAGTCTGCAACCCGACTTCGTGAAGCTGG ATTCGCTAGTAATCGCGCATCAGCCATGGCGCGGTGAATACGTTCCCGGGCCTTGTACACACCGCCCGTCAAGCCA TGAAAGCCGGGGGTACCTGAAGTACGTAACCGCAAGGAGCGTCCTAGGGTAAAACTGGTAATTGGGGCTAAGTCAT A |
| GX182404.8.1529 |
| AGAGTTTGATCATGGCTCAGATTGAACGCTGGCGGCAGGCCTAACACATGCAAGTCGAACGGTAACAGGAAGAAGC TTGCTTCTTTGCTGACGAGTGGCGGACGGGTGAGTAATGTCTGGGAAACTGCCTGATGGAGGGGGATAACTACTGG AAACGGTAGCTAATACCGCATAACGTCGCAAGACCAAAGAGGGGGACCTTCGGGCCTCTTGCCATCGGATGTGCCC AGATGGGATTAGCTAGTAGGTGGGGTAACGGCTCACCTAGGCGACGATCCCTAGCTGGTCTGAGAGGATGACCAGC CACACTGGAACTGAGACACGGTCCAGACTCCTACGGGAGGCAGCAGTGGGGAATATTGCACAATGGGCGCAAGCCT GATGCAGCCATGCCGCGTGTATGAAGAAGGCCTTCGGGTTGTAAAGTACTTTCAGCGGGGAGGAAGGGAGTAAAGT TAATACCTTTGCTCATTGACGTTACCCGCAGAAGAAGCACCGGCTAACTCCGTGCCAGCAGCCGCGGTAATACGGA GGGTGCAAGCGTTAATCGGAATTACTGGGCGTAAAGCGCACGCAGGCGGTTTGTTAAGTCAGATGTGAAATCCCCG GGCTCAACCTGGGAACTGCATCTGATACTGGCAAGCTTGAGTCTCGTAGAGGGGGGTAGAATTCCAGGTGTAGCGG TGAAATGCGTAGAGATCTGGAGGAATACCGGTGGCGAAGGCGGCCCCCTGGACGAAGACTGACGCTCAGGTGCGAA AGCGTGGGGAGCAAACAGGATTAGATACCCTGGTAGTCCACGCCGTAAACGATGTCGACTTGGAGGTTGTGCCCTT GAGGCGTGGCTTCCGGAGCTAACGCGTTAAGTCGACCGCCTGGGGAGTACGGCCGCAAGGTTAAAACTCAAATGAA TTGACGGGGGCCCGCACAAGCGGCGGAGCATGTGGATTAATTCGATGCAACGCGAAGAACCTTACCTGGGTTTGAC ATGCACAGGACGCGTCTAGAGATAGGCGTTCCCTTGTGGCCTGTGTGCAGGTGGTGCATGGCTGTCGTCAGCTCGT GTCGTGAGATGTTGGGTTAAGTCCCGCAACGAGCGCAACCCTTGTCTCATGTTGCCAGCACGTAATGGTGGGGACT CGTGAGAGACTGCCGGGGTCAACTCGGAGGAAGGTGGGGATGACGTCAAGTCATCATGCCCCTTATGTCCAGGGCT TCACACATGCTACAATGGCCGGTACAAAGGGCTGCGATGCCGCGAGGTTAAGCGAATCCTTAAAAGCCGGTCTCAG TTCGGATCGGGGTCTGCAACTCGACCCCGTGAAGTCGGAGTCGCTAGTAATCGCAGATCAGCAACGCTGCGGTGAA TACGTTCCCGGGCCTTGTACACACCGCCCGTCACGTCATGAAAGTCGGTAACACCCGAAGCCAGTGGCCTAACCCT CGGGAGGGAGCTGTCGAAGGTGGGATCGGCGATTGGGACGAAGTCGTAACAAGGTAACCGTAGGGGAACCTGCGGT TG |
| FJ950694.1.1472 |
| CGCCCTGATTGACGGCTATACACATGCAAGTCGAACGGTAACAGGAAACAGCTTGCTTCTTTGCTGACGAGTGGCG GACGGGTGAGTAATGTCTGGGAAACTGCCTGATGGAGGGGGATAACTACTGGAAACGGTAGCTAATACCGCATAAC GTCGCAAGACCAAAGAGGGGGACCTTCGGGCCTCTTGCCATCGGATGTGCCCAGATGGGATTAGCTAGTAGGTGGG GTAACGGCTCCATCCCTAGGCGAGCCGAATCCTTAGCCTGGTCTGAGAGGAATGACCAGCCACACTGGGACTGAGA ACACGGTCCAGACTCCTACGGGAGGCAGCAGTGGGGAATATTGCACAATGGGCGCAAGCCTGATGCAGCCATGCCG CGTGTATGAAGAAGGCCTTCGGGTTGTAAAGTACTTTCAGCGGGGAGGAAGGGAGTAAAGTTAATACCTTTGCTC ATTGACGTTACCCGCAGAAGAAGCACCGGCTAACTCCGTGCCAGCAGCCGCGGTAATACGGAGGGTGCAAGCGTTA ATCGGAATTACTGGGCGTAAAGCGCACGCAGGCGGTTTGTTAAGTCAGATGTGAAATCCCCGGGCTCAACCTGGGA ACTGCATCTGATACTGGCAAGCTTGAGTCTCGTAGAGGGGGGTAGAATTCCAGGTGTAGCGGTGAAATGCGTAGAG |
| ATCTGGAGGAATACCGGTGGCGAAGGCGGCCCCCTGGACGAAGACTGACGCTCAGGTGCGAAAGCGTGGGGAGCAA ACAGGATTAGATACCCTGGTAGTCCACGCCGTAAACGATGTCGACTTGGAGGTTGTGCCCTTGAGGCGTGGCTTCC GGAGCTAACGCGTTAAGTCGACCGCCTGGGGAGTACGGCCGCAAGGTTAAAACTCAAATGAATTGACGGGGGCCCG CACAAGCGGTGGAGCATGTGGTTTAATTCGATGCAACGCGAAGAACCTTACCTGGTCTTGACATCCACGGAAGTT TTCAGAGATGAGAATGTGCCTTCGGGAACCGTGAGACAGGTGCTGCATGGCTGTCGTCAGCTCGTGTTGTGAAATG TTGGGTTAAGTCCCGCAACGAGCGCAACCCTTATCCTTTGTTGCCAGCGGTCCGGCCGGGAACTCAAAGGAGACTG CCAGTGATAAACTGGAGGAAGGTGGGGATGACGTCCAGGTCATCATGGCCCTTACGAACCAGGGCTACACACGTGC CTACAATGGACGCATCCAAAGAGAGAGCGAACCCTGCCCGCGAGAGCAAGCGGACCTCATAAAGTGCGTCGTAGTC CGGATTGGAGTCTGCAACTCGACTCCATGAAGTCGGAATCGCTAGTAATCGTGGATCAGAATGCCACGGTGAATAC GTTCCCGGGCCTTGTACACACCGCCCGTCACACCATGGGAGTGGGTTGCAAAAGAAGTAGGTAGCTTAACCTTCGG GAGGGCGCTTACCACTTTGGATGCGAGG |
| GQ448506.1.1374 |
| AGAGTTTGATCATGGCTCAGGATGAACGCTAGCTACAGGCTTAACACATGCAAGTCGAGGGGCAGCATGGTCTTAG CTTGCTAAGGCCGATGGCGACCGGCGCACGGGTGAGTAACACGTATCCAACCTGCCGTCTACTCTTGGACAGCCTT CTGAAAGGAAGATTAATACAAGATGGCATCATGAGTCCGCATGTTCACATGATTAAAGGTATTCCGGTAGACGATG GGGATGCGTTCCATTAGATAGTAGGCGGGGTAACGGCCCACCTAGTCTTCGATGGGTAGGGGTTCTGAGAGGAAGG TCCCCCACATTGGAACTGAGACACGGCCCAAACTCATACGGGAGGCAGCAGTGGGGAATATTGCACAATGGGGGAA ACCCTGATGCAGCGACGCCGCGTGAAGGATGAAGTATTTCGGTATGTAAACTTCTATCAGCAGGGAAGAAAATGAC GGTACCTGACTAAGAAGCCCCGGCTAACTACGTGCCAGCAGCCGCGGTAATACGTAGGGGGCAAGCGTTATCCGGA TTTACTGGGTGTAAAGGGAGCGTAGACGGCAGTGCAAGTCTGAAGTGAAAGCCCGGGGCTCAACCCCGGGACTGCT TTGGAAACTGTGCAGCTAGAGTGTCGGAGAGGCAAGCGGAATTCCTAGTGTAGCGGTGAAATGCGTAGATATTAGG AGGAACACCAGTGGCGAAGGCGGCTTGCTGGACGATGACTGACGTTGAGGCTCGAAAGCGTGGGGAGCAAACAGGA TTAGATACCCTGGTAGTCCACGCCGTAAACGATGACTACTAGGTGTCGGGGAGCAAAGCTCTTCGGTGCCGCAGCC AACGCAATAAGTAGTCCACCTGGGGAGTACGTTCGCAAGAATGAAACTCAAAGGAATTGACGGGGACCCGCACAAG CGGTGGAGCATGTGGTTTAATTCGAAGCAACGCGAAGAACCTTACCTGCTCTTGACATCCCTCTGACCGCTCTTTA ATCGGAGCTTTCCTTCGGGACAGAGGAGACAGGTGGTGCATGGTTGTCGTCAGCTCGTGTCGTGAGATGTTGGGTT AAGTCCCGCAACGAGCGCAACCCTTATGGTCAGTTACTACGCAAGAGGACTCTGGCCAGACTGCCGTTGACAAAAC GGAGGAAGGTGGGGATGACGTCAAATCATCATGCCCTTTATGACTTGGGCTACACACGTACTACAATGGCGTTAAA CAAAGAGAAGCGAGACCGCGAGGTGGAGCAAAACTCGGAAACAACGTCCCAGTTCGGACTGCAGGCTGCAACTCGC CTGCACGAAGTCGGAATTGCTAGTAATCGCAGATCAGCATGCTGCGGTGAATACGTTCCCGGGCCTTGCACTCACC GCCCGT |
| HQ802983.1.1440 |
| TAAGATGAACGCTGGCGGCGTGCTTAACACATGCAAGTCCTATGAAGCGCTTAAACGGATTTCTTCGGATTGAAGT TTTTGTGACTGAGTGGCGGACGGGTGAGTAACGCGTGGGTAACTTGCCTCATACAGGGGGATAACAGTTAGAAATG ACTGCTAATACCGCATAAGCGCACAGTGCTGCATGGCACAGTGTGAAAAACTCCGGTGGTATGAGATGGACCCGCG TCTGATTAGCTAGTTGGTGGGGTAACGGCCTACCAAGGCGACGATCAGTAGCCGGCCTGAGAGGGTGAACGGCCAC ATTGGGACTGAGACACGGCCCAAACTCCTACGGGAGGCAGCAGTGGGGAATATTGCACAATGGGGGAAACCCTGAT GCAGCGACGCCGCGTGAGCGAAGAAGTATTTCGGTATGTAAAGCTCTATCAGCAGGGAAGAAAATGACGGTACCTG ACTAAGAAGCACCGGCTAAATACGTGCCAGCAGCCGCGGTAATACGTATGGTGCAAGCGTTATCCGGATTTACTGG GTGTAAAGGGAGCGTAGACGGTTGTGTAAGTCTGATGTGAAAGCCCGGGGCTCAACCCCGGGACTGCATTGGAAAC TATGTAACTAGAGTGTCGGAGAGGTAAGCGGAATTCCTAGTGTAGCGGTGAAATGCGTAGATATTAGGAGGAACAC CAGTGGCGAAGGCGGCTTACTGGACGATCACTGACGTTGAGGCTCGAAAGCGTGGGGAGCAAACAGGATTAGATAC CCTGGTAGTCCACGCCGTAAACGATGACTACTAGGTGTCGGGGCCCATAAGGGCTTCGGTGCCGCAGCAAACGCAA TAAGTATTCCACCTGGGGAGTACGTTCGCAAGAATGAAACTCAAAGGAATTGACGGGGACCCGCACAAGCGGTGGA GCATGTGGTTTAATTCGAAGCAACGCGAAGAACCTTACCTGGTCTTGACATCCCACTGACCGGACAGTAATGTGTC CTTTCCTCCGGGACAGTGGAGACAGGTGGTGCATGGTTGTCGTCAGCTCGTGTCGTGAGATGTTGGGTTAAGTCCC GCAACGAGCGCAACCCCTATCCTTAGTAGCCAGCAGTAAGATGGGCACTCTAGGGAGACTGCCAGGGATAACCTGG AGGAAGGTGGGGATGACGTCAAATCATCATGCCCCTTATGACTTGGGCTACACACGTGCTACAATGGCGTAAACAA AGTGAAGCGAAGTCGTGAGGCCAAGCAAATCACAAAAATAACGTCTCAGTTCGGATTGTAGTCTGCAACTCGACTA CAAGAAGCTGGAATCGCTAGTAATCGCAGATCAGAATGCTGCGGTGAATACGTTCCCGGGTCTTGTACACACCGCC CGTCACACCATGGGAGTCGAAAATGCCCGAAGTCGGTGACCTAACGAAAGAAGGAGCCGCCGAAGGCAGGTT |
| DQ793824.1.1370 |
| ATGAACGCTGGCGGCGTGCCTAACACATGCAAGTCGAACGAAGCGCTTGAACGGATATCTTCGGACTGAAGTTCTT GCGACTGAGTGGCGGACGGGTGAGTAACGCGTGGGTAACCTGCCTCATACAGGGGGATAACAGTTAGAAATGACTG CTAATACCGCATAAGCGCACAGCTTCGCATGGAGCAGTGTGAAAAACTCCGGTGGTATGAGATGGACCCGCGTCAG ATTAGCTAGTTGGCAGGGTAACGGCCTACCAAGGCGACGATCTGTAGCCGACCTGAGAGGGTGACCGGCCACATTG GGACTGAGACACGGCCCAAACTCCTACGGGAGGCAGCAGTGGGGAATATTGCACAATGGGGGAAACCCTGATGCAG CGACGCCGCGTGAGCGAAGAAGTATTTCGGTATGTAAAGCTCTATCAGCAGGGAAGATAATGACGGTACCTGACTA AGAAGCTCCGGCTAAATACGTGCCAGCAGCCGCGGTAATACGTATGGAGCAAGCGTTATCCGGATTTACTGGGTGT AAAGGGAGCGTAGACGGTTTGACAAGTCTGATGTGAAATTCCAGGGCTTAACCCTGGACCTGCATTGGAAACTGTC GGACTAGAGTGTCGGAGAGGTGAGTGGAATTCCTAGTGTAGCGGTGAAATGCGTAGATATTAGGAGGAACACCAGT GGCGAAGGCGGCTCACTGGACGATAACTGACGTTGAGGCTCGAAAGCGTGGGGAGCAAACAGGATTAGATACCCTG |
| GTAGTCCACGCCGTAAACGATGTGTACTAGGTGTTGGGGAGCAAAGCTCTTCGGTGCCGTCGCAAACGCAGTAAGT ACACCACCTGGGGAGTACGTTCGCAAGAATGAAACTCAAAGGAATTGACGGGGACCCGCACAAGCGGTGGAGCATG TGGTTTAATTCGAAGCAACGCGAAGAACCTTACCAAATCTTGACATCGGAGTGACCGCTCTTTAATCGGAGCTTTC CTTCGGGACACTCCAGACAGGTGGTGCATGGTTGTCGTCAGCTCGTGTCGTGAGATGTTGGGTTAAGTCCCGCAAC GAGCGCAACCCTTATCCTTAGTAGCCAGCAAGTGAAGTTGGGCACTCTAGGGAGACTGCCAGGGATAACCTGGAGG AAGGTGGGGATGACGTCAAATCATCATGCCCCTTATGATTTGGGCTACACACGTGCTACAATGGCGTAAACAAAGG GAAGCGATCACGTGAGTGTGAGCAAATCTCAAAAATAACGTCCCAGTTCGGACTGTAGTCTGCAACCCGACTACAC GAAGCTGGAATCGCTAGTAATCGCAGGTCAGCATACTGCGGTGAATACGTTCCCGGGTCTTGCACACACCGCCCGT CA |
| GQ448468.1.1366 |
| AGAGTTTGATCCTGGCTCAGGATGAACGCTGACAGAATGCTTAACACATGCAAGTATACTTGATCCTTCGGGTGAT GGTGGCGGACGGGTGAGTAACGCGTAAAGAACTTGCCCTGCAGTCTGGGACAACATTTGGAAACGAATGCTAATCC CGCATAAGCCCACAGCTCGGCATCGAGCAGAGGGAAAAGGAGTGATCTGCTTTGAGATGGCCTCGCGTCCGATTAG CTGGTTGGTGAGGTGACGGCCCATCAAGGCAACGATCGGTAGCCGGACTGAGAGGTTGAACGGCCACATTGGGATT GAGACACGGCCCTTACTCCTACGGGAGGCAGCAGTGGGGAATATTGGACAATGGACCAAAAGTCTGATCCAGCAAT TCTGTGTGCACGATGAAGTTTTTCGGAATGTAAAGTGCTTTCAGTTGGGACGAAGTAAGTGACGGTACCAACAGAA GAAGCGACGGCTAAATACGTGCCAGCAGCCGCGGTAATACGTATGTCGCAAGCGTTATCCGGATTTATTGGGCGTA AAGCGCGTCTAGGCGGTTTGGTAAGTCTGATGTGAAAATGCGGGGCTCAACTCCGTATTGCGTTGGAAACTGCCAA ACTAGAGTACTGGAGAGGTGGGCGGAACTACAAGTGTAGAGGTGAAATTCGTAGATATTTGTAGGAATGCCGATGG GGAAGCCAGCCCACTGGACAGATACTGACGCTAAAGCGCGAAAGCGTGGGTAGCAAACAGGATTAGATACCCTGGT AGTCCACGCCGTAAACGATGATTACTAGGTGTTGGGGGTCGAACCTCAGCGCCCAAGCTAACGCGATAAGTAATCC GCCTGGGGAGTACGTACGCAAGTATGAAACTCAAAGGAATTGACGGGGACCCGCACAAGCGGTGGAGCATGTGGTT TAATTCGACGCAACGCGAGGAACCTTACCAGCGTTTGACATCCTAAGAAATTAGCAGAGATGCTTTTGTGCCCCTT CGGGGGAACTTAGTGACAGGTGGTGCATGGCTGTCGTCAGCTCGTGTCGTGAGATGTTGGGTTAAGTCCCGCAACG AGCGCAACCCCTTTCGTATGTTGCCATCATTAAGTTGGGCACTCATGCGATACTGCCTGCGATGAGCAGGAGGAAG GTGGGGATGACGTCAAGTCATCATGCCCCTTATACGCTGGGCTACACACGTGCTACAATGGGTAGTACAGAGAGTC GCAAACCTGCGAGGGGGAGCTAATCTCAGAAAACTATTCTCAGTTCGGATTGTACTCTGCAACTCGAGTACATGAA GTTGGAATCGCTAGTAATCGCAAATCAGCTATGTTGCGGTGAATACGTTCTCGGGTCTTGTACACACCGCCCGT |
| EU774020.1.1361 |
| AGAGTTTGATCCTGGCTCAGGACGAACGCTGGCGGCGTGCCTAACACATGCAAGTCGAGCGATTCTCTTCGGAGAA GAGCGGCGGACGGGTGAGTAACGCGTGGGTAACCTGCCCTGTACACACGGATAACATACCGAAAGGTATGCTAATA CGGGATAATATATAAGAGTCGCATGACTTTTATATCAAAGATTTTTCGGTACAGGATGGACCCGCGTCTGATTAGC TTGTTGGCGGGGTAACGGCCCACCAAGGCGACGATCAGTAGCCGACCTGAGAGGGTGATCGGCCACATTGGAACTG AGACACGGTCCAAACTCCTACGGGAGGCAGCAGTGGGGAATATTGCACAATGGGCGCAAGCCTGATGCAGCAACGC CGCGTGAGCGATGAAGGCCTTCGGGTCGTAAAGCTCTGTCCTCAAGGAAGATAATGACGGTACTTGAGGAGGAAGC CCCGGCTAACTACATGCCAGCAGCCGCGGTAATACGTATGTCGCAAGCGTTATCCGGATTTATTGGGCGTAAAGCG CGTCTAGGTGGTTTGGTAAGTCTGATGTGAAAATGCGGGGCTCAACTCCGTATTGCGTTGGAAACTGCCAAACTAG AGTACTGGAGAGGTAGGCGGAACTACAAGTGTAGAGGTGAAATTCGTAGATATTTGTAGGAATGCCGATGGGGAAG CCAGCCTACTGGACAGATACTGACGCTAAAGCGCGAAAGCGTGGGTAGCAAACAGGATTAGATACCCTGGTAGTCC ACGCCGTAAACGATGATTACTAGGTGTTGGGGGTCGAACCTCAGCGCCCAAGCTAACGCGATAAGTAATCCGCCTG GGGAGTACGTACGCAAGTATGAAACTCAAAGGAGTTGACGGGGACCCGCACAAGCGGTGGAGCATGTGGTTTAATT CGACGCAACGCGAGGAACCTTACCAGCGTTTGACATCCTAGGAATGAGAAAGAGATTTCTTAGTGCTCCTTCGGGA GAACCTAGAGACAGGTGGTGCATGGCTGTCGTCAGCTCGTGTCGTGAGATGTTGGGTTAAGTCCCGCAACGAGCGC AACCCCTATTGTATGTTGCCATCATTAAGTTGGGCACTCATGCGATACTGCCTGCGATGAGCAGGAGGAAGGTGGG GATGACGTCAAGTCATCATGCCCCTTATACGCTGGGCTACACACGTGCTACAATGGGCAGTACAGAGAGAAGCAAT ACCGCGAGGTGGAGCCAAACTTAAAAACCAGTCTCAGTTCGGATTGTAGGCTGAAACTCGCCTACATGAAGCTGGA GTTACTAGTAATCGCGAATCAGAATGTCGCGGTGAATACGTACCCGGGTCTTGTACACACCGCCCGTCA |
| GQ491183.1.1360 |
| GATGAACCCTTGCGGCGTGCTTAACACATGCAAGTCGAACGGAAACATTTATTGAAGCTTCGGCAGATCTAGCT TGTTTCTAGTGGCGGACGGGTGAGTAACGCGTGGGCAACCTGCCTCACACTGGGGGATAACAGTCAGAAATGGCTG CTAATACCGCATAAGCGCACAGCATCGCATGATGCAGTGTGAAAAACTCCGGTGGTGTGAGATGGACCCGCGTTGG ATTAGCTAGTTGGCAGGGCAGCGGCCTACCAAGGCGACGATCCATAGCCGGCCTGAGAGGGTGAACGGCCACATTG GGACTGAGACACGGCCCAGACTCCCACGGGAGGCAGCAGTGGGGAATATTGCACAATGGGGGAAACCCTGATGCAG CGACGCCGCGTGAAGGAAGAAGTATCTCGGTATGTAAACTTCTATCAGCAGGGAAGAAAATGACGGTACCTGACTA AGAAGCCCCGGCTAACTACGTGCCAGCAGCCGCGGTAATACGTAGGGGGCAAGCGTTATCCGGATTTACTGGGTGT AAAGGGAGCGTAGACGGTGTTGCAAGTCTGATGTGAAAGGCGGGGGCTCAACCCCTGGACTGCATTGGAAACTGTG ATACTCGAGTGCCGGAGAGGTAAGCGGAATTCCTAGTGTAGCGGTGAAATGCGTAGATATTAGAAGGAATACCAGT GGCGAAGGCGGCTTACTGGACGGTAACTGACGTTGAGGCTCGAAAGCGTGGGGAGCAAACAGATTAAGAAACCTC TGGGTAGTCCACGCCCGTAAACGAAGGAATAAAGGGGTCGGGAGCAGAGCTTTTCGGTGCCGCAGCAAACCCAATA AGTATTCCACCTTGAGAGGACGTTCGCAAGAATGAAACTCAAAGGAATTGACGGGGGACCCGCACAAGCGGTGGAG CATGTGGTTTAATTCGAAGCAACGCGAAGAACCTTACCAAGTCTTGACATCCCTCTGACCGCACCTTAACCGGTGC TTTCCTTCGGGACAGAGGAGACAGGTGGTGCATGGTTGTCGTCAGCTCGTGTCGTGAGATGTTGGGTTAAGTCCCG |
| CAACGAGCGCAACCCTTATCCTTAGTAGCCAGCGGTCCGGCCGGGCACTCTGGGGAGACTGCCAGGGATAACCTGG AGGAAGGTGGGGATGACGTCAAATCATCATGCCCCTTATGATTTGGGCTACACACGTGCTACAATGGCGTAAACAA AGGGAAGCGATCACGTGAGTGCGAGCAAATCTCAAAAATAACGTCCCAGTTCGGACTGTAGTCTGCAACCCGACTA CACGAAGCTGGAATCGCTAGTAATCGCAGGTCAGCATACTGCGGTGAATACGTTCCCGGGTCTTGTAC |
| GQ491426.1.1332 |
| GCTCAGGATGAACGCTGGCGGCGTGCTTAACACATGCAAGTCGAGCGAAGCACTTGCCATTGACTCTTCGGAAGAT TTGGCATTTGACTGAGCGGCGGACGGGTGAGTAACGCGTGGGTAACCTGCCTCATACGGGGGAATAACAGTTAGAA ATGGCTGCTAATGCCGCATAACCGCACAGGACCGCATGGACTGGTGTGAAAAACTGAGGTGGTATGAGATGGGCCC GCGTCTGATTAGGTTAGTTGGCGGGGTAACGGCCCACCAAGCCGACGATCAGTAGCCGACCTGAGAGGGACCGGCC ACATTGGGACTGAGACATGGCCCAGACTCCTACGGGAGGCAGCAGTGGGGAATATTGCACAATGGAGGAAACTCTG ATGCAGCGACGCCGCATGAAGGAAGAAGTATCTCGGTATGTAAACTTCTATCAGCAGGGAAGAAAATGACGGTACC TGACTAAGAAGCCCCGGCTAACTACGTGCCAGCAGCCGCGGTAATACGTAGGGGGCAAGCGTTATCCGGATTTACT GGGTGTAAAGGGAGCGTAGACGGACGGGCAAGTCTGATGTGAAAGCCCGGGGCTTAACCCCGGGACTGCATTGGAA ACTGTCCATCTTGAGTGCCGGAGAGGTAAGCGGAATTCCTAGTGTAGCGGTGAAATGCGTAGATATTAGGAGGAAC ACCAGTGGCGAAGGCGGCTTACTGGACGGTAACTGACGTTGAGGCTCGAAAGCGTGGGGAGCAAACAGGATTAGAT ACCCTGGTAGTCCACGCCGTAAACGATCAATAATGGGTGTCGGGTTGCAAAGCAATCCGGTGCCGCAGCAAACGCA GTAAGTATTCCCCCTCGGGAGTACGTTCGCAAGAATGAAACTCAAAGGAAGGACGGGGATCCGCACAAGCGGCGG AGCATGTGGTTTAATTAGAAGCAACGCGAAGAACCTTACCAAGTCTTGACATCTGCCTGACCGTTCCTTAACCGGA ACTATCTTTCGGGACAGGCAAGACAGGTGGTGCATGGTTGTCGTCAGCTCGTGTCGTGAGATGTTGGGTTAAGTCC CGCAACGAGCGCAACCCCTGTCCTTAGTAGCCAGCAGTCCGGCTGGGCACTCTAGGGAGACTGCCGGGGGTAACCC GGAGGAAGGCGGGGAGGAGGTCAAATCATCATGCCCCCCCTGATTTGGGCTACACACGTGGTACAATGGCGTAAAC AAAGGGAAGCGGAGTGGTGACGCTGAGCAAATCTCAAAAATAACGTCCCACTTCGGACTGCAGTCTGCAACTCGAC TGCACGAAGCTGGAATCGCTAGTAATCGCGAATCAGAATG |
| GQ493039.1.1311 |
| GATGAACGCTGGCGGCGTGCCTAACACATGCAAGTCGAGCGATTTACTTCGGTAAAGAGCGGCGGACGGGTGAGTA ACGCGTGGGTAACCTGCCCTGTACACACGGATAACATACCGAAAGGTATGCTAATACGAGATAATATGCTTTTATC GCATGGTAGAAGTATCAAAGCTTTTGCGGTACAGGATGGACCCGCGTCTGATTAGCTAGTTGGTAAGGTAACGGCT TACCAAGGCAACGATCAGTAGCCGACCTGAGAGGGTGATCGGCCACATTGGAACTGAGACACGGTCCAAACTCCTA CGGGAGGCAGCAGTGGGGAATATTGCACAATGGGCGAAAGCCTGATGCAGCAACGCCGCGTGAGCGATGAAGGCCT TCGGGTCGTAAAGCTCTGTCCTCAAGGAAGATAATGACGGTACTTGAGGAGGAAGCCCCGGCTAACTACGTGCCAG CAGCCGCGGTAATACGTAGGGGGCTAGCGTTATCCGGAATTACTGGGCGTAAAGGGTGCGTAGGTGGTTTCTTAAG TCAGAGGTGAAAGGCTACGGCTCAACCGTAGTAAGCCTTTGAAACTGGGAAACTTGAGTGCAGGAGAGGAGAGTGG AATTCCTAGTGTAGCGGTGAAATGCGTAGATATTAGGAGGAACACCAGTTGCGAAGGCGGCTCTCTGGACTGTAAC TGACACTGAGGCACGAAAGCGTGGGGAGCAAACAAGATTAGNTNCCCTGGTAGTCCNCGCCGTNNCCGCCCATAAAG AGCTGTCGGAGGTTACCCCCTTCGGTGGCGCAGGTAACGCAATAAAGAATTCCGCCTGGGAAGGAACGCTTCGCAA GAGTGAAATTAAAAGGAATAGACGGGGACCCGCTCAAGTAGTGGAGCATGTGGTTTAATTCGAAGCAACGCGAAGA ACTTTCTCTAAGCTTGACATCCTTTTGACCGATGCCTAATAGCATCAATCCCTTCTGGGACAGAAGTGACAGGTGG TGCATGGTTGTTGTCAGCTCGTGTCGTGAGATGTTGGGTTAAGTCCCGCAACGAGCGCAACCCTTGCCTTTAGTTG CCAGCATTAAGTTGGGCACTCTATAGGGACTGCCAGGGATAACCTGGAGGAAGGTGGGGATGACGTCAAATCATCA TGCCCCTTATGCTTAGGGCTACACACGTGCTACAATGGGTGGTACAGAGGGCAGCCAAGTCGTGAGGCGGAGCTAA TCCCTTAAAGCCATTCTCAGTTCGGATTGTAGGCTGAAACTCGCCTACATGAAGCTGGAGTTACTAGTAATCGCAG ATCAGAATGATGCGGTGAA |
| JN387556.1.1324 |
| CGTAAGTAACCTGCCCTGTACACACGGATAACATACCGAAAGGTATGCTAATACGGATAATATATTTTGATCGCA TGGTCGAGATATCAAAGCTCCGGCGGTACACCAGGACCCCCGACAGAGGAGCTAGTTGGTAGTAATGTCACCAAG GCGACGATCAGAAGCCGAACTGAGAGGGGGATCCGCACATGACTGAGACACGGTCAAACTCCTACGGGAGGCAGCA GTGGGGAATATGCCAATGGGCGAAAGCTGATGCAGCACGCGCGTGAGCGATGAGGCTCGGGTCGTAAAGCTCGTCT CAAGGAAGATAATGACGGTACTTGAGGAGGAAGCCCCGGCTAACTACGTGCCAGCAGCCGCGGTAATACGTAGGGG GCTAGCGTTATCCGGAATTACTGGGCGTAAAGGGTGCGTAGGCGGTCTTTCAAGTCAGGAGTGAAAGGCTACGGCT CAACCGTAGTAAGCTCTTGAAACTGTAAGACTTGAGTGCAGGAGAGGAGAGTGGAATTCCTAGTGTAGCGGTGAAA TGCGTAGATATTAGGAGGAACACCAGTTGCGAAGGCGGCTCTCTGGACTGTAACTGACGCTGAGGCACGAAAGCGT GGGGAGCAAACAGGATTAGATACCCTGGTAGTCCACGCCGTAAACGATGAGTACTAGCTGTCGGAGGTTACCCCCT TCGGTGGCGCAGCTAACGCATTAAGTACTCCGCCTGGAAGTACGCTCGCAAGAGTGAAACTCAAAGGAATTGACG GGGACCCGCACAAGTAGCGGAGCATGTGGTTTAATTCGAAGCAACGCGAAGAACCTTACCTAAGCTTGACATCCCA CTGACCCTTCCCTAATCGGAAGCTTCCCTTCGGGACAGTGGTGACAGGTGGTGCATGGTTGTCGTCAGCTCGTGTC GTGAGATGTTGGGTTAAGTCCCGCAACGAGCGCAACCCTTGCCTTTAGTTGCCAGCATTAAGTTGGGCACTCTAGA GGGACTGCCAGGGATAACCCGGAGGAGTGGGGATGACGTCAAATCATCATGCCCTTATGCTAGGCTACACACGTGC TACAATGGGTGGTCAGAGGCCAGCCAGTCGTGAGGCCGAGCTATCCCATAAGCCATTCTCGTCCGGATTGTAGGCT GAACTCGCCTACATGAGCTGGAATTACAAGTATGCGATCGATGCTGCGTGATGCGTCCGGGTCTTGTACACACCGC CCGTCACACCATGGGAGTTGGGGGCGCCCGAAGCCGGATTGCTAACCTTTTGGAAGCGTCCGTCGAAGGTGAAACC AATAACTGGGGTGAAGTCGTAACAAGGTAACC |
| EU775983.1.1288 |
| GAAAGCGGCGGACGGGTGAGTAACGCGTAGGCAACCTGCCCATACAGAGGATAGCATCTGGAAACGGATATTAA TACCTCATAATACTTAGAGATCACATGGTAACTAAGTCAAAGATTTATCGGTATGGGATGGGCCTGCGTCTGATTA GCTAGTTGGTGGGGTAACGGCTCACCAAGGCGACGATCAGTAGCCGACCTGAGAGGGTGATCGGCCACATTGGAAC TGAGACACGGTCCAAACTCCTACGGGAGGCAGCAGTGGGGAATATTGCACAATGGGCGCAAGCCTGATGCAGCAAC GCCGCGTGAGCGATGAAGGCCTTCGGGTCGTAAAGCTCTGTCCTCAAGGAAGATAATGACGGTACTTGAGGAGGAA GCCCCGGCTAACTACGTGCCAGCAGCCGCGGTAATACGTAGGGGGCTAGCGTTATCCGGATTTACTGGGCGTAAAG GGTGCGTAGGCGGTCTTTCAAGTCAGGAGTTAAAGGCTACGGCTCAACCGTAGTAAGCTCCTGATACTGTCTGACT TGAGTGCAGGAGAGGAAAGCGGAATTCCCAGTGTAGCGGTGAAATGCGTAGATATTGGGAGGAACACCAGTAGCGA AGGCGGCTTTCTGGACTGTAACTGACGCTGAGGCACGAAAGCGTGGGGAGCAAACAGGATTAGATACCCTGGTAGT CCACGCTGTAAACGATGAGTACTAGGTGTCGGAGGTTACCCCCTTCGGTGCCGCAGCTAACGCATTAAGTACTCCG CCTGGGGAGTACGCACGCAAGTGTGAAACTCAAAGGAATTGACGGGGACCCGCACAAGTAGCGGAGCATGTGGTTT AATTCGAAGCAACGCGAAGAACCTTACCTAGGCTTGACATCCTTCTGACCGAGGACTAATCTCCTCTTTCCCTCCG GGGACAGAAGTGACAGGTGGTGCATGGTTGTCGTCAGCTCGTGTCGTGAGATGTTGGGTTAAGTCCCGCAACGAGC GCAACCCTTGTCTTTAGTTGCCATCATTAAGTTGGGCACTCTAGAGAGACTGCCAGGGATAACCTGGAGGAAGGTG GGGATGACGTCAAATCATCATGCCCCTTATGCCTAGGGCTACACACGTGCTACAATGGGTGGTACAGAGGGCAGCC AAGCCGTGAGGTGGAGCAAATCCCTTAAAGCCATTCTCAGTTCGGATTGTAGGCTGAAACTCGCCTACATGAAGCT GGAGTTACTAGTAATCGCAGATCAGAATGCTGCGGTGAATGCGTTCCCGGGTCTTGCACACACCGCCCGTCA |
| |

| **OTUs in Table 5** |
|---|
| GQ449137. 1.1391 |
| CTGGCTCAGGATGAACGCTAGCGACAGGCTTAACACATGCAAGTCGAGGGGCATCACGGAGGTAGCAATACCTTC TGGTGGCGACCGGCGCACGGGTGAGTAACACGTATGCAACCTGCCCTGTACAGAGGGACAAGCGGTGGAAACGCCG TCTAATCCCGCATGCACTCTTCCGGGGGCATCCCCGGGAGAGTAAAGGAGAGATCCGGTACAGGATGGACATGCGG CGCATTAGTTAGTTGGCGGGGTAACGGCCCACCAAGACGACGATGCGTAGGGGTTCTGAGAGGAAGGTCCCCCACA TTGGAACTGAGACACGGTCCAAACTCCTACGGGAGGCAGCAGTGAGGAATATTGGTCAATGGGCGGAAGCCTGAAC CAGCCAAGTCGCGTGAGGGAAGACGGCCCTACGGGTTGTAAACCTCTTTTGTCGGGGAGCAATGCCGCCTTTGCGA AGGCGGAGGGAGAGTACCCGAAGAAAAAGCACCGGCTAACTACGTGCCAGCAGCCGCGGTAATACGTAGGGTGCAA GCGTTAATCGGAATTACTGGGCGTAAAGCGTGCGCAGGCGGTTCTGTAAGACAGATGTGAAATCCCCGGGCTCAAC CTGGGAATTGCATTTGTGACTGCAGGACTAGAGTTCATCAGAGGGGGGTGGAATTCCAAGTGTAGCAGTGAAATGC GTAGATATTTGGAAGAACACCAATGGCGAAGGCAGCCCCCTGGGATGCGACTGACGCTCATGCACGAAAGCGTGGG GAGCAAACAGGATTAGATACCCTGGTAGTCCACGCCCTAAACGATGTCTACTGGTTGTTGGGGATTAATATCCTTG GTAACGAAGCTAACGCGTGAAGTAGACCGCCTGGGGAGTACGGTCGCAAGATTAAAACTCAAAGGAATTGACGGGG ACCCGCACAAGCGGTGGATGATGTGGATTAATTCGATGATACGCGAGGAACCTTACCCGGGCTCAAACGGCACAGT GATACTTTTGAAAGGAGGTAGCTCTACGGAGACTGTGCCGAGGTGCTGCATGGTTGTCGTCAGCTCGTGCCGTGAG GTGTCGGCTTAAGTGCCATAACGAGCGCAACCCCTATTGTCAGTTGCCAGCAGGTAAAGCTGGGGACTCTGACGAG ACTGCCGGCGCAAGCTGAGAGGAAGGCGGGGATGACGTCAAATCAGCACGGCCCTTACGTCCGGGGCGACACACGT GTTACAATGGCAGGCACAGCGGGAAGCCACCCGGCGACGGGGAGCGGAACCCGAAAGCCTGTCTCAGTTCGGATCG GAGTCTGCAACTCGACTCCGTGAAGCTGGATTCGCTAGTAATCGCGCATCAGCCATGGCGCGGTGAATACGTTCCC GGGCCTTGTACACACCGCCCGTA |
| HK555938.1.1357 |
| ACGGCACCCCTCTCCGGAGGGAAGCGAGTGGCGAACGGCTGAGTAACACGTGGAGAACCTGCCCCCTCCCCCGGGA TAGCCGCCCGAAAGGACGGGTAATACCGGATACCCCCGGGCGCCGCATGGCGCCCGGGCTAAAGCCCCGACGGGAG GGGATGGCTCCGCGGCCCATCAGGTAGACGGCGGGGTGACGGCCCACCGTGCCGACAACGGGTAGCCGGGTTGAGA GACCGACCGGCCAGATTGGGACTGAGACACGGCCCAGACTCCTACGGGAGGCAGCAGTGGGGAATCTTGCGCAATG GGGGGAACCCTGACGCAGCGACGCCGCGTGCGGGACGGAGGCCTTCGGGTCGTAAACCGCTTTCAGCAGGGAAGAG TCAAGACTGTACCTGCAGAAGAAGCCCCGGCTAACTACGTGCCAGCAGCCGCGGTAATACGTAGGGGGCGAGCGTT ATCCGGATTCATTGGGCGTAAAGCGCGCGTAGGCGGCCCGGCAGGCCGGGGGTCGAAGCGGGGGGCTCAACCCCCC GAAGCCCCCGGAACCTCCGCGGCTTGGGTCCGGTAGGGGAGGGTGGAACACCCGGTGTAGCGGTGGAATGCGCAGA TATCGGGTGGAACACCGGTGGCGAAGGCGGCCCTCTGGGCCGAGACCGACGCTGAGGCGCGAAAGCTGGGGGAGCG AACAGGATTAGATACCCTGGTAGTCCCAGCCGTAAACGATGGACGCTGGGTGTGGGGGGACGATCCCCCCGTGCCG CAGCCNACGCATTAAGCGTCCCGCCTGGGGAGTACGGCCGCAAGGCTAAAACTCAAAGGAATTGACGGGGGCCCGC ACAAGCAGCGGAGCATGTGGCTTAATTCGAAGCAACGCGAAGAACCTTACGGCGCATCCCCCCGAGGCCCACGGGG GGTCCGCCGCGTGGGTCAGAGGAGCGCATACGGGAGGTGCATGGTTGTCGTCAGCTCGTGTCGTGAGATGTTGGGT TAAGTCCCGCAACGAGCGCAACCCCCGCCGCGTGTTGCCATCGGGTGATGCCGGGAACCCACGCGGGACCGCCGCC GTCAAGGCGGAGGAGGGCGGGGACGACGTCAAGTCATCATGCCCCTTATGCCCTGGGCTGCACACGTGCTACAATG GCCGGTACAGAGGGATGCCACCCCGCGAGGGGGAGCGGATCCCGGAAAGCCGGCCCCAGTTCGGATTGGGGGCTGC AACCCGCCCCCATGAAGTCGGAGTTGCTAGTAATCGCGGATCAGCATGCCGCGGTGAATGCGTTCCCGGGCCTTGT ACACACCGCCCGTCACACCACCCGAGTCGTCTGCACCCGAAGTCGCCGGCCCAACCGCAAGGGGG |
| GQ358246.1.1466 |
| AGAGTTGATCTGGCTCAGATTGAACGCTGGCGGCAGGCTTAATACATGCAAGTCGAACGGTAACAGCAAAAAGCT TGCTTTTTTGGCTGACGAGTGGCGGACGGGTGAGTAATACCTAGGAAGCTGCCTAAACGAGGGGGATAACACCTGG AAACGGGTGCTAATACCGCATGATACCGCAAGGTCAAAGGTTGGTTTACCAATCGCGTTTAGATGCGCCTAGGAGG GATTAGCTAGTTGGTGGGGTAACGGCTCACCAAGGCGATGATCAGTAGCCGGTCTGAGAGGATGAACGGCCACATT GGGACTGAGACACGGCCCAGACTCCTACGGGAGGCAGCAGTGGGGAATCTTCCGCAATGGGCGAAAGCCTGACGGA GCAATGCCGCGTGAGTGATGAAGGGATTCGTCCCGTAAAGCTCTGTTGTATATGACGAATGTGCAGATTGTGAATA ATGATTTGTAATGACGGTAGTATACGAGGAAGCCACGGCTAACTACGTGCCAGCAGCCGCGGTAATACGTAGGTGG CGAGCGTTGTCCGGAATTATTGGGCGTAAAGAGCATGTAGGCGGTTTTTTAAGTCTGGAGTGAAAATGCGGGGCTC AACCCCGTATGGCTCTGGATACTGGAAGACTTGAGTGCAGGAGAGGAAAGGGGAATTCCCAGTGTAGCGGTGAAAT GCGTAGATATTGGGAGGAACACCAGTGGCGAAGGCGCCTTTCTGGACTGTGTCTGACGCTGAGATGCGAAAGCCAG GGTAGCGAACGGGATTAGATACCCCGGTAGTCCTGGCCGTAAACGATGGGTACTAGGTGTGGGAGGTATCGACCCC TTCCGTGCCGGAGTTAACGCAATAAGTACCCCGCCTGGGGAGTACGTCCGCAAGGATGAAACTCAAAGGAATTGAC GGGGGCCCGCACAAGCGGTGGAGTATGTGGTTTAATTCGACGCAACGCGAAGAACCTTACCAAGGCTTGACATTGA TTGAAAGACCTAGAGATAGGTCCCTCTCTTCGGAGACAAGAAAACAGGTGGTGCATGGCTGTCGTCAGCTCGTGTC GTGAGATGTTGGGTTAAGTCCCGCAACGAGCGCAACCCCTATCCTATGTTACCAGCGGGTAATGCCGGGGACTCAT AGGAGACTGCCAAGGACAACTTGGAGGAAGGCGGGGATGACGTCAAGTCATCATGCCCCTTATGTCTTGGGCTACA CACGTACTACAATGGTCGGCAACAGAGGGAAGCAAAGCCGCGAGGCAGAGCAAACCCCAGAAACCCGATCTCAGTT CGGATTGCAGGCTGCAACTCGCCTGCATGAAGTCGGAATCGCTAGTAATCGCAGGTCAGCATACTGCGGTGATTAC TATCCCGGGCGTTGTACTCACCGCCCGTCAGGCGGAGTTCGTACTTCAAATGTGCCACACTGGG |
| New.ReferenceOTU82 |
| TACGTAGGTGGCAAGCGTTGTCCGGATTTACTGGGCGTAAAGGAGCGTAGGCGGATTTTAAGTGGATGTGAAA TACCCGGGCTCAACCTGGGTGCTGCATTCCAAACTGGAAATCTAGAGTGCAGGAGGGGAAAGTGGAATTCCTAGTG TAGCGGTGAAATGCGTAGAGATTAGGAAGAACACCAGTGGCGAAGGCGACTTTCTGGACTGTAACTGACGCTGAGG CTCGAAAGCGTGGGGAGCAAACAGG |
| New.ReferenceOTU52 |
| TACGTAGGTGGCGAGCGTTATCCGGATTTACTGGGCGTAAAGGGAGCGTAGGCGGATGATTAAGTGGGATGTGAAA TACCCGGGCTCAACTTGGGTGCTGCATTCCAAACTGGTTATCTAGAGTGCAGGAGAGGAGAGTGGAATTCCTAGTG TAGCGGTGAAATGCGTAGAGATTAGGAAGAACACCAGTGGCGAAGGCGACTCTCTGGACTGTAACTGACGCTGAGG CTCGAAAGCGTGGGGAGCAAACAGG |
| GQ138615.1.1402 |
| AGAGTTTGATCCTGGCTCAGGATGAACGCTAGCGATAGGCCTAACACATGCAAGTCGAGGGGCAGCACATGAGTAG CAATACGATGGTGGCGACCGGCGCACGGGTGAGTAACACGTATGCAACCTACCTTTAACAGGGGAATAACCCGTTG AAAAACGGACTAATACTCCATAACACAGGGGTCCCGCATGGGAATATTTGTTAAAGATTTATCGGTTGAAGATGGG CATGCGTTCCATTAGCTAGTTGGTAGGGTAAAGGCCTACCAAGGCGACGATGCGTAGCCGACCTGAGAGGGTGAAC GGCCACACTGGGACTGAGACACGGCCCACACTCCTACGGGAGGCAGCAGTAGGGAATCTTCGGCAATGGGCGAAAG CCTGACCGAGCAACGCCGCGTGAATGATGAAGGCCTTCGGGTTGTAAAATTCTGTTATAAGGAAGAACGACTTTA GTAGGAAATGGCTAGAGTGTGACGGTACCTTATGAGAAAGCCACGGCTAACTACGTGCCAGCAGCCGCGGTAATAC GTAGGTGGCGAGCGTTATCCGGAATTATTGGGCGTAAAGAGCGCGCAGGTGGTTGATTAAGTCTGATGTGAAAGCC CACGGCTTAACCGTGGAGGGTCATTGGAAACTGGTCGACTTGAGTGCAGAAGAGGGAAGTGGAATTCCATGTGTAG CGGTGAAATGCGTAGAGATATGGAGGAACACCAGTGGCGAAGGCGGCTTCCTGGTCTGTAACTGACACTGAGGCGC GAAAGCGTGGGGAGCAAACAGGATTAGATACCCTGGTAGTCCACGCCGTAAACGATGAGTGCTAAGTGTTGGGGGT CGAACCTCAGTGCTGAAGTTAACGCATTAAGCACTCCGCCTGGGGAGTACGGTCGCAAGACTGAAACTCAAAGGAA TTGACGGGGACCCGCACAAGCGGTGGAGCATGTGGTTTAATTCGAAGCAACGCGAAGAACCTTACCAGGTCTTGAC ATACCATTGACCGTTCTAGAGATAGGATTTTCCCTTCGGGGACAATGGATACAGGTGGTGCATGGTTGTCGTCAGC TCGTGTCGTGAGATGTTGGGTTAGGTCCCGCAACGAGCGCAACCCCTGTCGTTAGTTGCCAGCATTCAGTTGGGGA CTCTAACGAGACTGCCAGTGACAAACTGGAGGAAGGTGGGGATGACGTCAAATCATCATGCCCCTTATGACCTGGG CTACACACGTGCTACAATGGTTGGTACAAAGAGAAGCGAAGCGGTGACGTGGAGCAAACCTCATAAAGCCAATCTC AGTTCGGATTGTAGGCTGCAACTCGCCTACATGAAGTTGGAATCGCTAGTAATCGCGAATCAGAATGTCGCGGTGA ATACGTTCCCGGGTCTTGTACACACCGCCCGTCA |
| JN681884.1.1409 |
| TGCAAGTAGAACGCTGAAGACTGGTGCTTGCACCGGTTGGAAGAGTTGCGAACGGGTGAGTAACGCGTAGGTAACC TGCCTGATAGCGGGGGATAACTATTGGAAACGATAGCTAATACCGCATAACAGGGAATAACACATGTTATTTTTTT GAAAGGGGCAATTGCTCCACTATCAGATGGACCTGCGTTGTATTAGCTAGTAGGTGAGGTAACGGCTCACCTAGGC GACGATACATAGCCGACCTGAGAGGGTGATCGGCCACACTGGGACTGAGACACGGCCCAGACTCCTACGGGAGGCA GCAGTAGGGAATCTTCGGCAATGGGGGCAACCCTGACCGAGCAACGCCGCGTGAGTGAAGAAGGTTTTCGGATCGT AAAGCTCTGTTGTAAGAGAAGAACGTTGAGTAGAGTGGAAAGTTACTCAAGTGACGGTATCTTACCAGAAAGGGAC GGCTAACTACGTGCCAGCAGCCGCGGTAATACGTAGGTCCCGAGCGTTGTCCGGATTTATTGGGCGTAAAGCGAGC GCAGGCGGTTTAATAAGTCTGAAGTTAAAGGCAGTGGCTCAACCATTGTTCGCTTTGGAAACTGTTAAACTTGAGT GCAGAAGGGGAGAGTGGAATTCCATGTGTAGCGGTGAAATGCGTAGATATATGGAGGAACACCGGTGGCGAAAGCG GCTCTCTGGTCTGTAACTGACGCTGAGGCTCGAAAGCGTGGGTAGCGAACAGGATTAGATACCCTGGTAGTCCACG CCGTAAACGATGAGTGCTAGGTGTTGGGTCCTTTCCGGGACTCAGTGCCGACGCTAACGCATTAAGCACTCCGCCT |
| GGGGAGTACGACCGCAAGGTTGAAACTCAAAGGAATTGACGGGGGCCCGCACAAGCGGTGGAGCATGTGGTTTAAT TCGAAGCAACGCGAAGAACCTTACCAGGTCTTGACATCCCGATGCTATCCCTAGAGATAGGGAGTTACTTCGGTAC ATCGGTGACAGGTGGTGCATGGTTGTCGTCAGCTCGTGTCGTGAGATGTTGGGTTAAGTCCCGCAACGAGCGCAAC CCCTATTGTTAGTTGCCATCATTCAGTTGGGCACTCTAGCGAGACTGCCGGTAATAAACCGGAGGAAGGTGGGGAT GACGTCAAATCATCATGCCCCTTATGACCTGGGCTACACACGTGCTACAATGGTTGGTACAACGAGTTGCGAGTCG GTGACGGCAAGCTAATCTCTTAAAGCCAATCTCAGTTCGGATTGTAGGCTGCAACTCGCCTACATGAAGTCGGAAT CGCTAGTAATCGCGGATCAGCACGCCGCGGTGAATACGTTCCCGGGCCTTGTACACACCGCCCGTCACACCACGAG AGTTTGTAACACCCAAAGTCGGTGAGGTAACCTTCGGAGCC |
| GU303759.1.1517 |
| AGAGTTTGATCATGGCTCAGGACGAACGCCGGCGGCGTGCCTAATACATGCAAGTAGAACGCTGAAGACTTTAGCT TGCTAAAGTTGGAAGAGTTGCGAACGGGTGAGTAACGCGTAGGTAACCTGCCTACTAGCGGGGGATAACTATTGGA AACGATAGCTAATACCGTATAACAGCATTTAACACATGTTAGATGCTTGAAAGGAGCAATTGCTTCACTAGTAGAT GGACCTGCGTTGTATTAGCTAGTTGGTGAGGTAACGGCTCACCAAGGCGACGATACATAGCCGACCTGAGAGGGTG ATCGGCCACACTGGGACTGAGACACGGCCCAGACTCCTACGGGAGGCAGCAGTAGGGAATCTTCGGCAATGGGGGC AACCCTGACCGAGCAACGCCGCGTGAGTGAAGAAGGTTTTCGGATCGTAAAGCTCTGTTGTAAGAGAAGAACGTGT GTGAGAGTGGAAAGTTCACACAGTGACGGTAACTTACCAGAAAGGGACGGCTAACTACGTGCCAGCAGCCGCGGTA ATACGTAGGTCCCGAGCGTTGTCCGGATTTATTGGGCGTAAAGCGAGCGCAGGCGGTTTAATAAGTCTGAAGTTAA AGGCAGTGGCTTAACCATTGTTCGCTTTGGAAACTGTTAGACTTGAGTGCAGAAGGGGAGAGTGGAATTCCATGTG TAGCGGTGAAATGCGTAGATATATGGARGGAAACACCGGTGGCGAAAGCGGCTCTCTGGTCTGTAACTGACGCTGA GGCTCGAGAAGCGTGGGGAGCAAACAGGATTAGATACCCTGGTAGTCCACGCCGTAAGCGATGAGTGCTAGGTGTT AGGCCCTTTCCGGGGCTTAGTGCCGCAGCTAACGCATTAAGCACTCCGCCTGGGGAGTACGACCGCAAGGTTGAAA CTCAAAGGAATTGACGGGGGCCCGCACAAGCGGTGGAGCATGTGGTTTAATTCGAAGCAACGCGAAGAACCTTACC AGGTCTTGACATCCCGATGCTATTCCTAGAGATAGGAAGTTTCTTCGGAACATCGGTGACAGGTGGTGCATGGTTG TCGTCAGCTCGTGTCGTGAGATGTTGGGTTAAGTCCCGCAACGAGCGCAACCCCTATTGTTAGTTGCCATCATTAA GTTGGGCACTCTAGCGAGACTGCCGGTAATAAACCGGAGGAAGGTGGGGATGACGTCAAATCATCATGCCCCTTAT GACCTGGGCTACACACGTGCTACAATGGCGGTCAACAGAGGGAAGCAATACTGTGAAGTGGAGCAAACCCCTAAAA GCCGTCCCAGTTCGGATTGCAGGCTGCAACCCGCCTGTATGAAGTTGGAATCGCTAGTAATCGCGGATCAGCATGC CGCGGTGAATACGTTCCCGGGCCTTGTACACACCGCCCGTCACACCATGAGAGTCGGGAACACCCGAAGTCCGTAG CCTAACTTTCACGAGGGGGCGCGGCCGAAGGTGGGTTCGATAATTGGGGTGAAGTCGTAACAAGGTAACCGTA |
| New.ReferenceOTU114 |
| TACGTAGGTCCCGAGCGTTGTCCGGATTTATTGGGCGTAAAGCGAGCGCAGGCGGTTTAATAAGTCTGAAGTTAAA GGCAGTGGCTTAACCATTTTTCGCTTTGGAAACTGTTAGACTTGAGTGCAGAAGGGGAGAGTGGAATTCCATGTGT AGCGGTGAAATGCGTAGATATATGGAGGAACACCGGTGGCGAAAGCGGCTCTCTGGTCTGTAACTGACGCTGAGGC TCGAAAGCGTGGGGAGCAAACAGG |
| EU774881.1.1422 |
| AGAGTTTGATCCTGGCTCAGGACGAACGCTGGCGGCGTGCCTAATACATGCAAGTCGAGCGAGCGGAACTAACAGA TTTACTTCGGTAATGACGTTAGGAAAGCGAGCGGCGGATGGGTGAGTAACACGTGGGGAACCTGCCCCATAGTCTG GGATACCACTTGGAAACAGGTGCTAATACCGGATAAGAAAGCAGATCGCATGATCAGCTTTTAAAAGGCGGCGTAA GCTGTCGCTATGGATGGCCCCGCGGTGCATTAGCTAGTTGGTAAGGTAAAGGCTTACCAAGGCAATGATGCATAG CCGAGTTGAGAGACTGATCGGCCACATTGGGACTGAGACACGGCCCAAACTCCTACGGGAGGCAGCAGTAGGGAAT CTTCCACAATGGACGCAAGTCTGATGGAGCAACGCCGCGTGAGTGAAGAAGGTTTTCGGATCGTAAAGCTCTGTTG TTGGTGAAGAAGGATAGAGGTAGTAACTGGCCTTTATTTGACGGTAATCAACCAGAAAGTCACGGCTAACTACGTG CCAGCAGCCGCGGTAATACGTAGGTGGCAAGCGTTGTCCGGATTTATTGGGCGTAAAGCGAGCGCAGGCGGTTTAA TAAGTCTGAAGTTAAAGGCAGTGGCTTAACCATTGTTCGCTTTGGAAACTGTTAGACTTGAGTGCAGAAGGGGAGA GTGGAATTCCATGTGTAGCGGTGAAATGCGTAGATATATGGAGGAACACCGGTGGCGAAAGCGGCTCTCTGGTCTG TAACTGACGCTGAGGCTCGAAAGCGTGGGGAGCAAACAGGATTAGATACCCTGGTAGTCCACGCCGTAAACGATGA GTGCTAGGTGTTAGGCCCTTTCCGGGGCTTAGTGCCGCAGCTAACGCATTAAGCACTCCGCCTGGGGAGTACGACC GCAAGGTTGAAACTCAAAGGAATTGACGGGGGCCCGCACAAGCGGTGGAGCATGTGGTTTAATTCGAAGCAACGCG AAGAACCTTACCAGGTCTTGACATCCCGATGCTATTCCTAGAGATAGGAAGTTTCTTCGGAACATCGGTGACAGGT GGTGCATGGTTGTCGTCAGCTCGTGTCGTGAGATGTTGGGTTAAGTCCCGCAACGAGCGCAACCCCTATTGTTAGT TGCCATCATTAAGTTGGGCACTCTAGCGAGACTGCCGGTAATAAACCGGAGGAAGGTGGGGATGACGTCAAGTCAT CATGCCCCTTATGACCTGGGCTACACACGTGCTACAATGGGCAGTACAACGAGAAGCGAGCCTGCGAAGGCAAGCG AATCTCTGAAAGCTGTTCTCAGTTCGGACTGCAGTCTGCAACTCGACTGCACGAAGCTGGAATCGCTAGTAATCGC GGATCAGCACGCCGCGGTGAATACGTTCCCGGGCCTTGCACACACCGCCCGTCA |
| AB469559.1.1551 |
| AGAGTTTGATCCTGGCTCAGGACGAACGCTGGCGGCGTGCCTAATACATGCAAGTGGAACGCACAGTTAGTATGTA GTTTACTACAACATTACTTGTGAGTCGCGAACGGGTGAGTAACGCGTAGGTAACCTGCCTTGTAGCGGGGGATAAC TATTGGAAACGATAGCTAATACCGCATAACAGTTGATAACTCATGTTATTAGCTTGAAAGATGCAACAGCATCACT ACGAGATGGACCTGCGTTGTATTAGCTAGTTGGTGAGGTAAAGGCTCACCAAGGCCACGATACATAGCCGACCTGA GAGGGTGATCGGCCACATTGGGACTGAGACACGGCCCAAACTCCTACGGGAGGCAGCAGTAGGGAATCTTCGGCAA TGGGGGCAACCCTGACCGAGCAACGCCGCGTGAGTGAAGAAGGTTTTCGGATCGTAAAGCTCTGTTGTAAGAGAAG |
| AACGTTGATGAGAGTGGAAAATTCATCAAGTGACGGTATCTTACCAGAAAGGACGGCTAACTACGTGCCAGCAGC CGCGGTAATACGTAGGTCCCGAGCGTTGTCCGGATTTATTGGGCGTAAAGCGAGCGCAGGCGGTTTCGTAAGTCTG AAGTTAAAGGCAGTGGCTCAACCATTGTTCGCTTTGGAAACTGCGAGACTTGAGTGCAGAAGGGGAGAGTGGAATT CCATGTGTAGCGGTGAAATGCGTAGATATATGGAGGAACACCGGTGGCGAAAGCGGCTCTCTGGTCTGTAACTGAC GCTGAGGCTCGAAAGCGTGGGGAGCAAACAGGATTAGATACCCTGGTAGTCCACGCCGTAAACGATGAGTGCTAGG TGTTAGGCCCTTTCCGGGGCTTAGTGCCGCAGCTAACGCATTAAGCACTCCGCCTGGGGAGTACGACCGCAAGGTT GAAACTCAAAGGAATTGACGGGGGCCCGCACAAGCGGTGGAGCATGTGGTTTAATTCGAAGCAACGCGAAGAACCT TACCAGGTCTTGACATCCCGATGCCCGCTCTAGAGATAGAGTTTTACTTTTGTACATCGGTGACAGGTGGTGCATG GTTGTCGTCAGCTCGTGTCGTGAGATGTTGGGTTAAGTCCCGCAACGAGCGCAACCCCTATTGTTAGTTGCCATCA TTGAGTTGGGCACTCTAGCGAGACTGCCGGTAATAAACCGGAGGAAGGTGGGGATGACGTCAAATCATCATGCCCC TTATGACCTGGGCTACACACGTGCTACAATGGCTGGTACAACGAGTCGCAAGCCGGTGACGGCAAGCTAATCTCTT AAAGCCAGTCTCAGTTCGGATTGTAGGCTGCAACTCGCCTACATGAAGTCGGAATCGCTAGTAATCGCGGATCAGC ACGCCGCGGTGAATACGTTCCCGGGCCTTGTACACACCGCCCGTCACACCACGAGAGTTTGTAACACCCGAAGTCG GTGAGGTAACCTTTTAGGAGCCAGCCGCCTAAGGTGGGATAGATGATTGGGGTGAAGTCGTAACAAGGTAGCCGTA TCGGAAGGTGCGGCTG |
| HK557089.3.1395 |
| AGACTTTAGCTTGCTAAAGTTGGAAGAGTTGCGAACGGGTGAGTAACGCGTAGGTAACCTGCCTACTAGCGGGGGA TAACTATTGGAAACGATAGCTAATACCGCATAACAGCATTTAACCCATGTTAGATGCTTGAAAGGAGCAATTGCTT CACTAGTAGATGGACCTGCGTTGTATTAGCTAGTTGGTGAGGTAACGGCTCACCAAGGCGACGATACATAGCCGAC CTGAGAGGGTGATCGGCCACACTGGGACTGAGACACGGCCCAGACTCCTACGGGAGGCAGCAGTAGGGAATCTTCG GCAATGGGGGCAACCCTGACCGAGCAACGCCGCGTGAGTGAAGAAGGTTTTCGGATCGTAAAGCTCTGTTGTAAGA GAAGAACGTGTGTGAGAGTGGAAAGTTCACACAGTGACGGTAACTTACCAGAAAGGGACGGCTAACTACGTGCCAG CAGCCGCGGTAATACGTAGGTCCCGAGCGTTGTCCGGATTTATTGGGCGTAAAGCGAGCGCAGGCGGTTTAATAAG TCTGAAGTTAAAGGCAGTGGCTTAACCATTGTTCGCTTTGGAAACTGTTAGACTTGAGTGCAGAAGGGGAGAGTGG AATTCCATGTGTAGCGGTGAAATGCGTAGATATATGGAGGAACACCGGTGGCGAAAGCGGCTCTCTGGTCTGTAAC TGACGCTGAGGCTCGAAAGCGTGGGGAGCAAACAGGATTAGATACCCTGGTAGTCCACGCCGTAAACGATGAGTGC TAGGTGTTAGGCCCTTTCCGGGGCTTAGTGCCGCAGCTAACGCATTAAGCACTCCGCCTGGGGAGTACGACCGCAA GGTTGAAACTCAAAGGAATTGACGGGGGCCCGCACAAGCGGTGGAGCATGTGGTTTAATTCGAAGCAACGCGAAGA ACCTTACCAGGTCTTGACATCCCGATGCTATTCCTAGAGATAGGAAGTTTCTTCGGAACTGTGAGACTTGAGGGCA GAAGGGTAGAGTGCACTTGTATGGGGAGCTGTGGAATGCGTTCCCGCAACGAGCGCAACCCCTATTGTTAGTTGCC ATCATTAAGTTGGGCACTCTAGCGAGACTGCCGGTAATAAACCGGAGGAAGGTGGGGATGACGTCAAATCATCATG CCCCTTATGACCTGGGCTACACACGTGCTACAATGGTTGGTACAACGAGTCGCGAGTCGGTGACGGCAAGCAAATC TCTTAAAGCCAATCTCAGTTCGGATTGTAGGCTGCAACTCGCCTACATGAAGTCGGAATCGCTAGTAATCGCGGAT CAGCACGCCGCGGTGAATACGTTCCCGGGCCTTGTACACACCGCCCGTCACACCACGAGAGTTTGTAACACCCGAA GTCGGTGAGGTANCCTTTTAGGAGC |
| EU358719.1.1513 |
| AGAGTTTGATCCTGGCTCAGGACGAACGCTGGCGGCGTGCCTAATACATGCAAGTAGAACGCTGAAGAAAGGAGCT TGCTTCTTTTGGATGAGTTGCGAACGGGTGAGTAACGCGTAGGTAACCTGCCTTGTAGCGGGGGATAACTATTGGA AACGATAGCTAATACCGCATAACAGCTTTTGACACATGTTAGAAGCTTGAAAGATGCAATTGCATCACTACGAGAT GGACCTGCGTTGTATTAGCTAGTAGGTAGGGTAACGGCCTACCTAGGCGACGATACATAGCCGACCTGAGAGGGTG ATCGGCCACACTGGGACTGAGACACGGCCCAGACTCCTACGGGAGGCAGCAGTAGGGAATCTTCGGCAATGGGGGC AACCCTGACCGAGCAACGCCGCGTGAGTGAAGAAGGTTTTCGGATCGTAAAGCTCTGTTGTAAGAGAAGAACGTGT GTGAGAGTGGAAAGTTCACACAGTGACGGTAACTTACCAGAAAGGGACGGCTAACTACGTGCCAGCAGCCGCGGTA ATACGTAGGTCCCGAGCGTTGTCCGGATTTATTGGGCGTAAAGCGAGCGCAGGCGGTTTAATAAGTCTGAAGTTAA AGGCAGTGGCTTAACCATTGTTCGCTTTGGAAACTGTTAAACTTGAGTGCAGAAGGGGAGAGTGGAATTCCATGTG TAGCGGTGAAATGCGTAGATATATGGAGGAACACCGGTGGCGAAAGCGGCTCTCTGGTCTGTAACTGACGCTGAGG CTCGAAAGCGTGGGGAGCAAACAGGATTAGATACCCTGGTAGTCCACGCCGTAAACGATGAGTGCTAGGTGTTAGG CCCTTTCCGGGGCTTAGTGCCGCAGCTAACGCATTAAGCACTCCGCCTGGGGAGTACGACCGCAAGGTTGAAACTC AAAGGAATTGACGGGGGCCCGCACAAGCGGTGGAGCATGTGGTTTAATTCGAAGCAACGCGAAGAACCTTACCAGG TCTTGACATCCCAGTGACCGCTCTAGAGATAGAGTTTTCTTCGGAACACTGGTGACAGGTGGTGCATGGTTGTCG TCAGCTCGTGTCGTGAGATGTTGGGTTAAGTCCCGCAACGAGCGCAACCCTTATTGTTAGTTGCCATCATTCAGTT GGGCACTCTAGCGAGACTGCCGGTAATAAACCGGAGGAAGGTGGGGATGACGTCAAATCATCATGCCCCTTATGAC CTGGGCTACACACGTGCTACAATGGTTGGTACAACGAGTCGCAAGTCGGTGACGGCAAGCAAATCTCTTAAAGCCA ATCTCAGTTCGGATTGTAGGCTGCAACTCGCCTACATGAAGCAGGAATTGCTAGTAATGGCAGGTCAGCATACTGC CGTGAATACGTTCCCGGGCCTTGTACACACCGCCCGTCACACCATGAGAGTCTGTAACACCCGAAGTCGGTAGTCT AACTACGGAGGACGCCGCCGAAGGTGGGACAGATAATTGGGGTGAAGTCGTAACAAGGTAGCCGTA |
| HQ748204.1.1442 |
| CTAATACATGCGAGGAGAACGCTGAAGACTTTCTTTTGCTATAGTTGGAGAGTTGCTAACGGGTGAGTAACGCGT AGGTGACCTGCCTACTAGCGGGGGATAACTATTGCAAACGATAGCTAATACCGCATAACAGCCTTTAACCCATGTT AGATGCTTGAAAGGAGCAATTGCTTCACTAGTAGATGGACCTGCGTTGTATTAGCTAGTTGGTGAGGTAACGGCTC ACCAAGGCGACGATACATAGCCGACCTGAGAGGGTGATCGGCCACACTGGGACTGAGACACGGCCCATACTCCTAC GGGAGGCACCAGTAGGGAATCTTCGGGAATGGGGGCAACCCTGACCGAGCAACGCCGCGTGAGTGAAGAAGGTTTT |
| CGGATCGTAAAGCTCTGTTGTAAGAGAAGAACGTGTGTGAGAGTGGAAAGTTCACACTGTGACGGTAACTTACCAG AAAGGGACGGCTAACTACGTGCCAGCAGCCGCGGTAATACGTAGGTCCCGAGCGTTGTCCGGATTTATTGGGCGTA AAGCGAGCGCAGGCGGTTTAATAAGTCTGAAGTTAAAGGCAGTGGCTTAACCATTGTTCGCTTTGGAAACTGTTAG ACTTGAGTGCATAAGGGGAGAGTGGAATTCCATGTGTAGCGGTGAAATGCGTAGATATATGGAGGAACACCGGTGG CGAAAGCGGCTCTCTGGTCTGTAACTGACGTTGAGGCTCGAAAGCGTGGGGAGCAAACAGGATTAGATACCCTGGT AGTCCACGCTGTAAACGATGAGTGGTAGGTGTTAGGCCCTTTCTGGGGTTTAGTGCCGCAGATTACGCATTAAGCC ATTCGCCTGGGGAGTACGACCGCAAGGTTGAAACTTAAAGGAATTGACGGGGGCCCGCACAAGCGGTGGAGCATGT GGTTTAATTAGAAGCAACGCGAAGAACCTTACCAGGTCTTGACATCCCGATGCTATTCTTAGAGATAGGAAGTTTC TTCGGAACATCGGTGACAGGTGGTGCATGGTTGTCGTCAGCTCGTGTCGAGAGATGTTGGGTTAAGTCCCTCAACG AGCGCAACCCCTATTTTTATTTGCCATCATTAAGTTGGGCAATCTAGCGAGACTGCCGGTAATAAACCGGAGGAAG GTGGGGATGACGTCAAATCATCATGCTCCTTATGTCATGGGGTACACACGTGGTACAATGGTTGGTACAACGAGTC GCGAGTTGGTGAAGGCAAGCAAATCTCTTAAAGCCAATATCAGTTCGGATTGTAGGCTGCAAATAGCCTACATGTA GTCGGAATTGTTAGTAATCGGGGATCAGCACTCCGCGGTGAATACGTTTCCGGGCCTTGTACACCCCGCCCGTCTA CACCACGAGAGTTTGTAACACCCGAAGTCGGTGAGGTAACTCTTTAGGAGCCAGCCGCCTAAGGTGGGATAGA |
| GQ338727.1.1397 |
| CTACCTGCAGTCGACGAACACCTTATTTGATTTTCTTCGGAACTGAAGATTTGGTGATTGAGTGGCGGACGGGTGA GTAACGCGTGGGTAACCTGCCCTGTACAGGGGGATAACAGTCAGAAATGACTGCTAATACCGCATAAGACCACAGC ACCGCATGGTGCAGGGGTAAAAACTCCGGTGGTACAGGATGGACCCGCGTCTGATTAGCTGGTTGGTGAGGTAACG GCTCACCAAGGCGACGATCAGTAGCCGGCTTGAGAAAGTGAACGGCCACATTGGGACTGAGACACGGCCCAAACTC CTACGGGAGGCAGCAGTGGGGAATATTGCACAATGGGGGAAACCCTGATGCAGCGACGCCGCGTGAGTGAAGAAGT ATCTCGGTATGTAAAGCTCTATCAGCAGGGAAGAAAATGACGGTACCTGACTAAGAAGCCCCGGCTAACTACGTGC CAGCAGCCGCGGTAATACGTAGGGGGCAAGCGTTATCCGGAATTACTGGGTGTAAAGGGTGCGTAGGTGGTATGGC AAGTCAGAAGTGAAAACCCAGGGCTTAACTCTGGGACTGCTTTTGAAACTGTCAGACTGGAGTGCAGGAGAGGTAA GCGGAATTCCTAGTGTAGCGGTGAAATGCGTAGATATTAGGAGGAACATCAGTGGCGAAGGCGGCTTACTGGACTG AAACTGACACTGAGGCACGAAAGCGTGGGGAGCAAACAGGATTAGATACCCTGGTAGTCCACGCCGTAAACGATGA ATACTAGGTGTCGGGGCCGTAGAGGCTTCGGTGCCGCAGCCAACGCAGTAAGTATTCCACCTGGGGAGTACGTTCG CAAGAATGAAACTCAAAGGAATTGACGGGGACCCGCACAAGTAGCGGAGCATGTGGTTTAATTCGAAGCAACGCGA AGAACCTTACCTAAGCTTGACATCCTTTTGACCGATGCCTAATCGCATCTTTCCCTTCGGGGACAGAAGTGACAGG TGGTGCATGGTTGTCGTCAGCTCGTGTCGTGAGATGTTGGGTTAAGTCCCGCAACGAGCGCAACCCTTGCCTTTAG TTGCCATCATTAAGTTGGGCACTCTAGAGGGACTGCCAGGGATAACCTGGAGGAAGGTGGGGATGACGTCAAATCA TCATGCCCCTTATGCTTAGGGCTACACACGTGCTACAATGGGTGGTACAGAGGGCAGCGAAGTCGTGAGGCCAAGC TAATCCCTTAAAGCCATTCTCAGTTCGGATTGTAGGCTGAAACCCGCCTACATGAAGCTGGAGTTACTAGTAATCG CAGATCAGAATGCTGCGGTGAATGCGTTCCCGGGTCTTGTACACACCGCCCGTCACACCATGGGAGTTGGGGGCGC CCGAAGCCGGCTAGCTACTTTGGAAGCGT |
| HQ803964.1.1435 |
| GGGGGGCTTAACACATGCAAGTCGAACGAAGCGCTTTCGCTTTAATCTTCGGAGGAAAGAGGAAGTGACTGAGTGG CGGACGGGTGAGTAACGCGTGGGTAACCTGCCTCATACAGGGGGATAACAGTTAGAAATGGCTGCTAATACCGCAT AAGCATACAGCACCGCATGGTGCAGTGTGAAAAACTCCGGTGGTATAAGATGGACCCGCGTCTGATTAGGTAGTTG GTGGGGTAACGGCCTACCAAGCCGACGATCAGTAGCCGACCTGAGAGGGTGACCGGCCACATTGGGACTGAGACAC GGCCCAAACTCCTACGGGAGGCAGCAGTGGGGAATATTGCACAATGGGGGAAACCCTGATGCAGCGACGCCGCGTG AAGGAAGAAGTATTTCGGTATGTAAACTTCTATCAGCAGGGAAGAAAATGACGGTACCTGACTAAGAAGCCCCGGC TAACTACGTGCCAGCAGCCGCGGTAATACGTAGGGGGCAAGCGTTATCCGGATTTACTGGGTGTAAAGGGAGCGTA GACGGAAGTGCAAGTCTGAAGTGAAAGCCCGGGGCTCAACCCCGTGACTGCTTTGGAAACTGTGCTTCTAGAGTGT CGGAGAGGTAAGCGGAATTCCTAGTGTAGCGGTGAAATGCGTAGATATTAGGAGGAACATCAGTGGCGAAGGCGGC TTACTGGGCGATAACTGACGTTGAGGCTCGAAAGCGTGGGGAGCAAACAGGATTAGATACCCTGGTAGTCCACGCC GTAAACGATGAATACTAGGTGTCGGGAAGCACAGCTTTTCGGTGCCGCCGCAAACGCATTAAGTATTCCACCTGGG GAGTACGTTCGCAAGAATGAAACTCAAAGGAATTGACGGGGACCCGCACAAGCGGTGGAGCATGTGGTTTAATTCG AAGCAACGCGAAGAACCTTACCAAGTCTTGACATCCCGGTGACCGGACAGTAATGTGTCCTTTTCTTCGGAACACC GGTGACAGGTGGTGCATGGTTGTCGTCAGCTCGTGTCGTGAGATGTTGGGTTAAGTCCCGCAACGAGCGCAACCCT TATCCCCAGTAGCCAGCGGTTCGGCCGGGCACTCTGAGGAGACTGCCAGGGATAACCTGGAGGAAGGTGGGGATGA CGTCAAATCATCATGCCCCTTATGACTTGGGCTACACACGTGCTACAATGGCGTAAACAAAGGAAGCGAGACCGT GAGGTGGAGCAAATCCCAAAAATAACGTCTCAGTTCGGACTGTAGTCTGCAACCCGACTACACGAAGCTGGAATCG CTAGTAATCGCAGATCAGAATGCTGCGGTGAATACGTTCCCGGGTCTTGTACACACCGCCCGTCACACCATGGGAG TTGGAAATGCCCGAAGTCAGTGACCCAACCGCAAGGAGGGAGCTGCCGAAGGCAGGTTCGATAACTG |
| FJ951866.1.1493 |
| AGAGTTTGATCCTGGCTCAGGATGAACGCTGGCGGCGTGCTTAACACATGCAAGTCGAACGAAGCACTTTAACTTG ATTTTTCGGAATGATTGTTCTTGTGACTGAGTGGCGGACGGGTGAGTAACGCGTGGGTAACCTGCCTCATACAGG GGGATAACAGTTAGAAATGACTGCTAATACCGCATAAGCGCACGGTATCGCATGATACAGTGTGAAAAACTCCGGT GGTATGAGATGGACCCGCGTCTGATTAGCTAGTTGGCGGGGTAACGGCCCACCAAGGCGACGATCAGTAGCCGACC TGAGAGGGTGACCGGCCACATTGGGACTGAGACACGGCCCAAACTCCTACGGGAGGCAGCAGTGGGGAATATTGCA CAATGGGCGAAAGCCTGATGCAGCGACGCCGCGTGAACGAAAAAGTATTTCGGTATGTAAAGTTCTATCAGCAGGG AAGATAATGACGGTACCTGACTAAGAAGCACCGGCTAAATACGTGCCAGCAGCCGCGGTAATACGTATGGTGCAAG |
| CGTTATCCGGATTTACTGGGTGTAAAGGGAGCGCAGGCGGTACGGCAAGTCTGATGTGAAAGCCCGGGGCTCAACC CCGGTACTGCATTGGAAACTGTCGAACTAGAGTGTCGGAGGGGTAAGCGGAATTCCTAGTGTAGCGGTGAAATGCG TAGATATTAGGAGGAACACCAGTGGCGAAGGCGGCTTACTGGACGACAACTGACGCTGAGGCGCGAAAGCGTGGGG AGCAAACAGGATTAGATACCCTGGTAGTCCACGCTGTAAACGATGAATACTAGGTGTGGGAGGACTGACCCCTTCC GTGCCGCAGTTAACACAATAAGTATTCCACCTGGGGAGTACGGCCGCAAGGCTGAAACTCAAAGGAATTGACGGGG GCCCGCACAAGCAGTGGATTATGTGGTTTAATTCGACGCAACGCGAAGAACCTTACCAGGACTTGACATCCAACTA ACGAAGTAGAGATACATTAGGTGCCCTTCGGGGAAAGTTGAGACAGGTGGTGCATGGTTGTCGTCAGCTCGTGTCG TGAGATGTTGGGTTAAGTCCCGCAACGAGCGCAACCCCTATTGTTAGTTGCTACGCAAGAGCACTCTAGCGAGACT GCCGTTGACAAAACGGAGGAAGGCGGGGACGACGTCAAATCATCATGCCCCTTATGTCCTGGGCTACACACGTAAT ACAATGGCCGTCAACAAAGGGAAGCAAAGCCGCGAGGTGGAGCAAATCCCCAAAAACGGTCTCAGTTCGGATTGCA GGCTGCAACTCGCCTGCATGAAGCTGGAATTGCTAGTAATCGTGGATCAGCATGCCACGGTGAATACGTTCCCGGG CCTTGTACACACCGCCCGTCACACCATGAGAGTCGGGAACACCCGAAGTCCGTAGTCTAACCGCAAGGAGGGCGCG GCCGAAGGTGGGTCCGGTAATTGGGGTGAAGTCGTAACAAGGTAACCGT |
| EU772870.1.1289 |
| AGTGGCGAACGGGTGAGTAACGCGTGAGGAACCTGCCTTTCAGTGGGGGACAACAGTTGGAAACGACTGCTAATAC CGCATGATACTTTTTGGAGGCATCTCTGAAAAGTCAAAGCTTTATGTGCTGAAAGATGGTCTCGCGTCTGATTAGC TAGTTGGTGAGGTAACGGCTCACCAAGGCGACGATCAGTAGCCGGTCTGAGAGGATGAACGGCCACATTGGGACTG AGATACGGCCCAGACTCCTACGGGAGGCAGCAGTGGGGAATATTGGCAATGGGGGAAACCCTGACCCAGCAACGC CGCGTGAAGGAAGAAGGCCTTCGGGTTGTAAACTTCTTTTACCAGGGACGAAGAACGTGACGGTACCTGGAGAAAA AGCAACGGCTAACTACGTGCCAGCAGCCGCGGTAATACGTAGGTTGCAAGCGTTATCCGGATTTATTGGGCGTAAA GCGCGTCTAGGCGGTTTGGTAAGTCTGATGTGAAAATGCGGGGCTCAACTCCGTATTGCGTTGGAAACTGCTAAAC TAGAGTACTGGAGAGGTAGGCGGAACTACAAGTGTAGAGGTGAAATTCGTAGATATTTGTAGGAATGCCGATGGGG AAGCCAGCCTACTGGACAGATACTGACGCTAAAGCGCGAAAGCGTGGGGAGCAAACAGGATTAGATACCCTGGTAG TCCACGCTGTAAACGATGAGTACTAGGTGTCGGAGGTTACCCCCTTCGGTGCCGCAGCTAACGCATTAAGTACTCC GCCTGGGGAGTACGCACGCAAGTGTGGAACTCAAAGGAATTGACGGGGACCCGCACAAGTAGCGGAGCATGTGGTT TAATTCGAAGCAACGCGAAGAACCTTACCTAGGCTTGACATCCTTCTGACCGAGGACTAATCTCCTCTTTCCCTCC GGGGACAGAAGTGACAGGTGGTGCATGGTTGTCGTCAGCTCGTGTCGTGAGATGTTGGGTTAAGTCCCGCAACGAG CGCAACCCTTGTCTTTAGTTGCCATCATTTAGTTGGGCACTCTGGAGAGACTGCCAGGGATAACCTGGAGGAAGGT GGGGATGACGTCAAATCATCATGCCCCTTATGCCTAGGGCTACACACGTGCTACAATGGGTGGTACAGAGGGCAGC TAAGCCGTGAGGTGGAGCAAATCCCTTAAAGCCATTCTCAGTTCGGATTGTAGGCTGAAACTCGCCTACATGAAGC TGGAGTTACTAGTAATCGCAGATCAGAATGCTGCGGTGAATGCGTTCCCGGGTCTTGTACACACCGCCCGTCA |
| GQ448468.1.1366 |
| AGAGTTTGATCCTGGCTCAGGATGAACGCTGACAGAATGCTTAACACATGCAAGTATACTTGATCCTTCGGGTGAT GGTGGCGGACGGGTGAGTAACGCGTAAAGAACTTGCCCTGCAGTCTGGGACAACATTTGGAAACGAATGCTAATCC CGCATAAGCCCACAGCTCGGCATCGAGCAGAGGGAAAAGGAGTGATCTGCTTTGAGATGGCCTCGCGTCCGATTAG CTGGTTGGTGAGGTGACGGCCCATCAAGGCAACGATCGGTAGCCGGACTGAGAGGTTGAACGGCCACATTGGGATT GAGACACGGCCCTTACTCCTACGGGAGGCAGCAGTGGGGAATATTGGACAATGGACCAAAAGTCTGATCCAGCAAT TCTGTGTGCACGATGAAGTTTTTCGGAATGTAAAGTGCTTTCAGTTGGGACGAAGTAAGTGACGGTACCAACAGAA GAAGCGACGGCTAAATACGTGCCAGCAGCCGCGGTAATACGTATGTCGCAAGCGTTATCCGGATTTATTGGGCGTA AAGCGCGTCTAGGCGGTTTGGTAAGTCTGATGTGAAAATGCGGGGCTCAACTCCGTATTGCGTTGGAAACTGCCAA ACTAGAGTACTGGAGAGGTGGGCGGAACTACAAGTGTAGAGGTGAAATTCGTAGATATTTGTAGGAATGCCGATGG GGAAGCCAGCCCACTGGACAGATACTGACGCTAAAGCGCGAAAGCGTGGGTAGCAAACAGGATTAGATACCCTGGT AGTCCACGCCGTAAACGATGATTACTAGGTGTTGGGGGTCGAACCTCAGCGCCCAAGCTAACGCGATAAGTAATCC GCCTGGGGAGTACGTACGCAAGTATGAAACTCAAAGGAATTGACGGGGACCCGCACAAGCGGTGGAGCATGTGGTT TAATTCGACGCAACGCGAGGAACCTTACCAGCGTTTGACATCCTAAGAAATTAGCAGAGATGCTTTTGTGCCCCTT CGGGGGAACTTAGTGACAGGTGGTGCATGGCTGTCGTCAGCTCGTGTCGTGAGATGTTGGGTTAAGTCCCGCAACG AGCGCAACCCCTTTCGTATGTTGCCATCATTAAGTTGGGCACTCATGCGATACTGCCTGCGATGAGCAGGAGGAAG GTGGGGATGACGTCAAGTCATCATGCCCCTTATACGCTGGGCTACACACGTGCTACAATGGGTAGTACAGAGAGTC GCAAACCTGCGAGGGGGAGCTAATCTCAGAAAACTATTCTCAGTTCGGATTGTACTCTGCAACTCGAGTACATGAA GTTGGAATCGCTAGTAATCGCAAATCAGCTATGTTGCGGTGAATACGTTCTCGGGTCTTGTACACACCGCCCGT |
| EU774020.1.1361 |
| AGAGTTTGATCCTGGCTCAGGACGAACGCTGGCGGCGTGCCTAACACATGCAAGTCGAGCGATTCTCTTCGGAGAA GAGCGGCGGACGGGTGAGTAACGCGTGGGTAACCTGCCCTGTACACACGGATAACATACCGAAAGGTATGCTAATA CGGGATAATATATAAGAGTCGCATGACTTTTATATCAAAGATTTTTCGGTACAGGATGGACCCGCGTCTGATTAGC TTGTTGGCGGGGTAACGGCCCACCAAGGCGACGATCAGTAGCCGACCTGAGAGGGTGATCGGCCACATTGGAACTG AGACACGGTCCAAACTCCTACGGGAGGCAGCAGTGGGGAATATTGCACAATGGGCGCAAGCCTGATGCAGCAACGC CGCGTGAGCGATGAAGGCCTTCGGGTCGTAAAGCTCTGTCCTCAAGGAAGATAATGACGGTACTTGAGGAGGAAGC CCCGGCTAACTACATGCCAGCAGCCGCGGTAATACGTATGTCGCAAGCGTTATCCGGATTTATTGGGCGTAAAGCG CGTCTAGGTGGTTTGGTAAGTCTGATGTGAAAATGCGGGGCTCAACTCCGTATTGCGTTGGAAACTGCCAAACTAG AGTACTGGAGAGGTAGGCGGAACTACAAGTGTAGAGGTGAAATTCGTAGATATTTGTAGGAATGCCGATGGGGAAG CCAGCCTACTGGACAGATACTGACGCTAAAGCGCGAAAGCGTGGGTAGCAAACAGGATTAGATACCCTGGTAGTCC ACGCCGTAAACGATGATTACTAGGTGTTGGGGGTCGAACCTCAGCGCCCAAGCTAACGCGATAAGTAATCCGCCTG |
| GGGAGTACGTACGCAAGTATGAAACTCAAAGGAGTTGACGGGGACCCGCACAAGCGGTGGAGCATGTGGTTTAATT CGACGCAACGCGAGGAACCTTACCAGCGTTTGACATCCTAGGAATGAGAAAGAGATTTCTTAGTGCTCCTTCGGGA GAACCTAGAGACAGGTGGTGCATGGCTGTCGTCAGCTCGTGTCGTGAGATGTTGGGTTAAGTCCCGCAACGAGCGC AACCCCTATTGTATGTTGCCATCATTAAGTTGGGCACTCATGCGATACTGCCTGCGATGAGCAGGAGGAAGGTGGG GATGACGTCAAGTCATCATGCCCCTTATACGCTGGGCTACACACGTGCTACAATGGGCAGTACAGAGAGAAGCAAT ACCGCGAGGTGGAGCCAAACTTAAAAACCAGTCTCAGTTCGGATTGTAGGCTGAAACTCGCCTACATGAAGCTGGA GTTACTAGTAATCGCGAATCAGAATGTCGCGGTGAATACGTACCCGGGTCTTGTACACACCGCCCGTCA |
| HQ782658.1.1415 |
| AATGCTTAACACATGCAAGTCTACTTGATCCTTCGGGTGATGGTGGCGGACGGGTGAGTAACGCGTAAAGAACTTG CCTTGCAGTCTGGGACAACGTCTGGAAACGGACGCTAATACCGGATATTATGCGAGAGTCGCATGGCTCTTTCATG AAAGCTATATGCGCTGCAGGAGAGCTTTGCGTCCCATTAGTTAGTTGGTGAGGTAACGGCTCACCAAGACCGCGAT GGGTAGCCGGCCTGAGAGGGTGAACGGCCACAAGGGGACTGAGACACGGCCCTTACTCCTACGGGAGGCAGCAGTG GGGAATATTGGACAATGGACCAAAAGTCTGATCCAGCAATTCTGTGTGCACGATGACGGTCTTAGGATTGTAAAGT GCTTTCAATCGGGAAAAAGAAAGTGATGGTACCGATAGAAGAAGCGACGGCTAAATACGTGCCAGCAGCCGCGGTA ATACGTATGTCGCAAGCGTTATCCGGATTTATTGGGCGTAAAGCGCGTCTAGGCGGTCTGGTAAGTCTGATGTGGA AATGCGGGGCTCAACTCCGTATTGCGTTGGAAACTGCCAGACTAGAGTACTGGAGAGGTGGGCGGAACTACAAGTG TAGAGGTGAAATTCGTAGATATTTGTAGGAATGCCGATAGAGAAGTCAGCTCACTGGACAGATACTGACGCTGAAG CGCGAAAGCATGGGGAGCAAACAGGATTAGATACCCTGGTAGTCCATGCCGTAAACGATGATTACTAAGCGTCGGG GGTCGAACCTCGGCACTCAAGCTAACGCGATAAGTAATCCGCCTGGGGAGTACGTACGCAAGTATGAAACTCAAAG GAATTGACGGGGACCCGCACAAGTGGTGGAGCATGTGGTTTAATTGGACGCAACGCGAGGAACTTTACCAGCGTGT GACATCCTAGGAATGAGAAAGAGATTTTCAGTGCTCCTTCGGGAGAACCCAGAGACAGGTGGTGCATGGCTGTGG TCAGCTCGTGTCGTGAGATGTTGGGTTAAGTCCCGCAACGAGCGCAACCCCTATTGTATGTTGCCATCATTAAGTT GGGCAATCATGCGATGCTGCCTGCGACGAGCAGGAGGAAGGTGGGGATGAGGTCAAGTCATCATGCCCGTTATATG CTGGGCTACACACGTGCTACAATGGGCAGTACAGAGAGAAGCAAATATGCGAGGAGGAGCAAATGTCAGAAAGCTG TTCGTAGTTCGGATTGTACTCTGCAACTGGAGTACATGAAGTTGGAATCAGTAGTAATCGCAAATCAGCAATGTTG CGGTGAATACGTTCTCGGGTCTGGTACACACCGCCCGTCACACCACGAGAGTTGATTGCACCTGAAGTAGCAGGCC TAACCGTAAGGAAGGGTGGTCCGAGGGTGTGGTTAGCGATTGGGGTG |
| DQ794633.1.1395 |
| AGAGTTTGATCCTGGCTCAGGATGAACGCTGGCGGCGTGCTTAACACATGCAAGTCGAGCGAAGCGCTTTACGGA TTTCTTCGGATTGAAGTGATTGTGACTGAGCGGCGGACGGGTGAGTAACGCGTGGGTAACCTGCCTCATACAGGGG GATAACAGTTAGAAATGACTGCTAATACCGCATAAGCGCACAGTACCGCATGGTACGGTGTGAAAAACTCCGGTG GTATGAGATGGACCCGCGTCTGATTAGGTAGTTGGTGGGGTAACGGCCTACCAAGCCAACGATCAGTAGCCGACCT GAGAGGGCGACCGGCCACATTGGGACTGAGACACGGCCCAAACTCCTACGGGAGGCAGCAGTGGGGAATATTGCAC AATGGGGGAAACCCTGATGCAGCGACGCCGCGTGAAGGATGAAGTATTTCGGTATGTAAACTTCTATCAGCAGGGA AGAAAATAACGGTACCTGAGTAAGAAGCCCCGGCTAACTACGTGCCAGCAGCCGCGGTAATACGTAGGGGGCAAGC GTTATCCGGATTTACTGGGTGTAAAGGGAGCGTAGACGGAAGTGCAAGTCTGATGTGAAAACCCGAGGCTCAACCA CGGGACTGCATTGGAAACTGTGCTTCTAGAGTGCCGGAGAGGTAAGCGGAATTCCTAGTGTAGCGGTGAAATGCGT AGATATTAGGAGGAACACCAGTGGCGAAGGCGGCTTACTGGACGGTAACTGACGTTGAGGCTCGAAAGCGTGGGGA GCAAACAGGATTAGATACCCTGGTAGTCCACGCCGTAAACGATGACTTACTAGGGTGTCGGGCAGCAAAGCTGTTC GGTTGCCGCAGCCATCGCAATAAGTAGTCCACCTGGGGGAGTACGTTCGCAAGAATGAAACTCAAAGGAATTGACG GGGACCCGCACAAGCGGTGGAGCATGTGGTTTAATTCGAAGCAACGCGAAGAACCTTACCTGCTCTTGACATCCCT CTGACCGGCAAGTAATGTTGCCTTTCCTTCGGGACAGAGGAGACAGGTGGTGCATGGTTGTCGTCAGCTCGTGTCG TGAGATGTTGGGTTAAGTCCCGCAACGAGCGCAACCCCTATCTTCAGTAGCCAGCATTTAAGGTGGGCACTCAGGA GAGACTGCCAGGGATAACCTGGAGGAAGGTGGGGATGACGTCAAATCATCATGCCCCTTATGAGCAGGGCTACACA CGTGCTACAATGGCGTAAACAAAGGGAAGCGAAAGGGTGACCTGGAGCAAATCTCAGAAATAACGTCTCAGTTCGG ATTGTAGTCTGCAACTCGACTACATGAAGCTGGAATCGCTAGTAATCGCGAATCAGCATGTCGCGGTGAATACGTT CCCGGGTCTTGTACTCACCGCCCGTCA |
| FN668375.4306350.4307737 |
| AGAGTTTGATCCTGGCTCAGGATGAACGCTGGCGGCGTGCCTAACACATGCAAGTTGAGCGATTTACTTCGGTAAA GAGCGGCGGACGGGTGAGTAACGCGTGGGTAACCTACCCTGTACACACGGATAACATACCGAAAGGTATGCTAATA CGGGATAATATATTTGAGAGGCATCTCTTGAATATCAAAGGTGAGCCAGTACAGGATGGACCCGCGTCTGATTAGC TAGTTGGTAAGGTAACGGCTTACCAAGGCGACGATCAGTAGCCGACCTGAGAGGGTGATCGGCCACATTGGAACTG AGACACGGTCCAAACTCCTACGGGAGGCAGCAGTGGGGAATATTGCACAATGGGCGAAAGCCTGATGCAGCAACGC CGCGTGAGTGATGAAGGCCTTCGGGTCGTAAAACTCTGTCCTCAAGGAAGATAATGACGGTACTTGAGGAGGAAGC CCCGGCTAACTACGTGCCAGCAGCCGCGGTAATACGTAGGGGGCTAGCGTTATCCGGATTTACTGGGCGTAAAGGG TGCGTAGGCGGTCTTTCAAGTCAGGAGTGAAAGGCTACGGCTCAACCGTAGTAAGCTCTTGAAACTGGGAGACTTG AGTGCAGGAGAGGAGAGTGGAATTCCTAGTGTAGCGGTGAAATGCGTAGATATTAGGAGGAACACCAGTTGCGAAG GCGGCTCTCTGGACTGTAACTGACGCTGAGGCACGAAAGCGTGGGGAGCAAACAGGATTAGATACCCTGGTAGTCC ACGCTGTAAACGATGAGTACTAGGTGTCGGGGGTTACCCCCTTCGGTGCCGCAGCTAACGCATTAAGTACTCCGCC TGGGAAGTACGCTCGCAAGAGTGAAACTCAAAGGAATTGACGGGGACCCGCACAAGTAGCGGAGCATGTGGTTTAA TTCGAAGCAACGCGAAGAACCTTACCTAAGCTTGACATCCCAATGACATCTCCTTAATCGGAGAGTTCCCTTCGGG GACATTGGTGACAGGTGGTGCATGGTTGTCGTCAGCTCGTGTCGTGAGATGTTGGGTTAAGTCCCGCAACGAGCGC |
| AACCCTTGTCTTTAGTTGCCATCATTAAGTTGGGCACTCTAGAGAGACTGCCAGGGATAACCTGGAGGAAGGTGGG GATGACGTCAAATCATCATGCCCCTTATGCTTAGGGCTACACACGTGCTGATTATGCTAAGGAAATAGGATTTACT GGACAATTCTTAATAGAGCCTAAGCCAAAAGAGCCTACTAAACATCAATATGATTTTGATACTGCTACTGTTTTAG GATTTTTAAGAAAGTATAATCTGGATAAATACTTCAAAGTGAATATAGAAGCAAACCATGCAACACTTGCAGGACA TACTTTCCAACATGAATTAA |
| GQ867445.1.1457 |
| CGCTGGCGGCGTGCTTAACACATGCAAGTCGAACGAAGCGATTTGGAGGAAGTTTTCGGATGAAATCTGAATTGAC TGAGTGGCGGACGGGTGAGTAACGCGTGGGTAACCTGCCTCACACAGGGGGACAACAGTTAGAAATGGCTGCTAAT ACCGCATAAGCGCACAGCTTCGCATGAAGCAGTGTGAAAAACTCCGGTGGTGTGAGATGGACCCGCGTCTGATTAG GTAGTTGGTGGGGTAACGGCCTACCAAGCCGACGATCAGTAGCCGACCTGAGAGGGTGACCGGCCACATTGGGACT GAGACACGGCCCAAACTCCTACGGGAGGCAGCAGTGGGGAATATTGCACAATGGGGGAAACCCTGATGCAGCGACG CCGCGTGAGTGAAGAAGTATTTCGGTATGTAAAGCTCTATCAGCAGGGAAGAAAATGACGGTACCTGACTAAGAAG CCCCGGCTAACTACGTGCCAGCAGCCGCGGTAATACGTAGGGGGCAAGCGTTATCCGGATTTACTGGGTGTAAAGG GAGCGTAGACGGCTTGGCAAGTCTGAAGTGAAAGCCCGGGGCTCAACCCCGGGACTGCTTTGGAAACTGTCAGGCT AGAGTGCTGGAGAGGTAAGTGGAATTCCTAGTGTAGCGGTGAAATGCATAGATATTAGGAGGAACACCAGTGGCGA AGGCGGCTTACTGGACAGTAACTGACGTTGAGGCTCGAAAGCGTGGGGAGCAAACAGGATTAGATACCCTGGTAGT CCACGCCGTAAACGATGAATACTAGGTGTTGGGGAGCAAAGCTCTTCGGTGCCGTCGCAAACGCAATAAGTATTCC ACCTGGGAAGTACGTTCGCAAGAATGAAACTCAAAGGAATTGACGGGGACCCGCACAAGCGGTGGAGCATGTGGTT TAATTCGAAGCAACGCGAAGAACCTTACCAAGTCTTGACATCCCATTGAAAAGCCCGTAACGGGGTTCCCTCTTCG GAGCAATGGAGACAGGTGGTGCATGGTTGTCGTCAGCTCGTGTCGTGAGATGTTGGGTTAAGTCCCGCAACGAGCG CAACCCTTATCCTAAGTAGCCAGCAGGTAGAGCTGGGCACTCTTGGGAGACTGCCAGGGACAACCTGGAGGAAGGT GGGGATGACGTCAAATCATCATGCCCCTTATGATTTGGGCTACACACGTGCTACAATGGCGTAAACAAAGGAAGC GAAGCTGTGAAGCTAAGCAAATCTCAAAAATAACGTCTCAGTTCGGATTGTAGTCTGCAACTCGACTACATGAAGC TGGAATCGCTAGTAATCGCGGATCAGAATGCCGCGGTGAACACGTTCCCGGGTCTTGTACACACCGCCCGTCACAC CATGGGAGTCAGTAACGCCCGAAGCCAGTGACCTAACCGCAAGGAAGGAGCTGTCGAAGGCGGACCGATAACTGG GGTGAAGTCGTAA |

### References

1. Nitzan, O., Elias, M., Peretz, A. & Saliba, W. Role of antibiotics for treatment of inflammatory bowel disease. World J. Gastroenterol. 22, 1078-1087 (2016).
2. Khan, K. J. et al. Antibiotic therapy in inflammatory bowel disease: a systematic review and meta-analysis. Am. J. Gastroenterol. 106, 661-673 (2011).
3. Knights, D., Lassen, K. G. & Xavier, R. J. Advances in inflammatory bowel disease pathogenesis: linking host genetics and the microbiome. Gut 62, 1505-1510 (2013).
4. Garrett, W. S. et al. Communicable ulcerative colitis induced by T-bet deficiency in the innate immune system. Cell 131, 33-45 (2007).
5. Couturier-Maillard, A. et al. NOD2-mediated dysbiosis predisposes mice to transmissible colitis and colorectal cancer. J. Clin. Invest. 123, 700-711 (2013).
6. Moon, C. et al. Vertically transmitted faecal IgA levels determine extra-chromosomal phenotypic variation. Nature 521, 90-93 (2015).
7. David, L. A. et al. Diet rapidly and reproducibly alters the human gut microbiome. Nature 505, 559-563 (2014).
8. Muegge, B. D. et al. Diet drives convergence in gut microbiome functions across mammalian phylogeny and within humans. Science 332, 970-974 (2011).
9. Lee, D. et al. Diet in the pathogenesis and treatment of inflammatory bowel diseases. Gastroenterology 148, 1087-1106 (2015).
10. Levine, A., Sigall Boneh, R. & Wine, E. Evolving role of diet in the pathogenesis and treatment of inflammatory bowel diseases. Gut 67, 1726-1738 (2018).
11. Chassaing, B. et al. Dietary emulsifiers impact the mouse gut microbiota promoting colitis and metabolic syndrome. Nature 519, 92-96 (2015).
12. Desai, M. S. et al. A Dietary Fiber-Deprived Gut Microbiota Degrades the Colonic Mucus Barrier and Enhances Pathogen Susceptibility. Cell 167, 1339-1353.e21 (2016).
13. Hou, J. K., Abraham, B. & El-Serag, H. Dietary intake and risk of developing inflammatory bowel disease: a systematic review of the literature. Am. J. Gastroenterol. 106, 563-573 (2011).
14. Ruemmele, F. M. et al. Consensus guidelines of ECCO/ESPGHAN on the medical management of pediatric Crohn's disease. J Crohns Colitis 8, 1179-1207 (2014).
15. Cohen-Dolev, N. et al. Differences in outcomes over time with exclusive enteral nutrition compared with steroids in children with mild to moderate crohn's disease: results from the GROWTH CD study. J Crohns Colitis 12, 306-312 (2018).
16. Sigall-Boneh, R. et al. Partial enteral nutrition with a Crohn's disease exclusion diet is effective for induction of remission in children and young adults with Crohn's disease. Inflamm. Bowel Dis. 20, 1353-1360 (2014).
17. Lee, D. et al. Comparative Effectiveness of Nutritional and Biological Therapy in North American Children with Active Crohn's Disease. Inflamm. Bowel Dis. 21, 1786-1793 (2015).
18. Borrelli, O. et al. Polymeric diet alone versus corticosteroids in the treatment of active pediatric Crohn's disease: a randomized controlled open-label trial. Clin. Gastroenterol. Hepatol. 4, 744-753 (2006).
19. Kaakoush, N. O. et al. Effect of exclusive enteral nutrition on the microbiota of children with newly diagnosed Crohn's disease. Clin. Transl. Gastroenterol. 6, e71 (2015).
20. Quince, C. et al. Extensive modulation of the fecal metagenome in children with crohn's disease during exclusive enteral nutrition. Am. J. Gastroenterol. 110, 1718-29; quiz 1730 (2015).
21. Gerasimidis, K. et al. Decline in presumptively protective gut bacterial species and metabolites are paradoxically associated with disease improvement in pediatric Crohn's disease during enteral nutrition. Inflamm. Bowel Dis. 20, 861-871 (2014).
22. Schwerd, T. et al. Exclusive enteral nutrition in active pediatric Crohn disease: Effects on intestinal microbiota and immune regulation. J. Allergy Clin. Immunol. 138, 592-596 (2016).
23. Lewis, J. D. et al. Inflammation, antibiotics, and diet as environmental stressors of the gut microbiome in pediatric crohn's disease. Cell Host Microbe 18, 489-500 (2015).
24. D'Argenio, V. et al. An altered gut microbiome profile in a child affected by crohn's disease normalized after nutritional therapy. Am. J. Gastroenterol. 108, 851-852 (2013).
25. Wu, G. D. et al. Linking long-term dietary patterns with gut microbial enterotypes. Science 334, 105-108 (2011).
26. Gurry, T. et al. Predictability and persistence of prebiotic dietary supplementation in a healthy human cohort. Sci. Rep. 8, 12699 (2018).
27. Wu, G. D. et al. Comparative metabolomics in vegans and omnivores reveal constraints on diet-dependent gut microbiota metabolite production. Gut 65, 63-72 (2016).
28. Obregon-Tito, A. J. et al. Subsistence strategies in traditional societies distinguish gut microbiomes. Nat. Commun. 6, 6505 (2015).
29. Smits, S. A. et al. Seasonal cycling in the gut microbiome of the Hadza hunter-gatherers of Tanzania. Science 357, 802-806 (2017).
30. Cerquetella, M. et al. Inflammatory bowel disease in the dog: differences and similarities with humans. World J. Gastroenterol. 16, 1050-1056 (2010).
31. Jergens, A. E. & Simpson, K. W. Inflammatory bowel disease in veterinary medicine. Front Biosci (Elite Ed) 4, 1404-1419 (2012).
32. Peiravan, A. et al. Genome-wide association studies of inflammatory bowel disease in German shepherd dogs. PLoS ONE 13, e0200685 (2018).
33. Vázquez-Baeza, Y., Hyde, E. R., Suchodolski, J. S. & Knight, R. Dog and human inflammatory bowel disease rely on overlapping yet distinct dysbiosis networks. Nat. Microbiol. 1, 16177 (2016).
34. Suchodolski, J. S., Dowd, S. E., Wilke, V., Steiner, J. M. & Jergens, A. E. 16S rRNA gene pyrosequencing reveals bacterial dysbiosis in the duodenum of dogs with idiopathic inflammatory bowel disease. PLoS ONE 7, e39333 (2012).
35. Simpson, K. W. et al. Adherent and invasive Escherichia coli is associated with granulomatous colitis in boxer dogs. Infect. Immun. 74, 4778-4792 (2006).
36. Allenspach, K., Wieland, B., Gröne, A. & Gaschen, F. Chronic enteropathies in dogs: evaluation of risk factors for negative outcome. J Vet Intern Med 21, 700-8 (2007).
37. Coelho, L. P. et al. Similarity of the dog and human gut microbiomes in gene content and response to diet. Microbiome 6, 72 (2018).
38. Kalenyak, K., Isaiah, A., Heilmann, R. M., Suchodolski, J. S. & Burgener, I. A. Comparison of the intestinal mucosal microbiota in dogs diagnosed with idiopathic inflammatory bowel disease and dogs with food-responsive diarrhea before and after treatment. FEMS Microbiol. Ecol. 94, (2018).
39. Minamoto, Y., Dhanani, N., Markel, M. E., Steiner, J. M. & Suchodolski, J. S. Prevalence of Clostridium perfringens, Clostridium perfringens enterotoxin and dysbiosis in fecal samples of dogs with diarrhea. Vet. Microbiol. 174, 463-473 (2014).
40. Ziese, A.-L. et al. Effect of probiotic treatment on the clinical course, intestinal microbiome, and toxigenic Clostridium perfringens in dogs with acute hemorrhagic diarrhea. PLoS ONE 13, e0204691 (2018).
41. Gevers, D. et al. The treatment-naive microbiome in new-onset Crohn's disease. Cell Host Microbe 15, 382-392 (2014).
42. Islam, K. B. M. S. et al. Bile acid is a host factor that regulates the composition of the cecal microbiota in rats. Gastroenterology 141, 1773-1781 (2011).
43. Wells, J. E. & Hylemon, P. B. Identification and characterization of a bile acid 7alpha-dehydroxylation operon in Clostridium sp. strain TO-931, a highly active 7alpha-dehydroxylating strain isolated from human feces. Appl. Environ. Microbiol. 66, 1107-1113 (2000).
44. Kitahara, M., Takamine, F., Imamura, T. & Benno, Y. Clostridium hiranonis sp. nov., a human intestinal bacterium with bile acid 7alpha-dehydroxylating activity. Int. J. Syst. Evol. Microbiol. 51, 39-44 (2001).
45. Banaszkiewicz, A. et al. Enterotoxigenic Clostridium perfringens infection and pediatric patients with inflammatory bowel disease. J Crohns Colitis 8, 276-281 (2014).
46. Segata, N. et al. Metagenomic microbial community profiling using unique clade-specific marker genes. Nat. Methods 9, 811-814 (2012).
47. Kitahara, M., Takamine, F., Imamura, T. & Benno, Y. Assignment of Eubacterium sp. VPI 12708 and related strains with high bile acid 7alpha-dehydroxylating activity to Clostridium scindens and proposal of Clostridium hylemonae sp. nov., isolated from human faeces. Int. J. Syst. Evol. Microbiol. 50 Pt 3, 971-978 (2000).
48. Zaneveld, J. R., McMinds, R. & Vega Thurber, R. Stress and stability: applying the Anna Karenina principle to animal microbiomes. Nat. Microbiol. 2, 17121 (2017).
49. Nagy-Szakal, D. et al. Monotonous diets protect against acute colitis in mice: epidemiologic and therapeutic implications. J. Pediatr. Gastroenterol. Nutr. 56, 544-550 (2013).
50. Mandigers, P. J. J., Biourge, V., van den Ingh, T. S. G. A. M., Ankringa, N. & German, A. J. A randomized, open-label, positively-controlled field trial of a hydrolyzed protein diet in dogs with chronic small bowel enteropathy. J. Vet. Intern. Med. 24, 1350-1357 (2010).
51. Marks, S. L., Laflamme, D. P. & McAloose, D. Dietary trial using a commercial hypoallergenic diet containing hydrolyzed protein for dogs with inflammatory bowel disease. Vet. Ther. 3, 109-118 (2002).
52. Buffie, C. G. et al. Precision microbiome reconstitution restores bile acid mediated resistance to Clostridium difficile. Nature 517, 205-208 (2015).
53. Sorg, J. A. & Sonenshein, A. L. Bile salts and glycine as cogerminants for Clostridium difficile spores. J. Bacteriol. 190, 2505-2512 (2008).
54. Dunn, K. A. et al. Early Changes in Microbial Community Structure Are Associated with Sustained Remission After Nutritional Treatment of Pediatric Crohn's Disease. Inflamm. Bowel Dis. 22, 2853-2862 (2016).
55. Duvallet, C., Gibbons, S. M., Gurry, T., Irizarry, R. A. & Alm, E. J. Meta-analysis of gut microbiome studies identifies disease-specific and shared responses. Nat. Commun. 8, 1784 (2017).
56. Oliveira, F. S. et al. MicrobiomeDB: a systems biology platform for integrating, mining and analyzing microbiome experiments. Nucleic Acids Res. 46, D684-D691 (2018).
57. Gonzalez, A. et al. Qiita: rapid, web-enabled microbiome meta-analysis. Nat. Methods 15, 796-798 (2018).
58. Theriot, C. M., Bowman, A. A. & Young, V. B. Antibiotic-Induced Alterations of the Gut Microbiota Alter Secondary Bile Acid Production and Allow for Clostridium difficile Spore Germination and Outgrowth in the Large Intestine. mSphere 1, (2016).
59. van Nood, E. et al. Duodenal infusion of donor feces for recurrent Clostridium difficile. N. Engl. J. Med. 368, 407-415 (2013).
60. Wang, Y. et al. Fecal microbiota transplantation for refractory immune checkpoint inhibitor-associated colitis. Nat. Med. (2018). doi:10.1038/s41591-018-0238-9
61. Kozich, J. J., Westcott, S. L., Baxter, N. T., Highlander, S. K. & Schloss, P. D. Development of a dual-index sequencing strategy and curation pipeline for analyzing amplicon sequence data on the MiSeq Illumina sequencing platform. Appl. Environ. Microbiol. 79, 5112-5120 (2013).
62. Caporaso, J. G. et al. QIIME allows analysis of high-throughput community sequencing data. Nat. Methods 7, 335-336 (2010).
63. Schloss, P. D. et al. Introducing mothur: open-source, platform-independent, community-supported software for describing and comparing microbial communities. Appl. Environ. Microbiol. 75, 7537-7541 (2009).
64. Edgar, R. C. Search and clustering orders of magnitude faster than BLAST. Bioinformatics 26, 2460-2461 (2010).
65. Quast, C. et al. The SILVA ribosomal RNA gene database project: improved data processing and web-based tools. Nucleic Acids Res. 41, D590-6 (2013).
66. Pruesse, E. et al. SILVA: a comprehensive online resource for quality checked and aligned ribosomal RNA sequence data compatible with ARB. Nucleic Acids Res. 35, 7188-7196 (2007).
67. Core Team, R. R: A Language and Environment for Statistical Computing. (2017).
68. Huber, W. et al. Orchestrating high-throughput genomic analysis with Bioconductor. Nat. Methods 12, 115-121 (2015).
69. McMurdie, P. J. & Holmes, S. phyloseq: an R package for reproducible interactive analysis and graphics of microbiome census data. PLoS ONE 8, e61217 (2013).
70. Pielou, E. C. The measurement of diversity in different types of biological collections. J. Theor. Biol. 13, 131-144 (1966).
71. Aβhauer, K. P., Wemheuer, B., Daniel, R. & Meinicke, P. Tax4Fun: predicting functional profiles from metagenomic 16S rRNA data. Bioinformatics 31, 2882-2884 (2015).
72. Love, M. I., Huber, W. & Anders, S. Moderated estimation of fold change and dispersion for RNA-seq data with DESeq2. Genome Biol. 15, 550-550 (2014).
73. Li, H. et al. The Sequence Alignment/Map format and SAMtools. Bioinformatics 25, 2078-2079 (2009).
74. Li, H. & Durbin, R. Fast and accurate long-read alignment with Burrows-Wheeler transform. Bioinformatics 26, 589-595 (2010).
75. Browne, H. P. et al. Culturing of "unculturable" human microbiota reveals novel taxa and extensive sporulation. Nature 533, 543-546 (2016).
76. Goodman, A. L. et al. Extensive personal human gut microbiota culture collections characterized and manipulated in gnotobiotic mice. Proc Natl Acad Sci USA 108, 6252-6257 (2011).
77. De MAN, J. C., Rogosa, M. & Sharpe, M. E. A MEDIUM FOR THE CULTIVATION OF LACTOBACILLI. Journal of Applied Bacteriology 23, 130-135 (1960).
78. Lunter, G. & Goodson, M. Stampy: a statistical algorithm for sensitive and fast mapping of Illumina sequence reads. Genome Res. 21, 936-939 (2011).
79. Friedman, E. S. et al. FXR-Dependent Modulation of the Human Small Intestinal Microbiome by the Bile Acid Derivative Obeticholic Acid. Gastroenterology (2018). doi:10.1053/j.gastro.2018.08.022
80. Leinonen, R., Sugawara, H., Shumway, M. & International Nucleotide Sequence Database Collaboration. The sequence read archive. Nucleic Acids Res. 39, D19-21 (2011).

Although the presently disclosed subject matter and its advantages have been described in detail, it should be understood that various changes, substitutions and alterations can be made herein without departing from the scope of the present disclosure as defined by the appended claims.

## Claims

1. A method for determining responsiveness of a companion animal having an intestinal disorder to a diet, wherein the intestinal disorder is chronic enteropathy, wherein the diet is a therapeutic hydrolyzed protein diet and wherein the companion animal is a dog, comprising:
a) measuring an amount of an intestinal microorganism in a fecal sample from the companion animal;
b) comparing the amount of the intestinal microorganism with a reference amount of the intestinal microorganism, wherein the reference amounts of the intestinal microorganisms are determined based on the amounts of the intestinal microorganisms in a plurality of healthy companion animals; and
c) determining that the companion animal is responsive to the diet when the amount of the intestinal microorganism is lower than the reference amount of the intestinal microorganism, or determining that the companion animal is non-responsive to the diet when the amount of the intestinal microorganism is higher than the reference amount of the intestinal microorganism, wherein the intestinal microorganism comprises a bacterium comprising a 16S rRNA comprising a nucleotide sequence that is identical to the 16S rRNA nucleotide sequence of FJ957494.1.1454 (SEQ ID NO: 13)

2. The method of claim 1, wherein the reference amount of an intestinal microorganism derived from a mean amount of the intestinal microorganism in a plurality of healthy companion animals.

## Patentansprüche

1. Verfahren zur Bestimmung des Ansprechens eines Haustiers mit einer Darmstörung auf eine Diät, wobei es sich bei der Darmstörung um chronische Enteropathie handelt, wobei es sich bei der Diät um eine therapeutische Hydrolysiertes-Protein-Diät handelt und wobei es sich bei dem Haustier um einen Hund handelt, umfassend:
a) Messen einer Menge eines Darm-Mikroorganismus in einer Kotprobe von dem Haustier;
b) Vergleichen der Menge des Darm-Mikroorganismus mit einer Referenzmenge des Darm-Mikroorganismus, wobei die Referenzmengen der Darm-Mikroorganismen bezogen auf die Mengen der Darm-Mikroorganismen in mehreren gesunden Haustieren bestimmt werden; und
c) Bestimmen, dass das Haustier auf die Diät anspricht, wenn die Menge des Darm-Mikroorganismus geringer als die Referenzmenge des Darm-Mikroorganismus ist, oder Bestimmen, dass das Haustier nicht auf die Diät anspricht, wenn die Menge des Darm-Mikroorganismus höher als die Referenzmenge des Darm-Mikroorganismus ist, wobei der Darm-Mikroorganismus ein Bakterium umfasst, das eine 16S-rRNA umfasst, die eine Nukleotidsequenz umfasst, die mit der 16S-rRNA-Nukleotidsequenz von FJ957494.1.1454 (SEQ ID NO: 13) identisch ist.

2. Verfahren nach Anspruch 1, wobei die Referenzmenge eines Darm-Mikroorganismus von einer mittleren Menge des Darm-Mikroorganismus in mehreren gesunden Haustieren abgeleitet ist.

## Revendications

1. Procédé de détermination de la réactivité d'un animal de compagnie souffrant d'un trouble intestinal à un régime alimentaire, le trouble intestinal étant une entéropathie chronique, ledit régime alimentaire étant un régime alimentaire thérapeutique à base de protéines hydrolysées et l'animal de compagnie étant un chien, le procédé comprenant :
a) la mesure de la quantité d'un micro-organisme intestinal dans un échantillon fécal provenant de l'animal de compagnie ;
b) la comparaison de la quantité du micro-organisme intestinal à une quantité de référence du micro-organisme intestinal, les quantités de référence des micro-organismes intestinaux étant déterminées sur la base des quantités des micro-organismes intestinaux chez une pluralité d'animaux de compagnie sains ; et
c) la détermination du fait que l'animal de compagnie est sensible au régime alimentaire lorsque la quantité du micro-organisme intestinal est inférieure à la quantité de référence du micro-organisme intestinal, ou la détermination du fait que l'animal de compagnie n'est pas sensible au régime alimentaire lorsque la quantité du micro-organisme intestinal est supérieure à la quantité de référence du micro-organisme intestinal, le micro-organisme intestinal comprenant une bactérie comprenant un ARNr 16S comprenant une séquence nucléotidique qui est identique à la séquence nucléotidique de l'ARNr 16S de FJ957494.1.1454 (SEQ ID NO : 13).

2. Procédé selon la revendication 1, dans lequel la quantité de référence d'un micro-organisme intestinal est déduite à partir d'une quantité moyenne du micro-organisme intestinal chez une pluralité d'animaux de compagnie sains.
